# EUROPEAN PATENT SPECIFICATION

(11) **EP 1 585 748 B1**
(45) Date of publication and mention of the grant of the patent: **27.12.2006**
(21) Application number: 03781250.0
(22) Date of filing: 19.12.2003
(51) Int. Cl.: C07D 487/04, A61K 31/519, A61P 1/04

(54) **PYRAZOLO 3,4-d PYRIMIDINE DERIVATIVES AND THEIR USE I N THE TREATMENT OF H.PYLORI INFECTION**
PYRAZOL-3,4-d-PYRIMIDINDERIVATE UND IHRE VERWENDUNG BEI DER BEHANDLUNG VON H.PYLORI-INFEKTIONEN
DERIVES DE PYRAZOLO 3,4-d PYRIMIDINE ET UTILISATION DE CEUX-CI POUR LE TRAITEMENT DE L'INFECTION PAR H.PYLORI

(30) Priority: 20.12.2002 SE 0203825
(43) Date of publication of application: 19.10.2005
(73) Proprietor: AstraZeneca AB, 151 85 Södertälje (SE)
(72) Inventor: Basarab, Gregory, Waltham, MA 02451 (US); Eyermann, Joseph, Waltham, MA 02451 (US); Govravaram, Madhusudhan, Waltham, MA 02451 (US); Green, Oluyinka, Waltham, MA 02451 (US); MacPherson, Lawrence, Waltham, MA 02451 (US); Morningstar, Marshall, Waltham, MA 02451 (US); NGUYEN, Thanh, San Diego, CA 92121 (US)
(86) International application number: PCT/SE2003/002033
(87) International publication number: WO 2004/056831

(56) References cited:
- WO-A1-03/002567
- WO-A2-90/06116
- MENDZ GEORGE L. ET AL.: 'Purine metabolism and microaerophily of helicobacter pylori' ARCH. MICROBIOL. vol. 168, 1997, pages 448 - 456, XP002956504

## Description

### Field of the invention

The present invention relates to novel fused heterocycles, their pharmaceutical compositions and methods of use. In addition, the present invention relates to therapeutic methods for the treatment and prevention of various diseases caused by *Helicobacter pylori (H. pylori)* infection.

### Background of the invention

*Helicobacter pylori* (*H. pylori*) is a highly motile, S-shaped, microaerophilic gram-negative bacterium that colonizes in the stomach. *H. pylori* infection is widespread with seroprevalence in the developed world between 30-60%. Infection with the bacterium is usually contracted during childhood and patients remain infected for life unless treated. H. *pylori* infection has been shown to result in the development of gastritis, peptic ulcer, and mucosa-associated lymphoid tissue (MALT) lymphoma and has been linked to gastric adenocarcinoma (Go, M.F. and D.T. Smoot, *Helicobacter pylori*, gastric MALT lymphoma, and adenocarcinoma of the stomach. Seminars in Gastrointestinal Disease, 2000, 11(3): p. 134-141). Eradication of *H. pylori* infection is currently achieved using combination therapy of antimicrobial and antisecretory agents (Malfertheiner, P., A. Leodolter, and.U. Peitz, Cure of *Helicobacter pylori*-associated ulcer disease through eradication. Bailliere's Best Practice and Research in Clinical Gastroenterology, 2000, 14(1): p. 119-132). However, compliance to these therapies is compromised due to adverse side effects and cumbersome dosing regimens. In addition, increasing prevalence of *H. pylori* strains resistant to existing antimicrobial therapies threatens to limit the use of these treatments (Qureshi, W.A. and D.Y. Graham, *Antibiotic-resistant H. pylori infection and its treatment.* Current Pharmaceutical Design, 2000, 6(15): p. 1537-1544). Given these considerations, an ideal therapy for H. *pylori* infection would be a novel antimicrobial monotherapy that is selective for *H. pylori* eradication. The selectivity attribute is expected to aid in minimizing side effects due effects on gut flora.

*H. pylori,* like all Gram positive and Gram negative bacteria, utilize a cell wall comprised of crosslinked peptidoglycan units to maintain shape and resist high osmotic pressure potentials. Bacterial cell wall biosynthesis is a validated target for antimicrobial activity; cephalosphorins, penicillins and glycopeptides are antimicrobial agents, which block cell wall biosynthesis (Walsh, C., *Molecular mechanisms that confer antibacterial resistance.* Nature, 2000, 406: p. 775-781). Cell wall biosynthesis requires the enzyme MurI, a glutamate racemase, and therefore this enzyme is essential for bacterial viability (Doublet, P., *et al., The murI gene of Escherichia coli is an essential gene that encodes a glutamate racemase activity.* Journal of Bacteriology, 1993, 175(10): p. 2970-9).

The present invention describes compounds, which specifically inhibit *H. pylori* MurI, compositions of such compounds and methods of use. The compounds disclosed herein represent a valuable contribution to the development of selective therapies directed to diseases resulting from *H. pylori* infection.

### Summary of the invention

In accordance with the present invention, the applicants have hereby discovered novel compounds that inhibit the MurI enzyme and thereby inhibit cell wall biosynthesis in *H. pylori* bacterium. The present invention includes pharmaceutically acceptable salts or prodrugs of such compounds. Also in accordance with the present invention applicants provide pharmaceutical compositions and a method to use invention compounds in the treatment of infections.

### Detailed description of the invention

In a first embodiment, the present invention provides a novel compound having structural formula (I): 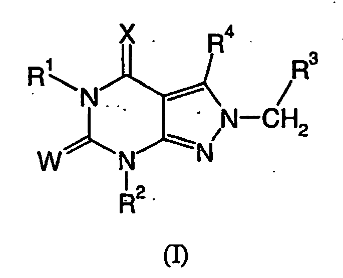 wherein,
X is S, O, or NR²⁰, provided that when W is O, then X is not O,
X and the double bond to which it is attached can be replaced with 2 hydrogen atoms,
W is S, O, or NR²⁰; provided that when X is O, then W is not O;
R¹ is H, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, hydroxy, amino, or optionally substituted heterocycle;
R² is H, optionally substituted alkyl, optionally substituted alkylcycloalkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, or optionally substituted heterocycle;
R³ is a monocyclic or bicyclic, saturated or unsaturated, ring system comprising 0, 1, 2 or 3 heteroatoms independently selected from N, O, or S, the ring being substituted by 0, 1, 2 or 3 substituents selected from =O, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}. -O(CH₂)ₘOR^{a}, -S(CH₂)ₘHet, -S(CH₂)ₘC(=O)Het, -S(CH₂)ₘC(=O)N^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵,-R⁵-Het, -Het-R⁵, -Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, O, or S, the ring system being substituted by 0, 1, 2 or 3 substituents selected from B(OH)₂, vicinal -OCH₂CH₂O-, vicinal - OC₁₋₂haloalkylO-, vicinal -OCH₂O-, vicinal -CH₂OCH₂O-, =O, halogen, -R^{b}OR^{a}, -SR^{a}, -OR^{a}, C₁₋₆alkyl, C₁₋₆haloalkyl, -CN, -S(=O)ₙR^{c}, -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, - S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, - C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}, - C(=O)NR^{a}(R^{b}Het), -C(=O)OR^{a}, -OC(=O)R^{a}, -C(=O)OR^{b}NR^{a}R^{a}, -C(=O)R^{a}, -C(=O)R^{b}NR^{a}R^{a}; -C(=NOR^{a})R^{a}, -C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), - S(=O)₂NR^{a}(R^{b}C(=O)OR^{a}), aminocarbonyl, phenyl, benzyl; or R⁴ is represented by -(CH₂)ₙR⁵-Het, -(CH₂)ₙR^{d}, -Het, -Het-Het, R⁵, -R⁵-Het, -He t-R⁵, -Het-OR⁵, R⁵-R⁵, or -R⁵-OR⁵; or R⁴ is represented by Chalky, -NC₁₋₆alkyl, or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)_{z}CH₂OH, -(CH₂)_{z}CO₂H, or -(CH₂)_{z}CO₂C₁₋₆ alkyl wherein z is 1,2,3,4,5, or 6;
R⁵ is independently at each instance, phenyl substituted by 0, 1, 2, or 3 groups selected from halogen, C₁₋₆haloalkyl, -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR ^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a},-C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a},-C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, or - S(=O)₂NR^{a}R^{b}C(=O)OR^{a};
R²⁰ is, independently at each instance, H, -CN, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R^{a} is, independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, vicinyl -OCH₂CH₂O-, vicinyl -OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, O, or S;
m is 1, 2 or 3;
n is 0, 1 or 2;
When "optionally substituted" is used, it refers to at least one substituent selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic
or a pharmaceutically acceptable salt thereof.

In an additional embodiment the present invention provides a compound having a structural formulaformula (I) as recited above 1 wherein:
R¹ is H, or C₁₋₆alkyl, or -(CH₂)ₙcycloalkyl or -(CH₂)₁₋₂Het wherein C₁₋₆alkyl or - (CH₂)ₙcycloalkyl or -(CH₂)₁₋₂Het is optionally substituted by 1, 2 or 3 substituents selected from Het, halogen, -CN, -OR^{a}, -NR^{a}R^{a}, -C(=O)OR^{a}, -C(=O)NR^{a}R^{a}, -OC(=O)C₁₋₄alkyl, - S(=O)ₙR^{c}, -S(=O)ₙNR^{a}R^{a} or -NR^{a}C(=O)C₁₋₄alkyl and n is 0, 1 or 2.

In an additional embodiment the present invention provides a compound having a structural formula (I) as recited above wherein:
R² is -(CH₂)₁₋₃cycloalkyl or -C₁₋₁₂alkyl wherein -(CH2)₁₋₃cycloalkyl or -C₁₋₁₂alkyl is optionally substituted with 0, 1, 2 or 3 substituents selected from Het, S(=O)ₙR^{c}, -S(=O)ₙNR^{a}R^{a} halogen, -CN, -OR^{a}, -NR^{a}R^{a}, -C(=O)OR ^{a}, -C(=O)R^{a}, -C(=O)NR^{a}R^{a}, -OC(=O)C₁₋₄alkyl, or -NR^{a}C(=O)C₁₋₄alkyl and n is 0, 1 or 2.

In an additional embodiment the present invention provides a compound having a structural formula (I) as recited above wherein:
R³ is selected from formulas (i), (ii), (iii) or (iv) set forth below: 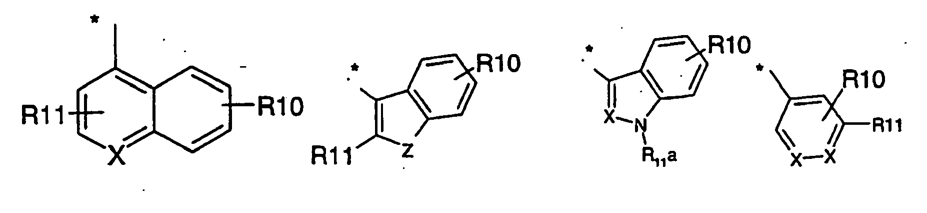 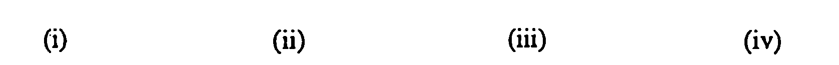
wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.

In an additional embodiment the present invention provides a compound having structural formulaa (I) as recited above wherein:
R⁴ is selected from formulas (a) to (z) or (aa) or (ab) set forth below: 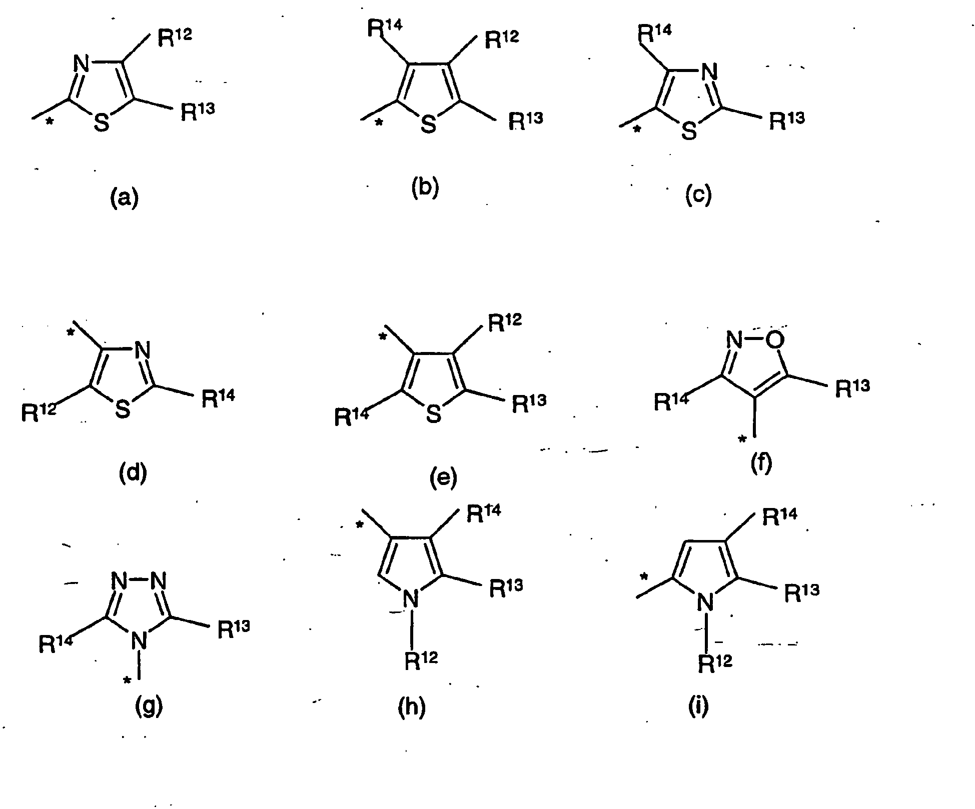 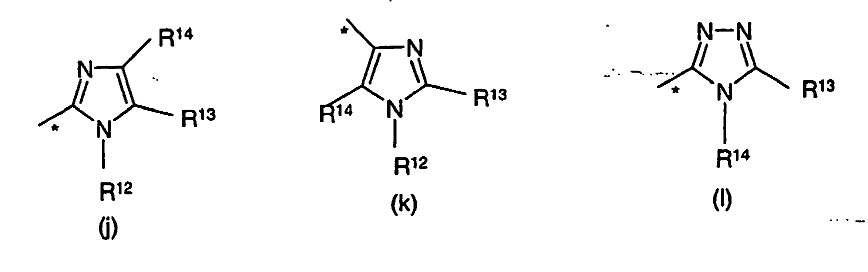 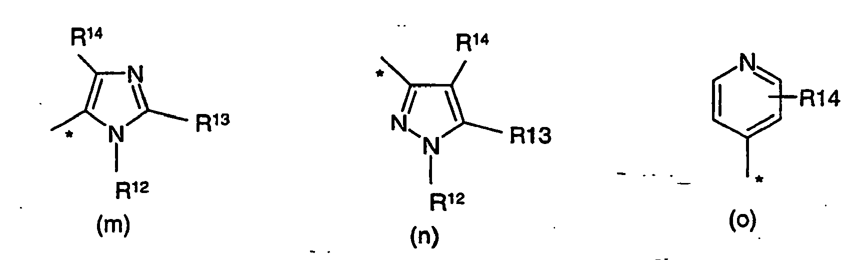 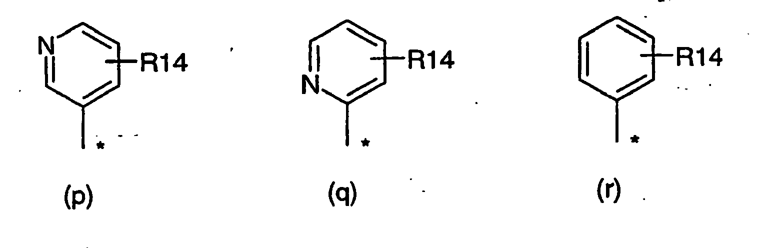 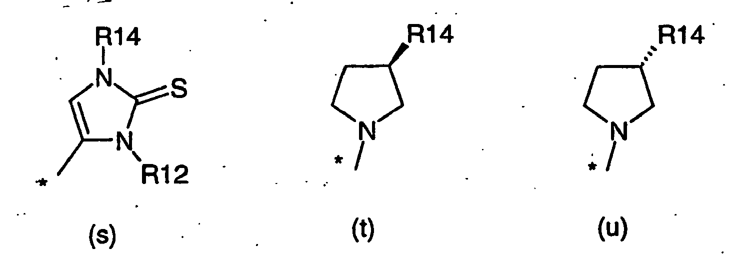 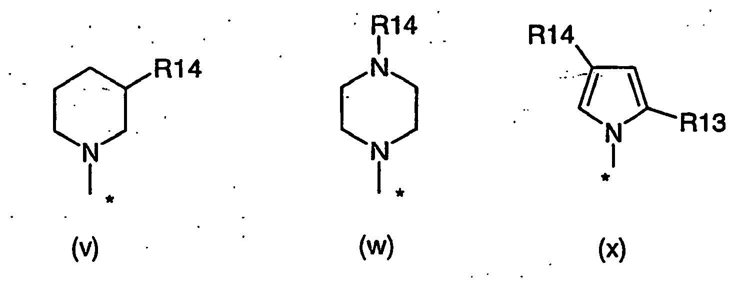 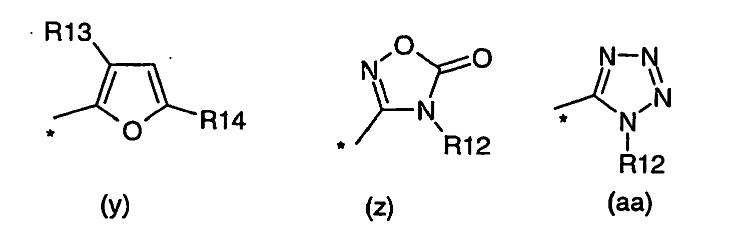 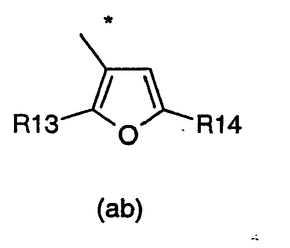
wherein * is the location wherein R⁴ is attached to the ring system and wherein
wherein R¹², R¹³ and R¹⁴ are each independently represented by H, Het, C₁₋₆alkyl, -CN, -NR^{a}R^{a}, -nitro, -C(=O)R^{a}, -C(=O)NR^{a}R^{a}, -C(=O)NR^{a}S(=O)₂R^{a}, -C(=O)NR^{a}-Het, - C(=O)NR^{a}NR^{a}R^{a}, -C(=O)NR^{a}(R^{b}NR^{a}R^{a} ), -C(=O)NR^{a}(R^{b}OR^{a}), -C(=O)NR^{a}(R^{b}S(=O)₂R^{a}), - C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), or -S(=O)₂NR^{a}(R^{b}C(=O)OR^{a}.

In an additional embodiment the present invention provides a compound having structural formula (I) as recited above wherein:
X is S, O, or NR²⁰, provided that when W is O, then X is not 0; or X and the double bond to which it is attached can be 2 hydrogen atoms,
W is S, O, or NR²⁰; provided that when X is O, then W is not O;
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}
R¹ is CH₃, CH₂CH₃, CH₂CN, CF₃, (CH₂)₂OH, cyclopropyl, isopropyl, CH₂CCH, (CH₂)₂N(CH₂)₂, (CH₂)₂N(C=NH)NH₂, -CH₂-2-pyridyl, -CH₂-3-pyridyl, -CH₂-4-pyridyl, - (CH₂)₂-1-imidazolyl, -(CH₂)₂-1-pyrazolyl, -(CH₂)₂-1-piperidyl, -(CH₂)ₘ-(1-methylpiperidin-4-yl), -CH₂-(1-methylpiperidin-3-yl), -(CH₂)₂-(morpholin-4-yl),
R² is -CH₂CH₂CH₃, -CH₂-cyclopropyl, -CH₂CH(CH₃)₂, -CH₂CH₂CH₂F, -CH₂-cyclobutyl, -CH₂C(CH₃)₃, -CH₂CH₂CH(CH₃)₂, -CH₂CF₃, -CH₂-methylphenyl, -CH₂-phenol, - CH₂-(3,5-dimethylisoxazol-4-yl), -CH₂-S-phenyl, -CH₂-phenylcarboxyl, or -CH₂SCF₃;
R³ is selected from formulas (i), (ii), (iii) or (iv) set forth below: 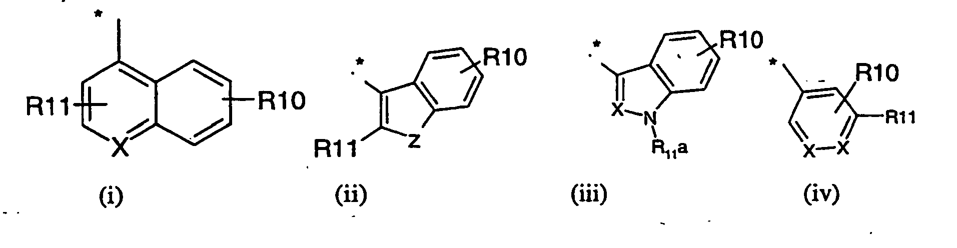 wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.
R⁴ is selected from formulas (a) to (z) or (aa) or (ab) set forth below: 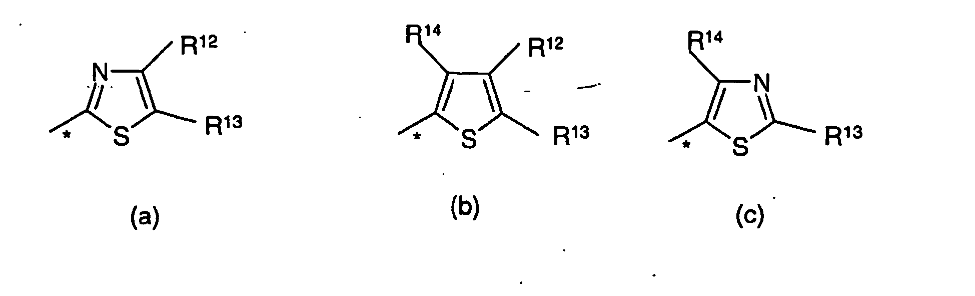 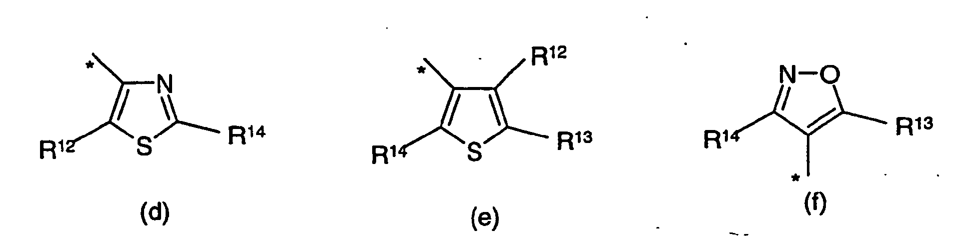 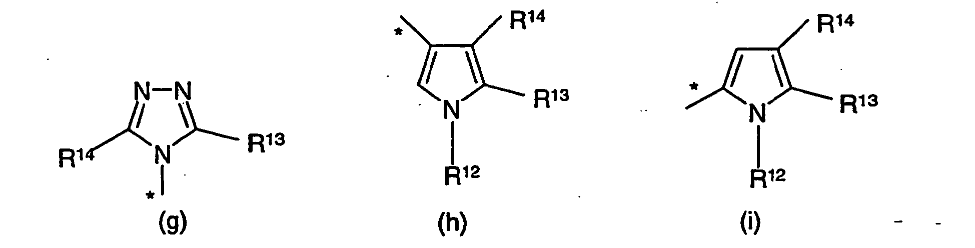 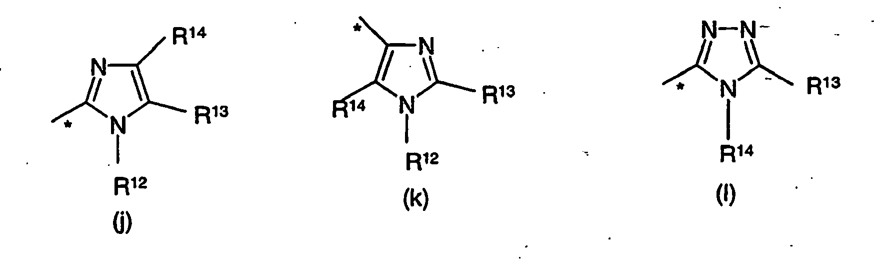 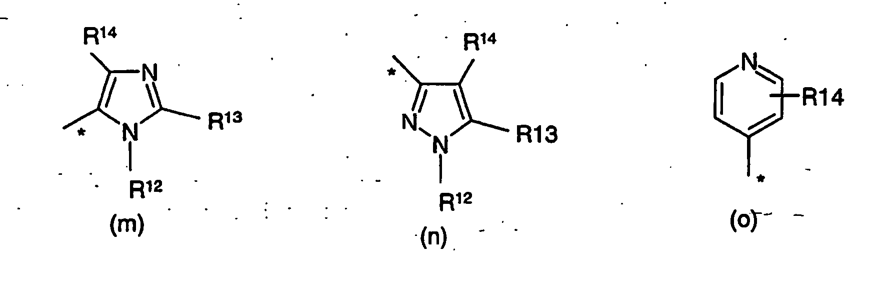 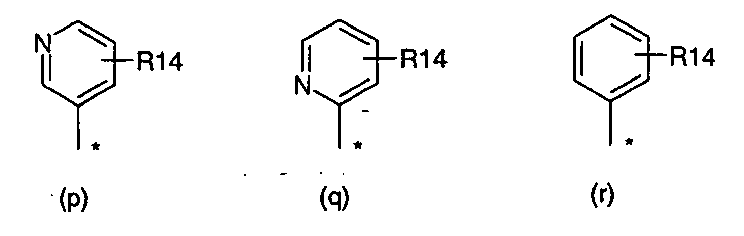 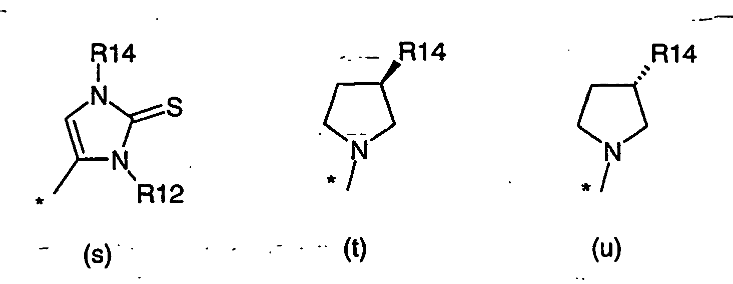 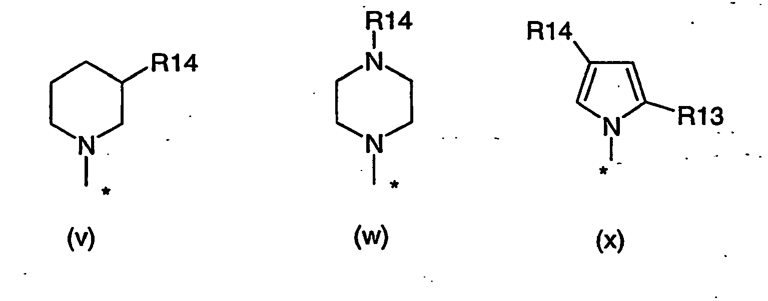 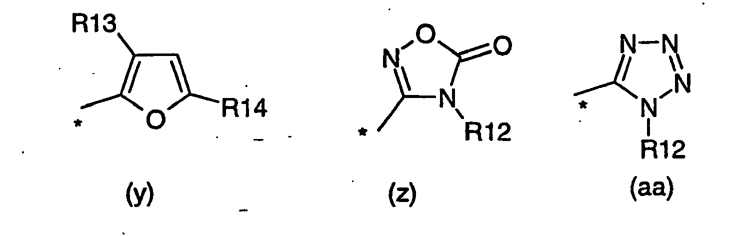 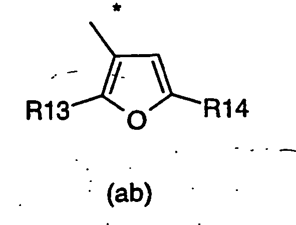
wherein * is the location wherein R⁴ is attached to the ring system and wherein
wherein R¹², R¹³ and R¹⁴ are each independently represented by H, Het, C₁₋₆alkyl, -CN, -NR^{a}R^{a}, -nitro, -C(=O)R^{a}, -C(=O)NR^{a}R^{a}, -C(=O)NR^{a}S(=O)₂R^{a}, -C(=O)NR^{a}-Het,-C(=O)NR^{a}NR^{a}R^{a}, -C(=O)NR^{a}(R^{b}NR^{a}R^{a}), *-*C(=O)NR(R^{b}OR^{a}), -C(=O)NR^{a}(R^{a}S(=O)₂R^{a}),-C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, - C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, - NR^{a}C(=O)OR^{a}, -NR^{a}S(=O)2R^{b}, -C(=NOR^{a})R^{a}, -S(=O)₂R^{a}, -S(=O)₂NR^{a}R^{a},-S(=O)₂NR^{a}(R^{b}C(=O)NR^{a}R^{a}), or -S(=O)₂NR^{a}(R^{b}C(=O)OR^{a}.

In an additional embodiment the present invention provides a compound selected from:
5-[2-[(6-chloroquinolin-4-yl)methyl]-7-(cyclopropylmethyl)-6-oxo-4-thioxo-4,5,6,7-tetrahydro-2H-pyrazolo[3,4-d]pyrimidin-3-yl]-1-methyl-1H-pyrrole-3-carbonitrile;
5-[2-[(6-chloroquinolin-4-yl)methyl]-7-(cyclopropylmethyl)-4-imino-5-methyl-6-oxo-4,5,6,7-tetrahydro-2*H*-pyrazolo[3,4-*d*]pyrimidin-3-yl]-1-methyl-1*H*-pyrrole-3-carbonitrile;
5-[(4Z)-2-[(6-chloroquinolin-4-yl)methyl]-7-(cyclopropylmethyl)-5-methyl-4-(methylimino)-6-oxo-4,5,6,7-tetrahydro-2*H*-pyrazolo[3,4-*d*]pyrimidin-3-yl]-1-methyl-1*H*-pyrrole-3-carbonitrile;
5-[2-[(6-chloroquinolin-4-yl)methyl]-7-(cyclopropylmethyl)-6-imino-5-methyl-4-oxo-4,5,6,7-tetrahydro-2H-pyrazolo[3,4-d]pyrimidin-3-yl]-1-methyl-1H-pyrrole-3-carbonitrile;
5-[2-[(6-chloroquinolin-4-yl)methyl]-7-(cyclopropylmethyl)-5-methyl-4-oxo-6-thioxo-4,5,6,7-tetrahydro-2H pyrazolo[3,4-*d*]pyrimidin-3-yl]-1-methyl-1*H* pyrrole-3-carbonitrile;
5-[(6Z)-2-[(6-chloroquinolin-4-yl)methyl]-7-(cyclopropylmethyl)-5-methyl-6-(methylimino)-4-oxo-4,5,6,7-tetrahydro-2H-pyrazolo[3,4-d]pyrimidin-3-yl]-1-metbyl-1H-pyrrole-3-carbonitrile;
N-[(6Z)-2-[(6-chloroquinolin-4-yl)methyl]-3-(4-cyano-1-methyl-1H-pyrrol-2-yl)-7-(cyclopropylmethyl)-5-methyl-4-oxo-2,4,5,7-tetrahydro-6H-pyrazolo[3,4-d]pyrimidin-6-ylidene]acetamide;
N-[(6Z)-2-[(6-chloroquinolin-4-yl)methyl]-3-(4-cyano-1-methyl-1H-pyrrol-2-yl)-7-(cyclopropylmethyl)-5-methyl-4-oxo-2,4,5,7-tetrahydro-6H-pyrazolo[3,4-d]pyrimidin-6-ylidene]methanesulfonamide;
5-((6Z)-2-[(6-chloroquinolin-4-yl)methyl]-7-(cyclopropylmethyl)-6-{[2-(dimethylamino)ethyl]imino}-5-methyl-4-oxo-4,5,6,7-tetrahydro-2H-pyrazolo[3,4-d]pyrimidin-3-yl)-1-methyl-1H-pyrrole-3-carbonitrile;
N-1--[2-[(6-chloroquinolin-4-yl)methyl]-3-(4-cyano-1-methyl-1H-pyrrol-2-yl)-7-(cyclopropylmethyl)-5-methyl-4-oxo-2,4,5,7-tetrahydro-6H-pyrazolo[3,4-d]pyrimidin-6-ylidene]-N~2~,N~2~-dimethylglycinamide;
5-[2-[(6-chloroquinolin-4-yl)methyl]-7-(cyclopropylmethyl)-6-oxo-4,5,6,7-tetrahydro-2H-pyrazolo[3,4-d]pyrimidin-3-yl]-1-methyl-1H-pyrrole-3-carbonitrile;
5-[2-[(6-chloroquinolin-4-yl)methyl]-7-(cyclopropylmethyl)-5-methyl-6-oxo-4,5,6,7-tetrahydro-2H-pyrazolo[3,4-d]pyrimidin-3-yl]-1-methyl-1H-pyrrole-3-carbonitrile;
5-[2-[(6-chloroquinolin-4-yl)methyl]-7-(cyclopropylmethyl)-6-oxo-6,7-dihydro-2H-pyrazolo[3,4-d]pyrimidin-3-yl]-1-methyl-1H-pyrrole-3-carbonitrile;
2-[(6-chloroquinolin-4-yl)methyl]-7-(cyclopropylmethyl)-5-methyl-3-(1-methyl-1*H*-imidazol-5-yl)-4-thioxo-2,4,5,7-tetrahydro-6*H*-pyrazolo[3,4-*d*]pyrimidin-6-one;
(4Z)-2-[(6-chloroquinolin-4-yl)methyl]-7-(cyclopropylmethyl)-5-methyl-3-(1-methyl-1*H-*imidazol-5-yl)-4-(methylimino)-2,4,5,7-tetrahydro-6*H*-pyrazolo[3,4-*d*]pyrimidin-6-one.

In a further embodiment the present invention provides a compound having the structural formula (II): 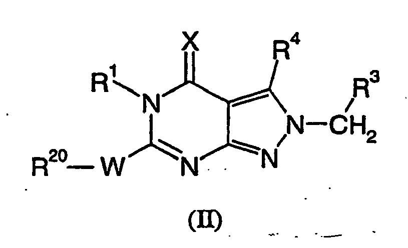 wherein,
X is S, O, or NR²⁰,
X and the double bond to which it is attached can be replaced with 2 hydrogen atoms,
W is S, O, or NR²¹;
R¹ is H, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, hydroxy, amino, or optionally substituted heterocycle, wherein the substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R³ is a monocyclic or bicyclic, saturated or unsaturated, ring system comprising 0, 1, 2 or 3 heteroatoms independently selected from N, 0, or S, the ring being substituted by 0, 1, 2 or 3 substituents selected from =0, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵,-R⁵-Het, -Het-R⁵, -Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, 0, or S, the ring system being substituted by 0, 1, 2 or 3 substituents selected from B(OH)₂, vicinal -OCH₂CH₂O-, vicinal - OC₁₋₂haloalkylO-, vicinal -OCH₂O-, vicinal -CH₂OCH₂O-, =O, halogen, -R^{b}OR^{a}, -SR^{a}, -OR^{a}, C₁₋₆alkyl, C₁₋₆haloalkyl, -CN, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, - S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}), - C(=O)NR^{a}(R^{b}Het), -C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), - S(=O)₂NR^{a}(R^{b}C(=O)OR^{a}), aminocarbonyl, phenyl, benzyl; or R⁴ is represented by -(CH₂)ₙR⁵-Het, -(CH₂)ₙR^{d}, -Het, -Het-Het, R⁵, -R⁵-Het, -Het-R⁵, -Het-OR⁵, R⁵-R⁵, or -R⁵-OR⁵; or R⁴ is represented by C₁₋₆alky, -NC₁₋₆alkyl, or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)_{z}CH₂OH, -(CH₂)_{z}CO₂H, or -(CH₂)_{z}CO₂C₁₋₆ alkyl wherein z is 1,2,3,4,5, or 6;
R⁵ is independently at each instance, phenyl substituted by 0, 1, 2, or 3 groups selected from halogen, C₁₋₆haloalkyl, -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, or - S(=O)₂NR^{a}R^{b}C(=O)OR^{a};
R²⁰ is, independently at each instance, H, -CN, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, wherein the substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄)alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²¹ is, independently at each instance, H, -CN, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}; optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, wherein the substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²⁰ and R²¹ and the N to which they are attached in combination can also form a 3 to 10 member N-linked saturated or unsaturated heterocycle having either 1, or 2 heteroatoms independently selected from N, O, or S wherein the heterocycle is substituted with R^{e};
R^{a} is, independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, 0 or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, 0 or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, vicinyl -OCH₂CH₂O-, vicinyl -OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, O, or S;
R^{e} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
m is 1, 2 or 3;
n is 0, 1 or 2;
or a pharmaceutically acceptable salt thereof.

In an additional embodiment the present invention provides a compound having structural formula (II) as recited above wherein:
R¹ is H, or C₁₋₆alkyl, or -(CH₂)ₙcycloalkyl wherein C₁₋₆alkyl or -(CH₂)ₙcycloalkyl is optionally substituted by 1, 2 or 3 substituents selected from Het, halogen, -CN, -OR^{a}, -NR^{a}R^{a}, -C(=O)OR^{a}, -C(=O)NR^{a}R^{a}, -OC(=O)C₁₋₄alkyl or -NR^{a}C(=O)C₁₋₄alkyl and n is 0, 1 or 2.

In an additional embodiment the present invention provides a compound having structural formula (II) as recited above wherein:
R³ is selected from formulas (i), (ii), (iii) or (iv) set forth below: 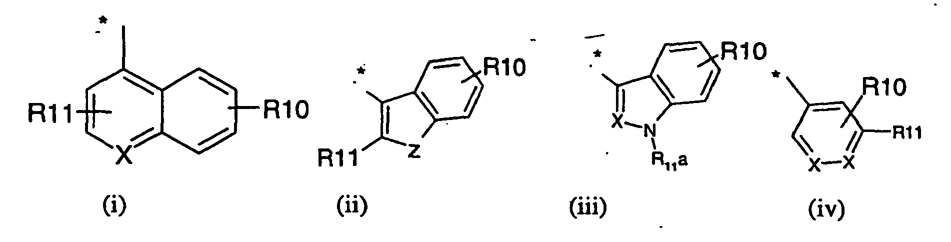
wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.

In an additional embodiment the present invention provides a compound having structural formula (III) as recited above wherein:
R⁴ is selected from formulas (a) to (z) or (aa) or (ab) set forth below: 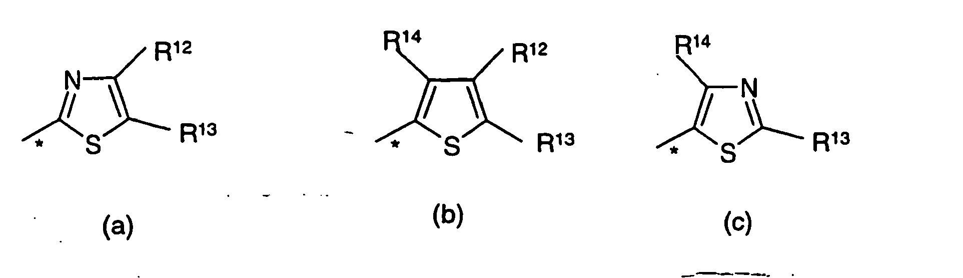 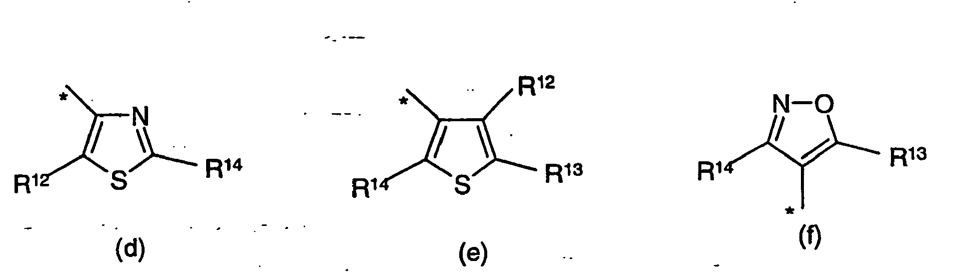 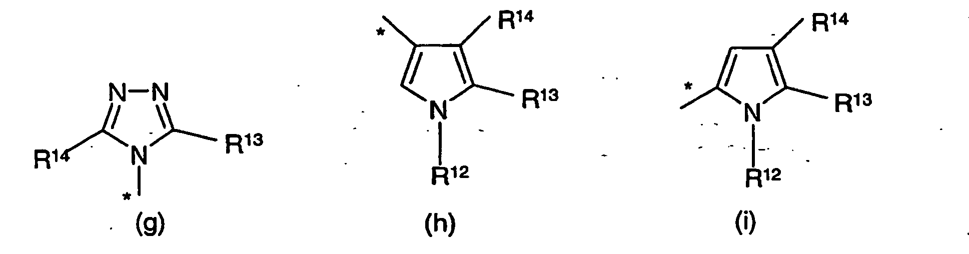 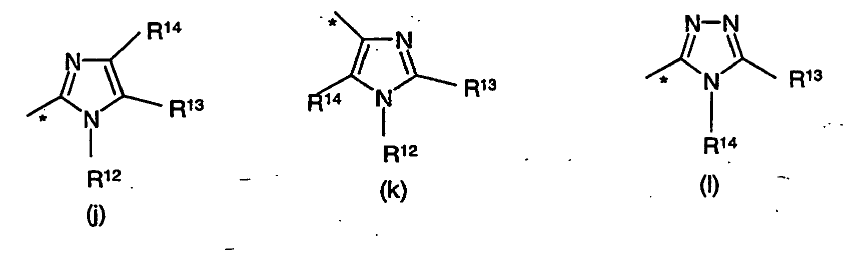 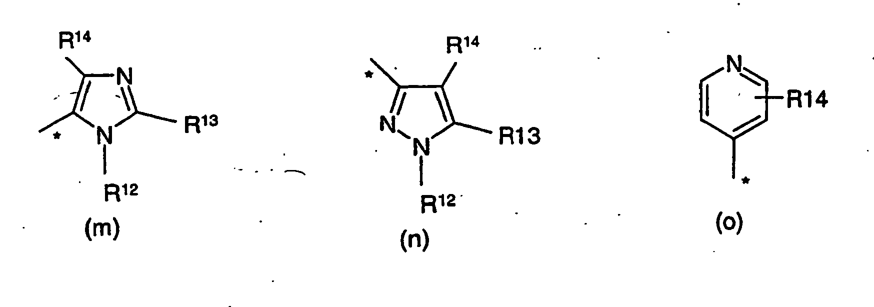 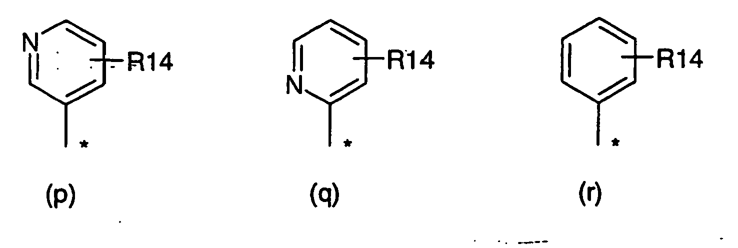 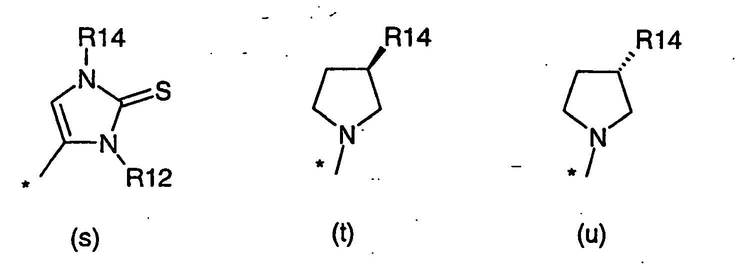 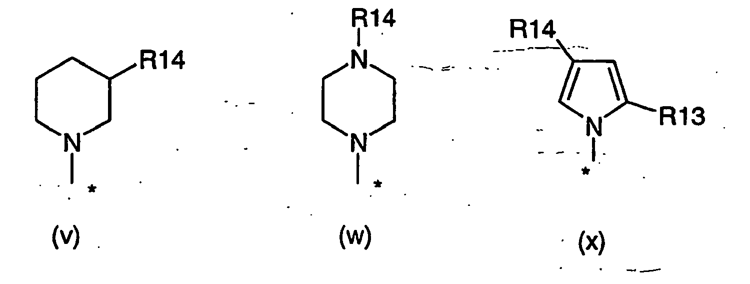 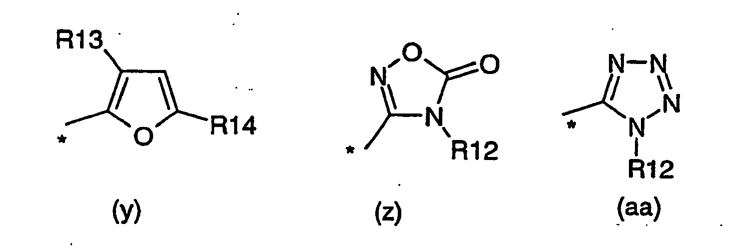 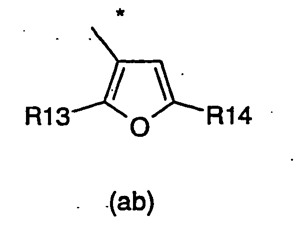
wherein * is the location wherein R⁴ is attached to the ring system and wherein
wherein R¹², R¹³ and R¹⁴ are each independently represented by H, Het, C₁₋₆alkyl, -CN, - NR^{a}R^{a}, -nitro, -C(=O)R^{a}, -C(=O)NR^{a}R^{a}, -C(=O)NR^{a}S(=O)₂R^{a}, -C(=O)NR^{a}-Het,-C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂Ra), - C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a},-C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, - NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), or -S(=O)₂NR^{a}(R^{b}C(=O)OR^{a}.

In an additional embodiment the present invention provides a compound having structural formula (II) as recited above wherein:
X is S, 0, or NR²⁰; or X and the double bond to which it is attached can be 2 hydrogen atoms,
W is S, O, or NR²¹;
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ and R²¹ and the N to which they are attached in combination can also form a 3 to 10 member N-linked saturated or unsaturated heterocycle having either 1, or 2 heteroatoms independently selected from N, O, or S wherein the heterocycle is substituted with R^{e};
R¹ is CH₃, CH₂CH₃, CH₂CN, CF₃, (CH₂)₂OH, cyclopropyl, isopropyl, CH₂CCH, (CH₂)₂N(CH₂)₂, (CH₂)₂N(C=NH)NH₂, -CH₂-2-pyridyl, -CH₂-3-pyridyl, -CH₂-4-pyridyl, -(CH₂)₂-1-imidazolyl, -(CH₂)₂-1-pyrazolyl, -(CH₂)₂-1-piperidyl, -(CH₂)ₘ-(1-methylpiperidin-4-yl), -CH₂-(1-methylpiperidin-3-yl), -(CH₂)₂-(morpholin-4-yl),
R³ is selected from formulas (i), (ii), (iii) or (iv) set forth below: 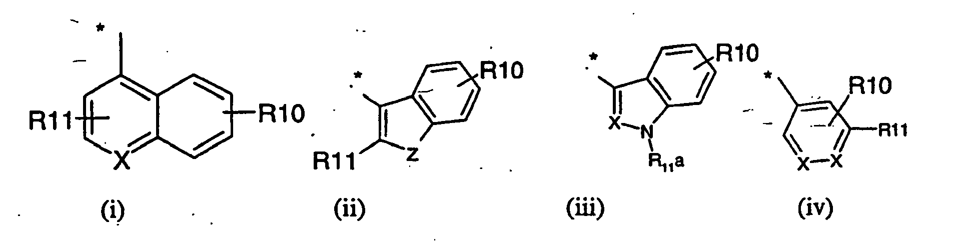
wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen,-CN, nitro, OR^{a}, CF₃, -NR^{a}R^{a}, - C(=O)OR^{a}, -C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2. R⁴ is selected from formulas (a) to (z) or (aa) or (ab) set forth below: 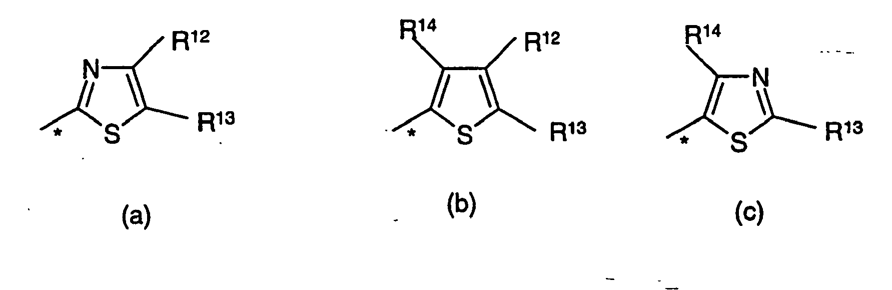 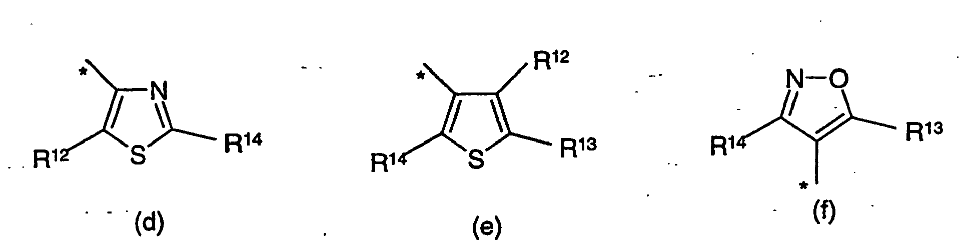 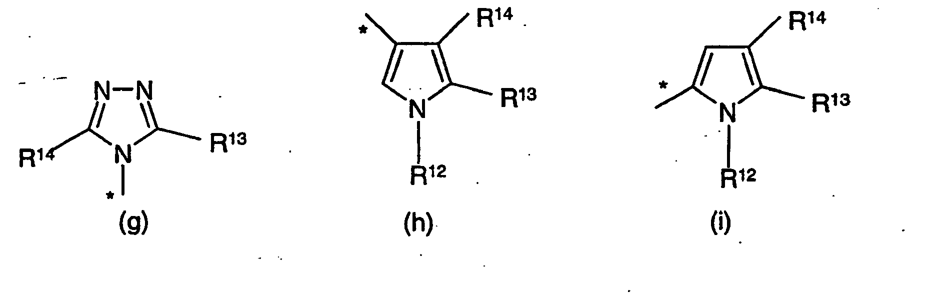 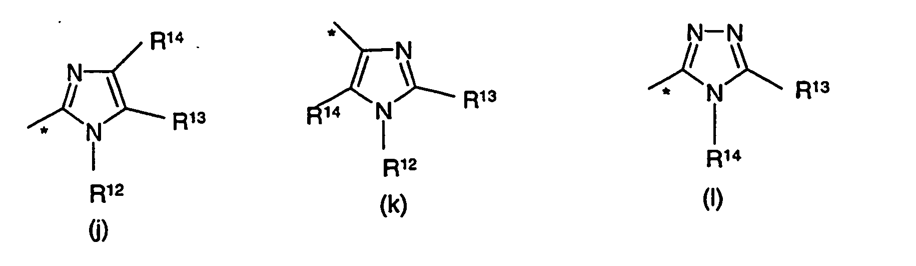 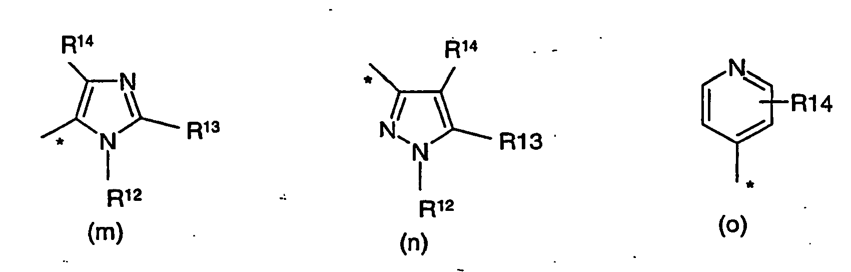 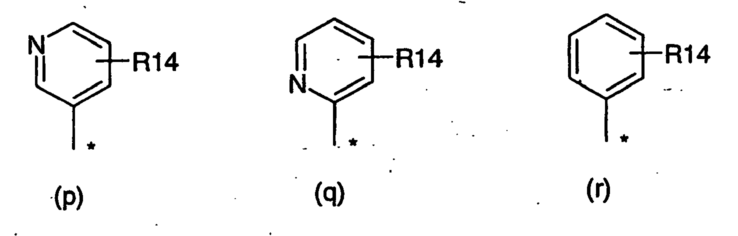 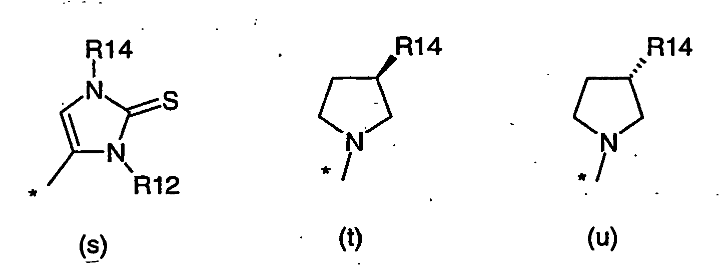 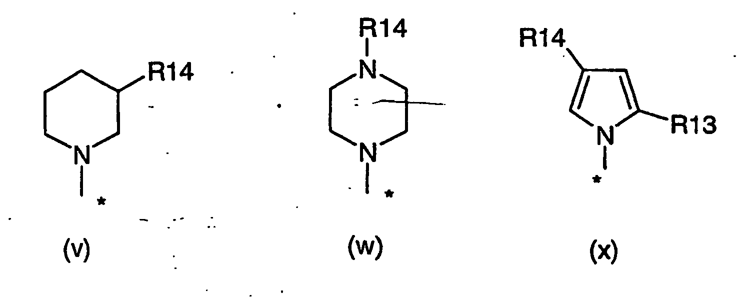 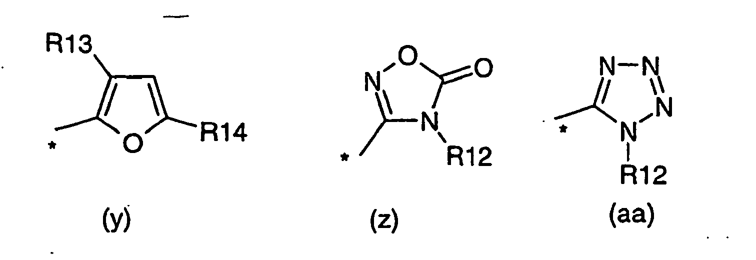 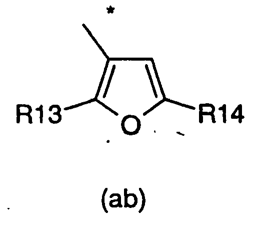 wherein * is the location wherein R⁴ is attached to the ring system and wherein
wherein R¹², R¹³ and R¹⁴ are each independently represented by H, Het, C₁₋₆alkyl, -CN, - NR^{a}R^{a}, -nitro, -C(=O)R^{a}, -C(=O)NR^{a}R^{a}, -C(=O)NR^{a}S(=O)₂R^{a}, -C(=O)NR^{a}-Het,-C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), - C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, - NR^{a}C(=O)OR^{a} , NR^{a}S(=O)₂R^{b}, -C(=NOR^{a})R^{a}, -S(=O)₂R^{a}, -S(=O)₂NR^{a}R^{a}, - S(=O)₂NR^{a}(R^{b}C(=O)NR^{a}R^{a}), or *-*S(=O)₂NR^{a}(R^{b}C(=O)OR^{a}.

In an additional embodiment the present invention provides a compound selected from:
5-{6-amino-2-[(6-chloroquinolin-4-yl)methyl]-5-methyl-4-oxo-4,5-dihydro-2H-pyrazolo[3,4-d]pyrimidin-3-yl)-1-methyl-1H-pyrrole-3-carbonitrile;
N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-3-methylbutanamide
N,N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-3-methylbutanamide;
N'-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-N,N-dimethylimidoformamide;
5-{2-[(6-chloroquinolin-4-yl)methyl]-6-[(cyclopropylmethyl)(methyl)amino]-5-methyl-4-oxo-4,5-dihydro-2H-pyrazolo[3,4-d]pyrimidin-3-yl}-1-methyl-1H-pyrrole-3-carbonitrile;
5-{2-[(6-chloroquinolin-4-yl)methyl]-6-[(cyclopropylmethyl)amino]-5-methyl-4-oxo-4,5-dihydro-2H-pyrazolo[3,4-d]pyrimidin-3-yl}-1-methyl-1H-pyrrole-3-carbonitrile;
N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]propane-1-sulfonamide;
ethyl 2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-ylcarbamate;
N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-N'-ethylurea;
5-[(4Z)-2-[(6-chloroquinolin-4-yl)methyl]-6-[(cyclopropylmethyl)amino]-5-methyl-4-(methylimino)-4,5-dihydro-2*H*-pyrazolo[3,4-*d*]pyrimidin-3-yl]-1-methyl-1*H*-pyrrole-3-carbonitrile;
5-[(4Z,6Z)-2-[(6-chloroquinolin-4-yl)methyl]-7-(cyclopropylmethyl)-5-methyl-4,6-bis(methylimino)-4,5,6,7-tetrahydro-2*H*-pyrazolo[3,4-*d*]pyrimidin-3-yl]-1-methyl-1*H* pyrrole-3-carbonitrile.

In a further embodiment the present invention provides a compound having structural formula (III) as recited above wherein: 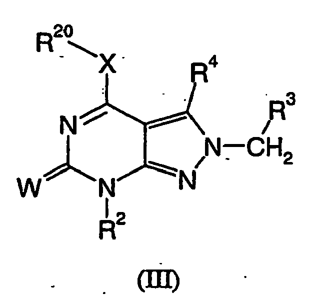 wherein,
X is S, O, NR²¹; or XR²⁰ is hydrogen;
W is S, O, or NR²⁰
R² is H, optionally substituted alkyl, optionally substituted alkylcycloalkyl, optionally substituted alkylcycloalkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, or optionally substituted heterocycle;
R³ is a monocyclic or bicyclic, saturated or unsaturated, ring system comprising 0, 1, 2 or 3 heteroatoms independently selected from N, O, or S, the ring being substituted by 0, 1, 2 or 3 substituents selected from =0, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵, -R⁵-Het, -Het-R⁵,-Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, O, or S, the ring system being substituted by 0, 1, 2 or 3 substituents selected from B(OH)₂, vicinal -OCH₂CH₂O-, vicinal - OC₁₋₂haloalkylO-, vicinal -OCH₂O-, vicinal -CH₂OCH₂O-, =O, halogen, -R^{b}OR^{a}, -SR^{a}, -OR^{a}, C₁₋₆alkyl, C₁₋₆haloalkyl, -CN, -S(=O)ₙR^{c}, -O(CH₂)ₘHet, -O(CH₂)ₘC(=O)Het,-O(CH₂)ₘC(=Q)NR^{a}R^{a}, -O(CH₂)ₘC(=O)OR^{a}, -O(CH₂)ₘNR^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}, -C(=O)NR^{a}(R^{b}Het), -C(=O)OR^{a}, -OC(=O)R^{a}, -C(=O)OR^{b}NR^{a}R^{a}, -C(=O)R^{a}, -C(=O)R^{b}R^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}(R^{b}(=O)NR^{a}R^{a}) S(=O)₂NR^{a}(R^{b}C(=O)OR^{a}), aminocarbonyl, phenyl, benzyl; or R⁴ is represented by -(CH₂)ₙR⁵-Het, -(CH₂)ₙR^{d}, -Het, -Het-Het, R⁵, -R⁵-Het, -Het-R⁵, -Het-OR⁵, R⁵-R⁵, or -R⁵-OR⁵; or R⁴ is represented by C₁₋₆alky, -NC₁₋₆alkyl, or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)_{z}CH₂OH, -(CH₂)_{z}CO₂H, or -(CH₂)_{z}CO₂C₁₋₆ alkyl wherein z is 1,2,3,4,5, or 6;
R⁵ is independently at each instance, phenyl substituted by 0, 1, 2, or 3 groups selected from halogen, C₁₋₆haloalkyl, -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}; -R^{b}OR^{a}, -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, or - S(=O)₂NR^{a}R^{b}C(=O)OR^{a};
R²⁰ is, independently at each instance, H, -CN, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²¹ is, independently at each instance, H, -CN, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}; or
R²⁰ and R²¹ and the N to which they are attached in combination can also form a 3 to 10 member N-linked saturated or unsaturated heterocycle having either 1, or 2 heteroatoms independently selected from N, O, or S wherein the heterocycle is substituted with R^{e};
R^{a} is, independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, 0 or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, vicinyl -OCH₂CH₂O-, vicinyl -OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, O, or S;
R^{e} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, 0 or S;
m is 1, 2 or 3;
n is 0, 1 or 2;
When "optionally substituted" is used, it refers to at least one substituent selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbaTnoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic
or a pharmaceutically acceptable salt thereof.

In an additional embodiment the present invention provides a compound having structural formula (III) as recited above wherein:
R² is -(CH₂)₁₋₃cycloalkyl or -C₁₋₁₂alkyl wherein -(CH₂)₁₋₃cycloalkyl or -C₁₋₁₂alkyl is optionally substituted with 0, 1, 2 or 3 substituents selected from Het, S(=O)ₙR^{c}, halogen, -CN, -OR^{a}, -NR^{a}R^{a}, -C(=O)OR^{a}, -C(=O)R^{a}, -C(=O)NR^{a}R^{a}, -OC(=O)C₁₋₄alkyl or -NR^{a}C(=O)C₁₋₄alkyl and n is 0, 1 or 2.

In an additional embodiment the present invention provides a compound having structural formula (III) as recited above wherein:
R³ is selected from formulas (i), (ii), (iii) or (iv) set forth below: 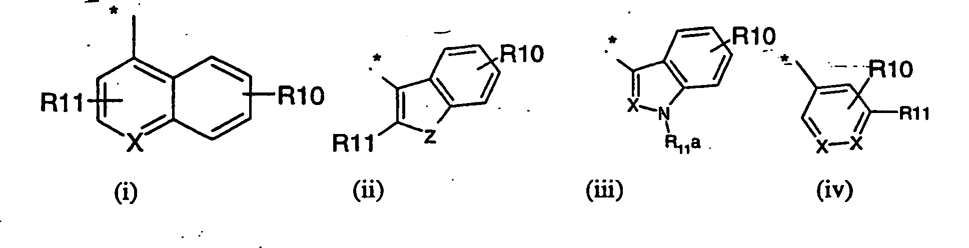
wherein * is the location where (i) or (ii) or (iii) or (iv) is attached to structural formula (I), and X is C or N; and Z is 0 or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.

In an additional embodiment the present invention provides a compound having structural formula (III) as recited above wherein:
R⁴ is selected from formulas (a) to (z) or (aa) or (ab) set forth below: 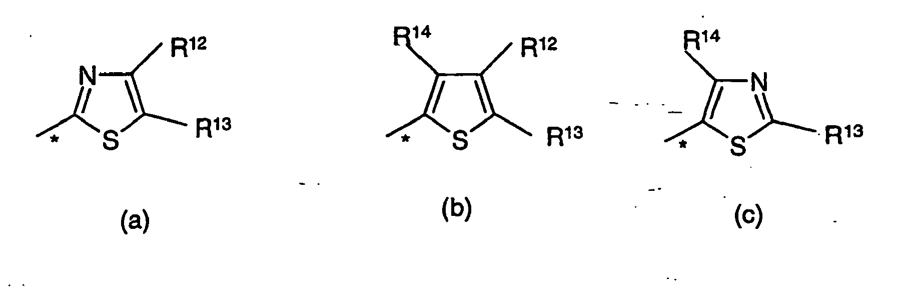 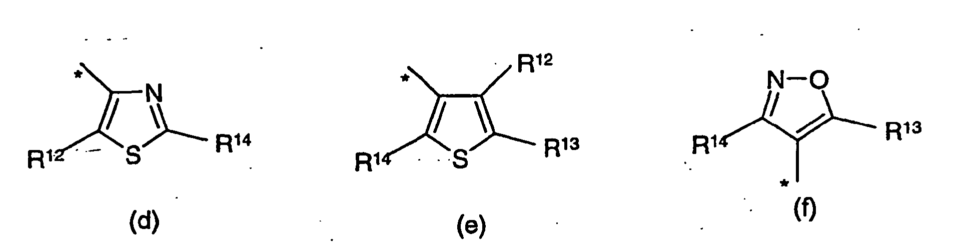 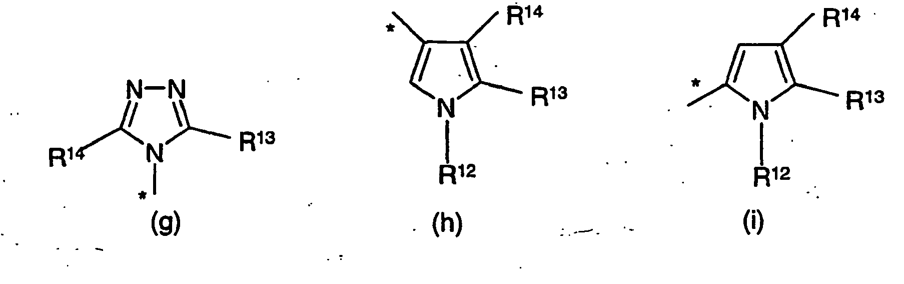 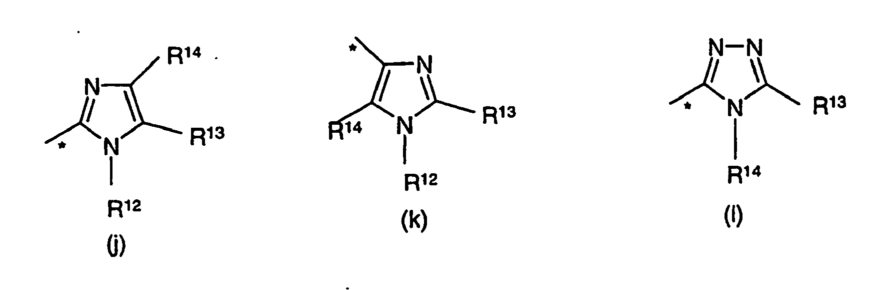 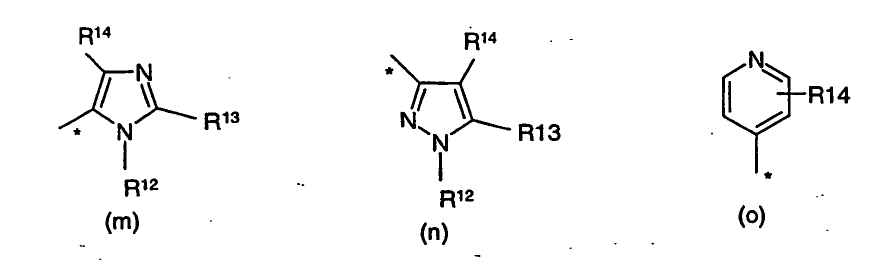 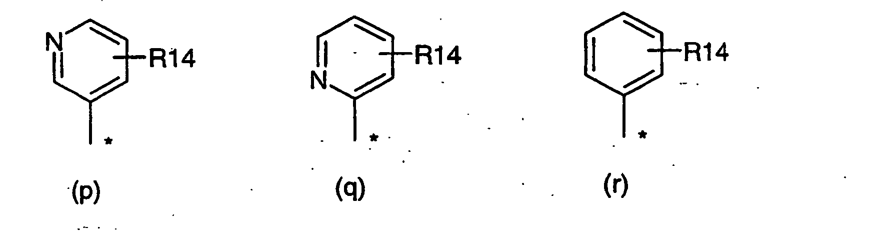 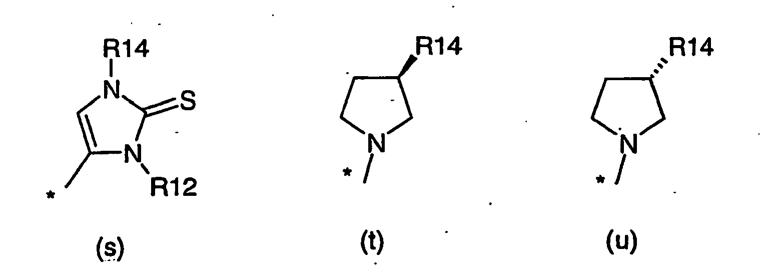 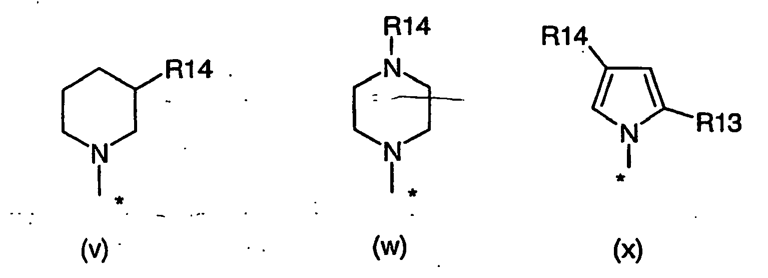 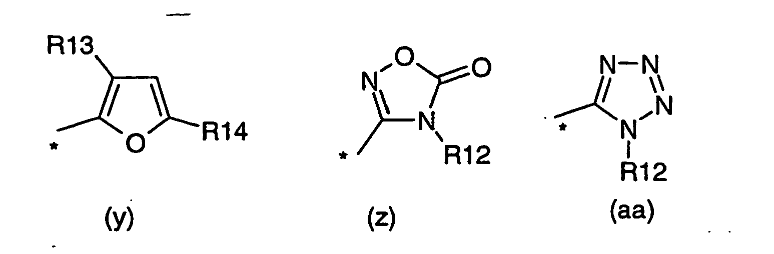 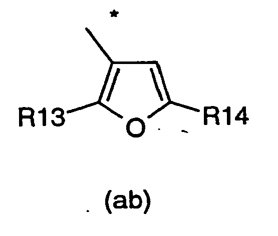
wherein * is the location wherein R⁴ is attached to the ring system and wherein
wherein R¹², R¹³ and R¹⁴ are each independently represented by H, Het, C₁₋₆alkyl, -CN, - NR^{a}R^{a}, -nitro, -C(=O)R^{a}, -C(=O)NR^{a}R^{a}, -C(=O)NR^{a}S(=O)₂R^{a}, -C(=O)NR^{a}-Het, - C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), - C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, - C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, - NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), or -S(=O)₂NR^{a}(R^{b}C(=O)OR^{a}.

In an additional embodiment the present invention provides a compound having structural formula (III) as recited above wherein:
X is S, 0, or NR²¹; or XR²⁰ is hydrogen,
W is S, O, or NR²⁰;
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ and R²¹ and the N to which they are attached in combination can.also form a 3 to 10 member N-linked saturated or unsaturated heterocycle having either 1, or 2 heteroatoms independently selected from N, 0, or S wherein the heterocycle is substituted with R^{e};
R¹ is CH₃, CH₂CH₃, CH₂CN, CF₃, (CH₂)₂OH, cyclopropyl, isopropyl, CH₂CCH, (CH₂)₂N(CH₂)₂, (CH₂)₂N(C=NH)NH₂, -CH₂-2-pyridyl, -CH₂-3-pyridyl, -CH₂-4-pyridyl, - (CH₂)₂-1-imidazolyl, -(CH₂)₂-1-pyrazolyl, -(CH₂)₂-1-piperidyl, -(CH₂)ₘ-(1-methylpiperidin-4-yl), -CH₂-(1-methylpiperidin-3-yl), -(CH₂)₂-(morpholin-4-yl),
R² is -CH₂CH₂CH₃, -CH₂-cyclopropyl, -CH₂CH(CH₃)₂, -CH₂CH₂CH₂F, -CH₂-cyclobutyl, -CH₂C(CH₃)₃, -CH₂CH₂CH(CH₃)₂, -CH₂CF₃, -CH₂-methylphenyl, -CH₂-phenol, - CH₂-(3,5-dimethylisoxazol-4-yl), -CH₂-S-phenyl, -CH₂-phenylcarboxyl, or -CH₂SCF₃;
R³ is selected from formulas (i), (ii), (iii) or (iv) set forth below: 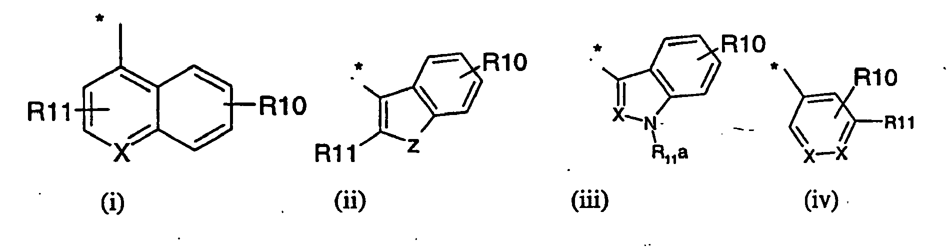 wherein * is the location where (i) or (ii) or (iii) or (iv) is attached to structural formula (I), and X is C or N; and Z is 0 or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a},-OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.
R⁴ is selected from formulas (a) to (z) or (aa) or (ab) set forth below: 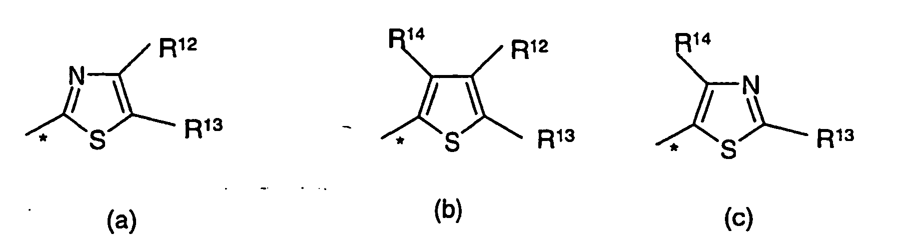 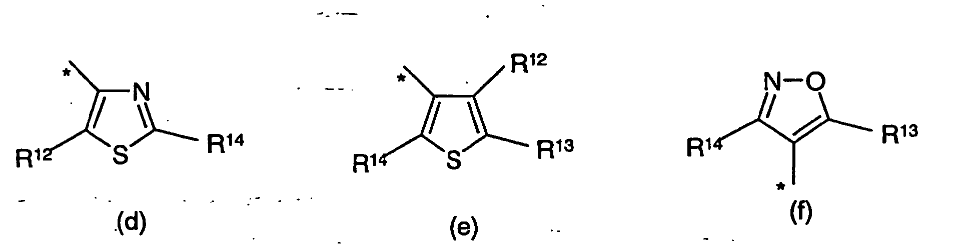 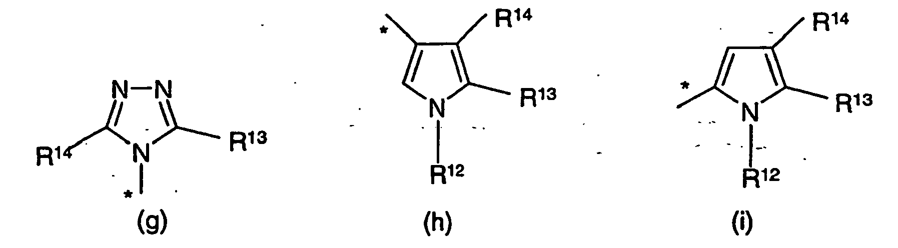 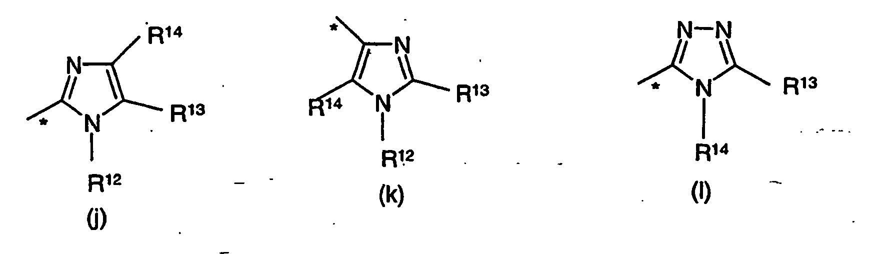 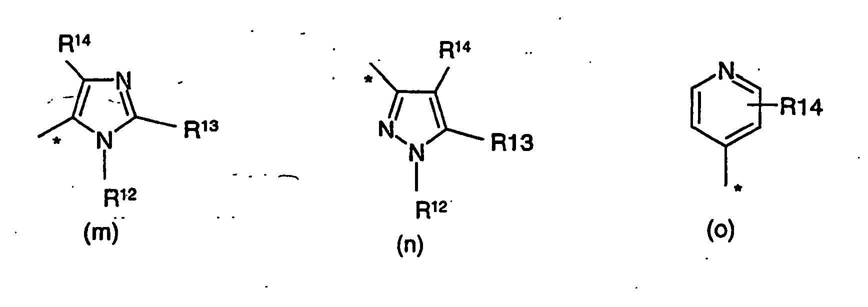 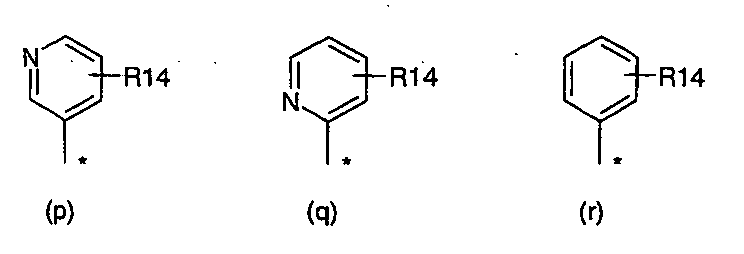 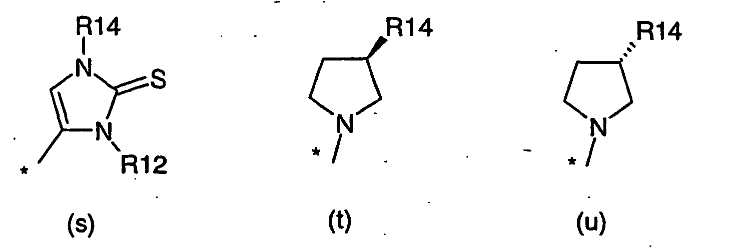 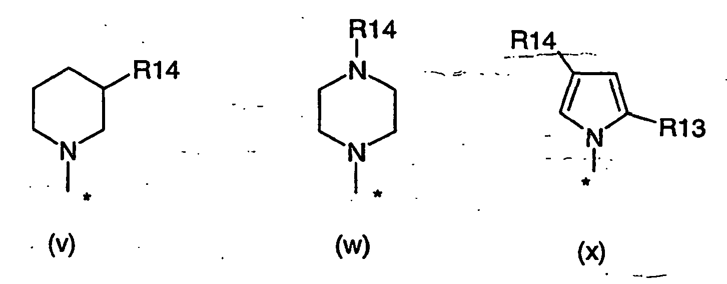 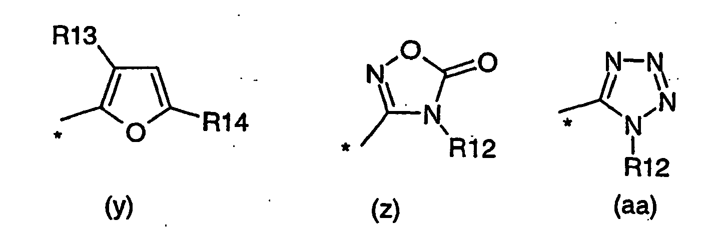 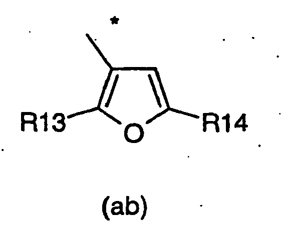
wherein * is the location wherein R⁴ is attached to the ring system and wherein
wherein R¹², R¹³ and R¹⁴ are each independently represented by H, Het, C₁₋₆alkyl, -CN, - NR^{a}R^{a}, -nitro, -C(=O)R⁴, -C(=O)NR^{a}R^{a}, -C(=O)NR^{a}S(=O)₂R^{a}, -C(=O)NR^{a}-Het, - C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, - C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, - NR^{a}C(=O)OR^{a}, -NR^{a}S(=O)₂R^{b}, -C(=NOR^{a})R^{a}, -S(=O)₂R^{a}, -S(=O)₂NR^{a}R^{a},-S(=O)₂NR^{a}(R^{b}C(=O)NR^{a}R^{a}), or -S(=O)₂NR^{a}(R^{b}C(=O)OR^{a}.

In an additional embodiment the present invention provides a compound selected from:
4-amino-7-isobutyl-2-(1-naphthylmethyl)-3-pyridin-4-yl-2,7-dihydro-6H-pyrazolo [3,4-d]pyrimidin-6-one;
7-isobutyl-4-(methylamino)-2-(1-naphthylmethyl)-3-pyridin-4-yl-2,7-dihydro-6H-pyrazolo[3,4-d]pyrimidin-6-one;
4-(dimethylamino)-7-isobutyl-2-(1-naphthylmethyl)-3-pyridin-4-yl-2,7-dihydro-6H-pyrazolo[3,4-d]pyrimidin-6-one;
7-isobutyl-4-(4-methylpiperazin-1-yl)-2-(1-naphthylmethyl)-3-pyridin-4-yl-2,7-dihydro-6H-pyrazolo[3,4-d]pyrimidin-6-one;
4-amino-2-[(6-chloroquinolin-4-yl)methyl]-7-isobutyl-3-(1-methyl-1H-pyrrol-2-yl)-2,7-dihydro-6H-pyrazolo[3,4-d]pyrimidin-6-one;
5-{4-amino-2-[(6-chloroquinolin-4-yl)methyl]-7-isobutyl-6-oxo-6,7-dihydro-2H-pyrazolo[3,4-d]pyrimidin-3-yl}-1-methyl-1H-pyrrole-3-carbonitrile;
5-[2-[(6-chloroquinolin-4-yl)methyl]-7-isobutyl-4-(methylamino)-6-oxo-6,7-dihydro-2H-pyrazolo[3,4-d]pyrimidin-3-yl]-1-methyl-1H-pyrrole-3-carbonitrile;
5-[2-[(6-chloroquinolin-4-yl)methyl]-4-(dimethylamino)-7-isobutyl-6-oxo-6,7-dihydro-2H-pyrazolo[3,4-d]pyrimidin-3-yl]-1-methyl-1H-pyrrole-3-carbonitrile;
5-[2-[(6-chloroquinolin-4-yl)methyl]-7-isobutyl-6-oxo-4-(propylamino)-6,7-dihydro-2H-pyrazolo[3,4-d]pyrimidin-3-yl]-1-methyl-1H-pyrrole-3-carbonitrile;
5-{2-[(6-chloroquinolin-4-yl)methyl]-4-[(2-hydroxyethyl)amino]-7-isobutyl-6-oxo-6,7-dihydro-2H-pyrazolo[3,4-d]pyrimidin-3-yl}-1-methyl-1H-pyrrole-3-carbonitrile.
5-[2-[(6-chloroquinolin-4-yl)methyl]-4-(hydroxyamino)-7-isobutyl-6-oxo-6,7-dihydro-2H-pyrazolo [3,4-d]pyrimidin-3-yl]-1-methyl-1H-pyrrole-3-carbonitrile;
5-[2-[(6-chloroquinolin-4-yl)methyl]-4-(cyclopropylamino)-7-isobutyl-6-oxo-6,7-dihydro-2H-pyrazolo[3,4-d]pyrimidin-3-yl]-1-methyl-1H-pyrrole-3-carbonitrile;
5-{2-[(6-chloroquinolin-4-yl)methyl]-4-hydrazino-7-isobutyl-6-oxo-6,7-dihydro-2H-pyrazolo[3,4-d]pyrimidin-3-yl}-1-methyl-1H-pyrrole-3-carbonitrile;
5-[2-[(6-chloroquinolin-4-yl)methyl]-4-(2,2-dimethylhydrazino)-7-isobutyl-6-oxo-6,7-dihydro-2H-pyrazolo[3,4-d]pyrimidin-3-yl]-1-methyl-1H-pyrrole-3-carbonitrile;
N-[2-[(6-chloroquinolin-4-yl)methyl]-3-(4-cyano-1-methyl-1H-pyrrol-2-yl)-7-isobutyl-6-oxo-6,7-dihydro-2H-pyrazolo[3,4-*d*]pyrimidin-4-yl]acetamide;
5-[2-[(6-chloroquinolin-4-yl)methyl]-7*-*(cyclopropylmethyl)-4-(methylthio)-6-oxo-6,7-dihydro-2H-pyrazolo[3,4-d]pyrimidin-3-yl]-1-methyl-1H-pyrrole-3-carbonitrile;
5-{2-[(6-chloroquinolin-4-yl)methyl]-7-(cyclopropylmethyl)-4-[(2-hydroxybutyl)amino]-6-oxo-6,7-dihydro-2H-pyrazolo[3,4-d]pyrimidin-3-yl}-1-methyl-1H-pyrrole-3-carbonitrile;
5-(2-[(6-chloroquinolin-4-yl)methyl]-7-(cyclopropylmethyl)-4-{[(2R)-2-hydroxypropyl]amino}-6-oxo-6,7-dihydro-2H-pyrazolo[3,4-d]pyrimidin-3-yl)-1-methyl-1H-pyrrole-3-carbonitrile;
5-[2-[(6-chloroquinolin-4-yl)methyl]-7-(cyclopropylmethyl)-4-methoxy-6-oxo-6,7-dihydro-2*H*-pyrazolo[3,4-*d*]pyrimidin-3-yl]-1-methyl-1*H*-pyrrole-3-carbonitrile;
5-[2-[(6-chloroquinolin-4-yl)methyl]-7-(cyclopropylmethyl)-6-oxo-4-(1*H*-pyrrol-1-yl)-6,7-dihydro-2*H*-pyrazolo[3,4-*d*]pyrimidin-3-yl]-1-methyl-1*H*-pyrrole-3-carbonitrile;
5-[(6Z)-2-[(6-chloroquinolin-4-yl)methyl]-7-(cyclopropylmethyl)-4-(methylamino)-6-(methylimino)-6,7-dihydro-2*H*-pyrazolo[3,4-*d*]pyrimidin-3-yl]-1-methyl-1*H*-pyrrole-3-carbonitrile;
5-[4-amino-2-[(6-chloroquinolin-4-yl)methyl]-7-(cyclopropylmethyl)-6-oxo-6,7-dihydro-2*H* pyrazolo[3,4-*d*]pyrimidin-3-yl]-1-methyl-1*H*-pyrrole-3-carbonitrile;

In a further embodiment the present invention provides a compound having structural formula (IV), 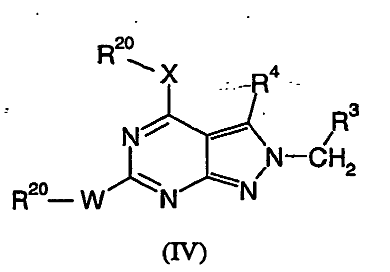 wherein,
X is S, O, NR²¹; or XR²⁰ is hydrogen;
W is S, O, or NR²¹;
R³ is a monocyclic or bicyclic, saturated or unsaturated, ring system comprising 0, 1, 2 or 3 heteroatoms independently selected from N, 0, or S, the ring being substituted by 0, 1, 2 or 3 substituents selected from =O, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵,-R⁵-Het, -Het-R⁵, - Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, 0, or S, the ring system being substituted by 0, 1, 2 or 3 substituents selected from B(OH)₂, vicinal -OCH₂CH₂O-, vicinal - OC₁₋₂haloalkylO-, vicinal -OCH₂O-, vicinal -CH₂OCH₂O-, =O, halogen, -R^{b}OR^{a}, -SR^{a}, -OR^{a}, C₁₋₆alkyl, C₁₋₆haloalkyl, -CN, -S(=O)ₙR^{c}, -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, - S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, - C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}), - C(=O)NR^{a}(R^{b}Het), -C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, -C(=NCN)R⁸, -S(=O)₂NR^{a}R^{a}, -NR^{a}S(=O)₂R^{a}, -S(=O)₂NR^{a}(R^{b}C(=O)NR^{a}R^{a}), - S(=O)₂NR^{a}(R^{b}C(=O)OR^{a}, aminocarbonyl, phenyl, benzyl; or R⁴ is represented by -(CH₂)ₙR⁵-Het, -(CH₂)ₙR^{d}, -Het, -Het-Het, R⁵, -R⁵-Het, -Het-R⁵, -Het-OR⁵, R⁵-R⁵, or -R⁵-OR⁵; or R⁴ is represented by C₁₋₆alky, -NC₁₋₆alkyl, or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)_{z}CH₂OH, -(CH₂)_{z}CO₂H, or -(CH₂)_{z}CO₂C₁₋₆ alkyl wherein z is 1,2,3,4,5, or 6;
R⁵ is independently at each instance, phenyl substituted by 0, 1, 2, or 3 groups selected from halogen, C₁₋₆haloalkyl, -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH2)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘ,NR^{a}R^{a}, -S(CH₂)ₘOR^{a}, -R^{b}OR^{a} -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a} -S(=O)₂NR^{a}R^{a}, -NR^{a}S(=O)₂R^{a}, -S(=O)₂NR^{a}R^{b}C(=O)NR^{a}R^{a}, or - S(=O)₂NR^{a}R^{b}C(=O)OR^{a};
R²⁰ is, independently at each instance, H, -CN, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, wherein such substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²¹ is, independently at each instance, H, -CN, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}; optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle wherein such substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²⁰ and R²¹ and the N to which they are attached in combination can also form a 3 to 10 member N-linked saturated or unsaturated heterocycle having either 1, or 2 heteroatoms independently selected from N, 0, or S wherein the heterocycle is substituted with R^{e};
R^{a} is, independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, vicinyl -OCH₂CH₂O-, vicinyl -OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, O, or S;
R^{e} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
m is 1,2 or 3;
n is 0, 1 or 2;
When "optionally substituted" is used, it refers to at least one substituent selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic
or a pharmaceutically acceptable salt thereof.

In an additional embodiment the present invention provides a compound having structural formula (IV) as recited above wherein:
R³ is selected from formulas (i), (ii), (iii) or (iv) set forth below: 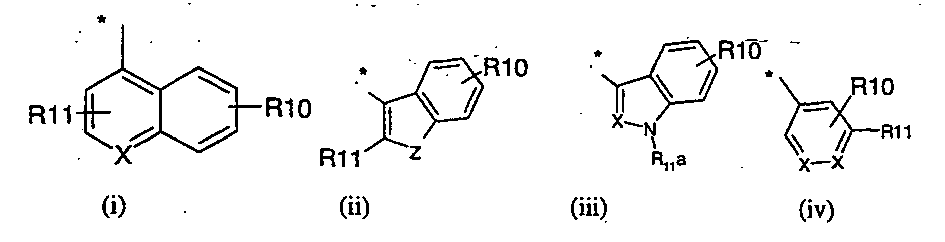
wherein * is the location where (i) or (ii) or (iii) or (iv) is attached to structural formula (I), and X is C or N; and Z is O or S,-wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}-C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or-S(=O)ₙR^{c} and n=1 or 2.

In an additional embodiment the present invention provides a compound having structural formula (IV) as recited above wherein:
R⁴ is selected from formulas (a) to (z) or (aa) or (ab) set forth below: 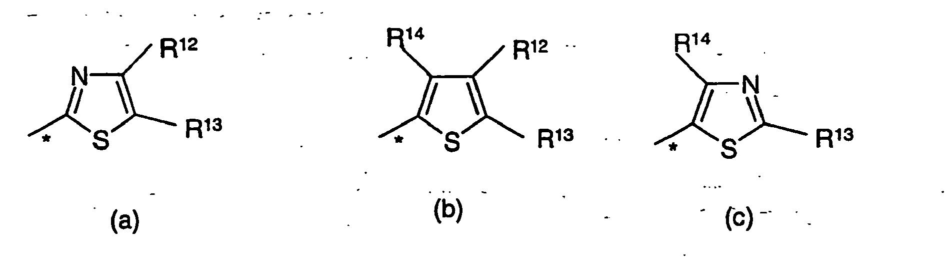 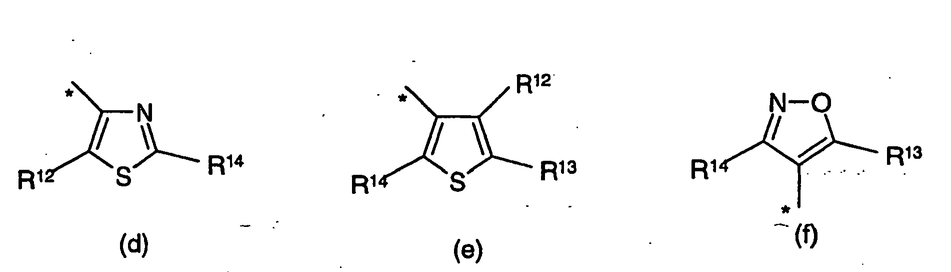 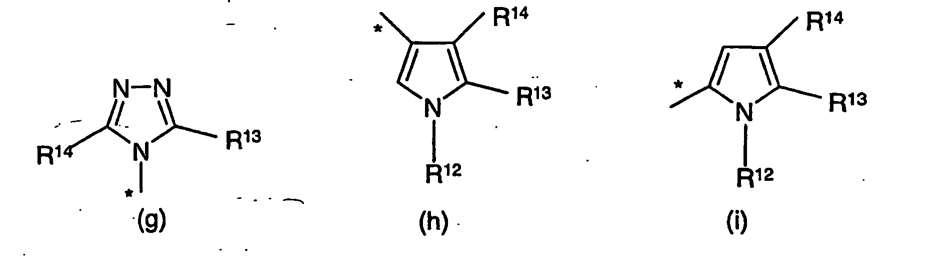 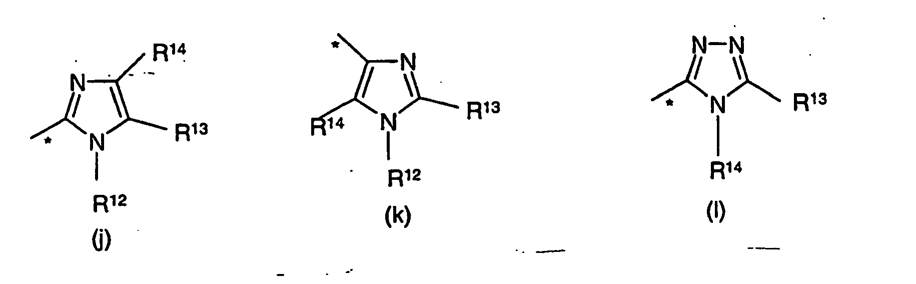 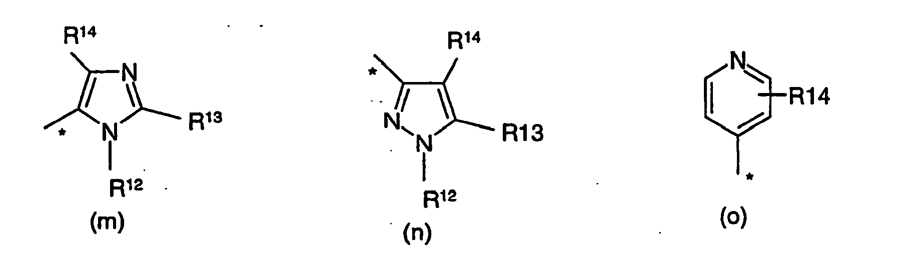 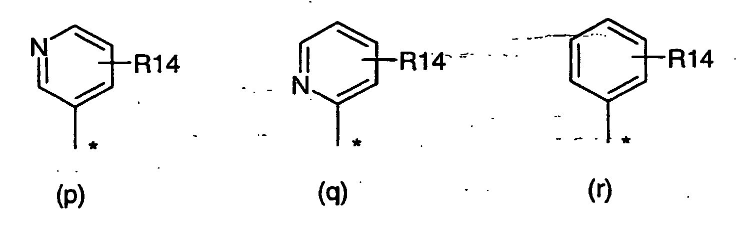 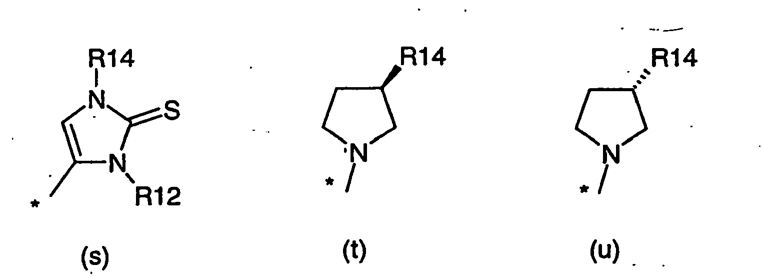 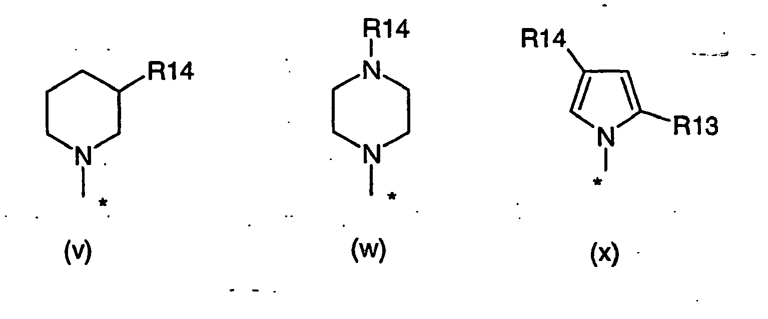 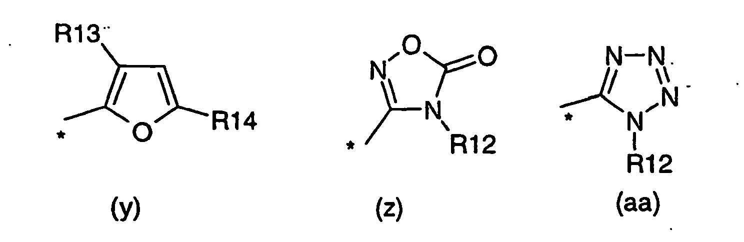 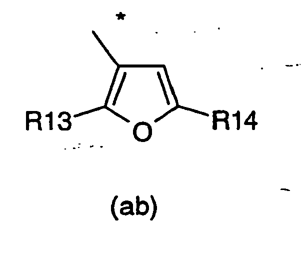
wherein * is the location wherein R⁴ is attached to the ring system and wherein
wherein R¹², R¹³ and R¹⁴ are each independently represented by H, Het, C₁₋₆alkyl, -CN, - NR^{a}R^{a}, -nitro, -C(=O)R^{a}, -C(=O)NR^{a}R^{a}, -C(=O)NR^{a}S(=O)₂R^{a}, -C(=O)NR^{a}-Het, - C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)R^{a}), - C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, - NR^{a}C(=O)OR^{a}, -NR^{a}S(=O)₂R^{b}, -C(=NOR^{a})R^{a}, -S(=O)₂R^{a}, -S(=O)₂NR^{a}R^{a}, - S(=O)₂NR^{a}(R^{b}C(=O)NR^{a}R^{a}), or -S(=O)₂NR^{a}(R^{b}C(=O)OR^{a}.

In an additional embodiment the present invention provides a compound having structural formula (IV) as recited above wherein:
X is S, O, or NR²¹; or X-R²⁰ is hydrogen
W is S, O, or NR²¹;
R¹ is CH₃, CH₂CH₃, CH₂CN, CF₃, (CH₂)₂OH, cyclopropyl, isopropyl, CH₂CCH, (CH₂)₂N(CH₂)₂, (CH₂)₂N(C=NH)NH₂, -CH₂-2-pyridyl, -CH₂-3-pyridyl, -CH₂-4-pyridyl, - (CH₂)₂-1-imidazolyl, -(CH₂)₂-1-pyrazolyl, -(CH₂)₂-1-piperidyl, -(CH₂)ₘ-(1-methylpiperidin-4-yl), -CH₂-(1-methylpiperidin-3-yl), -(CH₂)₂-(morpholin-4-yl),
R² is -CH₂CH₂CH₃, -CH₂-cyclopropyl, -CH₂CH(CH₃)₂, -CH₂CH₂CH₂F, -CH₂-cyclobutyl, -CH₂C(CH₃)₃, -CH₂CH₂CH(CH₃)₂, -CH₂CF₃, -CH₂-methylphenyl, -CH₂-phenol, - CH₂-(3,5-dimethylisoxazol-4-yl), -CH₂-S-phenyl, -CH₂-phenylcarboxyl, or -CH₂SCF₃;
R³ is selected from formulas (i), (ii), (iii) or (iv) set forth below: 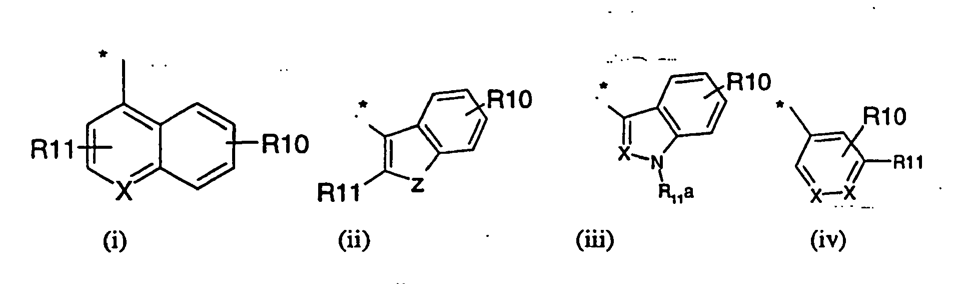 wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, - C(=O)OR^{a}, -C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.
R⁴ is selected from formulas (a) to (z) or (aa) or (ab) set forth below: 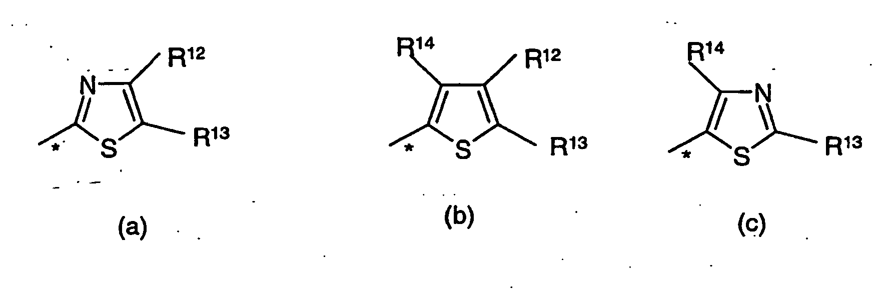 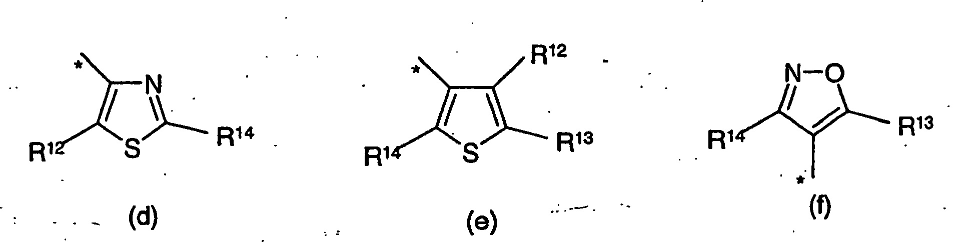 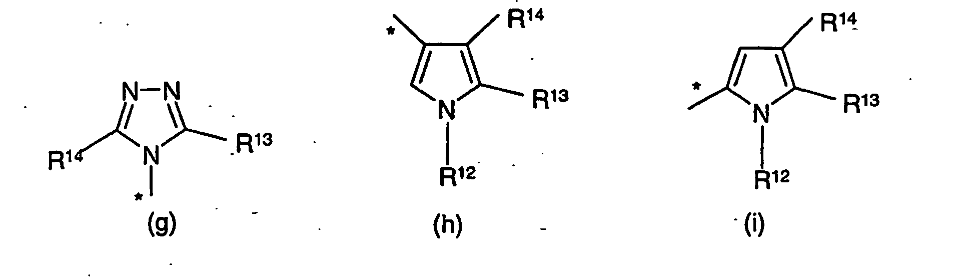 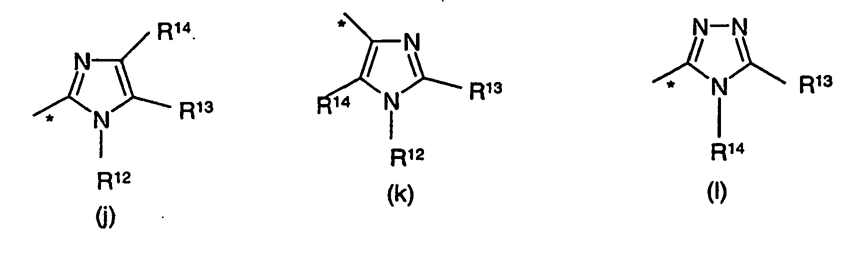 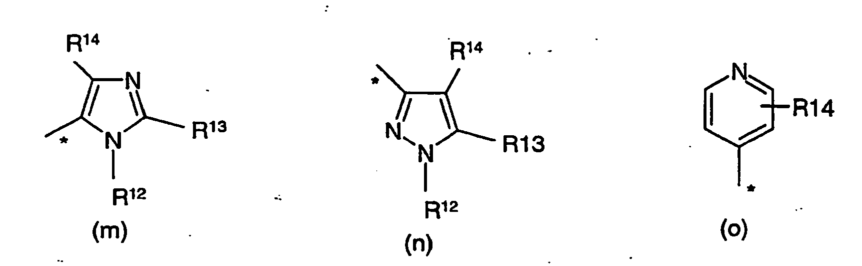 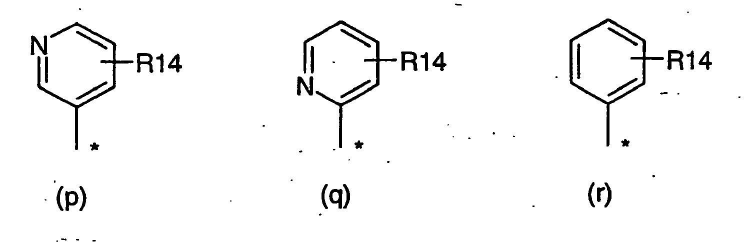 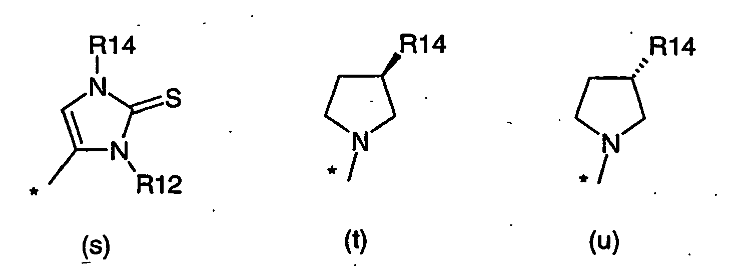 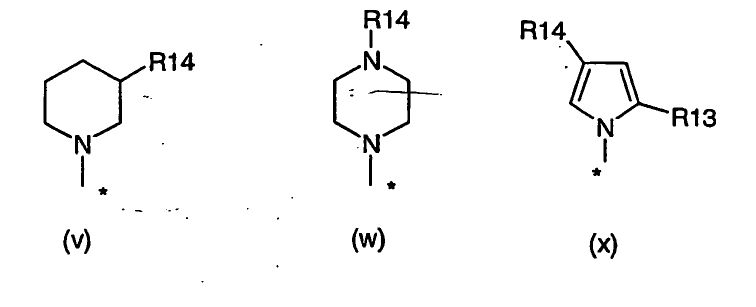 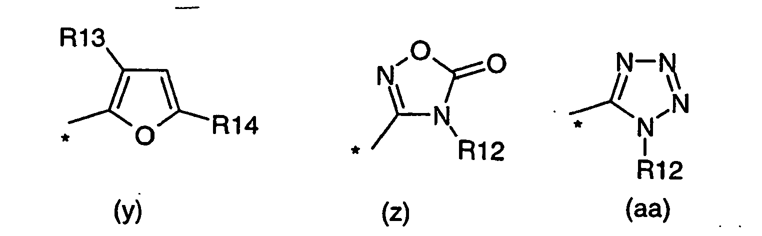 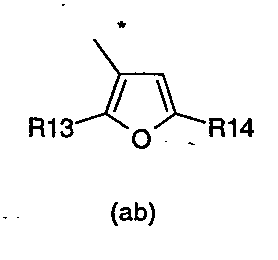 wherein * is the location wherein R⁴ is attached to the ring system and wherein
   wherein R¹², R¹³ and R¹⁴ are each independently represented by H, Het, C₁₋₆alkyl, -CN, - NR^{a}R^{a}, -nitro, -C(=O)R^{a}, -C(=O)NR^{a}R^{a}, -C(=O)NR^{a}S(=O)₂R^{a}, -C(=O)NR^{a}-Het, - C(=O)NR^{a}NR^{a}R^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), - C(=O)NR^{a}R^{b}Het, -C(=O)NR^{a}OR^{b}, -C(=O)R^{b}NR^{a}R^{a}, -C(=NOR^{a})R^{a}, -C(=NCN)R^{a}, - C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, - NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), or -S(-O)₂NR^{a}(R^{b}(-O)OR^{a}.
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ and R²¹ and the N to which they are attached in combination can also form a 3 to 10 member N-linked saturated or unsaturated heterocycle having either 1, or 2 heteroatoms independently selected from N, 0, or S wherein the heterocycle is substituted with R^{e};
R^{e} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, 0 or S.

In an additional embodiment the present invention provides a compound selected from:
5-[2-[(6-chloroquinolin-4-yl)methyl]-6-[(cyclopropylmethyl)amino]-4-(methylamino)-2*H-*pyrazolo[3,4-*d*]pyrimidin-3-yl]-1-methyl-1*H-*pyrrole-3-carbonitrile;
N-{3-(4-acetyl-1-methyl-1*H-*pyrrol-2-yl)-2-[(6-chloroquinolin-4-yl)methyl]A-methoxy-2*H-*pyrazolo[3,4-d]pyrimidin-6-yl}-2-cyclopropylacetamide.

In a further embodiment the present invention provides a compound according to any one of claims 1 to 24, for use as a medicament.

In a further embodiment the present invention provides the use of a compound as defined in any one of claims 1 to 24, in the manufacture of a medicament for the treatment or prophylaxis of disorders associated with *H*. *pylori* infection.

In a further embodiment the present invention provides a pharmaceutical composition comprising a compound as defined in any one of formulas (I), (II), (III), (IV) together with at least one pharmaceutically acceptable carrier, diluent or excipent.

### Definitions

The definitions set forth in this section are intended to clarify terms used throughout this application. The term "herein" means the entire application.

As used in this application, the term "optionally substituted," as used herein, means that substitution is optional and therefore it is possible for the designated atom to be unsubstituted. In the event a substitution is desired then such substitution means that any number of hydrogens on the designated atom is replaced with a selection from the indicated group, provided that the designated atom's normal valency is not exceeded, and that the substitution results in a stable compound. For example when a substituent is keto (i.e., =0), then 2 hydrogens on the atom are replaced.

When any variable (e.g., R¹, R⁴, R^{a}, R^{e} etc.) occurs more than one time in any constituent or formula for a compound, its definition at each occurrence is independent of its definition at every other occurrence. Thus, for example, if a group is shown to be substituted with 0-3 R¹, then said group may optionally be substituted with 0,1, 2 or 3 R¹ groups and R^{e} at each occurrence is selected independently from the definition of R^{e}. Also, combinations of substituents and/or variables are permissible only if such combinations result in stable compounds.

The compounds herein described may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. When required, separation of the racemic material can be achieved by methods known in the art. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic forms and all geometric isomeric forms of a structure are intended, unless the specific stereochemistry or isomeric form is specifically indicated.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

As used herein "acyl" refers to groups of the of the general formula -C(=O)-R, wherein R is hydrogen, hydrocarbyl radical, amino or alkoxy. Examples of acyl groups include, but are not limited to acetyl, propionyl, benzoyl, phenyl acetyl, carboethoxy, and dimethylcarbamoyl.

As used herein the term "amine" or "amino" refers to groups of the general formula-NRR', wherein R and R' are independently selected from hydrogen or a hydrocarbyl radical.

As used herein "aromatic" refers to hydrocarbyl groups having one or more polyunsaturated carbon rings having aromatic character, (e.g., 4n + 2 delocalized electrons) and comprising up to about 14 carbon atoms.

As used herein, "alkyl" or "alkylene" used alone or as a suffix or prefix, is intended to include both branched and straight-chain saturated aliphatic hydrocarbon groups having from 1 to 12 carbon atoms or if a specified number of carbon atoms is provided then that specific number would be intended. For example "C₁₋₆ alkyl" denotes alkyl having 1, 2, 3, 4, 5 or 6 carbon atoms. Examples of alkyl include, but are not limited to, methyl, ethyl, n-propyl, i-propyl, n-butyl, i-butyl, sec-butyl, t-butyl, pentyl, and hexyl. As used herein, "C₁₋₃ alkyl", whether a terminal substituent or an alkylene group linking two substituents, is understood to specifically include both branched and straight-chain methyl, ethyl, and propyl.

As used herein, "alkenyl" or "alkenylene" is intended to include from 2 to 12 hydrocarbon atoms of either a straight or branched configuration with one or more carbon-carbon double bonds that may occur at any stable point along the chain. Examples of "C₃₋₆alkenyl" include, but are not limited to, 1-propenyl, 2-propenyl, 1-butenyl, 2-butenyl, 3-butenyl, 3-methyl-2-butenyl, 2-pentenyl, 3-pentenyl, hexenyl.

As used herein, "alkynyl" or "alkynylene" is intended to include from 2 to 12 hydrocarbon chains of either a straight or branched configuration with one or more carbon-carbon triple bonds that may occur at any stable point along the chain. Examples of alkynyl include but are not limited to ethynyl, 1-propynyl, 2-propynyl, 1-butynyl, 2-butynyl, 3-butynyl.

As used herein, "alkoxy" or "alkyloxy" represents an alkyl group as defined above with the indicated number of carbon atoms attached through an oxygen bridge. Examples of alkoxy include, but are not limited to, methoxy, ethoxy, n-propoxy, isopropoxy, n-butoxy, isobutoxy, t-butoxy, n-pentoxy, isopentoxy, cyclopropylmethoxy, allyloxy and propargyloxy. Similarly, "alkylthio" or "thioalkoxy" represent an alkyl group as defined above with the indicated number of carbon atoms attached through a sulphur bridge.

As used herein, the term "aryl" is intended to mean aromatic groups including both monocyclic aromatic groups comprising 6 carbon atoms and polycyclic aromatic groups comprising up to about 14 carbon atoms.

As used herein the term "cycloalkyl" is intended to include saturated ring groups, having the specified number of carbon atoms. For example, "C₃₋₆ cycloalkyl" denotes such groups as cyclopropyl, cyclobutyl, cyclopentyl, or cyclohexyl.

As used herein the term "alkylcycloalkyl" is intended to mean an alkyl attached to the formula atom modified with a cycloalkyl. Examples of alkylcycloalkyl include, but are not limited to cyclopropylmethyl, cyclopentylmethyl, cyclohexylmethyl, cycloheptylmethyl, cyclopropylethyl, cyclopentylethyl, cyclohexylethyl, cycloheptylethyl, cyclopropylpropyl, cyclopentylpropyl, cyclohexylpropyl, cycloheptylpropyl.

As used herein "cycloalkenyl" refers to ring-containing hydrocarbyl groups having at least one carbon-carbon double bond in the ring, and having from 3 to 12 carbons atoms.

As used herein "cycloalkynyl" refers to ring-containing hydrocarbyl groups having at least one carbon-carbon triple bond in the ring, and having from 7 to 12 carbons atoms.

As used herein, "electronically neutral" refers to a stable compound having a no charge.

As used herein, "halo" or "halogen" refers to fluoro, chloro, bromo, and iodo. "Counterion" is used to represent a small, negatively charged species such as chloride, bromide, hydroxide, acetate, sulfate, tosylate, benezensulfonate, and the like.

As used herein, "haloalkyl" is intended to include both branched and straight-chain saturated aliphatic hydrocarbon groups having the specified number of carbon atoms, substituted with 1 or more halogen (for example --CᵥF_{w} where v=1 to 3 and w=1 to (2v+1)). Examples of haloalkyl include, but are not limited to, trifluoromethyl, trichloromethyl, pentafluoroethyl, pentachloroethyl, 2,2,2-trifluoroethyl, 2,2-difluoroethyl, heptafluoropropyl, and heptachloropropyl. "Haloalkoxy" is intended to mean a haloalkyl group as defined above with the indicated number of carbon atoms attached through an oxygen bridge; for example trifluoromethoxy, pentafluoroethoxy, 2,2,2-trifluoroethoxy, and the like. "Haloalkylthio" is intended to mean a haloalkyl group as defined above with the indicated number of carbon atoms attached through a sulphur bridge.

As used herein, the term "Het" is intended to mean a 5 or 6 member ring, saturated or unsaturated heterocycle containing 1, 2, 3 or 4 heteroatoms independently selected from N, 0, or S, and substituted by 0, 1, 2 or 3 substituents selected from halogen, C₁₋₄alkyl, -S(=O)ₙR^{c}, - C(=O) R^{a}, or -S(=O)₂NR^{a}R^{a}, vicinal -OCH₂CH₂O-, vicinal -OC₁₋₂haloalkylO-, vicinal -OCH₂O-, or vicinal -CH₂OCH₂O-, =O, halogen, cyano, -R^{b}OR^{a}, -R^{b}SR^{a}, -SR^{a}, -OR^{a}, C₁₋₆alkyl, C₁₋₆haloalkyl, -CN, nitro, -OH, -NHR^{a}, -NR^{a}₂, -NHC(=O)R^{a}, N=NR^{a}, -C(=O)NR^{a}R^{a}, -C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, -C(=O)NR^{a}R^{b} Het, -C(=O)OR^{a}, -OC(=O)R^{a}, -C(=O)OR^{b}NR^{a}R^{a}, - C(=O)R^{b}NR^{a}R^{a}, -C(=NOR^{a})R^{a}, -C(=NCN)R^{a}, -NR^{a}S(=O)₂R^{a}, -S(=O)₂NR^{a}R^{b}C(=O)NR^{a}R^{a}, or -S(=O)₂NR^{a}R^{b}C(=O)OR^{a};

As used herein, the term "heterocycle" or "heterocyclic" or hetercyclyl refers to a ring-containing monovalent and divalent structures having one or more heteroatoms, independently selected from N, 0 and S, as part of the ring structure and comprising from 3 to 20 atoms in the rings. Heterocyclic groups may be saturated or unsaturated, containing one or more double bonds, and heterocyclic groups may contain more that one ring. The heterocyclic rings described herein may be substituted on carbon or on a heteroatom atom if the resulting compound is stable. If specifically noted, nitrogen in the heterocycle may optionally be quaternized. It is understood that when the total number of S and 0 atoms in the heterocycle exceeds 1, then these heteroatoms are not adjacent to one another.

Examples of heterocycles include, but are not limited to, 1H-indazole, 2-pyrolidonyl, 2H, 6H-1, 5,2-dithiazinyl, 2H-pyrrolyl, 3H-indolyl, 4-piperidonyl, 4aH-carbazole, 4H-quinolizinyl, 6H-1, 2,5-thiadiazinyl, acridinyl, azetidine, aziridine, azocinyl, benzimidazolyl, benzofuranyl, benzothiofuranyl, benzothiophenyl, benzoxazolyl, benzthiazolyl, benzotriazolyl, benzotetrazolyl, benzisoxazolyl, benzisothiazolyl, benzimidazalonyl, carbazolyl, 4aH-carbazolyl, b-carbolinyl, chromanyl, chromenyl, cinnolinyl, decahydroquinolinyl, 2H,6H-1,5,2-dithiazinyl, dioxolane, furyl, 2,3-dihydrofuran, 2,5-dihydrofuran, dihydrofuro[2,3-b]tetrahydrofuran, furanyl, furazanyl, homopiperidinyl, imidazolidine, imidazolidinyl, imidazolinyl, imidazolyl, 1H-indazolyl, indolenyl, indolinyl, indolizinyl, indolyl, isobenzofuranyl, isochromanyl, isoindazolyl, isoindolinyl, isoindolyl, isoquinolinyl, isothiazolyl, isoxazolyl; morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxirane, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, purinyl, pyranyl, pyrrolidine, pyrroline, pyrrolidine, pyrazinyl, pyrazolidinyl, pyrazolinyl, pyrazolyl, pyridazinyl, pyridooxazole, pyridoimidazole, pyridothiazole, pyridinyl, N-oxide-pyridinyl, pyridyl, pyrimidinyl, pyrrolidinyl, pyrrolinyl, pyrrolyl, quinazolinyl, quinolinyl, 4H-quinolizinyl, quinoxalinyl, quinuclidinyl, carbolinyl, tetrahydrofuranyl, tetrahydroisoquinolinyl, thiophane, thiotetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, thiirane, triazinyl, 1,2,3-triazolyl, 1,2,4-triazolyl, 1,2,5-triazolyl, 1,3,4-triazolyl, xanthenyl.

In addition to the polycyclic heterocycles described above, heterocyclic or heterocycle compounds include polycyclic heterocyclic moieties wherein the ring fusion between two or more rings comprises more than one bond common to both rings and more than two atoms common to both rings. Examples of such bridged heterocycles include quinuclidine, diazabicyclo[2.2.1]heptane and 7-oxabicyclo[2.2.1]heptane.

As used herein, "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, laetic, maleic, tartaric, citric, ascorbic, palmitic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, the disclosure of which is hereby incorporated by reference.

"Prodrugs" are intended to include any covalently bonded carriers that release the active parent drug according to formula (I) in vivo when such prodrug is administered to a mammalian subject. Prodrugs of a compound of formula (I) are prepared by modifying functional groups present in the compound in such a way that the modifications are cleaved, either in routine manipulation or in vivo, to the parent compound. Prodrugs include compounds of formula (I) wherein a hydroxy, amino,-or sulfhydryl group is bonded to any group that, when the prodrug or compound of formula (I) is administered to a mammalian subject, cleaves to form a free hydroxyl, free amino, or free sulfhydryl group, respectively. Examples of prodrugs include, but are not limited to, acetate, formate and benzoate derivatives of alcohol and amine functional groups in the compounds of formula (I), and the like.

"Stable compound" and "stable structure" are meant to indicate a compound that is sufficiently robust to survive isolation to a useful degree of purity from a reaction mixture, and formulation into an efficacious therapeutic agent.

### Formulations

Compounds of the present invention may be administered orally, parenteral, buccal, vaginal, rectal, inhalation, insufflation, sublingually, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level as the most appropriate for a particular patient.

An effective amount of a compound of the present invention for use in therapy of infection is an amount sufficient to symptomatically relieve in a warm-blooded animal, particularly a human the symptoms of infection, to slow the progression of infection, or to reduce in patients with symptoms of infection the risk of getting worse.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or tablet disintegrating agents; it can also be an encapsulating material.

In powders, the carrier is a finely divided solid, which is in a mixture with the finely divided active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredients dispersed therein by, for example, stirring. The molten homogeneous mixture is then poured into convenient sized molds and allowed to cool and solidify.

Suitable carriers include magnesium carbonate, magnesium stearate, talc, lactose, sugar, pectin, dextrin, starch, tragacanth, methyl cellulose, sodium carboxymethyl cellulose, a low-melting wax, cocoa butter, and the like.

Some of the compounds of the present invention are capable of forming salts with various inorganic and organic acids and bases and such salts are also within the scope of this invention. Examples of such acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates like dimethyl, diethyl, dibutyl; diamyl sulfates; long chain halides such as decyl, lauryl, myristyl and stearyl halides; aralkyl halides like benzyl bromide and others. Non-toxic physiologically-acceptable salts are preferred, although other salts are also useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

In order to use a compound of the formula (I) or a pharmaceutically acceptable salt thereof for the therapeutic treatment (including prophylactic treatment) of mammals including humans, it is normally formulated in accordance with standard pharmaceutical practice as a pharmaceutical composition.

In addition to the compounds of the present invention, the pharmaceutical composition of this invention may also contain, or be co-administered (simultaneously or sequentially) with, one or more pharmacological agents of value in treating one or more disease conditions referred to herein.

The term composition is intended to include the formulation of the active component or a pharmaceutically acceptable salt with a pharmaceutically acceptable carrier. For example this invention may be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions.

Liquid form compositions include solutions, suspensions, and emulsions. Sterile water or water-propylene glycol solutions of the active compounds may be mentioned as an example of liquid preparations suitable for parenteral administration. Liquid compositions can also be formulated in solution in aqueous polyethylene glycol solution. Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

The pharmaceutical compositions can be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms.

### Synthesis

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Such methods include, but are not limited to, those described below. All references cited herein are hereby incorporated in their entirety by reference.

The novel compounds of this invention may be prepared using the reactions and techniques described herein. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed Such restrictions to the substituents, which are compatible with the reaction conditions, will be readily apparent to one skilled in the art and alternate methods must then be used.

Examples of such processes are illustrated below:

In an aspect of the invention, intermediate compounds of formula **Ib** may be formed by reacting compounds of formula **Ia** with R²-X in a solvent such as DMSO and a base such as K₂CO₃ with heat as set forth below:

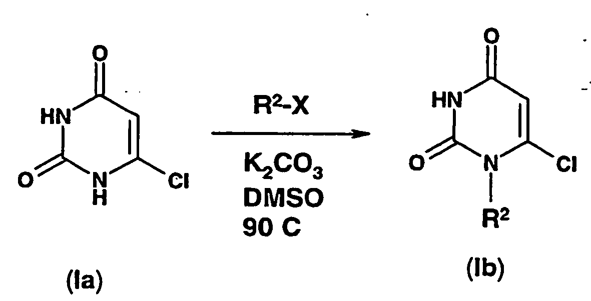

An intermediate compound of formula **Ic** may be formed by reacting a compound of Formula **Ib** with NH₂NH₂H₂O in ethanol and refluxed as follows:

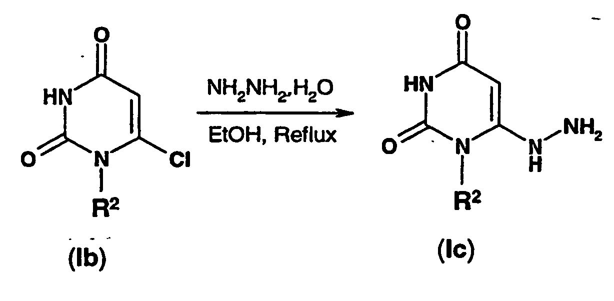

Intermediate compound of Formula **Id** may be formed by reacting compounds of formula **Ic** with R³-CHO in methanol as follows:

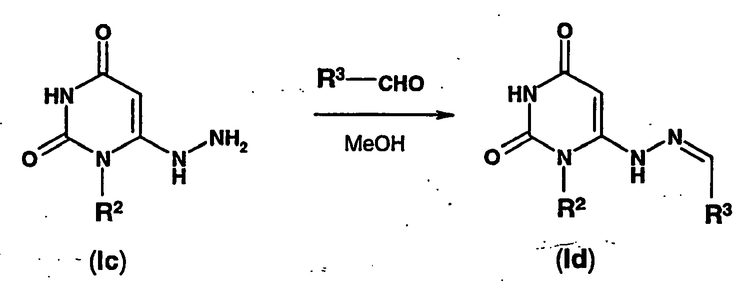

Intermediate compounds of formula **Ie** may by formed by reacting compounds of formula **Id** with R⁴-CHO and reflux in DMF with piperdine as follows:

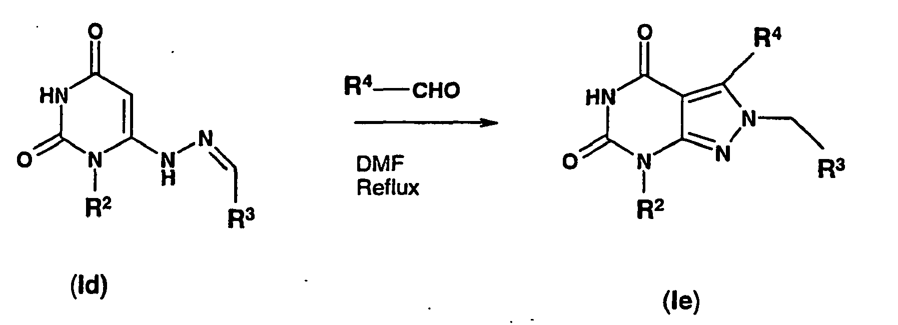

Compounds of formula **I** may by formed by reacting compounds of formula **Ie** with POCl₃, 3-nitro-1,2,4 triazole in pyridine at 70 C followed by amine (R²⁰-NH₂) as set forth below:

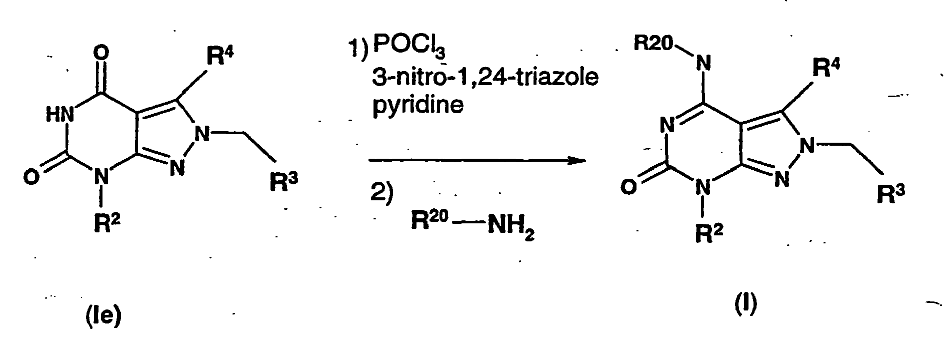

In an aspect of the invention, intermediate compounds of formula **IIb** may be formed by reacting compounds of formula **IIa** with R²-X in a solvent such as DMSO and a base such as K₂CO₃ with heat as set forth below:

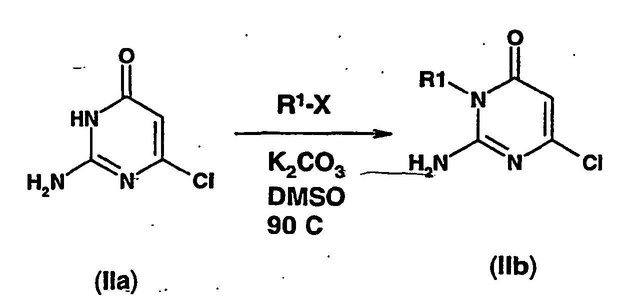

An intermediate compound of formula **IIc** may be formed by reacting compounds of formula **IIb** with NH₂NH₂H₂O in ethanol and refluxed as follows:

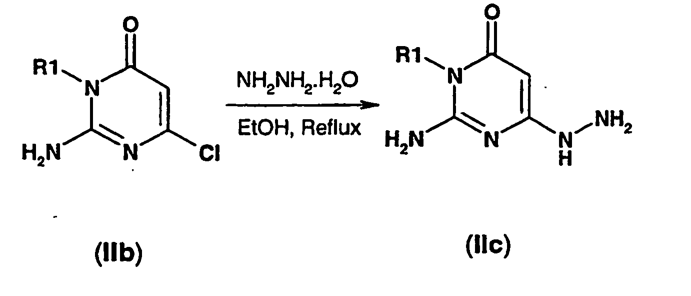

Intermediate compound of formula **IId** may be formed by reacting compounds of formula **IIc** with R³-CHO in methanol as follows:

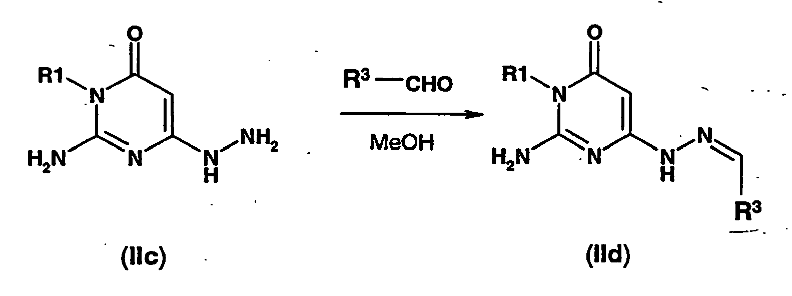

Intermediate compounds of formula **IIe** may by formed by reacting compounds of **IId** with R⁴-CHO and reflux in DMF with piperdine as follows:

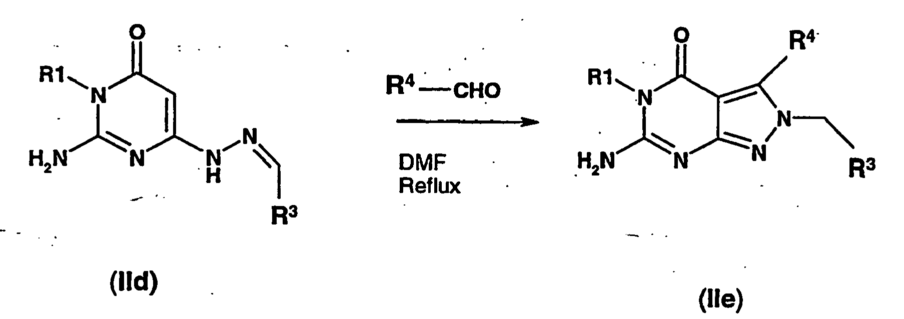

Compounds of formula **II** may by formed by reacting compounds of formula **IIe** with acyl chloride in (R²⁰-COCl) as set forth below:

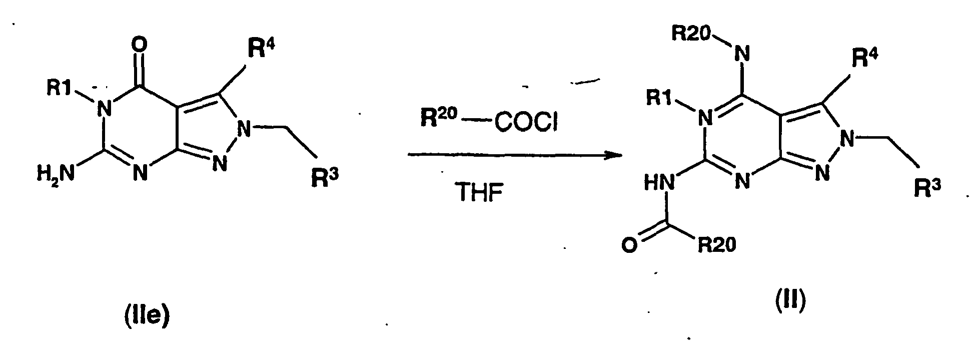

In an aspect of the invention, compounds of formula **III** may be formed by reacting compounds of formula **IIe** with R²-X in a solvent such as THF and a base such as DBU with heat as set forth below:

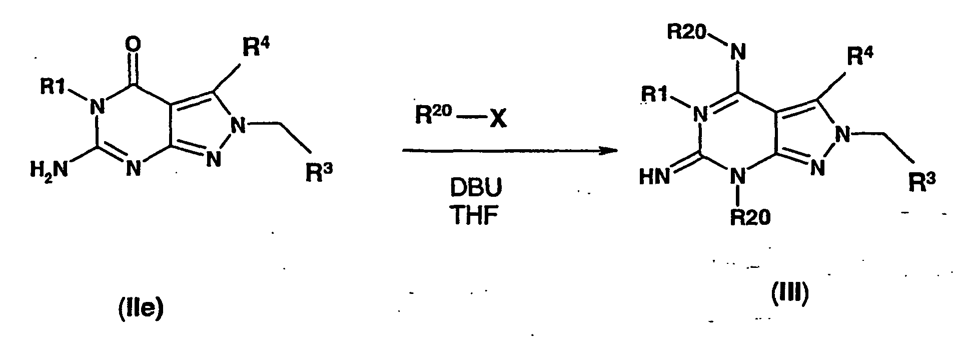

### Examples

Chemical abbreviations used in the Examples are defined as follows: Boc denotes t-butoxycarbonyl, Cbz denotes benzyloxycarbonyl, DCM denotes methylene chloride, DIPEA denotes diisopropylethylamine, DMF denotes N,N-dimethylformamide, DMSO denotes dimethyl sulfoxide, Et₂O denotes diethyl ether, EtOAc denotes ethyl acetate, TFA denotes trifluoroacetic acid, THF denotes tetrahydrofuran. Solvent mixture compositions are given as volume percentages or volume ratios. In cases where the NMR spectrum is complex, only diagnostic signals are reported.

Other terms used in the Examples are defined as follows: atm. denotes atmospheric pressure, equiv. denotes equivalent(s), h denotes hour(s), T_{b}denotes bath temperature, HPLC denotes high performance liquid chromatography, min denotes minutes, NMR denotes nuclear magnetic resonance, psi denotes pounds per square inch.
(i) temperatures are given in degrees Celsius (°C); unless otherwise stated, operations were carried out at room or ambient temperature, that is, at a temperature in the range of 18-25 °C;
(ii) organic solutions were dried over anhydrous magnesium sulfate or sodium sulfate; evaporation of solvent was carried out using a rotary evaporator under reduced pressure (600-4000 Pascals; 4.5-30 mm Hg) with a bath temperature of up to 60 °C;
(iii) chromatography means flash chromatography on silica gel or by FlashMaster^{™} II by Jones Chromatography using Isolute columns; thin layer chromatography (TLC) was carried out on silica gel plates;
(iv) in general, the course of reactions was followed by TLC or analytical HPLC and reaction times are given for illustration only;
(v) melting points are uncorrected and (dec) indicates decomposition;
(vi) final products had satisfactory proton nuclear magnetic resonance (NMR) spectra;
(vii) when given, NMR data is in the form of delta values for major diagnostic protons, given in parts per million (ppm) relative to tetramethylsilane (TMS) as an internal standard, determined at 300 or 500 MHz using deuterated chloroform (CDCl₃) or DMSO-_{d6} or CD₃OD as solvent; conventional abbreviations for signal shape are used; for AB spectra the directly observed shifts are reported; coupling constants (J) are given in Hz; Ar designates an aromatic proton when such an assignment is made;
(viii) reduced pressures are given as absolute pressures in pascals (Pa); elevated pressures are given as gauge pressures in bars;
(ix) solvent ratios are given in volume:volume (v/v) terms; and
(x) Mass spectra (MS) were run using an automated system with atmospheric pressure electrospray ionization (ESI). Generally, only spectra where parent masses are observed are reported. The lowest mass major ion is reported for molecules where isotope splitting results in multiple mass spectral peaks (for example when chlorine is present).

The invention will now be illustrated by the following non-limiting examples.

### Example 1

### 5-{2-[(6-chloroquinolin-4-yl)methyl]-7-(cyclopropylmethyl)-4-[(2-hydroxyethyl)amino]-6-oxo-6,7-dihydro-2H-pyrazolo[3,4-d]pyrimidin-3-yl}-1-methyl-1H-pyrrole-3-carbonitrile

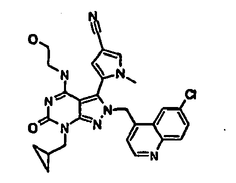

### (a) 6-Chloro-1-cyclopropylmethylpyrimidine-2,4[1H,3H]dione

6-Chlorouracil **I** (49.64 g, 0.34 mol; Lancaster) was dissolved in anhydrous DMSO (375 mL) and treated with solid K₂CO₃ (23.46 g, 0.17 mol) under nitrogen. The resulting white suspension was heated to ca. 80 - 90 °C and kept at this temperature for 2¼ h. Foaming was observed as the temperature increased, then the reaction mixture became mostly clear. Cyclopropylmethyl bromide (65 g, 0.48 mol) was added neat via syringe, resulting in a white fluffy precipitate. This was followed by a catalytic amount of KI (2.88 g, 0.017 mol). The reaction mixture was heated for 19 hr, becoming mostly homogenous, then turbid with white granular precipitate, and eventually orange as the reaction progressed, remaining heterogeneous. 375 mL 1 N NaOH (aq) was added to the hot reaction mixture, causing it to darken and clear. The heat was removed and the reaction mixture allowed to cool to room temperature while stirring. It was washed with 4 x 125 mL toluene and the organic washings discarded. The aqueous phase was brought to pH 2-3 by the addition of *ca.* 100 mL conc. HCl (aq.). 50 mL water was added, and precipitation began after about one hour at room temperature; cooling in ice completed the crystallization. The yellow-green solid was collected by filtration and washed with very cold ether to remove most of the color, then dried under vacuum. Yield: 29.37 g (43 %) of a light yellow solid.

### (b) 1-cyclopropylmethyl-6-hydrazinopyrimidine-2,4[1H,3H]dione

6-Chloro-1-cyclopropylmethyluracil (24.34 g, 0.12 mol) was suspended in absolute ethanol (245 mL) under nitrogen. Anhydrous hydrazine (11.69 g, 0.36 mol) was added via syringe in excess, resulting in a clear yellow solution. The reaction mixture was heated at 80 - 85 °C for one hour; bright yellow crystals began forming within minutes of the application of heat. The reaction mixture was cooled to room temperature and then in an ice bath, and the crude product collected by filtration. N₂H₄⁻xHCl was removed by trituration with cold water to give the product as a pale yellow solid, 20.47 g (86%). Mp 221 °C (dec).

### (c) 6-chloroquinoline-4-carbaldehyde [3-(cyclopropylmethyl)-2,6-dioxo-1,2,3,6-tetrahydropyrimidin-4-yl]hydrazone

To a suspension of 1-cyclopropylmethyl-6-hydrazinopyrimidine-2,4[1H,3H]dione (5.05 g) in methanol (75 mL) was added 6-chloroquinoline,4- carbaldehyde (5.30 g). After stirring overnight, the reaction was filtered, yielding a yellow solid (10 g).

### (d)5-{2-[(6-chloroquinolin-4-yl)methyl]-7-cyclopropylmethyl-4,6-dioxo-4,5,6,7-tetrahydro-2H-pyrazolo[3,4-d]pyrimidin-3-yl}-1-methyl-1H -pyrrole-3-carbonitrile

To a solution of 5-formyl-1-methyl-1*H*-pyrrole-3-carbonitrile (2.5 g) in DMF (50 mL) were added 6-chloroquinoline-4-carbaldehyde [3-(cyclopropylmethyl)-2,6-dioxo-1,2,3,6-tetrahydropyrimidin-4-yl]hydrazone (5.66 g) and piperdine (2 mL). After stirring overnight at T_{b}= 75 °C, the reaction was diluted with ethyl acetate and water. The organic solution was collected, washed with saturated NaHCO₃ and brine, dried (Na₂SO₄), filtered and concentrated. The residue was purified by FlashMaster™ yielding 8.63 g of white solid. Mass: 486 (M+H)⁺.

### (e) 5-{2-[(6-chloroquinolin-4-yl)methyl]-7-(cyclopropylmethyl)-4-[(2-hydroxyethyl)amino]-6-oxo-6,7-dihydro-2H-pyrazolo[3,4,d]pyrimidin-3-yl}-1-methyl-1H-pyrrole-3-carbonitrile

To a solution of 5-{2-[(6-chloroquinolin-4-yl)methyl]-7-cyclopropylmethyl-4,6-dioxo-4,5,6,7-tetrahydro-2*H*-pyrazolo[3,4-*d*]pyrimidiri-3-yl}-1-methyl-1*H*-pyrrole-3-carbonitrile (0.42 g) in pyridine (5 mL) were added 4-chlorophenyl phosphorodichloridate (0.5 mL) and 3-nitro-1,2,4 triazole (0.15 g). After heating (T_{b}= 50 °C) for 3 h, the reaction was diluted with ethyl acetate. The organic solution was washed with saturated NaHCO₃ and brine, dried (Na₂SO₄), filtered and concentrated. The residue was dissolved in THF (10 mL) and ethanolamine (1.0 mL). After 3h, the reaction was concentrated. This residue was purified by flash chromatography using a FlashMaster™ yielding 0.22 g, ES (M+H)⁺= 529.

Following the method of **Example 1e,** the following examples were made by reaction of 5-{2-[(6-chloroquinolin-4-yl)methyl]-7-(cyclolpropylmethyl)-4,6-dioxo-4,5,6,7-tetrahydro-2*H-*pyrazolo[3,4-*d*]pyrimidin-3-yl)-1-methyl-1*H*-pyrrole-3-carbonitrile with the appropriate amine.

### Example 2

### 5-{4-dimethylamino-2-[(6-chloroquinolin-4-yl)methyl]-7-isobutyl-6-oxo-6,7-dihydro-2H-pyrazolo[3,4-d]pyrimidin-3-yl}-1-methyl-1H-pyrrole-3-carbonitrile

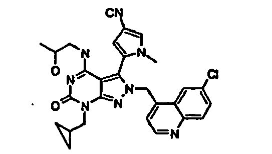

amine: 1-amino-2-propanol
1H NMR (300 MHz, CHLOROFORM-D) d ppm 0.51 (d, *J=*5.65 Hz, 4 H) 1.16 (d, 3 H) 1.46 (m, 1 H) 3.07 (d, *J*=2.64 Hz, 2 H) 3.98 (m, 4 H) 5.12 (m, 1 H) 5.65 (d, *J*=2.45 Hz, 1 H) 6.73 (s, 1 H) 6.81 (dd, *J*=4.24, 2.54 Hz, 1 H) 7.33 (s, 1 H) 7.72 (dd, *J*=8.85, 2.26 Hz, 1 H) 7.97 (d, *J*=2.07 Hz, 1 H) 8.11 (d, *J*=9.04 Hz, 1 H) 8.82 (d, *J*=4.33 Hz, 1 H); ES (M+H)⁺ = 544.

### Example 3

### 5-{2[(6-chloroquinolin-4-yl)methyl]-7-(cyclopropylmethyl)4-[(2-hydroxy-1-methylethyl)amino]-6-oxo-6,7-dihydro-2H-pyrazolo[3,4-d]pyrimidin-3-yl}-1-methyl-1H-pyrrole-3-carbonitrile

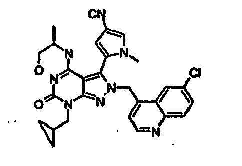

amine: 2-amino-1-propanol
ES (M+H)⁺ = 544.

### Example 4

### 5-{2-[(6-chloroquinolin-4-yl)methyl]-7-(cyclopropylmethyl)-4-[(3-hydroxypropyl)amino]-6-oxo-6,7-dihydro-2H-pyrazolo[3,4-d]pyrimidin-3-yl}-1-methyl-1H-pyrrole-3-carbonitrile

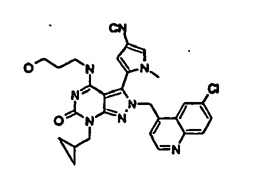

amine: 3-amino-1-propanol
1H NMR (300 MHz, CHLOROFORM-D) d ppm 0.51 (d, *J*=5.46 Hz, 4 H) 1.48 (m, 1 H) 1.68 (m, 2 H) 3.00 (s, 3 H) 3.70 (m, 4 H) 4.00 (m, 2 H) 5.63 (s, 1 H) 5.80 (m, 1 H) 6.69 (d, *J*=1.32 Hz, 1 H) 6.77 (d, *J*=4.52 Hz, 1 H) 7.29 (m, 1 H) 7.71 (dd, *J*=9.04, 2.07 Hz, 1 H) 7.96 (d, *J*=2.07 Hz, 1 H) 8.11 (d, *J*=9.04 Hz, 1 H) 8.82 (d, *J*=4.33 Hz, 1 H); ES (M+H)⁺ = 544.

Following the method of **Example 1c-e** and starting with 6-chloro-1-isobutylpyrimidine-2,4(1*H*,3*H*)-dione, the following examples were made by reaction of 5-{2-[(6-chloroquinolin-4-yl)methyl]-7-isobutyl-4,6-dioxo-4,5,6,7-tetrahydro-2*H*-pyrazolo[3,4-*d*]pyrimidin-3-yl}-1-methyl-1H-pyrrole-3-carbonitrile with the appropriate amine.

### Example 5

### 5-{4-amino-2-[(6-chloroquinolin-4-yl)methyl]-7-isobutyl-6-oxo-6,7-dihydro-2H-pyrazolo[3,4-d]pyrimidin-3-yl}-1-methyl-1H-pyrrole

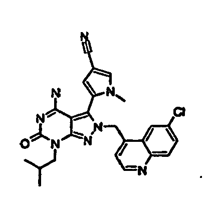

amine: ammonium hydroxide
1H NMR (500 MHz, CHLOROFORM-D) d ppm 1.00 (dd, *J*=10.40, 6.62 Hz, 6 H) 2.40 (m, 1 H) 3.02 (s, 3 H) 3.91 (d, *J*=7.57 Hz, 2 H) 5.64 (s, 2 H) 6.74 (s, 1 H) 6.77 (d, *J*=4.41 Hz, 1 H) 7.31 (d, *J*=1.26 Hz, 1 H) 7.72 (dd, *J*=9.14,2.21 Hz, 1 H) 8.00 (d, *J*=2.21 Hz, 1 H) 8.11 (d, *J*=8.83 Hz, 1 H) 8.82 (d, *J*=4.41 Hz, 1 H); ES (M+H)⁺ = 487.

### Example 6

### 5-{4-methylamino-2-[(6-chloroquinolin4-yl)methyl]-7-isobutyl-6-oxo-6,7-dihydro-2H-pyrazolo[3,4-d]pyrimidin-3-yl}-1-methyl-1H-pyrrole-3-carbonitrile

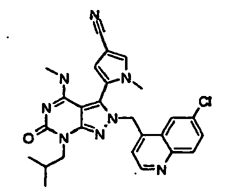

amine: methylamine
1H NMR (500 MHz, CHLOROFORM-D) d ppm 0.99 (m, 6 H) 2.44 (m, 1 H) 3.01 (m, 6 H) 3.96 (m, 2 H) 5.62 (s, 2 H) 6.73 (s, 1 H) 6.77 (d, *J*=3.47 Hz, 1 H) 7.34 (s, 1 H) 7.73 (d, *J*=8.51 Hz, 1 H) 8.00 (s, 1 H) 8.12 (d, *J*=8.83 Hz, 1 H) 8.82 (d, *J*=3.78 Hz, 1 H); ES (M+H)⁺ = 501.

### Example 7

### 5-{4-dimethylamino-2-[(6-chloroquinolin-4-yl)methyl]-7-isobutyl-6-oxo-6,7-dihydro-2H-pyrazolo[3,4-d]pyrimidin-3-yl}-1-methyl-1H-pyrrole-3-carbonitrile

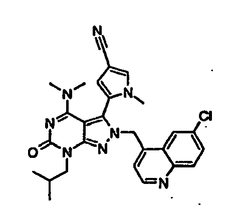

amine: dimethylamine
1H NMR (500 MHz, CHLOROFORM-D) d ppm 0.98 (m, 6 H) 2.41 (m, 1 H) 2.85 (s, 6 H) 2.99 (s, 3 H) 4.03 (m, 2 H) 5.73 (m, 1 H) 6.67 (s, 1 H) 6.74 (d, *J*=4.41 Hz, 1 H) 7.27 (m, 1 H) 7.72 (m, 1 H) 7.98 (s, 1 H) 8.12 (m, 1 H) 8.82 (d, *J=*4.10 Hz, 1 H); ES (M+H)⁺ = 515.

### Example 8

### 5-{4-propylamino-2-[(6-chloroquinolin-4-yl)methyl]-7-isobutyl-6-oxo-6,7-dihydro-2H-pyrazolo[3,4-d]pyrimidin-3-yl}-1-methyl-1H-pyrrole-3-carbonitrile

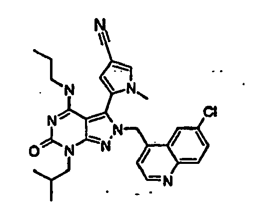

amine: propylamine
1H NMR (500 MHz, CHLOROFORM-D) d ppm 0.82 (t, *J*=7.41 Hz, 3 H) 1.01 (dd, *J*=11.35, 6.62 Hz, 6 H) 1.47 (m, 2 H) 2.44 (m, 1 H) 3.01 (s, 3 H) 3.46 (dd, *J*=22.07, 5.68 Hz, 2 H) 3.96 (m, *J*=15.45, 7.57 Hz, 1 H) 4.45 (m, 1 H) 5.63 (s, 2 H) 6.75 (d, *J=* 1.58 Hz, 1 H) 6.78 (d, *J*=4.41 Hz, 1 H) 7.35 (d, *J=* 1.58 Hz, 1 H) 7.73 (dd, *J*=9.14, 2.21 Hz, 1 H) 8.02 (d, *J*=2.21 Hz, 1 H) 8.12 (d, *J*=8.83 Hz, 1 H) 8.82 (d, *J*=4.10 Hz, 1 H); ES (M+H)⁺ = 529.

### Example 9

### 5-[2-[(6-chloroquinolin-4-yl)methyl]-4-(hydroxyamino)-7-isobutyl-6-oxo-6,7-dihydro-2H-pyrazolo[3,4-d]pyrimidin-3-yl]-1-methyl-1H-pyrrole-3-carbonitrile

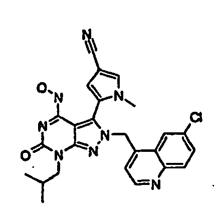

amine: hydroxylamine
1H NMR (300 MHz, DMSO-D6) d ppm 0.89 (dd, *J*=6.50, 1.98 Hz, 6 H) 2.25 (m, 1 H) 3.13 (s, 3 H) 3.68 (m, 2 H) 5.79 (m, *J*=8.85 Hz, 2 H) 6.77 (m, 1 H) 7.70 (d, *J*=1.70 Hz, 1 H) 7.80 (dd, *J*=9.04, 2.26 Hz, 1 H) 8.05 (d, *J*=9.04 Hz, 1 H) 8.14 (d, *J*=2.07 Hz, 1 H) 8.79 (d, *J*=4.33 Hz, 1 H) 9.41 (s, 1 H) 10.28 (s, 1 H); ES (M+H)⁺ = 503

### Example 10

### 5-[2-[(6-chloroquinolin-4-yl)methyl]-4-(cyclopropylamino)-7-isobutyl-6-oxo-6,7-dihydro-2H-pyrazolo[3,4-d]pyrimidin-3-yl]-1-methyl-1H-pyrrole-3-carbonitrile

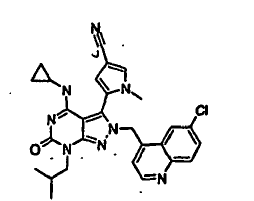

amine: cyclopropylamine
1H NMR (300 MHz, CHLOROFORM-D) d ppm 0.83 (m, 4 H) 0.99 (m, 6 H) 2.45 (m, 1H) 2.98 (s, 3 H) 3.83 (m, 1 H)3.96 (t, *J*=7.44 Hz, 1H) 5.65 (m, 1 H) 6.71 (d, *J*=0.94 Hz, 1 H) 6.76 (d, *J*=4.33 Hz, 1 H) 7.33 (s, 1 H) 7.71 (m, 1 H) 7.99 (d, *J*=1.70 Hz, 1H) 8.10 (m, 1 H) 8.81 (d, *J*=4.33 Hz, 1 H); ES (M+H)⁺ = 528

### Example 11

### 5-{2-[(6-chloroquinolin-4-yl)methyl]-4-[(2-hydroxyethyl)amino]-7-isobutyl-6-oxo-6,7-dihydro-2H-pyrazolo[3,4-d]pyrimidin-3-yl}-1-methyl-1H-pyrrole-3-carbonitrile

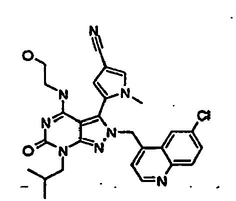

amine: ethanolamine
1H NMR (500 MHz, CHLOROFORM-D) d ppm 1.01 (dd, *J*=10.72,6.62 Hz, 4 H) 2.43 (m, 1 H) 3.05 (s, 3H) 3.65 (m, 4H) 3.95 (dd, *J*=9.62,7.72 Hz, 2 H) 5.10 (s, 1H) 5.65 (s, 2H) 6.73 (s, 1 H) 6.78 (d, *J*=4.41 Hz, 1 H) 7.33 (d, *J*=5.04 Hz, 1 H) 7.73 (dd, *J*=8.83,1.89 Hz, 1 H) 8.00 (d, *J*=1.89 Hz, 1 H) 8.12 (d, *J*=8.83 Hz, 1 H) 8.82 (d, *J*=4.41 Hz, 1 H); ES (M+H)⁺ = 531

### Example 12

### 5-{2-[(6-chloroquinolin-4-yl)methyl]4-hydrazino-7-isobutyl-6-oxo-6,7-dihydro-2H-pyrazolo[3,4-d]pyrimidin-3-yl}-1-methyl-1H-pyrrole-3-carbonitrile

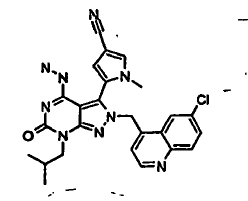

amine: hydrazine
1H NMR (300 MHz; CHLOROFORM-D) d ppm 1.00 (dd, *J*=6.50, 2.54 Hz, 6 H) 2.35 (m, *J=*13.85, 6.88 Hz, 1 H) 3.18 (m, 3 H) 3.83 (d, *J*=7.35 Hz, 3 H) 4.15 (s, 2 H) 5.65 (m, 2 H) 6.55 (d, *J=*1.13 Hz, 1 H) 6.71 (d, *J*=4.33 Hz, 1H) 7.22 (s, 1 H) 7.70 (dd, *J*=8.85, 2.07 Hz, 1 H) 7.92 (d, *J*=1.88 Hz, 1 H) 8.10 (d, *J*=9.04 Hz, 1 H) 8.79 (d, *J*=4.33 Hz, 1 H); ES (M+H)⁺ = 502

### Example 13

### 5-[2-[(6-chloroquinolin-4-yl)methyl]-4-(2,2-dimethylhydrazino)-7-isobutyl-6-oxo-6,7-dihydro-2H-pyrazolo[3,4-d]pyrimidin-3-yl]-1-methyl-1H-pyrrole-3-carbonitrile

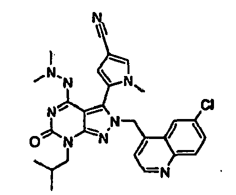

amine: dimethylhydrazine
1H NMR (300 MHz, CHLOROFORM-D) d ppm 1.01 (dd, *J*=6.59,1.51 Hz, 4 H) 2.29 (s, 6 H) 2.36 (m, 1 H) 3.23 (s, 3 H) 3.82 (d, *J*=7.54 Hz, 1 H) 5.67 (m, 1 H) 6.54 (d, *J*=1.70 Hz, 1 H) 6.73 (d, *J*=4.52 Hz, 1 H) 7.23 (d, *J=*1.51 Hz, 1 H) 7.70 (dd, *J*=8.95,2.17 Hz, 1 H) 7.92 (d, *J*=2.26 Hz, 1 H) 8.10 (d, *J*=9.04 Hz, 1 H) 8.80 (d, *J*=4.52 Hz, 1 H); ES (M+H)⁺ = 531

Following the method of **Example 1**, the following examples were made by reaction of 7-isobutyl-2-(1-naphthylmethyl)-3-pyridin-4-yl-2*H*-pyrazolo[3,4-*d*]pyrimidine-4,6(5*H*,7*H*)-dione with the appropriate amine. 7-isobutyl-2-(1-naphthylmethyl)-3-pyridin-4-yl-2*H-*pyrazolo[3,4-*d*]pyrimidine-4,6(5*H*,7*H*)-dione was synthesized in analogous method to Example 1 using for step(a) 1-chloro-2-methylpropane, step (c) 1-naphthaldehyde and step (d) isonicotinaldehyde.

### Example 14

### 4-amino-7-isobutyl-2-(1-naphthylmethyl)-3-pyridin-4-yl-2,7-dihydro-6H-pyrazolo[3,4-d]pyrimidin-6-one

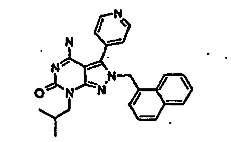

amine: ammonium hydroxide
1H NMR (500 MHz, CHLOROFORM-D) d ppm 1.01 (d, *J*=6.94 Hz, 6 H) 2.42 (m, 1 H) 3.96 (s, 2 H) 5.74 (s, 2 H) 6.76 (d, J= 7.25 Hz, 1H) 7.19 (d, *J*=5.99 Hz, 2 H) 7.31 (m, *J*=7.88 Hz, 1 H) 7.52 (m, 2 H) 7.82(d, *J*=8.20 Hz, 1 H) 7.90 (dd, *J*=7.72, 5.20 Hz, 2 H) 8.74 (d, *J*=5.99 Hz, 2 H); ES (M+H)⁺ = 425

### Example 15

### 7-isobutyl-4-(methylamino)-2-(1-naphthylmethyl)-3-pyridin-4-yl-2,7-dihydro-6H-pyrazolo[3,4-d]pyrimidin-6-one

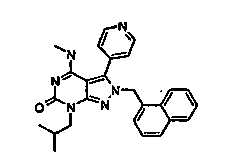

amine: methylamine
1H NMR (500 MHz, CHLOROFORM-D) d ppm 1.01 (d, *J*=6.94 Hz, 6 H) 2.46 (m, 1 H) 2.99 (d, *J*=4.73 Hz, 3 H) 4.57 (m, *J*=4.73 Hz, 1 H) 5.73 (s, 2 H) 6.76 (d, J= 7.25 Hz, 1H) 7.19 (d, *J*=5.99 Hz, 2 H) 7.31 (m, *J*=7.88 Hz, 1 H) 7.52 (m, 2 H) 7.82 (d, *J*=8.20 Hz, 1 H) 7.90 (dd, *J*=7.72, 5.20 Hz, 2 H) 8.74 (d, *J*=5.99 Hz, 2 H); ES (M+H)⁺= 439.

### Example 16

### 4-(dimethylamino)-7-isobutyl-2-(1-naphthylmethyl)-3-pyridin-4-yl-2,7-dihydro-6H-pyrazolo[3,4-d]pyrimidin-6-one

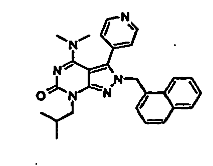

amine: dimethylamine
1H NMR (500 MHz, CHLOROFORM-D) d ppm 1.00 (d, *J*=6.62 Hz, 6 H) 2.43 (m, 1 H) 2.78 (s, 6 H) 4.01 (d, *J*=7.57 Hz, 2 H) 5.72 (s, 2 H) 6.75 (d, *J*=6.94 Hz, 1 H) 7.17 (d, *J*=4.73 Hz, 1 H) 7.35 (t, *J*=7.72 Hz, 1 H) 7.54 (m, 2 H) 7.83 (d, *J*=8.20 Hz, 1 H) 7.89 (dd, *J*=14.03, 8.04 Hz, 2 H) 8.66 (s, 2 H); ES (M+H)⁺ = 453.

### Example 17

### 7-isobutyl-4-(4-methylpiperazin-1-yl)-2-(1-napht6ylmethyl)-3-pyridin-4-yl-2,7-dihydro-6H-pyrazolo[3,4-d]pyrimidin-6-one

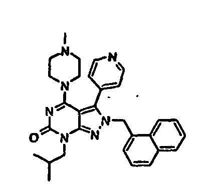

amine: 1-methylpiperazine
1H NMR (500 MHz, DMSO-D6) d ppm 1.01 (d, *J*=6.94 Hz, 6 H) 2.19 (s, 3 H) 2.43 (m, 1 H) 3.35 (s, 4 H) 4.01 (d, *J*=7.57 Hz, 2 H) 5.74 (s, 2 H) 6.77 (d, *J*=6.94 Hz, 1 H) 7.18 (m, 2 H) 7.36 (m, 1 H) 7.53 (m, 2 H) 7.83 (d, *J*=8.51 Hz, 1 H) 7.89 (dd, *J*=15.29, 8.04 Hz, 2 H) 8.69 (d, *J*=5.99 Hz, 2 H); ES (M+H)⁺ = 508.

### Example 18

### 4-amino-2-[(6-chloroquinolin-4-yl)methyl]-7-isobutyl-3-(1-methyl-1H-pyrrol-2-yl)-2,7-dihydro-6H-pyrazolo[3,4-d]pyrimidin-6-one

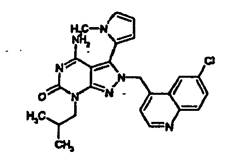

4-amino-2-[(6-chloroquinolin-4-yl)methyl]-7-isobutyl-3-(1-methyl-1*H*-pyrrol-2-yl)-2,7-dihydro-6*H*-pyrazolo[3,4-*d*]pyrimidin-6-one was synthesized in analogous method to Example 1 using for step (a) 1-chloro-2-methylpropane and for step (d) 1-methyl-1H pyrrole-2-carbaldehyde. 1H NMR (500 MHz, CHLOROFORM-D) d ppm 0.99 (dd, *J*=9.14, 6.62 Hz, 6 H) 2.33 (m, 1 H) 3.33 (s, 3 H) 3.90 (m, 2 H) 5.71 (d, *J*=15.76 Hz, 1 H) 5.87 (d, *J*=15.45 Hz, 1 H) 6.46 (m, 1 H) 7.08 (m, 1 H) 7.86 (dd, *J*=8.83, 1.89 Hz, 1 H) 8.04 (d, *J*=1.89 Hz, 1 H) 8.31 (d, *J*=9.14 Hz, 1 H) 9.01 (d, *J*=4.73 Hz, 1 H) 12.66 (s, 1 H); ES (M+H)⁺ 462.

### Example 19

### N-[2-[(6-chloroquinolin-4-yl)methyl]-3-(4-cyano-1-methyl-1H-pyrrol-2-yl)-7-isobutyl-6-oxo-6,7-dihydro-2H-pyrazolo[3,4-d]pyrimidin-4-yl]acetamide

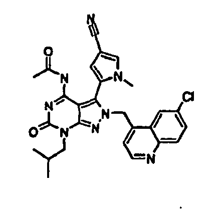

To a solution of 5-{4-amino-2-[(6-chloroquinolin-4-yl)methyl]-7-isobutyl-6-oxo-6,7-dihydro-2*H*-pyrazolo[3,4-d]pyrimidin-3-yl}-1-methyl-1*H*-pyrrole-3-carbonitrile (0.15g) in pyridine (3 mL) was added acetic anhydride (0.15 mL). After stirring at rtfor 4 h, the reaction was concentrated and purified by Flash Master (0.072 g). 1H NMR (300 MHz, CHLOROFORM-D) d ppm 1.00 (m, 6 H) 2.08 (m, 3 H) 2.39 (m, 1 H) 3.24 (s, 3 H) 3.91 (d, *J*=7.72 Hz, 2 H) 5.75 (m, 2 H) 6.61 (d, *J*=1.70 Hz, 1 H) 6.78 (d, *J*=4.33 Hz, 1 H) 7.30 (d, *J*=1.70 Hz, 1H) 7.72 (dd, *J*=9.04, 2.26 Hz, 1 H) 7.89 (d, *J*=2.07 Hz, 1H) 8.12 (d, *J*=8.85 Hz, 1 H) 8.83 (d, *J*=4.52 Hz, 1 H) 12.28 (s, 1H); ES (M+H)+ 529.

### Example 20

### 5-[2-[(6-chloroquinolin-4-yl)methyl]-7-(cyclopropylmethyl)-6-oxo4-thioxo-4,5,6,7-tetrahydro-2H-pyrazolo[3,4-d]pyrimidin-3-yl]-1-methyl-1H-pyrrole-3-carbonitrile

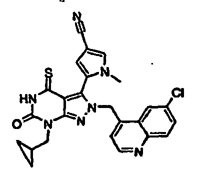

To a solution of 5-{2-[(6-chloroquinolin-4-yl)methyl]-7-(cyclolpropylmethyl)-4,6-dioxo-4,5,6,7-tetrahydro-2*H*-pyrazolo[3,4-*d*]pyrimidin-3-yl}-1-methyl-1*H*-pyrrole-3-carbonitrile (0.50 g) in pyridine (10 mL) was added P₄S₁₀ (0.72 g). After heating (T_{b}= 110 C) overnight, the reaction was concentrated. The residue was dissolved in EtOAc and water. The organic solution was collected, washed with saturated NaHCO₃ and brine, dried (Na₂SO₄), filtered and concentrated. The residue was purified by first by flash chromatography using a FlashMaster^{™} yielding 0.14 g, 1H NMR (300 MHz, CHLOROFORM-D) d ppm 0.56 (m, 4 H) 1.47 (m, 1 H) 3.05 (s, 3 H) 3.97 (m, 2 H) 5.70 (m, 2 H) 6.64 (d, *J*=1.70 Hz, 1 H) 6.79 (d, *J*=4.33 Hz, 1 H) 7.72 (dd, *J*=9.04, 2.26 Hz, 1 H) 7.94 (d, *J*=2.07 Hz, I H) 8.12 (d, *J*=9.04 Hz, 1 H) 8.84 (d, *J*=4.33 Hz, 1 H) 9.12 (s, 1 H); ES M+H⁺= 503.

### Example 21

### 5-[2-[(6-chloroquinolin-4-yl)methyl]-7-(cyclopropylmethyl)-4-(methylthio)-6-oxo-6,7-dihydro-2H-pyrazolo[3,4-d]pyrimidin-3-yl]-1-methyl-1H-pyrrole-3-carbonitrile

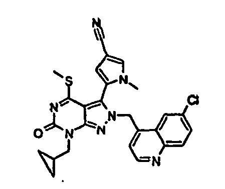

To a solution of 5-[2-[(6-chloroquinolin-4-yl)methyl]-7-(cyclopropylmethyl)-6-oxo-4-thioxo-4,5,6,7-tetrahydro-2*H* pyrazolo[3,4-d]pyrimidin-3-yl]-1-methyl-1*H*-pyrrole-3-carbonitrile (0.33 g) in THF (5 mL) were added iodomethane( 0.10 mL) and 1,8-diazabicycl[4.3.0]undec-7-ene (0.15 mL). After 2 h, the reaction was diluted with ethyl acetate and water. The slurry was filtered and washed with water to yield 250 mg product. 1H NMR (300 MHz, DMSO-D6) d ppm 0.56 (d, *J*=5.46 Hz, 4 H) 1.28 (m, 1 H) 2.55 (s, 3 H) 2.95 (s, 3 H) 4.07 (dd, *J*=7.25, 4.80 Hz, 2 H) 5.69 (d, *J*=2.45 Hz, 2 H) 6.71 (d, *J*=1.70 Hz, 1 H) 6.79 (d, *J*=4.52 Hz, 1 H) 7.29 (m, 1H) 7.72 (dd, *J*=9.04,2.26 Hz, 1 H) 7.99 (d, *J*=2.26 Hz, 1 H) 8.11 (d, *J*=9.04 Hz, 1 H) 8.83 (d, *J*=4.33 Hz, 1 H); ES M+H⁺= 516.

### Example 22

### 5-[2-[(6-chloroquinolin-4-yl)methyl]-7-(cyclopropylmethyl)-6-oxo:4,5,6,7-tetrahydro-2H-pyrazolo[3,4-d]pyrimidin-3-yl]-1-methyl-1H-pyrrole-3-carbonitrile

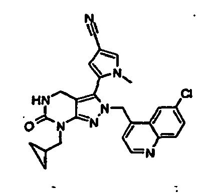

To a solution of 5-[2-[(6-chloroquinolin-4-yl)methyl]-7-(cyclopropylmethyl)-6-oxo-4-thioxo-4,5,6,7-tetrahydro-2*H*-pyrazolo[3,4-*d*]pyrimidin-3-yl]-1-methyl-1*H*-pyrrole-3-carbonitrile (0.17 g) in-methanol (1 mL) and THF (1.5 mL) was added NiCl₂-6H₂O ((0.11g). After the nickel had dissolved, sodium borohyride (0.5 g) was added. After 30 min., the reaction was concentrated. The residue was diluted with ethyl acetate and water. The organic solution was washed with saturated NaHCO₃ and brine, dried (Na₂SO₄), filtered and concentrated. The residue was purified by flash chromatography using a FlashMaster^{™} yielding 44 mg, 1H NMR (300 MHz, DMSO-D6) d ppm 0.34 (m, 4 H) 1.23 (m, 1 H) 3.19 (s, 3 H) 3.57 (d, *J*=6.97 Hz, 2 H) 4.15 (s, 2 H) 5.63 (s, 2 H) 6.68 (d, *J*=1.70 Hz, 1 H) 6.76 (d, *J*=4.33 Hz, 1 H) 6.90 (s, 1 H) 7.72 (d, *J*=1.70 Hz, 1 H) 7.79 (dd, *J*=9.04, 2.26 Hz, 1 H) 8.05 (d, *J*=8.85 Hz, 1 H) 8.17 (d, *J*=2.07 Hz, 1 H) 8.80 (d, *J*=4.52 Hz, 1 H); ES M+H⁺= 472.

### Example 23

### 5-[2-[(6-chloroquinolin-4-yl)methyl]-7-(cyclopropylmethyl)-4-hydrazino-6-oxo-6,7-dihydro-2H-pyrazolo[3,4-d]pyrimidin-3-yl]-1-methyl-1H-pyrrole-3-carbonitrile

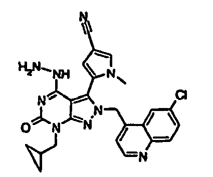

To a solution of 5-[2-[(6-chloroquinolin-4-yl)methyl]-7-(cyclopropylmethyl)-6-oxo-4-thioxo-4,5,6,7-tetrahydro-2*H*-pyrazolo[3,4-*d*]pyrimidin-3-yl]-1-methyl-1*H*-pyrrole-3-carbonitrile (03.0 g) in acetonitrile (5 mL) were added hydrazine hydrate (0.50 mL) and mercury (II) chloride (250 mg). After 5 h, the reaction was diluted with ethyl acetate and filtered through a celite bed. The residue was purified by flash chromatography using a FlashMaster^{™} yielding 120 mg, 1H NMR (300 MHz, DMSO-D6) d ppm 0.44 (m, 4 H) 1.34 (m, 1 H) 3.17 (m, 3 H) 3.80 (m, *J*=6.97, 6.97 Hz, 2 H) 5.60 (q, *J=* 15.89 Hz, 2 H) 6.48 (d, *J=* 1.70 Hz, 1H) 6.71 (d, *J*=4.52 Hz, 1 H) 7.22 (d, *J*=1.51 Hz, 1 H) 7.66 (dd, *J*=9.04,2.07 Hz, 1 H) 7.85 (d, *J*=2.07 Hz, 1 H) 8.02 (d, *J*=9.04 Hz, 1 H) 8.71 (d, *J*=4.33 Hz, 1 H); ES (M+H)⁺ = 500.

Following the method of Example 23, the following examples were made by reaction of 5-[2-[(6-chloroquinolin-4-yl)methyl]-7-(cyclopropylmethyl)-6-oxo-4-thioxo-4,5,6,7-tetrahydro-2*H*-pyrazolo[3,4-*d*]pyrimidin-3-yl]-1-methyl-1*H*-pyrrole-3-carbonitrile with the appropriate amine.

### Example 24

### 5-(2-[(6-chloroquinolin-4-yl)methyl]-7-(cyclopropylmethyl)-4-{[(2S)-2-hydroxypropyl]amino}-6-oxo-6,7-dihydro-2H-pyrazolo[3,4-d]pyrimidin-3-yl)-1-methyl-1H-pyrrole-3-carbonitrile

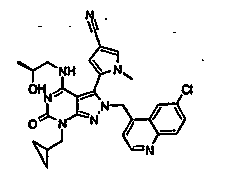

amine: (S)-(+)-1-amino-2-propanol
1H NMR (300 MHz, CHLOROFORM-D) d ppm 0.50 (d, *J*=4.52 Hz, 4 H) 1.16 (dd, *J*=6.22, 1.88 Hz, 3 H) 1.46 (m, 1 H) 2.50 (m, 1 H) 3.08 (d, *J*=3.01 Hz, 3 H) 3.89 (m, 2 H) 3.99 (m, *J*=6.78, 6.78 Hz, 1 H) 5.24 (m, 1 H) 5.66 (d, *J*=2.07 Hz, 2 H) 6.73 (d, *J*=1.70 Hz, 1 H) 6.81 (dd, *J*=4.24, 2.35 Hz, 1 H) 7.34 (s, 1 H) 7.72 (dd, *J*=8.85, 2.07 Hz, 1 H) 7.96 (d, *J*=2.26 Hz, I H) 8.12 (d, *J*=8.85 Hz, 1 H) 8.83 (d, *J*=4.52 Hz, 1 H); ES (M+H)⁺= 544

### Example 25

### 5-(2-[(6-chloroquinolin-4-yl)methyl]-7-(cyclopropylmethyl)-4-{[(2R)-2-hydroxypropyl]amino}-6-oxo-6,7-dihydro-2H-pyrazolo[3,4-d]pyrimidin-3-yl)-1-methyl-1H-pyrrole-3-carbonitrile

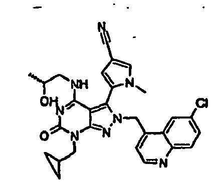

amine: (R)-(-1)-1-amino-2-propanol
1H NMR (300 MHz, DMSO-D6) d ppm 0.51 (d, *J*=5.46 Hz, 4H) 1.17 (m, 3H) 1.47 (m, 1 H) 3.07 (d, *J*=3.20 Hz, 3 H) 3.96 (m, 2H) 3.99 (m, 2H) 5.18 (s, 1 H) 5.66 (d, *J*=2.45 Hz, 2 H) 6.73 (d, *J*=1.70 Hz, 1 H) 6.81 (dd, *J*=4.14, 2.45 Hz, 1 H) 7.33 (s, 1 H) 7.72 (dd, *J*=9.04,2.07 Hz, 1 H) 7.96 (d, *J*=2.26 Hz, 1 H) 8.11 (d, *J*=9.04 Hz, 1 H) 8.83 (d, *J*=4.33 Hz, 1 H); ES (M+H)⁺= 544

### Example 26

### 5-{6-amino-2-[(6-chloroquinolin-4-yl)methyl-4-methyl-4-oxo-4,5-dihydro-2H-pyrazolo[3,4-d]pyrimidin-3-yl}-1-methyl-1H-pyrrole-3-carbonitrile

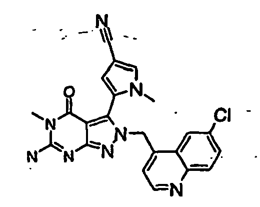

### (a) 2-amino-6-chloropyrimidin-4(3H)-one

2-Amino-4,6-dichloropyrimidine (10.6 g) was suspended in 1N NaOH (100 mL) and heated to reflux. Additional NaOH₍ₛ₎ (1.0 g) was added after 2 h and 4 h. After 5h, the solution was cooled with an ice bath and neutralized with acetic acid. The white precipitate was filtered, washed with water, and dried on a high vacuum to yield 9.28 g of white solid.

### (b) 2-amino-6-chloro-3-methylpyrimidin-4(3H)-one

To a suspension of 2-amino-6-chloropyrimidin-4(3H)-one (5.37 g) in ethanol (300 mL) was added NaOH₍ₛ₎ (1.94 g) and heated (T_{b}= 60 °C). After 30 min., iodomethane (3.0 mL) was added and the reaction heated to reflux. After 2h, additional NaOH₍ₛ₎ (2.36 g) and iodomethane (1.5 mL) were added. After 7 h, the reaction was concentrated. The residue was diluted with water and neutralized with acetic acid. The resulting solid was collected by filtration yielding 2.43 g white solid after drying on high vacuum.

### (c) 2-amino-6-hydrazino-3-methylpyrimidin-4(3H)-one

To a suspension of 2-amino-6-chloro-3-methylpyrimidin-4(3*H*)-one (2.43 g) in ethanol was added hydrazine hydrate (12.0 mL). After 7 h at reflux, the suspension was cooled, filtered and dried on high vacuum yielding 1.01 g of white solid.

### (d) 6-chloroquinoline-4-carbaldehyde (2-amino-1-methyl-6-oxo-1,6-dihydropyrimidin-4-yl)hydrazone

To a suspension of 2-amino-6-hydrazino-3-methylpyrimidin-4(3*H*)-one (1.01 g) in methanol (20 ml) was added 6-chloroquinoline 4- carbaldehyde (1.35 g). After 3h, the reaction was filtered yielding a yellow solid (2.14 g).

### (e) 5-{6-amino-2-[(6-chloroquinolin-4-yl)methyl]-5-methyl-4-oxo-4,5-dihydro-2H-pyrazolo[3,4-d]pyrimidin-3-yl}-1-methyl-1H-pyrrole-3-carbonitrile

To a suspension of 6-chloroquinoline-4-carbaldehyde (2-amino-1-methyl-6-oxo-1,6-dihydropyrimidin-4-yl)hydrazone (2.14 g) in DMF (25 mL) were added 5-formyl-1-methyl-1*H*-pyrrole-3-carbonitrile (0.86 g) and piperdine (0.50 mL). After stirring overnight at T_{b}= 60 °C, the solution was diluted with ethylacetate and water. The organic solution was collected, washed with sat'd NaHCO₃ and brine, dried (Na₂SO₄), filtered and concentrated. The residue was suspended in methanol and filtered (1.34 g). 1H NMR (300 MHz, DMSO-D6) d ppm 2.50 (m, 3 H) 3.28 (d, *J*=4.33 Hz, 6 H) 5.87 (m, 2 H) 6.74 (d, *J*=1.70 Hz, 1 H) 6.79 (d, *J*=4.33 Hz, 1 H) 6.94 (s, 2 H) 7.79 (m, 2 H) 8.07 (m, 2 H) 8.80 (d, *J*=4.33 Hz, 1 H); ES (M+H)⁺ 445.

### Example 27

### 5-[2-[(6-chloroquinolin-4-yl)methyl]-7-(cyclopropylmethyl)-6-imino-5-methyl-4-oxo-4,5,6,7-tetrahydro-2H-pyrazolo[3,4-d]pyrimidin-3-yl]-1-methyl-1H-pyrrole-3-carbonitrile

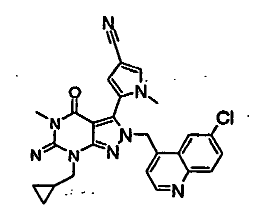

To a suspension of 5-{6-amino-2-[(6-chloroquinolin-4-yl)methyl]-5-methyl-4-oxo-4,5-dihydro-2H-pyrazolo[3,4-*d*]pyrimidin-3-yl}-1-methyl-1H-pyrrole-3-carbonitrile (0.12 g) in THF (2 mL) and 1,8-diazabicyclo[4.3.0]undec-7-ene (0.10 mL) was added (bromomethyl)cyclopropane (0.10 mL). The reaction was heated in a microwave for 1h at 140 °C. The reaction was then concentrated and purified by FlashMaster^{™} yielding 58 mg of brown foam. 1H NMR (300 MHz, DMSO-D6) d ppm 0.53 (m, 4 H) 1.40 (m, 1 H) 3.33 (s, 3 H) 3.39 (s, 3 H) 3.98 (m, 2 H) 5.68 (m, 1 H) 6.56 (d, *J*=1.70 Hz, 1 H) 6.81 (d, *J*=4.33 Hz, 1 H) 7.31 (d, *J*=1.51 Hz, 1 H) 7.71 (dd, *J*=9.04,2.07 Hz, 1 H) 7.84 (d, *J*=2.07 Hz, 1 H) 8.12 (d, *J*=8.85 Hz, 1 H) 8.83 (d, *J*=4.33 Hz, 1 H); ES (M+M)⁺ 499.

### Example 28-29

### N-[2-[(6-chloroquimolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-3-methylbutanamide

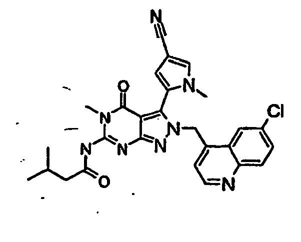

### N,N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-bis-3-methylbutanamide

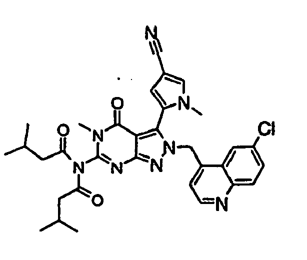

To a solution of 5-{6-amino-2-[(6-chloroquinolin-4-yl)methyl]-5-methyl-4-oxo-4,5-dihydro-2*H*-pyrazolo[3,4-*d*]pyrimidin-3-yl}-1-methyl-1*H*-pyrrole-3-carbonitrile (0.11 g) in THF (5 mL) and triethylamine (0.50 mL) was added isovaleryl chloride (0.05 mL). After stirring overnight, DMF (1.0 mL), 1,8-diazabicyclo[4.3.0]undec-7-ene(0.10 mL) and additional isovaleryl chloride (0.05 mL) were added. After 5 h additional, the reaction was warmed (T_{b}= 55 °C). After stirring overnight again, the reaction was then concentrated and purified by FlashMaster^{™} yielding two products: mono-acylated *N*-[2-[(6-chloroquinolin-4-yl)methyl]-3-(4-cyano-1-methyl-1*H*-pyrrol-2-yl)-5-methyl-4-oxo-4,5-dihydro-2*H*-pyrazolo[3,4-d]pyrimidin-6-yl]-3-methylbutanamide (52 mg) 1H NMR (300 MHz, CHLOROFORM-D) d ppm 1.01 (m, 6 H) 2.24 (m, 1 H) 2.40 (m, *J*=6.97 Hz, 2 H) 3.39 (s, 3 H) 3.50 (s, 3 H) 5.76 (dd, *J*=70.64, 15.64 Hz, 2 H) 6.60 (m, 1 H) 6.93 (d, *J*=1.51 Hz, 1 H) 7.36 (s, 1 H) 7.64 (m, 1 H) 7.71 (dd, *J*=9.04, 2.07 Hz, 1 H) 8.12 (d, *J=*9.23 Hz, 1 H) 8.86. (d, *J*=4.33 Hz, 1 H) 14.38 (s, 1 H); ES (M+H)⁺ 529 and bis-acylated *N*,*N*-[2-[(6-chloroquinolin-4-yl)methyl]-3-(4-cyano-1-methyl-1*H*-pyrrol-2-yl)-5-methyl-4-oxo-4,5-dihydro-2*H*-pyrazolo[3,4-*d*]pyrimidin-6-yl]-bis-3-methylbutanamide (49 mg) 1H NMR (300 MHz, CHLOROFORM-D) d ppm 0.95 (m, 12 H) 2.19 (m, 4 H) 2.65 (m, 2 H) 3.40 (m, 6 H) 5.90 (dd, *J*=81.01, 15.26 Hz, 1 H) 6.67 (d, *J*=1.70 Hz, 1 H) 7.06 (d, *J*=4.52 Hz, 1 H) 7.39 (d, *J*=1.51 Hz, 1H) 7.63 (d, *J=*2.07 Hz, 1 H) 7.71 (dd, *J*=8.95, 2.17 Hz, 1 H) 8.12 (d, *J*=9.04 Hz, 1 H) 8.86 (d, *J*=4.52 Hz, 1 H); ES (M+H)⁺ 613

### Example 30

### N'-[2-[(6-chloroqninolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-N,N-dimethylimidoformamide

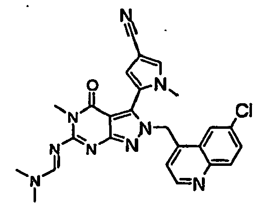

To a suspension of 5-{6-amino-2-[(6-chloroquinolin-4-yl)methyl]-5-methyl-4-oxo-4,5-dihydro-2*H*-pyrazolo[3,4-*d*]pyrimidin-3-yl}-1-methyl-1*H*-pyrrole-3-carbonitrile (0.12 g) in DMF (3 mL) and 1,8-diazabicyclo[4.3.0]undec-7-ene (0.20 mL) was added propanesulfonyl chloride (0.10 mL). After stirring overnight at rt, the solution was diluted with ethyl acetate and water. The organic solution was collected, washed with sat'd NaHCO₃ and brine, dried (Na₂SO₄), filtered and concentrated. The reaction was then concentrated and purified by FlashMaster^{™} yielding 65 mg of brown foam. 1H NMR (300 MHz, CHLOROFORM-D) d ppm 3.19 (d, *J*=9.98 Hz, 6 H) 3.45 (s, 3 H) 3.58 (s, 3 H) 5.80 (dd, *J*=77.14, 15.73 Hz, 2 H) 6.54 (d, *J*=1.70 Hz, 1H) 7.00 (d, *J*=4.52 Hz, 1H) 7.34 (d, *J*=1.70 Hz, 1H) 7.69 (m, 2H) 8.10 (m, 1 H) 8.78 (s, 1H) 8.82 (d, *J*=4.33 Hz, 1H); ES (M+H)⁺ 500.

### Example 31

### 5-[4-amino-2-[(6-chloroquinolin-4-yl)methyl]-7-(cyclopropylmethyl)-6-oxo-6,7-dihydro-2H-pyrazolo[3,4-d]pyrimidin-3-yl]-1-methyl-1H-pyrrole-3-carbonitrile

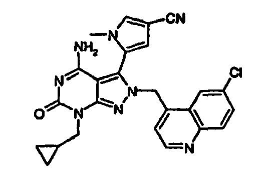

5-[2-[(6-chloroquinolin-4-yl)methyl]-7-(cyclopropylmethyl)-6-oxo-4-thioxo-4,5,6,7-tetrahydro-2*H*-pyrazolo[3,4-*d*]pyrimidin-3-yl]-1-methyl-1*H*-pyrrole-3-carbonitrile (0.5g, 1.0 mmol) was dissolved in 10 mL anhydrous THF. Anhydrous ammonia in methanol (20 mL of 2M solution) was added, followed by mercury (II) chloride (410 mg, 1.5 mmol, 1.5 eq). The mixture was heated to 60°C for 48h. Volatiles were evaporated and the residue dissolved in 100 mL ethylacetate, 50 mL dichloromethane, and 100 mL water. The organic layer was washed with water (100 mL) and dried over sodium sulfate. The residue was purified by chromatography on silica (gradient: dichloromethane to 10% methanol in dichloromethane), yielding 232 mg (48%) of the product as a yellow solid. ES M+H⁺ = 485. ¹H NMR (300MHz, DMSO-d₆): 8.80 (d, J = 6Hz, 1H); 8.16 (d, J = 3Hz, 1H); 8.06 (d, J = 9Hz, 1H); 7.80 (m, 2H); 6.85 (d, J = 3Hz, 1H); 6.81 (d, J = 6Hz, 1H); 5.87 (d, J =16Hz, 1H); 5.72 (d, J = 16Hz, 1H); 3.77 (m, 2H); 3.33 (s, 3H); 1.28 (m, 1H); 0.45-0.3 (m, br, 4H).

### Example 32

### 2-[(6-chloroquinolin-4-yl)methyl]-7-(cyclopropylmethyl)-5-methyl-3-(1-methyl-1H-imidazol-5-yl)4-thioxo-2,4,5,7-tetrahydro-6H-pyrazolo[3,4-d]pyrimidin-6-one

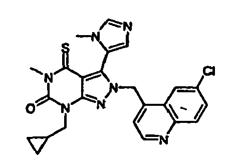

2-[(6-chloroquinolin-4-yl)methyl]-7-(cyclopropylmethyl)-5-methyl-3-(1-methyl-1*H*-imidazol-5-yl)-2*H*-pyrazolo[3,4-*d*]pyrimidine-4,6(5*H*,7*H*)-dione (250 mg, 0.53 mmol) and P4S 10 (470 mg, 1.06 mmol, 2.0 eq.) were stirred in 5 mL anhydrous pyridine. The mixture was heated in a microwave reactor at 150°C for 2h. 15 min. The reaction was diluted with 50 mL ethylacetate and 50 mL water. The aqueous layer was washed 2x30 mL with ethylacetate. The combined organic layers were washed with water (70 mL) and brine (50 mL) and dried over sodium sulfate. Volatiles were removed under vacuum and the residue purified by chromatography on silica (gradient: dichloromethane to 10% methanol in dichloromethane). Yield 40 %. FS+H⁺ = 492. ¹H NMR (300MHz, DMSO-*d*₆) 8.82 (d, J = 3.Hz, 1H); 8.59 (d, J = 6Hz, 1H); 8.16 (d, J = 2Hz, 1H); 7.80 (s, 1H); 7.38 (m, 1H); 7.03 (s, 1H); 6.80 (d, J = 6Hz, 1H); 5.90 (m, 2H); 3.90 (m, 2H); 3.67 (s, 3H); 3.16 (s, 3H); 1.40 (m, 1H); 0.5-0.4 (m, 4H).

### Example 33

### (4Z)-2-[(6-chloroquinolin-4-yl)methyl]-7-(cyclopropylmethyl)-5-methyl-3-(1-methyl-1H-imidazol-5-yl)-4-(methylimino)-2,4,5,7-tetrahydro-6H-pyrazolo[3,4-d]pyrimidin-6-one

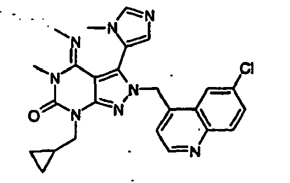

2-[(6-chloroquinolin-4-yl)methyl]-7-(cyclopropylmethyl)-5-methyl-3-(1-methyl-1*H*-imidazol-5-yl)-4-thioxo-2,4,5,7-tetrahydro-6*H*-pyrazolo[3,4-*d*]pyrimidin-6-one (210 mg, 0.43 mmol) was dissolved in 5 mL anhydrous THF. Methylamine (4 eq as 2.0 M solution in THF) added, and the solution heated to 60°C. Mercury (II) chloride (232 mg, 0.86mmol, 2 eq.) was added and the solution stirred at 60°C for one hour. As no reaction was evident (as judged by LC/MS of an aliquot), another 4 eq. amine was added, and the reaction heated to 110°C for one hour in the microwave reactor. The reaction mixture was diluted with 25 mL ethylacetate and filtered through celite. The celite was washed with small amount of solvent. The organic layer was washed with water (50 mL) and brine (50 mL), and dried over sodium sulfate. The volatiles were removed under vacuum and the residue purified by chromatography on silica (gradient: dichloromethane to 10% methanol in dichloromethane). Yield 35 %. ES+H⁺ = 489. ¹H NMR (300MHz, CDCl₃) 8.75 (d, J = 6Hz, 1H); 8.04 (d, J = 9Hz, 1H); 7.96 (d, J = 2Hz, 1H); 7.64 (dd, J = 9Hz, 2Hz, 1H); 7.52 (s, 1H); 7.20 (s, 1H); 6.65 (d, J = 6Hz, 1H); 5.63 (m, 2H); 3.87 (m, 2H); 3.31 (s, 3H); 2.98 (s, 3H); 2.66 (s, 3H); 1.37 (m, 1H); 0.5-0.41 (m, 4H).

### Utility

The compounds of the present invention have utility for the prevention and treatment of *H*. *pylori* infection. Methods of treatment target the prevention of cell wall biosynthesis through the MurI enzyme. Compounds that inhibit MurI activity control the production of cell wall biosynthesis. The inhibition of MurI. will inhibit growth of *H. pylori* and will-reduce or prevent the diseases resulting from *H. pylori* infection such as peptic ulcers, gastritis and MALT lymphoma. The compounds of the present invention have utility for the prevention and treatment of such disorders.

Compounds of the present invention have been shown to inhibit MurI, as determined by glutamate racemase activity assay described herein.

Compounds provided by this invention should also be useful as standards and reagents in determining the ability of a potential pharmaceutical to inhibit MurI. These would be provided in commercial kits comprising a compound of this invention.

### Abbreviations

As used herein "rt" denotes room temperature, "ug " denotes microgram, "mg" denotes milligram, "g" denotes gram, "uL" denotes microliter, "mL" denotes milliliter, "L" denotes liter, "nM" denotes nanomolar, "uM" denotes micromolar, "mM" denotes millimolar, "M" denotes molar, "nm" denotes nanometer, "DMSO" denotes dimethyl sulfoxide, "DTT" denotes dithiothreitol, "EDTA" denotes ethylenediaminetetraacetate,

### Assay

### Glutamate Racemase Activity Assay:

Glutamate racemase (MurI) activity was assayed by measuring the conversion of glutamate from D to L enantiomer. This reaction was coupled to the reduction of NAD⁺ to NADH by L-glutamate dehydrogenase (LGDH). LGDH from bovine liver was obtained as a lyophilized powder (Sigma #G-7882) and dissolved in 10 mM Tris (Sigma #T-6791), pH 7.5, buffer containing 0.1 mM EDTA (Fisher #BP118-500) and 50% glycerol (Sigma #G-9012). The assay mixture consisted of 100 mM Tris-HCl, pH 8.0, 10 mM β-NAD (Sigma #N-7004), 5 mM DTT (Sigma #D-5545), 0.03% PEG (mw 8000, Sigma #P-5413), 0.03 mg/mL BSA (Pierce #23210), 15 U/mL LGDH, D-glutamate (40 µM, Fluka #49460), and purified MurI (1 uM). The assay was performed in 96-well black microtiter plates (Greiner #XN2-9511) with a final assay volume of 100 µL. Compounds were prepared as 20 mM stock solutions in dimethyl sulfoxide (DMSO, Sigma #D-5879) and serial dilutions were prepared from these solutions using DMSO, 2 µL of which were added to the wells. Activity at room temperature was measured by monitoring the increase in fluorescence using a TECAN Ultra plate reader with 340 nm excitation and 465 nm emission filters. The compounds provided have measured IC50 of less then 400 µM

## Claims

1. A compound having the structural formula (I): 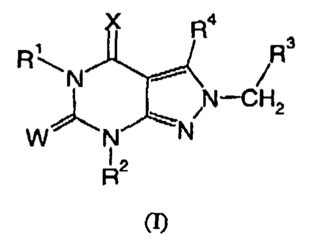 wherein,
X is S, 0, or NR²⁰, provided that when W is O, then X is not O,
X and the double bond to which it is attached can be replaced with 2 hydrogen atoms,
W is S, O, or NR²⁰; provided that when X is O, then W is not O;
R¹ is H, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, hydroxy, amino, or optionally substituted heterocycle;
R² is H, optionally substituted alkyl, optionally substituted alkylcycloalkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, or optionally substituted heterocycle;
R³ is a monocyclic or bicyclic, saturated or unsaturated, ring system comprising 0, 1, 2 or 3 heteroatoms independently selected from N, O, or S, the ring being substituted by 0, 1, 2 or 3 substituents selected from =O, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{u}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵,-R⁵-Het, -Het-R⁵, - Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, 0, or S, the ring system being substituted by 0, 1, 2 or 3 substituents selected from B(OH)₂, vicinal -OCH₂CH₂O-, vicinal -OC₁₋₂ haloalkylO-, vicinal -OCH₂O-, vicinal -CH₂OCH₂O-, =O, halogen, -R^{b}OR^{a}, -SR^{a}, -OR^{a}, C₁₋₆ alkyl, C₁₋₆haloalkyl, -CN, -S(=O)ₙR^{c}, -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR³R³, -NHC(=O)R^{a}, -NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het),-C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, - C(=NCN)R^{a}, -S(=O)₂NR^{a}R^{a},-NR^{a}S(=O)₂R^{a}, -S(=O)₂NR^{a}(R^{b}C(=O)NR^{a}R^{a}), - S(=O)₂NR^{a}(R^{b}C(=O)OR^{a}), aminocarbonyl, phenyl, benzyl; or R⁴ is represented by -(CH₂)ₙR⁵-Het, -(CH₂)ₙR^{d}, -Het, -Het-Het, R⁵, -R⁵-Het, -Het-R⁵, -Het-OR⁵, R⁵-R⁵, or -R⁵-OR⁵; or R⁴ is represented by C₁₋₆alky, -NC₁₋₆alkyl, or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)₂CH₂OH, -(CH₂)_{z}CO₂H, or -(CH₂)_{z}CO₂C₁₋₆alkyl wherein z is 1,2,3,4,5, or 6;
R⁵ is independently at each instance, phenyl substituted by 0, 1, 2, or 3 groups selected from halogen, C₁₋₆haloalkyl, -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, - C(=O)NR^{a}OR^{a}, -C(=O)NR^{a}R^{b}NR^{a}R^{a}, C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, or - S(=O)₂NR^{a}R^{b}C(=O)OR^{a};
R²⁰ is, independently at each instance, H, -CN, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R^{a} is, independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, 0 or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, vicinyl-OCH₂CH₂O-, vicinyl-OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, O, or S;
m is 1, 2 or 3;
n is 0, 1 or 2;
When "optionally substituted" is used, it refers to at least one substituent selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic
or a pharmaceutically acceptable salt thereof.

2. A compound as recited in Claim 1 wherein:
R¹ is H, or C₁₋₆alkyl, or -(CH₂)ₙcycloalkyl or -(CH₂)₁₋₂Het wherein C₁₋₆alkyl or-(CH₂)ₙcycloalkyl or -(CH₂)₁₋₂Het is optionally substituted by 1, 2 or 3 substituents selected from Het, halogen, -CN, -OR^{a}, -NR^{a}R^{a}, -C(=O)OR^{a}, -C(=O)NR^{a}R^{a}, -OC(=O)C₁₋₄alkyl, -S(=O)ₙR^{c}, -S(=O)ₙNR^{a}R^{a} or -NR^{a}C(=O)C₁₋₄alkyl and n is 0, 1 or 2.

3. A compound as recited in Claim 1 wherein:
R² is -(CH₂)₁₋₃cycloalkyl or -C₁₋₁₂alkyl wherein -(CH₂)₁₋₃cycloalkyl or -C₁₋₁₂alkyl is optionally substituted with 0, 1, 2 or 3 substituents selected from Het, S(=O)ₙR^{c}, -S(=O)ₙNR^{a}R^{a} halogen, -CN, -OR^{a}, -NR^{a}R^{a}, -C(=O)OR^{a}, -C(=O)R^{a}, -C(=O)NR^{a}R^{a}, -OC(=O)C₁₋₄alkyl, or -NR^{a}C(=O)C₁₋₄alkyl and n is 0, 1 or 2.

4. A compound as recited in Claim 1 wherein:
R³ is selected from formulas (i), (ii), (iii) or (iv) set forth below: 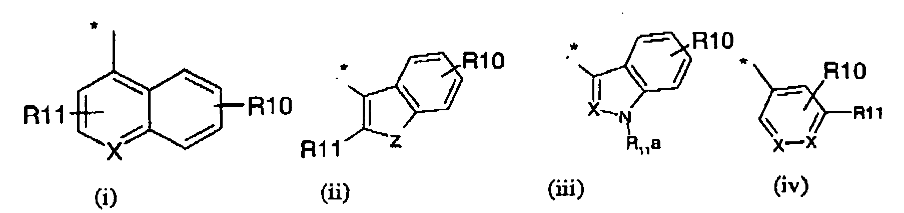
wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.

5. A compound as recited in Claim 1 wherein:
R⁴ is selected from formulas (a) to (z) or (aa) or (ab) set forth below: 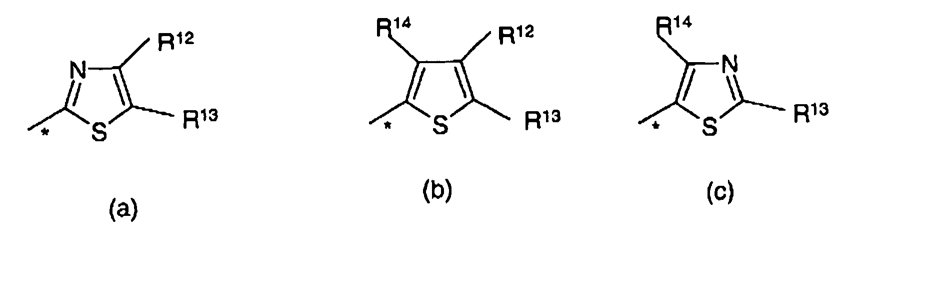 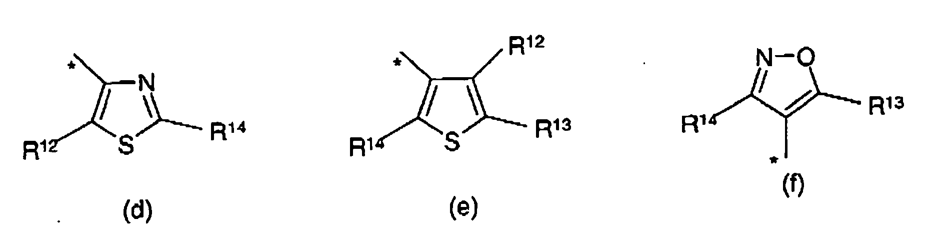 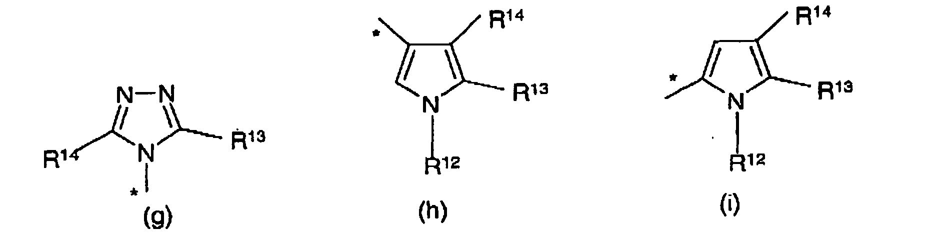 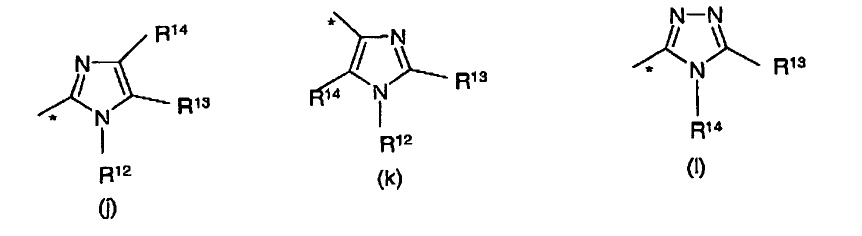 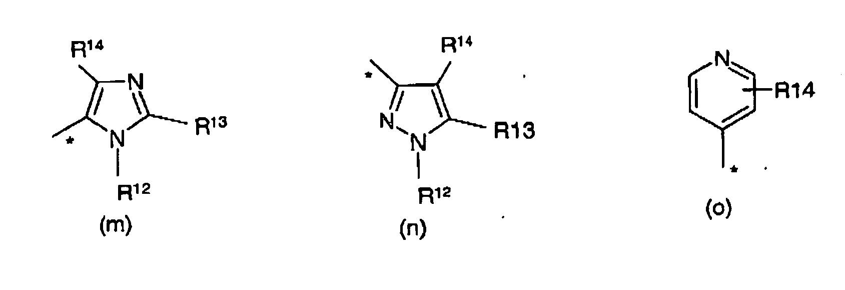 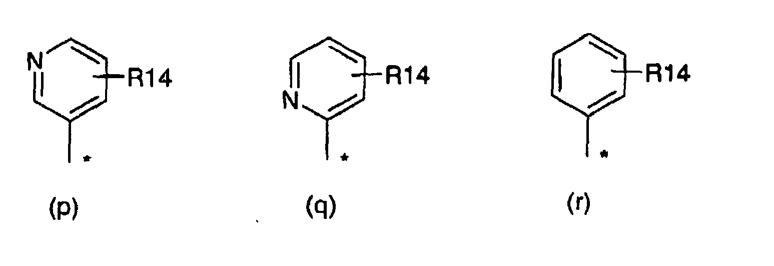 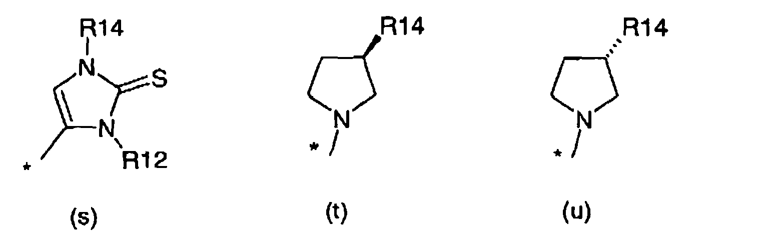 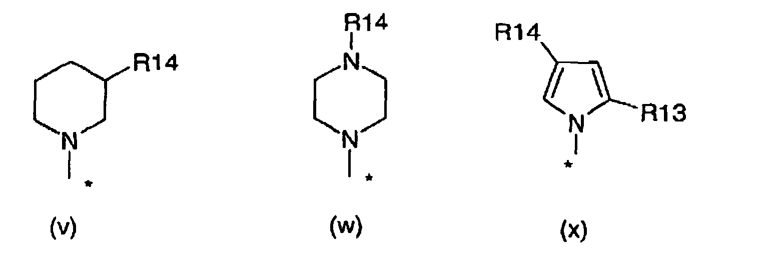 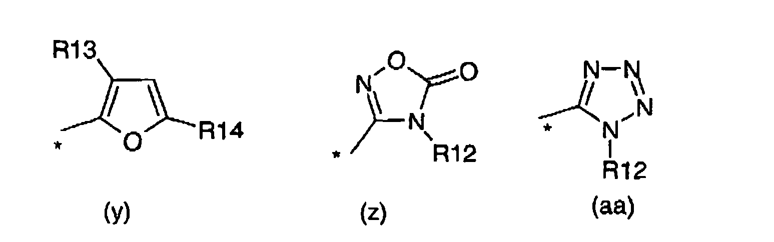 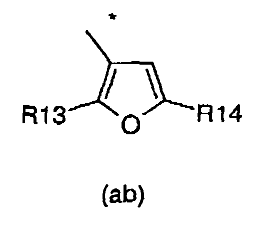
wherein* is the location wherein R⁴ is attached to the ring system and wherein
wherein R¹², R¹³ and R¹⁴ are each independently represented by H, Het, C₁₋₆alkyl, -CN, -NR^{a}R^{a}, - nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), or - S(=O)₂NR^{a}(R^{b}C(=O)OR^{a}.

6. A compound as recited in Claim 1 wherein:
X is S, O, or NR²⁰, provided that when W is O, then X is not O; or X and the double bond to which it is attached can be 2 hydrogen atoms,
W is S, 0, or NR²⁰; provided that when X is O, then W is not 0;
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}
R¹ is CH₃, CH₂CH₃, CH₂CN, CF₃, (CH₂)₂OH, cyclopropyl, isopropyl, CH₂CCH, (CH₂)₂N(CH₂)_{2.}(CH₂)₂N(C=NH)NH_{2,}-CH₂-2-pyridyl, -CH₂-3-pyridyl, -CH₂-4-pyridyl, -(CH₂)₂-1-imidazolyl, -(CH₂)₂-1-pyrazolyl, -(CH₂)₂-1-piperidyl, -(CH₂)ₘ-(1-methylpiperidin-4-yl), -CH₂-(1-methylpiperidin-3-yl), -(CH₂)₂-(morpholin-4-yl),
R² is -CH₂CH₂CH₃, -CH₂-cyclopropyl, -CH₂CH(CH₃)₂, -CH₂CH₂CH₂F, -CH₂-cyclobutyl, -CH₂C(CH₃)₃, -CH₂CH₂CH(CH₃)₂, -CH₂CF₃, -CH₂-methylphenyl, -CH₂-phenol, - CH₂-(3,5-dimethylisoxazol-4-yl), -CH₂-S-phenyl, -CH₂-phenylcarboxyl, or -CH₂SCF₃;
R³ is selected from formulas (i), (ii), (iii) or (iv) set forth below: 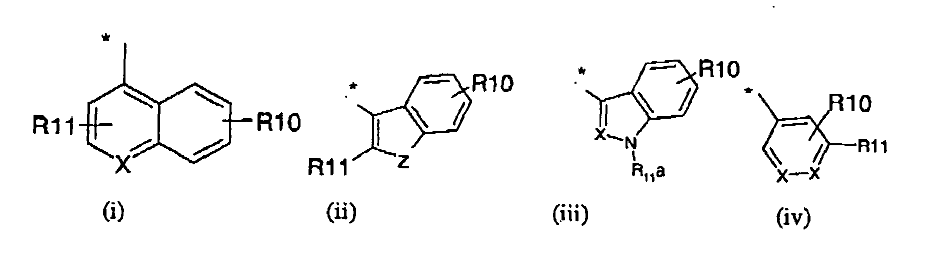 wherein * is the location where (i) or (ii) or (iii) or (iv) is attached to structural formula (I), and X is C or N; and Z is 0 or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.
R⁴ is selected from formulas (a) to (z) or (aa) or (ab) set forth below: 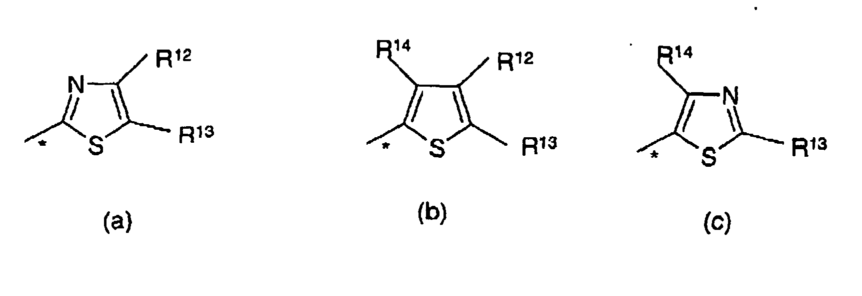 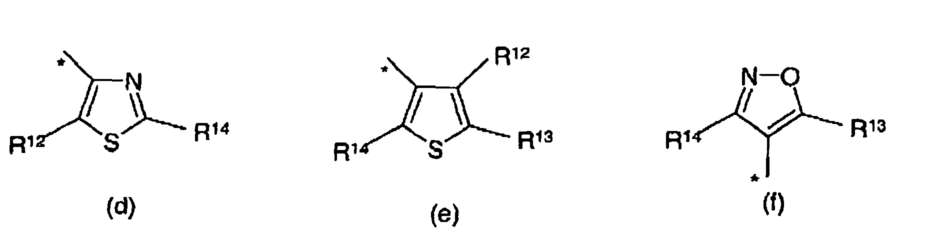 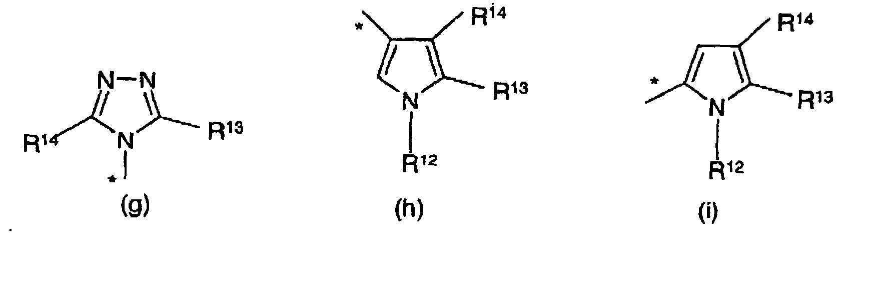 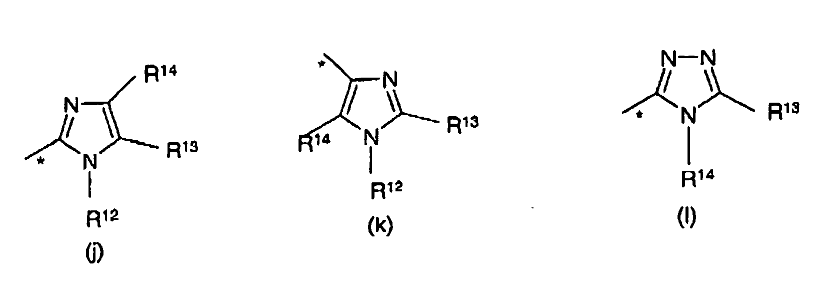 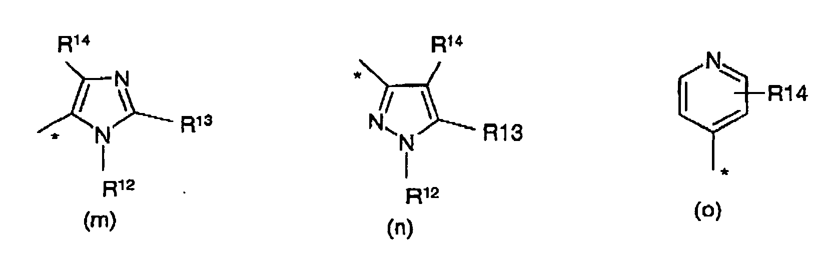 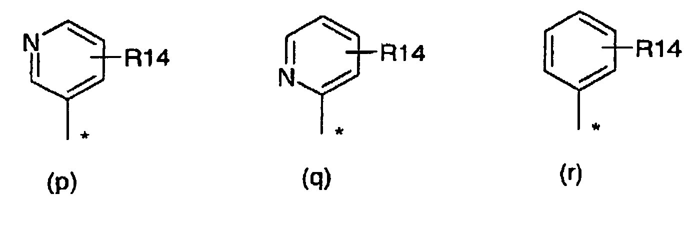 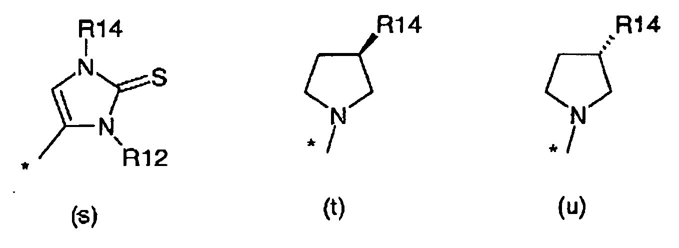 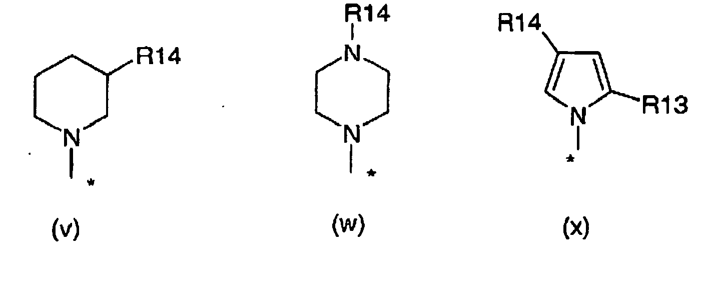 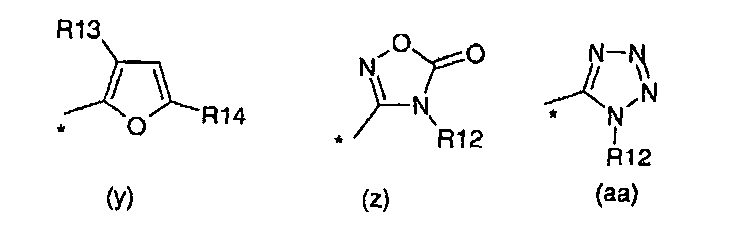 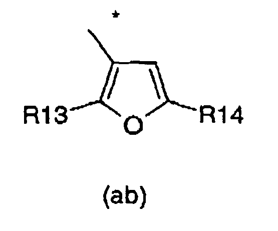
wherein * is the location wherein R⁴ is attached to the ring system and wherein
wherein R¹², R¹³ and R¹⁴ are each independently represented by H, Het, C₁₋₆alkyl, -CN, -NR^{a}R^{a}, - nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, -C(=O)NR³(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), or - S(=O)₂NR^{a}(R^{b}C(=O)OR^{a}.

7. A compound of formula (I) selected from:
5-[2-[(6-chloroquinolin-4-yl)methyl]-7-(cyclopropylmethyl)-6-oxo-4-thioxo,4,5,6,7-tetrahydro-2H-pyrazolo[3,4-d]pyrimidin-3-yl]-1-methyl-1H-pyrrole-3-carbonitrile;
5-[2-[(6-chloroquinolin-4-yl)methyl]-7-(cyclopropylmethyl)-4-imino-5-methyl-6-oxo-4,5,6,7-tetrahydro-2*H*-pyrazolo[3,4-d]pyrimidin-3-yl]-1-methyl-1*H*-pyrrole-3-carbonitrile;
5-[(4Z)-2-[(6-chloroquinolin-4-yl)methyl]-7-(cyclopropylmethyl)-5-methyl-4-(methylimino)-6-oxo-4,5,6,7-tetrahydro-2*H*-Pyrazolo[3,4-*d*]pyrimidin-3yl]-1-methyl-1*H*-pyrrole-3-carbonitrile;
5-[2-[(6-chloroquinolin-4-yl)methyl]-7-(cyclopropylmethyl)-6-imino-5-methyl-4-oxo-4,5,6,7-tetrahydro-2H-pyrazolo [3,4-d]pyrimidin-3-yl]-1-methyl-1H-pyrrole-3-carbonitrile;
5-[2-[(6-chloroquinolin-4-yl)methyl]-7-(cyclopropylmethyl)-5-methyl-4-oxo-6-thioxo-4,5,6,7-tetrahydro-2*H*-pyrazolo[3,4-*d*]pyrimidin-3-yl]-1-methyl-1*H*-pyrrole-3-carbonitrile;
5-[(6Z)-2-[(6-chloroquinolin-4-yl)methyl]-7-(cyclopropylmethyl)-5-methyl-6-(methylimino)-4-oxo-4,5,6,7-tetrahydro-2H-pyrazolo[3,4-d]pyrimidin-3-yl]-1-methyl-1H-pyrrole-3-carbonitrile;
N-[(6Z)-2-[(6-chloroquinolin-4-yl)methyl]-3-(4-cyano-1-methyl-1H-pyrrol-2-yl)-7-(cyclopropylmethyl)-5-methyl-oxo-2,4,5,7-tetrahydro-6H-pyrazolo[3,4-d]pyrimidin-6-ylidene]acetamide;
N-[(6Z)-2-[(6-chloroquinolin-4-yl)methyl]-3-(4-cyano-1-methyl-1H-pyrrol-2-yl)-7-(cyclopropylmethyl)-5-methyl-4-oxo-2,4,5,7-tetrabydro-6H-pyrazolo[3,4-d]pyrimidin-6-ylidene]methanesulfonamide;
5-((6Z)-2-[(6-chloroquinolin-4-yl)methyl]-7-(cyclopropylmethyl)-6-{[2-(dimethylamino)ethyl]imino}-5-methyl-4-oxo-4,5,6,7-tetrahydro-2H-pyrazolo[3,4-d]pyrimidin-3-yl)-1-methyl-1H-pyrrole-3-carbonitrile;
N-1-[2-[(6-chloroquinolin-4-yl)methyl]-3-(4-cyano-1-methyl-1H-pyrrol-2-yl)-7-(cyclopropylmethyl)-5-methyl-4-oxo-2,4,5,7-tetrahydro-6H-pyrazolo[3,4-d]pyrimidin-6-ylidene]-N-2-,N-2-dimethylglycinamide;
5-[2-[(6-chloroquinolin-4-yl)methyl]-7-(cyclopropylmethyl)-6-oxo-4,5,6,7-tetrahydro-2H-pyrazolo[3,4-d]pyrimidin-3-yl]-1-methyl-1H-pyrrole-3-carbonitri1e;
5-[2-[(6-chloroquinolin-4-yl)methyl]-7-(cyclopropylmethyl)-5-methyl-6-oxo-4,5,6,7-tetrahydro-2H-pyrazolo[3,4-d]pyrimidin-3-yl]-1-methyl-1H-pyirole-3-carbonitrile;
5-[2-[(6-chlozoquinolin-4-yl)methyl]-7-(cyclopropylmethyl)-6-oxo-6,7-dihydro-2H-pyrazolo[3,4-d]pyrimidin-3-yl]-1-methyl-1H-pyrrole-3-carbonitrile;
2-[(6-chloroquinolin-4-yl)methyl]-7-(cyclopropylmethyl)-5-methyl-3-(1-methyl-1*H*-imidazol-5-yl)-4-thioxo-2,4,5,7-tetrahydro-6*H*-pyrazolo[3,4-*d*]pyrimidin-6-one;
(4Z)-2-[(6-chloroquinolin-4-yl)methyl]-7-(cyclopropylmethyl)-5-methyl-3-(1-methyl-1*H-*imidazol-5-yl)-4-(methylimino)-2,4,5,7-tetrahydro-6*H*-pyrazolo[3,4-*d*]pyrimidin-6-one.

8. A compound having the structural formula (II): 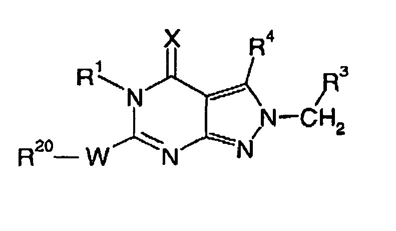 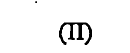 wherein,
X is S, O, or NR²⁰,
X and the double bond to which it is attached can be replaced with 2 hydrogen atoms,
W is S, O, or NR²¹;
R¹ is H, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, hydroxy, amino, or optionally substituted heterocycle, wherein the substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R³ is a monocyclic or bicyclic, saturated or unsaturated, ring system comprising 0, 1, 2 or 3 heteroatoms independently selected from N, O, or S, the ring being substituted by 0, 1, 2 or 3 substituents selected from =O, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵,-R⁵-Het, -Het R⁵, - Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, O, or S, the ring system being substituted by 0, 1, 2 or 3 substituents selected from B(OH)₂, vicinal -OCH₂CH₂O-, vicinal -OC₁₋₂ haloalkylO-, vicinal -OCH₂O-, vicinal -CH₂OCH₂O-, =0, halogen, -R^{b}OR^{a}, -SR^{a}, -OR^{a}, C₁₋₆ alkyl, C₁₋₆haloalkyl, -CN, -S(=O)ₙR^{c}, -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet,-S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), - C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, - C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), - S(=O)₂NR^{a}(R^{a}C(=O)OR^{a}), aminocarbonyl, phenyl, benzyl; or R⁴ is represented by -(CH₂)ₙR⁵-Het, -(CH₂)ₙR^{d}, -Het, -Het-Het, R⁵, -R⁵-Het, -Het-R⁵, -Het-OR⁵, R⁵-R⁵, or -R⁵-OR⁵; or R⁴ is represented by C₁₋₆alky, -NC₁₋₆alkyl), or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)_{z}CH₂OH, -(CH₂)_{z}CO₂H, or -(CH₂)_{z}CO₂C₁₋₆alkyl wherein z is 1,2,3,4,5, or 6;
R⁵ is independently at each instance, phenyl substituted by 0, 1, 2, or 3 groups selected from halogen, C₁₋₆haloalkyl, -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR⁸, -O(CH₂)ₘNR^{a}R^{a}, -O(CH₂)ₘOR^{a},-S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a},-C(=O)NR^{a}R^{a},-C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)NR^{a}R^{a}, -NR^{a}S(=O)₂R^{a}, -S(=O)₂NR^{a}R^{b}C(=O)NR^{a}R^{a}, or -S(=O)₂NR^{a}R^{b}C(=O)OR^{a};
R²⁰ is, independently at each instance, H, -CN, -S(=O)ₙR^{c}, -C(=O)R^{a},-C(=O)NR^{a}R^{a}, -C(=O)OR^{a}, -NR^{a}C(=O)R^{a}, or -OC(=O)R^{a}, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, wherein the substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²¹ is, independently at each instance, H, -CN, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}; optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, wherein the substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²⁰ and R²¹ and the N to which they are attached in combination can.also form a 3 to 10 member N-linked saturated or unsaturated heterocycle having either 1, or 2 heteroatoms independently selected from N, 0, or S wherein the heterocycle is substituted with R^{e};
R^{a} is, independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, vicinyl -OCH₂CH₂O-, vicinyl -OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, O, or S;
R^{c} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
mis 1, 2 or 3;
n is 0, 1 or 2;
or a pharmaceutically acceptable salt thereof.

9. A compound as recited in Claim 8 wherein:
R¹ is H, or C₁₋₆alkyl, or -(CH₂)ₙcycloalkyl wherein C₁₋₆alkyl or -(CH₂)ₙcycloalkyl is optionally substituted by 1, 2 or 3 substituents selected from Het, halogen, -CN, -OR^{a}, -NR^{a}R^{a}, -C(=4)OR^{a}, -C(=O)N^{a}R^{a}, -OC(=O)C₁₋₄alkyl or -NR^{a}C(=O)C₁₋₄alkyl and n is 0, 1 or 2.

10. A compound as recited in Claim 8 wherein:
R³ is selected from formulas (i), (ii), (iii) or (iv) set forth below: 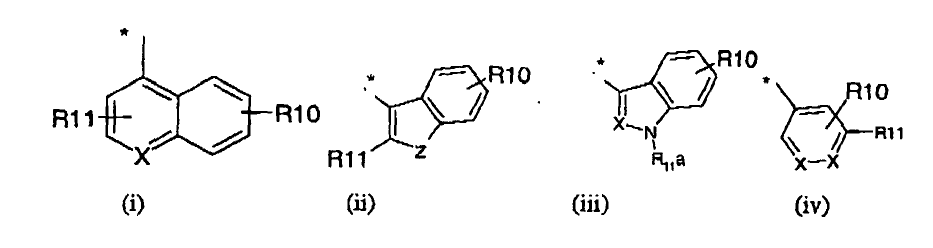
wherein * is the location where (i) or (ii) or (iii) or (iv) is attached to structural formula (I), and X is C or N; and Z is 0 or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.

11. A compound as recited in Claim 8 wherein:
R⁴ is selected from formulas (a) to (z) or (aa) or (ab) set forth below: 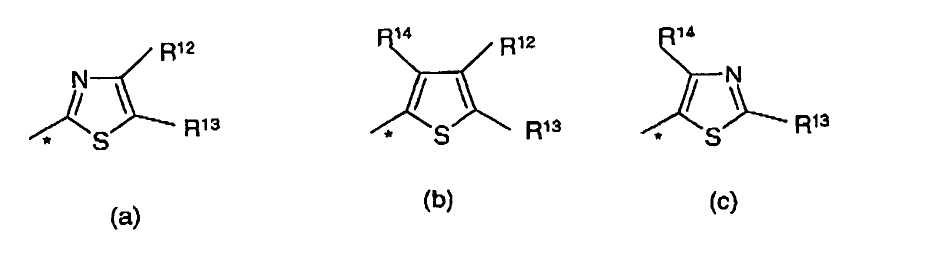 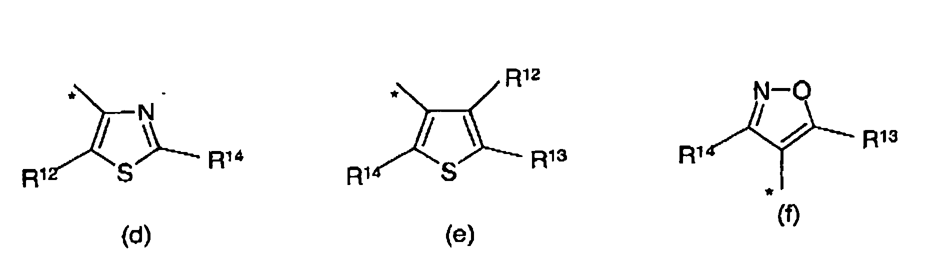 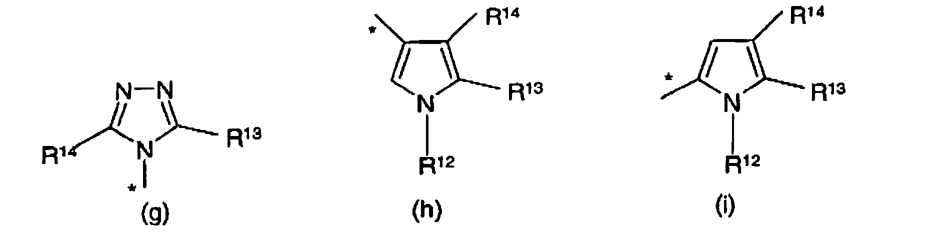 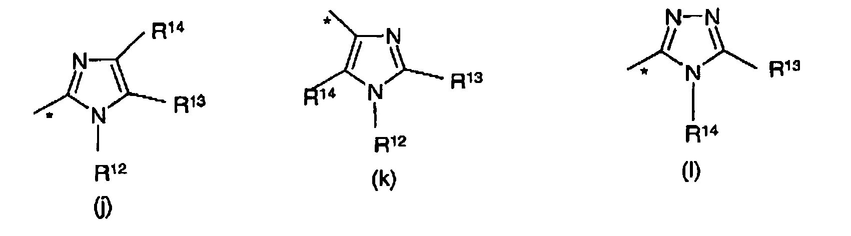 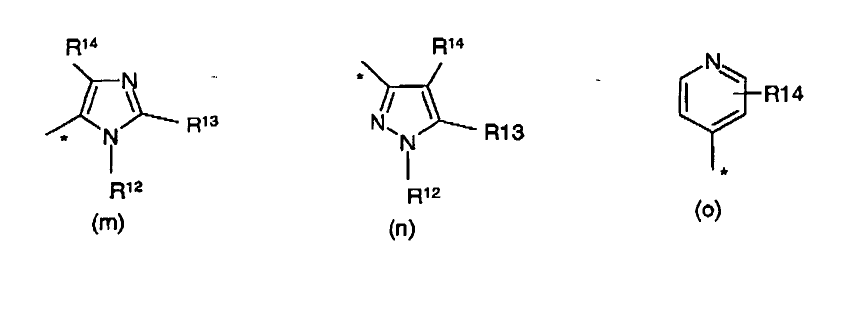 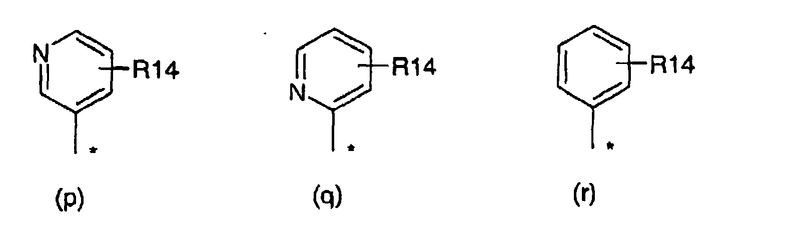 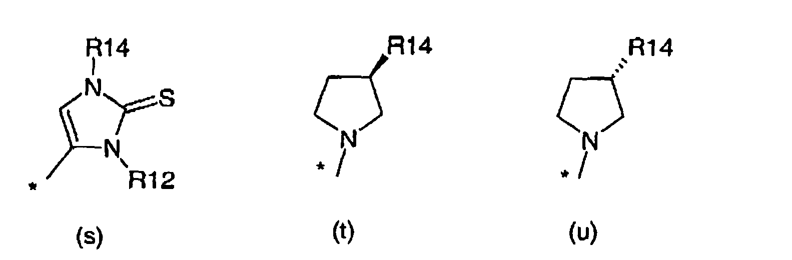 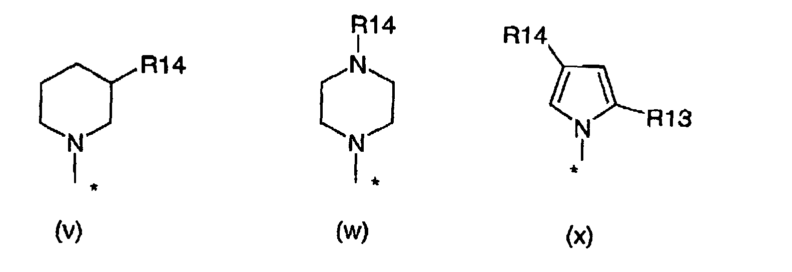 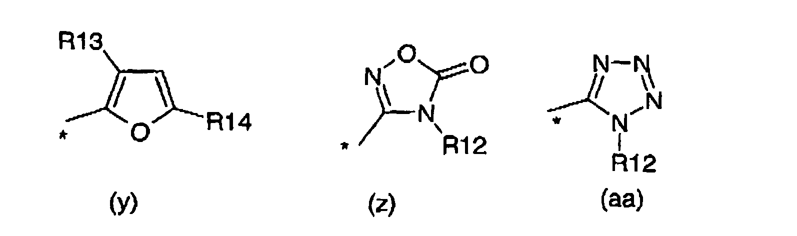 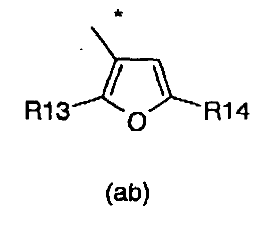
wherein * is the location wherein R⁴ is attached to the ring system and wherein
wherein R¹², R¹³ and R¹⁴ are each independently represented by H, Het, C₁₋₆alkyl, -CN, -NR^{a}R^{a}, -nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a} , -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), or - S(=O)₂NR^{a}(R^{b}C(=O)OR^{a}.

12. A compound as recited in Claim 8 wherein:
X is S, 0, or NR²⁰; or X and the double bond to which it is attached can be 2 hydrogen atoms,
W is S, O, or NR²¹;
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ and R²¹ and the N to which they are attached in combination can also form a 3 to 10 member N-linked saturated or unsaturated heterocycle having either 1, or 2 heteroatoms independently selected from N, O, or S wherein the heterocycle is substituted with R^{e};
R¹ is CH₃, CH₂CH₃, CH₂CN, CF₃, (CH₂)₂OH, cyclopropyl, isopropyl, CH₂CCH, (CH₂)₂N(CH₂)₂, (CH₂)₂N(C=NH)NH₂,-CH₂-2-pyridyl, -CH₂-3-pyridyl, -CH₂-4-pyridyl, -(CH₂)₂-1-imidazolyl, -(CH₂)₂-1-pyrazolyl, -(CH₂)₂-1-piperidyl -(CH₂)ₘ-(1-methylpiperidin-4-yl), -CH₂-(1-methylpiperidin-3-yl), -(CH₂)₂-(morpholin-4-yl),
R³ is selected from formulas (i), (ii), (iii) or (iv) set forth below: 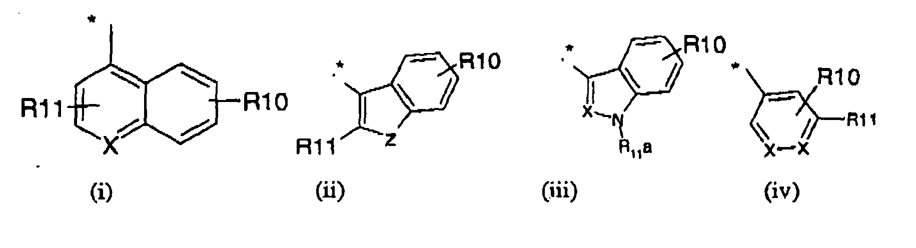 wherein * is the location where (i) or (ii) or (iii) or (iv) is attached to structural formula (I), and X is C or N; and Z is 0 or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.
R⁴ is selected from formulas (a) to (z) or (aa) or (ab) set forth below: 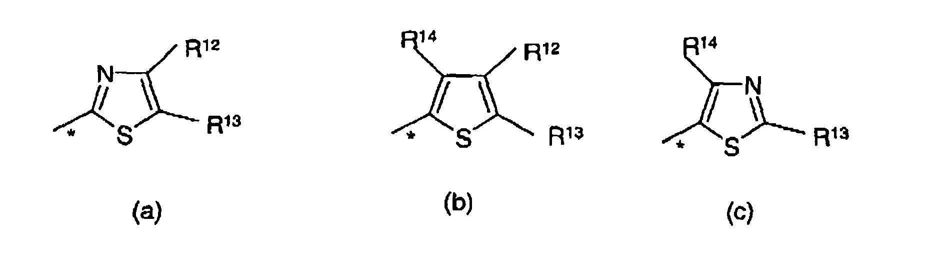 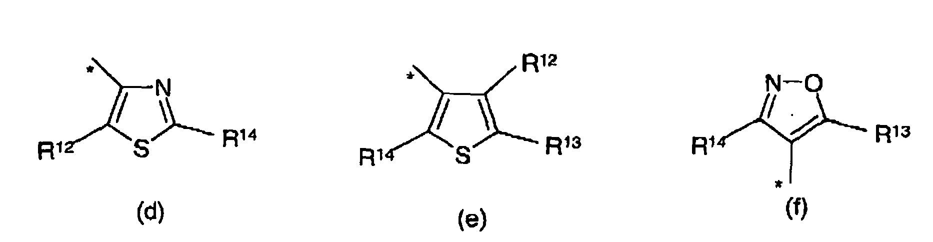 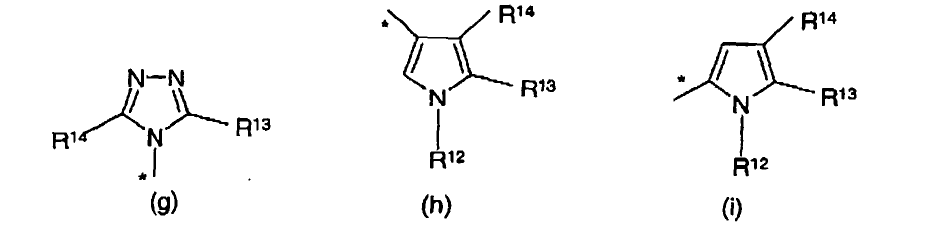 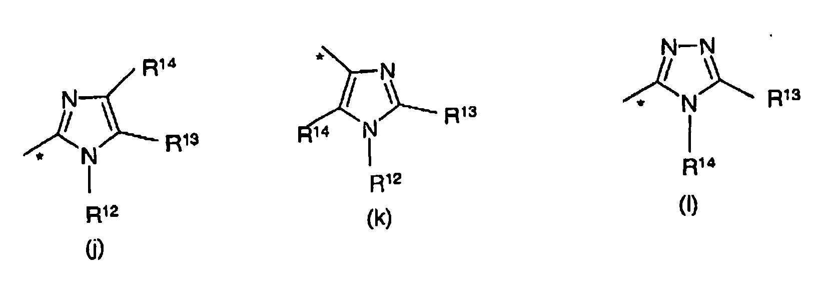 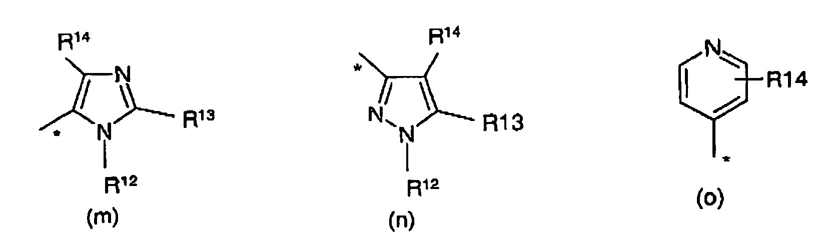 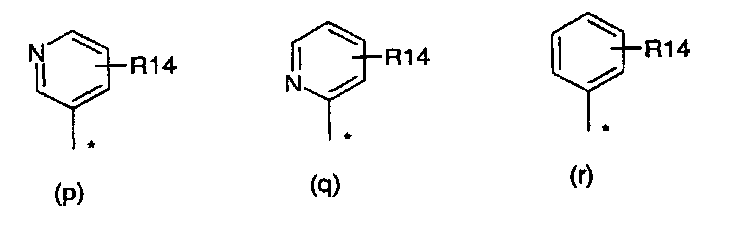 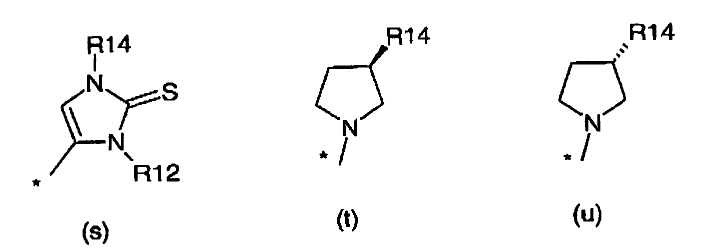 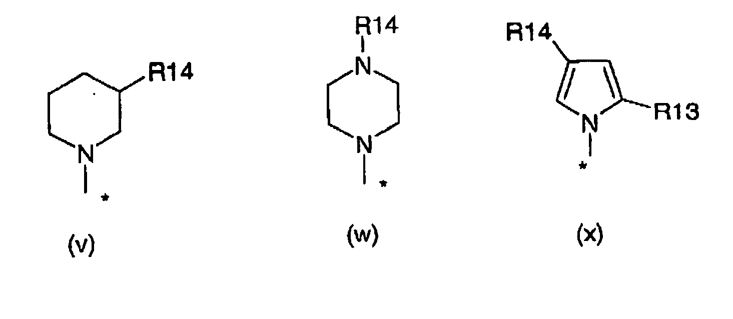 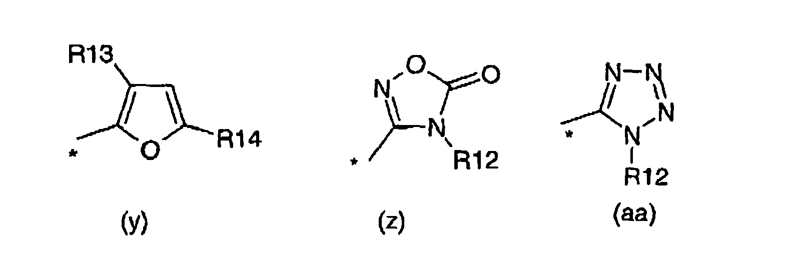 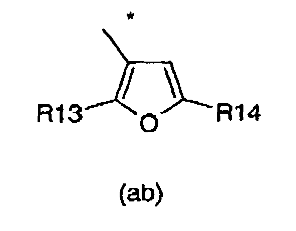
wherein * is the location wherein R⁴ is attached to the ring system and wherein
wherein R¹², R¹³ and R¹⁴ are each independently represented by H, Het, C₁₋₆alkyl, -CN, -NR^{a}R^{a}, - nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), or -S(=O)₂NR^{a}(R^{b}C(=O)OR^{a}.

13. A compound of formula (II) selected from:
5-{6-amino-2-[(6-chloroquinolin-4-yl)methyl]-5-methyl-4-oxo-4,5-dihydro-2H-pyrazolo[3,4-d]pyrimidin-3-yl}-1-methyl-1H-pyrrole-3-carbonitrile;
N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-3-methylbutanamide
N,N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-3-methylbutanamide;
N'-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-N,N-dimethylimidoformamide;
5-{2-[(6-chloroquinolin-4-yl)methyl]-6-[(cyclopropylmethyl)(methyl)amino]-5-methyl-4-oxo-4,5-dihydro-2H-pyrazolo[3,4-d]pyrimidin-3-yl}-1-methyl-1H-pyrrole-3-carbonitrile;
5-{2-[(6-chloroquinolin-4-yl)methyl]-6-[(cyclopropylmethyl)amino]-5-methyl-4-oxo-4,5-dihydro-2H-pyrazolo[3,4-d]pyrimidin-3-yl}-1-methyl-1H-pyrrole-3-carbonitrile;
N-[2-[(6-chloroquinolin-4-yl)methyl] -3-(4-cyano-1-methyl-1H-pyrrol-2-yl) 5-methyl-4-oxo-4,5-dihydro-2H-pyrazolo[3,4-d]pyrimidin-6-yl]propane-1-sulfonamide;
ethyl 2-[(6-chloroquinolin-4-yl)methyl]-3-(4-cyano-1-methyl-1H-pyrrol-2-yl)-5-methyl-4-oxo-4,5-dihydro-2H-pyrazolo [3,4-d]pyrimidin-6-ylcarbamate;
N-[2-[(6-chloroquinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-N'-ethylurea;
5-[(4Z)-2-[(6-chloroquinolin-4-yl)methyl)-6-[(cyclopropylmethyl)amino]-5-methyl-4-(methylimino)-4,5-dihydro-2*H*-pyrazolo[3,4-*d*]pyrimidin-3- yl]-1-methyl-1*H*-Pyrrole-3-carbonitrile;
5-[(4Z,6Z)-2-[(6-chloroquinolin-4-yl)methyl]-7-(cyclopropylmethyl)-5-methyl-4,6-bis(methylimino)-4,5,6,7-tetrahydro-2H-pyrazolo[3,4-d]pyrimidin-3-yl]-1-methyl-1H-pyrrole-3-carbonitrile.

14. A compound having the structural formula (III): 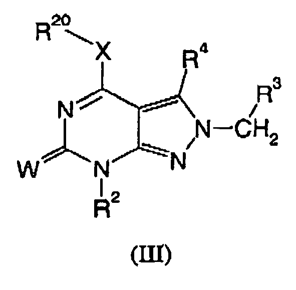 wherein,
X is S, O, NR²¹; or XR²⁰ is hydrogen;
W is S, O, or NR²⁰;
R² is H, optionally substituted alkyl, optionally substituted alkylcycloalkyl, optionally substituted alkylcycloalkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, or optionally substituted heterocycle;
R³ is a monocyclic or bicyclic, saturated or unsaturated, ring system comprising 0, 1, 2 or 3 heteroatoms independently selected from N, O, or S, the ring being substituted by 0, 1, 2 or 3 substituents selected from =O, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH2)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵,-R⁵-Het, -Het-R⁵, - Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, O, or S, the ring system being substituted by 0, 1, 2 or 3 substituents selected from B(OH)₂, vicinal -OCH₂CH₂O-, vicinal-OC₁₋₂ haloalkylO-, vicinal -OCH₂O-, vicinal -CH₂OCH₂O-, =O, halogen, -R^{b}OR^{a}, -SR^{a}, -OR^{a}, C₁₋₆ alkyl, C₁₋₆haloalkyl, -CN, -S(=O)ₙR^{e}, -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), - C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, - C(=NCN)R^{a}, -S(=O)₂NR^{a}R^{a},-NR^{a}S(-O)₂R^{a}, -S(=O)₂NR^{a}(R^{b}C(=O)NR^{a}R^{a}), - S(=O)₂NR^{a}(R^{b}C(=O)OR^{a}), aminocarbonyl, phenyl, benzyl; or R⁴ is represented by -(CH₂)ₙR⁵-Het, -(CH₂)ₙR^{d}, -Het, -Het-Het, R⁵, -R⁵-Het, -Het-R⁵, -Het-OR⁵, R⁵-R⁵, or -R⁵-OR⁵; or R⁴ is represented by C₁₋₆alky, -NC₁₋₆alkyl, or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)_{z}CH₂OH, -(CH₂)_{z}CO₂H, or -(CH₂)_{z}CO₂C₁₋₆alkyl wherein z is 1,2,3,4,5, or 6;
R⁵ is independently at each instance, phenyl substituted by 0, 1, 2, or 3 groups selected from halogen, C₁₋₆haloalkyl. -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, or - S(=O)₂NR^{a}R^{b}C(=O)OR^{a};
R²⁰ is, independently at each instance, H, -CN, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²¹ is, independently at each instance, H, -CN, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}; or
R²⁰ and R²¹ and the N to which they are attached in combination can also form a 3 to 10 member N-linked saturated or unsaturated heterocycle having either 1, or 2 heteroatoms independently selected from N, O, or S wherein the heterocycle is substituted with R^{e};
R^{a} is, independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}(=O)Rⁿ, -OC(=O)R^{a}, B(OH)₂, vicinyl -OCH₂CH₂O-, vicinyl-OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, O, or S;
R^{e} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
mis 1, 2 or 3;
n is 0, 1 or 2;
When "optionally substituted" is used, it refers to at least one substituent selected from cyclopropyl, halogen, nitro, cyano, hydroxy, trifluoromethyl, amino, carboxy, carboxamido, amidino, carbamoyl, mercapto, sulfamoyl, C₁₋₄alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄alkyl)sulfamoyl, N,N-C₁₋₄ alkyl)sulfamoyl, C₁₋₄ alkylsolfonylamino, and heterocyclic
or a pharmaceutically acceptable salt thereof.

15. A compound as recited in Claim 14 wherein:
R² is -(CH₂)₁₋₃cycloalkyl or -C₁₋₁₂alkyl wherein -(CH₂)₁₋₃cycloalkyl or -C₁₋₁₂alkyl is optionally substituted with 0, 1, 2 or 3 substituents selected from Het, S(=O)ₙR^{c}, halogen, -CN, -OR^{a}, -NR^{a}R^{a},-C(=O)OR^{a}, -C(=O)R^{a}, -C(=O)NR^{a}R^{a}, -OC(=O)C₁₋₄alkyl or -NR^{a}C(=O)C₁₋₄alkyl and n is 0, 1 or 2.

16. A compound as recited in Claim 14 wherein:
R³ is selected from formulas (i), (ii), (iii) or (iv) set forth below: 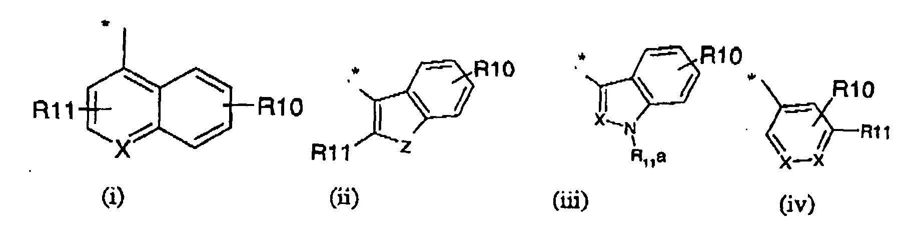
wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{e} and n=1 or 2.

17. A compound as recited in Claim 14 wherein:
R⁴ is selected from formulas (a) to (z) or (aa) or (ab) set forth below: 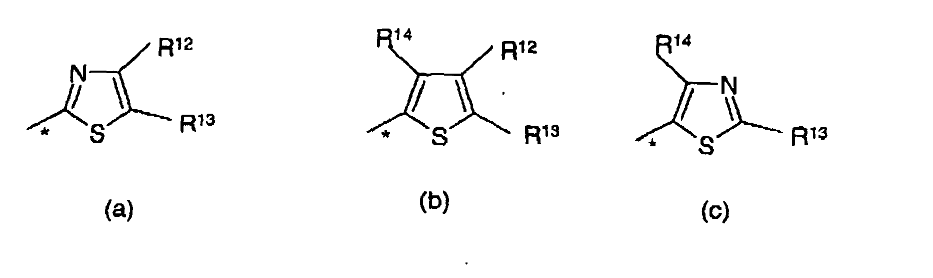 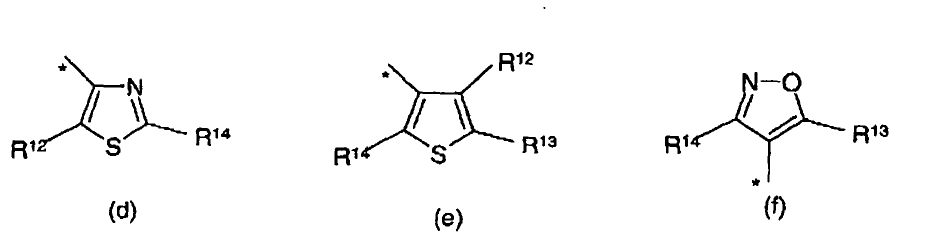 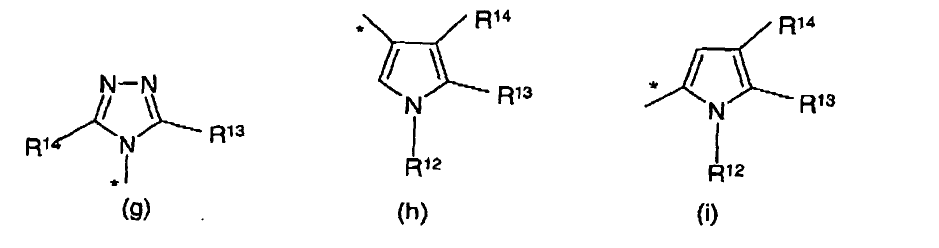 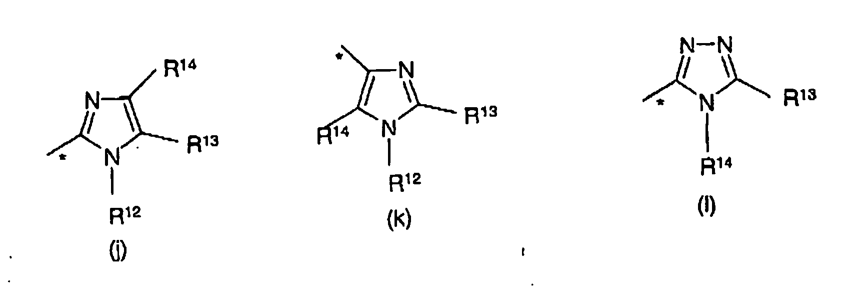 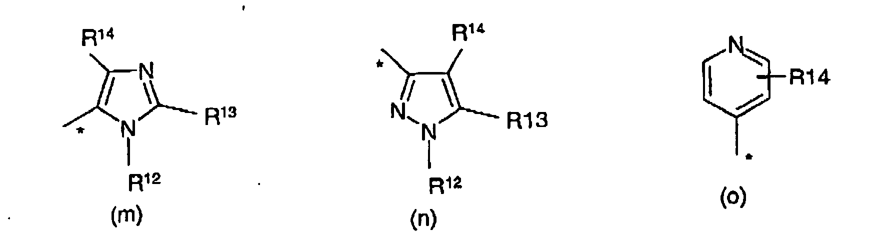 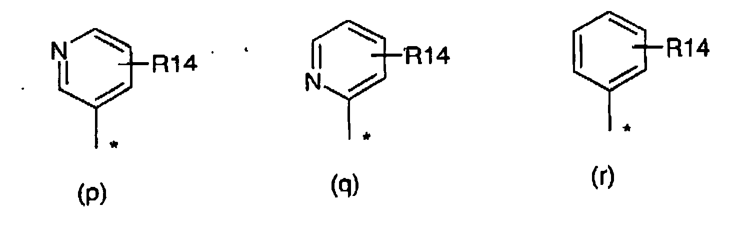 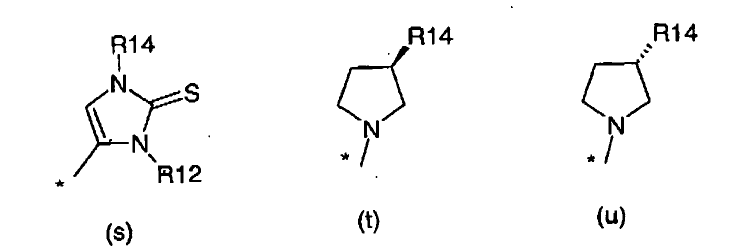 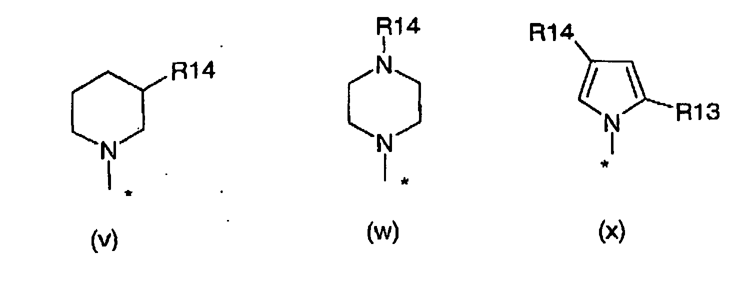 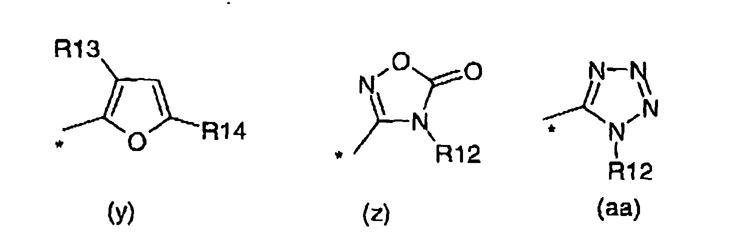 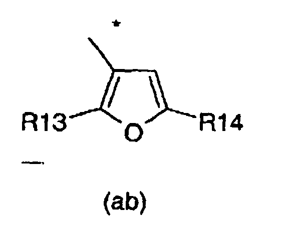
wherein * is the location wherein R⁴ is attached to the ring system and wherein
wherein R¹², R¹³ and R¹⁴ are each independently represented by H, Het, C₁₋₆alkyl, -CN, -NR^{a}R^{a}, - nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a},-C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b},-C(=NOR^{a})R^{a}, -S(=O)₂R^{a}, -S(=O)₂NR^{a}R^{a}, -S(=O)₂NR^{a}(R^{b}C(=O)NR^{a}R^{a}), or - S(=O)₂NR^{a}(R^{b}C(=O)OR^{a}.

18. A compound as recited in Claim 14 wherein:
X is S, O, or NR²¹; or XR²⁰ is hydrogen,
W is S, O, or NR²⁰;
R²⁰ is H, -CN, R³, -OR^{a}, -NR^{a}R^{a}, -Het, S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ and R²¹ and the N to which they are attached in combination can also form a 3 to 10 member N-linked saturated or unsaturated heterocycle having either 1, or 2 heteroatoms independently selected from N, O, or S wherein the heterocycle is substituted with R^{e};
R¹ is CH₃, CH₂CH₃, CH₂CN, CF₃, (CH₂)₂OH, cyclopropyl, isopropyl, CH₂CCH, (CH₂)₂N(CH₂)₂, (CH₂)₂N(C=NH)NH₂, -CH₂-2-pyridyl -CH₂-3-pyridyl, -CH₂-4-pyridyl, -(CH₂)₂-1-imidazolyl, -(CH₂)₂-1-pyrazolyl, -(CH₂)₂-1-piperidyl, -(CH₂)ₘ-(1-methylpiperidin-4-yl), -CH₂-(1-methylpiperidin-3-yl), -(CH₂)₂-(morpholin-4-yl),
R² is -CH₂CH₂CH₃, -CH₂-cyclopropyl, -CH₂CH(CH₃)₂, -CH₂CH₂CH₂F, -CH₂-cyclobutyl, -CH₂C(CH₃)₃, -CH₂CH₂CH(CH₃)₂, -CH₂CF₃, -CH₂-methylphenyl, -CH₂-phenol, - CH₂-(3,5-dimethylisoxazol-4-yl), -CH₂-S-phenyl, -CH₂-phenylcarboxyl, or -CH₂SCF₃;
R³ is selected from formulas (i), (ii), (iii) or (iv) set forth below: 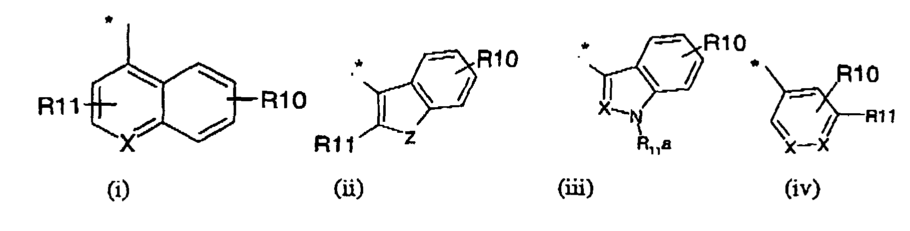 wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.
R⁴ is selected from formulas (a) to (z) or (aa) or (ab) set forth below: 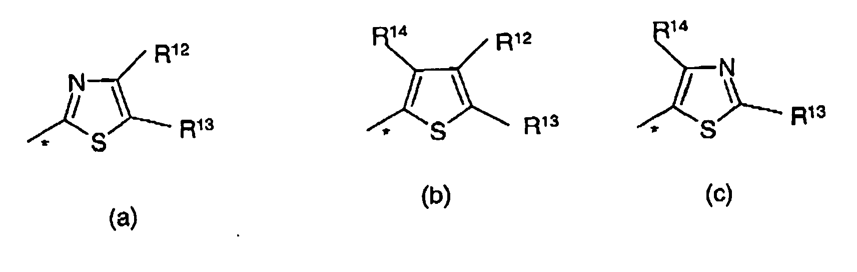 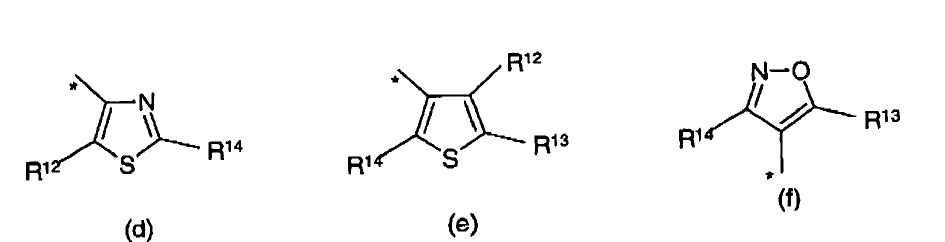 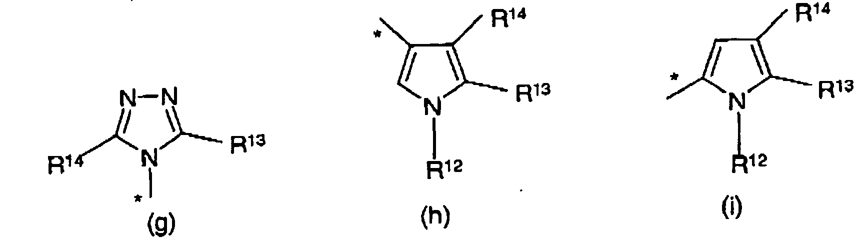 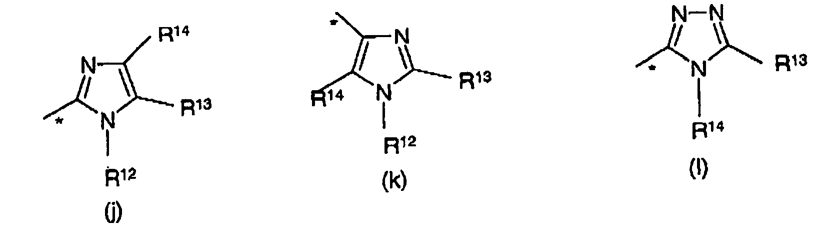 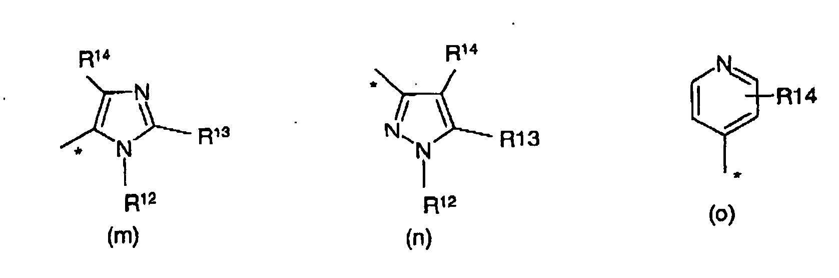 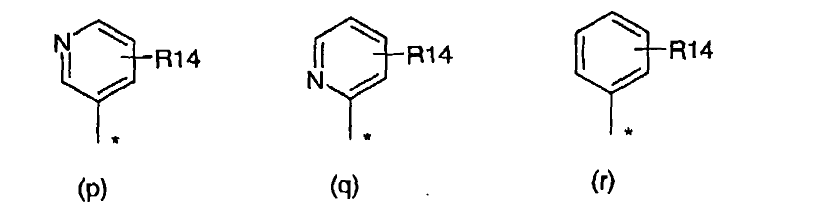 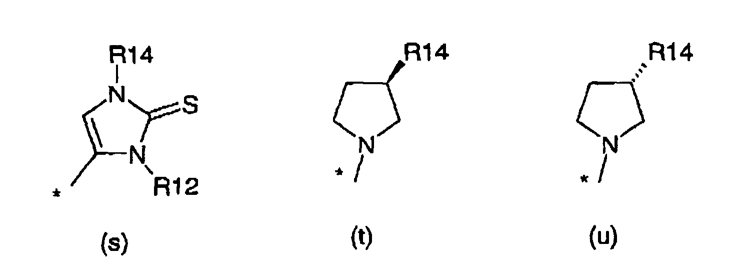 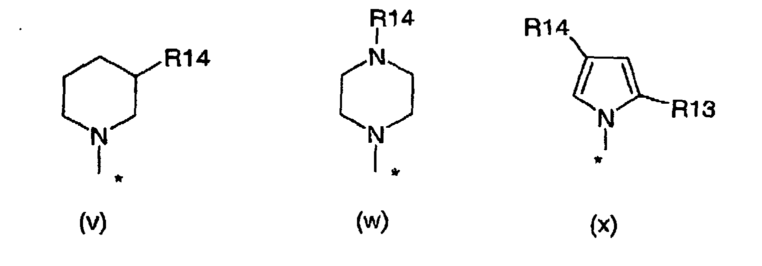 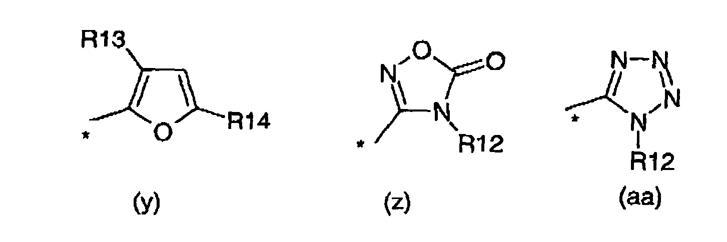 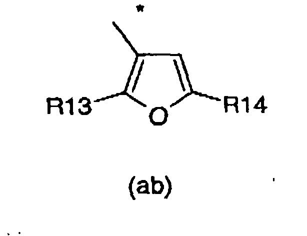
wherein * is the location wherein R⁴ is attached to the ring system and wherein
wherein R¹², R¹³ and R¹⁴ are each independently represented by H, Het, C₁₋₆alkyl, -CN, -NR^{a}R^{a}, - nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a},-C(=O)R^{a}, -OC(=O)R^{a}, -C(=O)R^{a}-SR^{a}, =S, -NR^{a}C(=O)R^{a}, -NR^{a}C(=O)OR^{a} , -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), or - S(=O)₂NR^{a}(R^{b}C(=O)OR^{a}.

19. A compound of formula (III) selected from:
4-amino-7-isobutyl-2-(1-naphthylmethyl)-3-pyridin-4-yl-2,7-dihydro-6H-pyrazolo[3,4-d]pyrimidin-6-one;
7-isobutyl-4-(methylamino)-2-(1-naphthylmethyl)-3-pyridin-4-yl-2,7-dihydro-6H-pyrazolo[3,4-d]pyrimidin-6-one;
4-(dimethylamino)-7-isobutyl-2-(1-naphthylmethyl)-3-pyridin-4-yl-2,7-dihydro-6H-pyrazolo[3,4-d]pyrimidin-6-one;
7-isobutyl-4-(4-methylpiperazin-1-yl)-2-(1-naphthylmethyl)-3-pyridin-4-yl-2,7-dihydro-6H-pyrazolo[3,4-d]pyrimidin-6-one;
4-amino-2-[(6-chloroquinolin-4-yl)methyl]-7-isobutyl-3-(1-methyl-1H-pyrrol-2-yl)-2,7-dihydro-6H-pyrazolo[3,4-d]pyrimidin-6-one;
5-{4-amino-2-[(6-chloroquinolin-4-yl)methyl]-7-isobutyl-6-oxo-6,7-dihydro-2H-pyrazolo[3,4-d]pyrimidin-3-yl}-1-methyl-1H-pyrrole-3-carbonitrile;
5-[2-[(6-chloroquinolin-4-yl)methyl]-7-isobutyl-4-(methylamino)-6-oxo-6,7-dihydro-2H-pyrazolo[3,4-d]pyrimidin-3-yl]-1-methyl-1H-pyrrole-3-carbonitrile;
5-[2-[(6-chloroquinolin-4-yl)methyl]-4-(dimethylamino)-7-isobutyl-6-oxo-6,7-dihydro-2H-pyrazolo[3,4-d]pyrimidin-3-yl]-1-methyl-1H-pyrrole-3-carbonitrile;
5-[2-[(6-chloroquinolin-4-yl)methyl]-7-isobutyl-6-oxo-4-(propylamino)-6,7-dihydro-2H-pyrazolo[3,4-d]pyrimidin-3-yl]-1-methyl-1H-pyrrole-3-carbonitrile;
5-{2-[(6-chloroquinolin-4-yl)methyl]-4-[(2-hydroxyethyl)amino]-7-isobutyl-6-oxo-6,7-dihydro-2H-pyrazolo[3,4-d]pyrimidin-3-yl}-1-methyl-1H-pyrrole-3-carbonitrile
5-[2-[(6-chloroquinolin-4-yl)methyl]-4-(hydroxyamino)-7-isobutyl-6-oxo-6,7-dihydro-2H-pyrazolo[3,4-d]pyrimidin-3-yl]-1-methyl-1H-pyrrole-3-carbonitrile;
5-[2-[(6-chloroquinolin-4-yl)methyl]-4-(cyclopropylamino)-7-isobutyl-6-oxo-6,7-dihydro-2H-pyrazolo[3,4-d]pyrimidin-3-yl]-1-methyl-1H-pyrrole-3-carbonitrile;
5-{2-[(6-chloroquinolin-4-yl)methyl]-4-hydrazino-7-isobutyl-6-oxo-6,7-dihydro-2H-pyrazolo[3,4-d]pyrimidin-3-yl}-1-methyl-1H-pyrrole-3-carbonitrile;
5-[2-[(6-chloroquinolin-4-yl)methyl]-4-(2,2-dimethylhydrazino)-7-isobutyl-6-oxo-6,7-dihydro-2H-pyrazolo[3,4-d]pyrimidin-3-yl]-1-methyl-1H-pyrrole-3-carbonitrile;
N-[2-[(6-chloroquinolin-4-yl)methyl]-3-(4-cyano-1-methyl-1H-pyrro-2-yl)-7-isobutyl-6-oxo-6,7-dihydro-2H-pyrazolo[3,4-d]pyrimidin-4-yl]acetamide;
5-[2-[(6-chloroquinolin-4-yl)methyl]-7-(cyclopropylmethyl)-4-(methylthio)-6-oxo-6,7-dihydro-2H-pyrazolo[3,4-d]pyrimidin-3-yl]-1-methyl-1H-pyrrole-3-carbonitrile;
5- {2-[(6-chloroquinolin-4-yl)methyl]-7-(cyclopropylmethyl)-4-[(2-hydroxybutyl)amino]-6-oxo-6,7-dihydro-2H-pyrzolo[3,4-d]pyrimidin-3-yl}-1-methyl-1H-pyrrole-3-carbonitrile ;
5-(2-[(6-chloroquinolin-4-yl)methyl]-7-(cyclopropylmethyl)-4-{[(2R)-2-hydroxypropyl]amino}-6-oxo-6,7-dihydro-2H-pyrazolo[3,4-d]pyrimidin-3-yl)-1-methyl-1H-pyrrole-3-carbonitrile;
5-[2-[(6-chloroquinolin-4-yl)methyl]-7-(cyclopropylmethyl)-4-methoxy-6-oxo-6,7-dihydro-2*H-*pyrazolo[3,4-*d*]pyrimidin-3-yl]-1-methyl-1*H*-pyrrole-3-carbonitrile;
5-[2-[(6-chloroquinolin-4-yl)methyl]-7-(cyclopropylmethyl)-6-oxo-4-(1*H*-pyrrol-1-yl)-6,7-dihydro-2*H*-pyrazolo[3,4-d]pyrimidin-3-yl]-1methy-1*H*-pyrrolre-3-carbonitrile;
5-[(6Z)-2-[(6-chloroqumolin-4-yl)methyl]-7-(cyclopropylmethyl)-4-(methylamino)-6-(methylimino)-6,7-dihydro-2*H*-pyrazolo[3,4-d]pyrimidin-3-yl]-1-methyl-1*H*-pyrrole-3-carbonitrile;
5-[4-amino-2-[(6-chloroquinolin-4-yl)methyl]-7-(cyclopropylmethyl)-6-oxo-6,7-dihydro-2*H-*pyrazolo[3,4-d]pyrimidin-3-yl]-1-methyl-1*H*-pyrrole-3-carbonitrile.

20. A compound having the structural formula (IV): 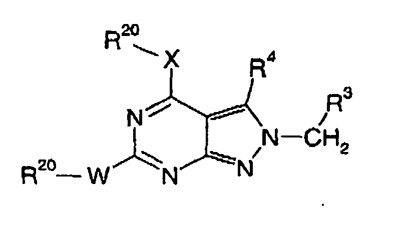 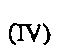 wherein,
X is S, O, NR²¹; or XR²⁰ is hydrogen;
W is S, O, or NR²¹;
R³ is a monocyclic or bicyclic, saturated or unsaturated, ring system comprising 0, 1, 2 or 3 heteroatoms independently selected from N, 0, or S, the ring being substituted by 0, 1, 2 or 3 substituents selected from =O, halogen, -OR^{a}, C₁₋₆alkyl, C₁₋₆haloalkyl, -CN, nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, aminocarbonyl, phenyl, benzyl; or R³ is represented by -Het, -Het-Het, R⁵,-R⁵-Het, -Het-R⁵, - Het-O-R⁵, -R⁵-R⁵, -R⁵-OR⁵;
R⁴ is a monocyclic or bicyclic, saturated or unsaturated, ring system, or a vicinal-fused derivative thereof, which may contain from 5 to 12, preferably 5 to 10, ring atoms, 0, 1, 2, 3 or 4 of which are heteroatoms independently selected from N, O, or S, the ring system being substituted by 0, 1, 2 or 3 substituents selected from B(OH)₂, vicinal -OCH₂CH₂O-, vicinal -OC₁₋₂ haloalkylO-, vicinal -OCH₂O-, vicinal -CH₂OCH₂O-, =O, halogen, -R^{b}OR^{a}, -SR^{a}, -OR^{a}, C₁₋₆ alkyl, C₁₋₆haloalkyl, -CN, -S(=O)ₙR^{c}, -O(CH₂)ₘHet, -O(CH₂)ₘC(=O)Het, O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), - C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, - C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), - S(=O)₂NR^{a}(R^{b}C(=O)OR^{a}), aminocarbonyl, phenyl, benzyl; or R⁴ is represented by -(CH₂)ₙR⁵-Het, -(CH₂)ₙR^{d}, -Het, -Het-Het, R⁵, -R⁵-Het, -Het-R⁵, -Het-OR⁵, R⁵-R⁵, or -R⁵-OR⁵; or R⁴ is represented by C₁₋₆alky, -NC₁₋₆alkyl, or -N(C₁₋₆alkyl)₂ wherein the C₁₋₆alkyl, -NC₁₋₆alkyl, -N(C₁₋₆alkyl) are substituted by 0, 1 or 2 substituents selected from R^{a}, OR^{a}, halogen or phenyl wherein R⁴ is not -(CH₂)_{z}CH₃, -(CH₂)_{z}CH₂OH, -(CH₂)₂CO₂H, or -(CH₂)_{z}CO₂C₁₋₆alkyl wherein z is 1,2,3,4,5, or 6;
R⁵ is independently at each instance, phenyl substituted by 0, 1, 2, or 3 groups selected from halogen, C₁₋₆haloalkyl, -OC₁₋₆haloalkyl, C₁₋₆alkyl, -CN, nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, or - S(=O)₂NR^{a}R^{b}C(=O)OR^{a};
R²⁰ is, independently at each instance, H, -CN, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}, optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle, wherein such substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²¹ is, independently at each instance, H, -CN, -S(=O)ₙR^{c}, -C(=O)R^{a}, -C(=O)NR^{a}R^{a}, -C(=O)OR^{a}, -NR^{a}C(=O)R^{a}, or -OC(=O)R^{a}; optionally substituted alkyl, optionally substituted alkenyl, optionally substituted alkynyl, optionally substituted cycloalkyl, optionally substituted cycloalkenyl, optionally substituted cycloalkynyl, optionally substituted aryl, optionally substituted alkoxy, optionally substituted amino, optionally substituted heterocycle wherein such substitution is selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic;
R²⁰ and R²¹ and the N to which they are attached in combination can also form a 3 to 10 member N-linked saturated or unsaturated heterocycle having either 1, or 2 heteroatoms independently selected from N, 0, or S wherein the heterocycle is substituted with R^{c};
R^{a} is, independently at each instance, H, C₁₋₆alkyl, -C(=O)C₁₋₆alkyl, C₁₋₄haloalkyl, phenyl, benzyl, or 5 or 6-memebered ring, saturated or unsaturated heterocycle containing 1,2,3, or 4 heteroatoms independently selected from N, 0 or S;
R^{b} is, independently at each instance, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;
R^{c} is C₁₋₆alkyl, C₁₋₄haloalkyl, phenyl or benzyl;
R^{d} is phenyl substituted by 0, 1 or 2 groups selected from -CN, halogen, nitro, C₁₋₆alkyl, C₁₋₄haloalkyl, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, vicinyl -OCH₂CH₂O-, vicinyl -OC₁₋₂haloalkylO-, vicinyl -OCH₂O-, vicinyl -CH₂OCH₂O-, phenyl, benzyl and a 5- or 6-membered ring, saturated or unsaturated heterocycle containing 1, 2, 3 or 4 heteroatoms independently selected from N, 0, or S;
R^{e} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, 0 or S;
m is 1, 2 or 3;
n is 0, 1 or 2;
When "optionally substituted" is used, it refers to at least one substituent selected from cyclopropyl, halogen, nitro, cyano, hydroxy, trifluoromethyl, amino, carboxy, carboxamido, amidino, carbamoyl, mercapto, sulfamoyl, C₁₋₄ alkyl, C₂₋₄ alkenyl, C₂₋₄ alkynyl, C₁₋₄ alkoxy, C₁₋₄ alkanoyl, C₁₋₄ alkanoyloxy, NH(C₁₋₄ alkyl), N(C₁₋₄ alkyl)₂, C₁₋₄ alkanoylamino, (C₁₋₄ alkanoyl)₂amino, N-(C₁₋₄ alkyl)carbamoyl, N,N-(C₁₋₄ alkyl)₂carbamoyl, (C₁₋₄)S, (C₁₋₄ alkyl)S(O), (C₁₋₄alkyl)S(O)₂, (C₁₋₄) alkoxycarbonyl, N-(C₁₋₄ alkyl)sulfamoyl, N,N-C₁₋₄ alkyl)sulfamoyl, C₁₋₄ alkylsolfonylamino, and heterocyclic
or a pharmaceutically acceptable salt thereof.

21. A compound as recited in Claim 20 wherein:
R³ is selected from formulas (i), (ii), (iii) or (iv) set forth below: 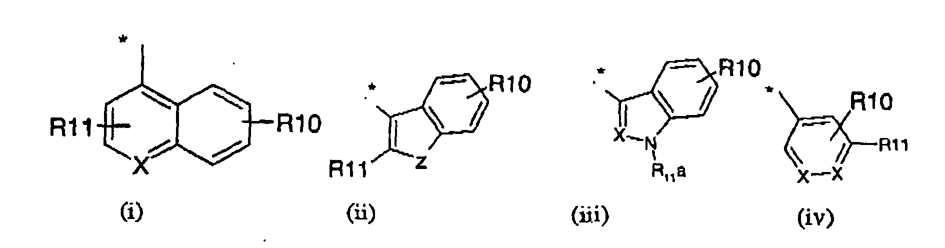
wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.

22. A compound as recited in Claim 20 wherein:
R⁴ is selected from formulas (a) to (z) or (aa) or (ab) set forth below: 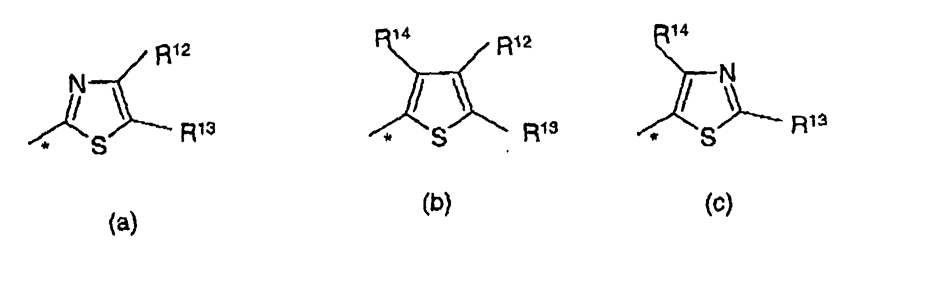 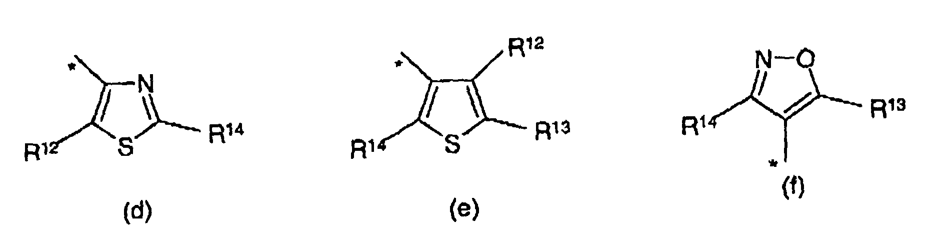 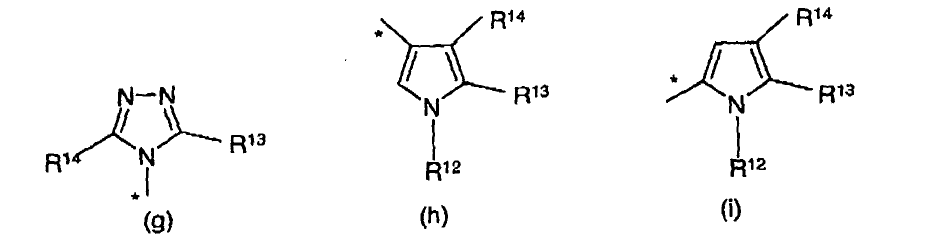 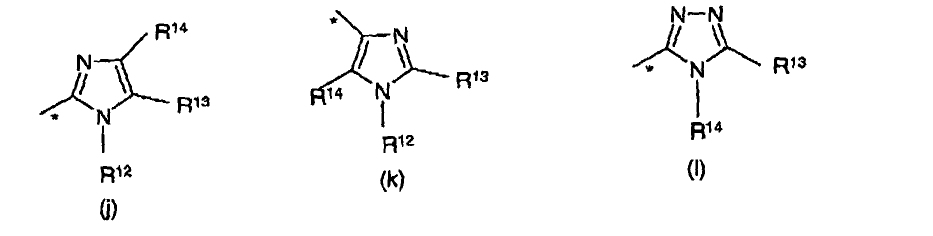 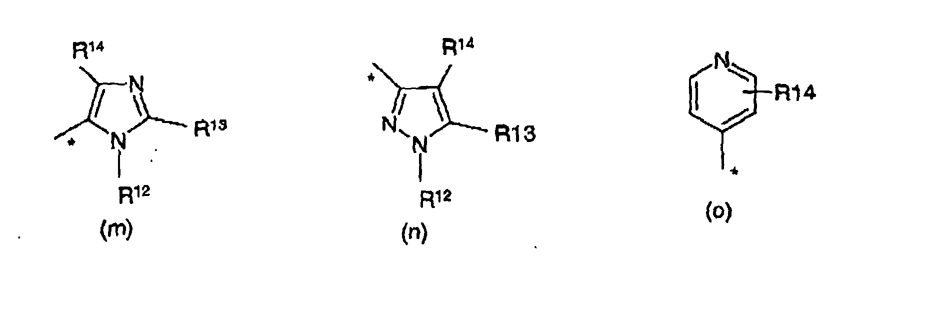 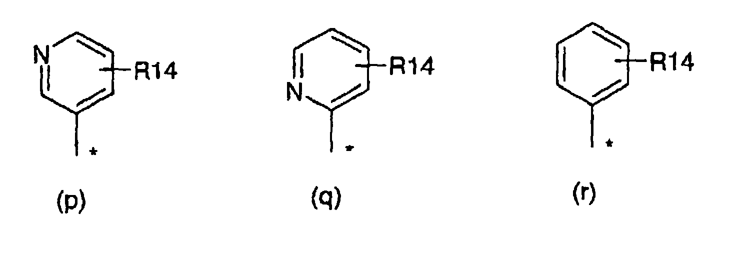 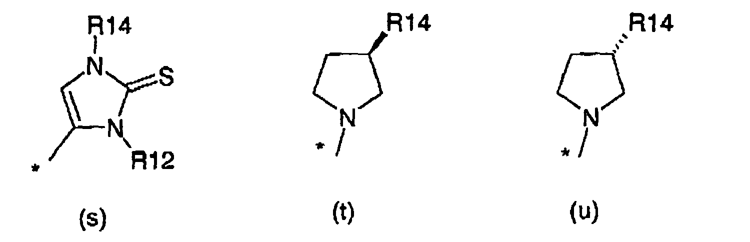 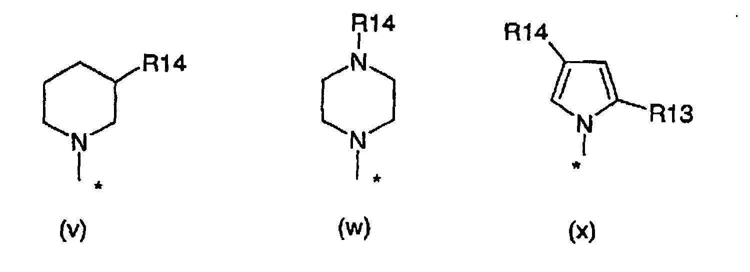 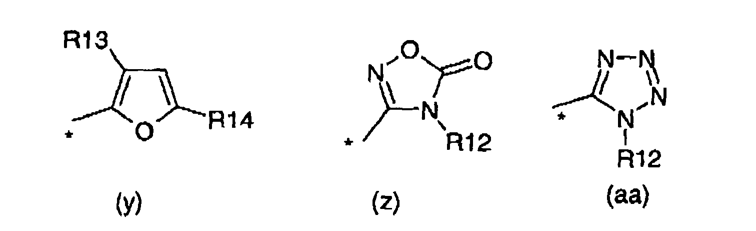 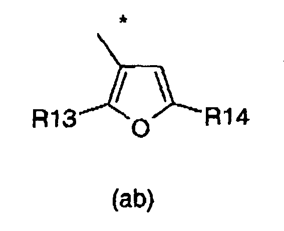
wherein * is the location wherein R⁴ is attached to the ring system and wherein
wherein R¹² , R¹³ and R¹⁴ are each independently represented by H, Het, C₁₋₆alkyl, -CN, -NR^{a}R^{a}, - nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(-O)NR^{a}OR^{a}, -C(=O)R^{b},NR^{a}R^{a}, -C(=NOR^{a}R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), or - S(=O)₂NR^{a}(R^{b}C(=O)OR^{a}.

23. A compound as recited in Claim 20 wherein:
X is S, 0, or NR²¹; or X-R²⁰ is hydrogen
W is S, O, or NR²¹;
R¹ is CH₃, CH₂CH₃, CH₂CN, CF₃, (CH₂)₂OH, cyclopropyl, isopropyl, CH₂CCH, (CH₂)₂N(CH₂)₂, (CH₂)₂N(C=NH)NH₂, -CH₂-2-pyridyl, -CH₂-3-pyridyl, -CH₂-4-pyridyl, -(CH₂)₂-1-imidazolyl, -(CH₂)₂-1-pyrazolyl, -(CH₂)₂-1-piperidyl, -(CH₂)ₘ-(1-methylpiperidin-4-yl), -CH₂-(1-methylpiperidin-3-yl),-(CH₂)₂-(morpholin-4-yl),
R² is -CH₂CH₂CH₃, -CH₂-cyclopropyl, -CH₂CH(CH₃)₂, -CH₂CH₂CH₂F, -CH₂-cyclobutyl, -CH₂C(CH₃)₃, -CH₂CH₂CH(CH₃)₂, -CH₂CF₃, -CH₂-methylphenyl, -CH₂-phenol, - CH₂-(3,5-dimethylisoxazol-4-yl), -CH₂-S-phenyl, -CH₂-phenylcarboxyl, or -CH₂SCF₃;
R³ is selected from formulas (i), (ii), (iii) or (iv) set forth below: 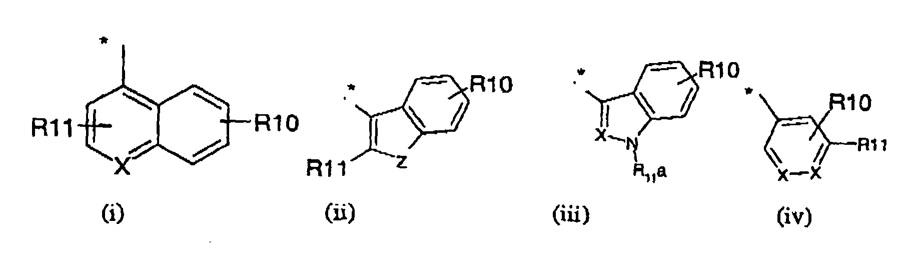 wherein * is the location where (i) or (ii) or (iii) or (iv) is attached to structural formula (I), and X is C or N; and Z is O or S, wherein R¹⁰ is at any position on the ring and R¹⁰ and R¹¹ are independently at each instance H, R^{a}, halogen, -CN, nitro, OR^{a}, CF₃, -NR^{a}R^{a}, -C(=O)OR^{a}, - C(=O)R^{a}, -C(=O)NR^{a}R^{a}, -OC(=O)C₁₋₄alkyl, -NR^{a}C(=O)C₁₋₄alkyl or -S(=O)ₙR^{c}; and wherein R^{11a} is R^{a}, -S(=O)₂NR^{a}R^{a} or -S(=O)ₙR^{c} and n=1 or 2.
R⁴ is selected from formulas (a) to (z) or (aa) or (ab) set forth below: 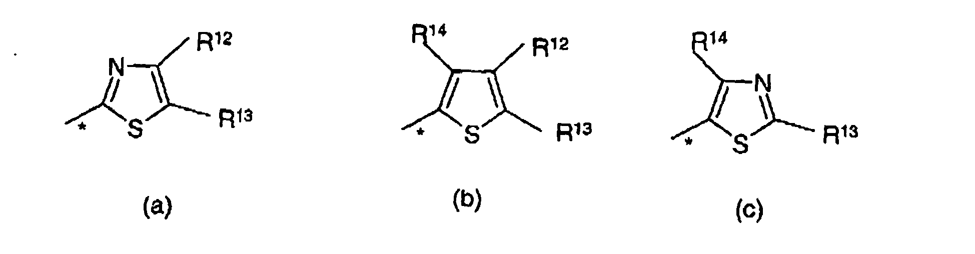 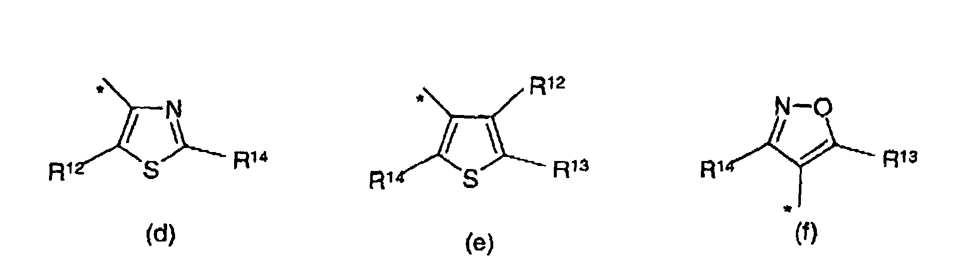 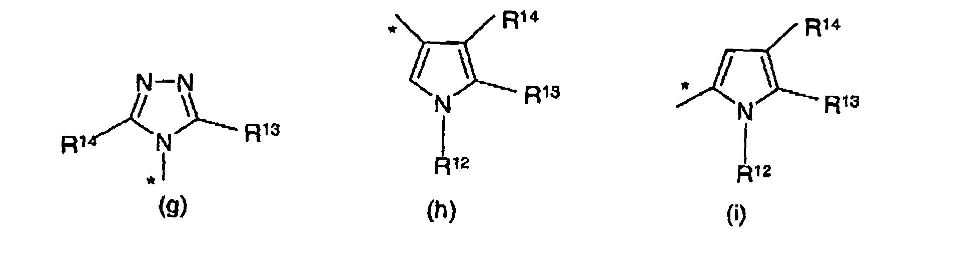 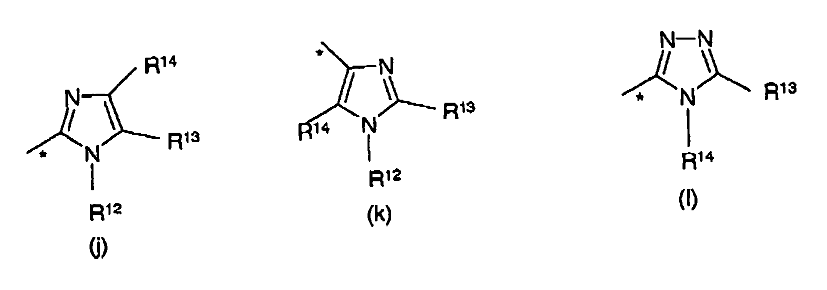 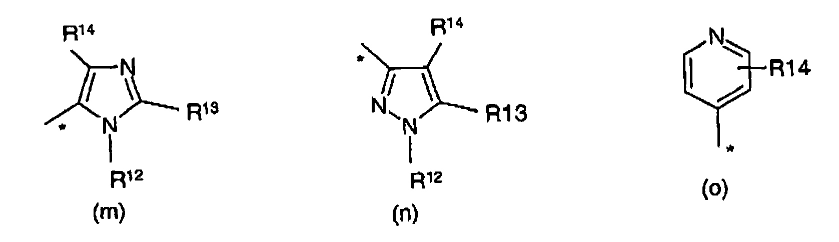 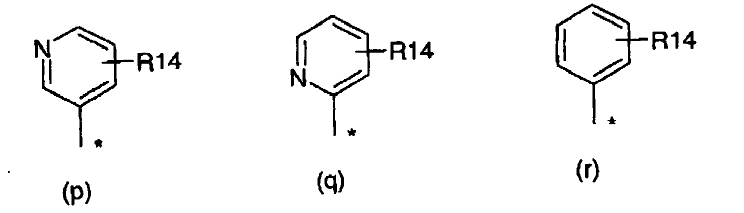 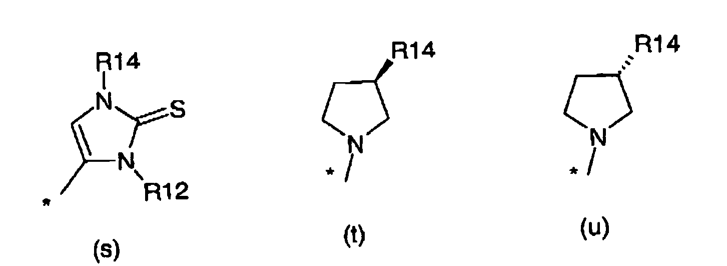 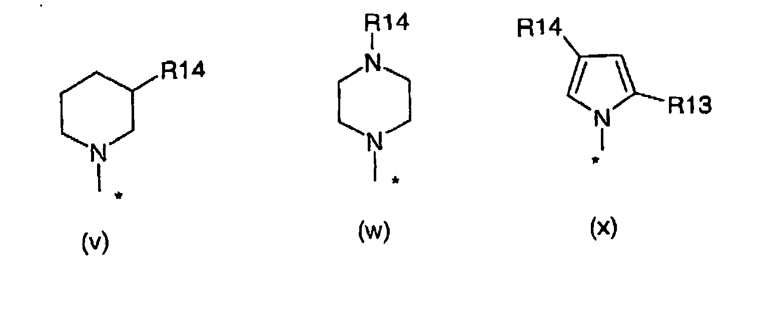 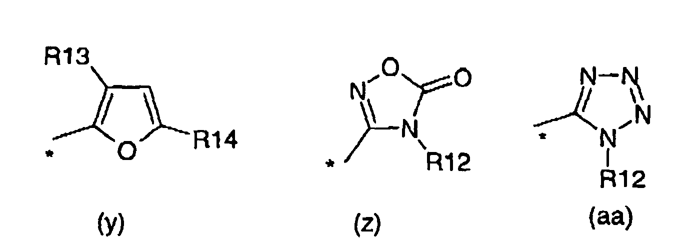 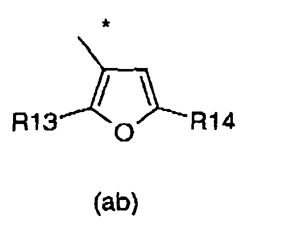 wherein * is the location wherein R⁴ is attached to the ring system and wherein
wherein R¹², R¹³ and R¹⁴ are each independently represented by H, Het, C₁₋₆alkyl, -CN, -NR^{a}R^{a}, - nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b},-C(=NOR^{a})R^{a}, -S(=O)₂R^{a}, -S(=O)₂NR^{a}R^{a}, -S(=O)₂NR^{a}(R^{b}C(=O)NR^{a}R^{a}), or - S(=O)₂NR^{a}(R^{b}C(=O)OR^{a}.
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ is H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, or -OC(=O)R^{a};
R²⁰ and R²¹ and the N to which they are attached in combination can also form a 3 to 10 member N-linked saturated or unsaturated heterocycle having either 1, or 2 heteroatoms independently selected from N, O, or S wherein the heterocycle is substituted with R^{c};
R^{e} is independently at each instance, H, C₁₋₆alkyl, -C(=O)C₁₋₄alkyl, C₁₋₄haloalkyl, phenyl, benzyl, or 5 or 6-memebered ring, saturated or unsaturated heterocycle containing 1,2,3, or 4 heteroatoms independently selected from N, O or S;

24. A compound of formula (IV) selected from:
5-[2-[(6-chloroquinolin-4-yl)methyl]-6-[(cyclopropylmethyl)amino]-4-(methylamino)-2*H-*pyrazolo[3,4-*d*]pyrimidin-3-yl]-1-methyl-1*H*-pyrrole-3-carbonitrile;
*N*-{3-(4-acetyl-1-methyl-1*H*-pyrrol-2-yl)-2-[(6-chloroquinolin-4-yl)methyl]-4-methoxy-2*H-*pyrazolo[3,4-*d*]pyrimidin-6-yl}-2-cyclopropylacetamide.

25. A compound according to any one of claims 1 to 24, for use as a medicament.

26. The use of a compound as defined in any one of claims 1 to 24, in the manufacture of a medicament for the treatment or prophylaxis of disorders associated with *H. pylori* infection.

27. A pharmaceutical composition comprising a compound as defined in any one of claims 1 to 24, together with at least one pharmaceutically acceptable carrier, diluent or excipient.

## Patentansprüche

1. Verbindung mit der Strukturformel (I): 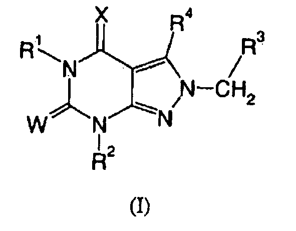 worin
X S, O oder NR²⁰ ist, mit der Maßgabe, dass wenn W 0 ist, dann X nicht O ist,
X und die Doppelbindung, an welche es gebunden ist, durch 2 Wasserstoffatome ersetzt werden kann,
W S, O oder NR²⁰ ist, mit der Maßgabe, dass wenn X O ist, dann W nicht O ist;
R¹ H, wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, Hydroxy, Amino oder wahlweise substituierter Heterocyclus ist;
R² H, wahlweise substituiertes Alkyl, wahlweise substituiertes Alkylcycloalkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino oder wahlweise substituierter Heterocyclus ist;
R³ ein monocyclisches oder bicyclisches, gesättigtes oder ungesättigtes Ringsystem ist, umfassend 0, 1, 2 oder 3 Heteroatome, welche unabhängig gewählt werden aus N, O oder S, wobei der Ring durch 0, 1, 2 oder 3 Substituenten substituiert ist, die gewählt sind aus =O, Halogen, -OR^{a}, C₁₋₆-Alkyl, C₁₋₆-Halogenalkyl, -CN, Nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, Aminocarbonyl, Phenyl, Benzyl; oder R³ wird durch -Het, -Het-Het, R⁵, -R⁵-Het, -Het-R⁵, -Het-O-R⁵, -R⁵-R⁵, -R⁵-OR⁵ angegeben;
R⁴ ein monocyclisches oder bicyclisches, gesättigtes oder ungesättigtes Ringsystem oder ein Vizinalkondensiertes Derivat davon ist, welches 5 bis 12, vorzugsweise 5 bis 10, Ringatome enthalten kann, wobei 0, 1, 2, 3 oder 4 davon Heteroatome sind, die unabhängig gewählt werden aus N, O oder S, wobei das Ringsystem durch 0, 1, 2 oder 3 Substituenten substituiert wird, welche gewählt werden aus B(OH)₂, vizinalem -OCH₂CH₂O-, vizinalem -OC₁₋₂-Halogenalkyl-O-, vizinalem -OCH₂O-, vizinalem -CH₂OCH₂O-, =O, Halogen, -R^{b}OR^{a}, -SR^{a}, -OR^{a}, C₁₋₆-Alkyl, C₁₋₆-Halogenalkyl, -CN, -S(=O)ₙR^{c} , -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het),-C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, - C(=NCN)R^{a}, -S(=O)₂NR^{a}R^{a}, -NR^{a}S(=O)₂R^{a}, -S(=O)₂NR^{a}R^{b}C(=O)NR^{a}R^{a}), - S(=O)₂NR^{a}(R^{b}C(=O)OR^{a} Aminocarbonyl, Phenyl, Benzyl; oder R⁴ wird durch - (CH₂)ₙR⁵-Het, -(CH₂)ₙR^{d}, -Het, -Het-Het, R⁵, -R⁵-Het, -Het-R⁵, -Het-OR⁵, R⁵-R⁵ oder -R⁵-OR⁵ angegeben; oder R⁴ wird durch C₁₋₆-Alkyl, -NC₁₋₆-Alkyl oder -N(C₁₋₆-Alkyl)₂ angegeben, wobei C₁₋₆-Alkyl, -NC₁₋₆-Alkyl, -N(C₁₋₆-Alkyl) durch 0, 1 oder 2 Substituenten substituiert sind, die aus R^{a}, OR^{a}, Halogen oder Phenyl gewählt sind, wobei R⁴ nicht -(CH₂)_{z}CH₃, -(CH₂)_{z}CH₂OH, -(CH₂)_{z}CO₂H, oder -(CH₂)_{z}-CO₂C₁₋₆-Alkyl ist, wobei z 1, 2, 3, 4, 5 oder 6 ist; R⁵ unabhängig bei jedem Auftreten Phenyl, das durch 0, 1, 2 oder 3 Gruppen substituiert ist, ist, gewählt aus Halogen, C₁₋₆-Halogenalkyl, -OC₁₋₆-Halogenalkyl, C₁₋₆-Alkyl, -CN, Nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a},-C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, oder - S(=O)₂NR^{a}R^{b}C(=O)OR^{a};
R²⁰ unabhängig bei jedem Auftreten H, -CN, wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, wahlweise substituierter Heterocyclus, -S(=O)ₙR^{c}, -C(=O)R^{a}, -C(=O)NR^{a}R^{a}, -C(=O)OR^{a}, -NR^{a}C(=O)R^{a}, oder-OC(=O)R^{a}; ist R^{a} unabhängig bei jedem Auftreten H, C₁₋₆-Alkyl, -C(=O)C₁₋₄-Alkyl , C₁₋₄-Halogenalkyl, Phenyl, Benzyl, oder ein 5- oder 6-gliedriger Ring, gesättigter oder ungesättigter Heterocyclus, welcher 1, 2, 3 oder 4 Heteroatome enthält, die unabhängig aus N, O oder S gewählt werden, ist;
R^{b} unabhängig bei jedem Auftreten C₁₋₆-Alkyl, -C(=O)-C₁₋₄-Alkyl, C₁₋₄-Halogenalkyl, Phenyl, Benzyl, oder ein 5- oder 6-gliedriger Ring, gesättigter oder ungesättigter Heterocyclus, welcher 1, 2, 3 oder 4 Heteroatome enthält, die unabhängig aus N, O oder S gewählt werden, ist;
R^{c} C₁₋₆-Alkyl, C₁₋₄-Halogenalkyl, Phenyl oder Benzyl ist;
R^{d} Phenyl ist, welches durch 0, 1 oder 2 Gruppen substituiert ist, gewählt aus -CN, Halogen, Nitro, C₁₋₆-Alkyl, C₁₋₄-Halogenalkyl, -OH, -OR^{c}, -NR^{a}R^{a}, -S-(=O)nR^{c}, -C(=O)NR^{a}R^{a}, -C(=O)OR^{a}, -NR^{a}C(=O)R^{a}; -OC(=O)R^{a}, B(OH)₂, Vizinyl-OCH₂CH₂O-, Vizinyl-OC₁₋₂Halogenalkyl-O-, Vizinyl-OCH₂O-, Vizinyl-CH₂OCH₂O-, Phenyl, Benzyl und einem 5- oder 6- gliedrigem Ring, gesättigtem oder ungesättigtem Heterocyclus, welcher 1, 2, 3 oder 4 Heteroatome enthält, unabhängig gewählt aus N, O oder S;
m 1, 2 oder 3 ist;
n 0, 1 oder 2 ist.
Wenn "wahlweise substituiert" verwendet wird, bezieht es sich auf mindestens einen Substituenten, der aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl, C₂₋₄-Alkenyl, C₂₋₄-Alkinyl, C₁₋₄ -Alkoxy, C₁₋₄-Alkanoyl, C₁₋₄-Alkanoyloxy, NH (C₁₋₄ - Alkyl), N(C₁₋₄-Alkyl)₂, C₁₋₄-Alkanoylamino, (C₁₋₄-Alkanoyl)₂-amino, N-(C₁₋₄-Alkyl)carbamoyl, N,N-(C₁₋₄-Alkyl)₂-carbamoyl, (C₁₋₄)S, (C₁₋₄-Alkyl)S(O), (C₁₋₄-Alkyl)S(O)₂, (C₁₋₄)-Alkoxycarbonyl, N-(C₁₋₄-Alkyl)sulfamoyl, N,N-(C₁₋₄-Alkyl)sulfamoyl, C₁₋₄-Alkylsulfonylamino und einem Heterocyclus gewählt wird,
oder ein pharmazeutisch annehmbares Salz davon.

2. Verbindung wie in Anspruch 1 angegeben, wobei:
R¹ H oder C₁₋₆-Alkyl oder (CH₂)ₙ-Cycloalkyl oder -(CH₂)₁₋₂Het ist, wobei C₁₋₆-Alkyl oder (CH₂)ₙ-Cycloalkyl oder -(CH₂)₁₋₂Het wahlweise durch 1, 2 oder 3 Substituenten substituiert ist, die aus Het, Halogen, -CN, OR^{a}, -NR^{a}R^{a}, -C(=O)OR^{a}, -C(=O)NR^{a}R^{a}, -OC(=O)C₁₋₄-Alkyl, -S(=O)ₙR^{c}, -S(=O)ₙNR^{a}R^{a} oder -NR^{a}C-(=O)C₁₋₄-Alkyl gewählt werden und n 0, 1 oder 2 ist.

3. Verbindung, wie in Anspruch 1 angegeben, worin:
R²-(CH₂)₁₋₃-Cycloalkyl oder -C₁₋₁₂-Alkyl ist, worin -(CH₂)₁₋₃-Cycloalkyl oder -C₁₋₁₂-Alkyl wahlweise durch 0, 1, 2 oder 3 Substituenten substituiert ist, welche aus Het, S (=O) ₙR^{c}, -S(=O)ₙNR^{a}R^{a}, Halogen, -CN, -OR^{a}, -NR^{a}R^{a}, -C(=O)OR^{a}, -C(=O)R^{a}, -C(=O)NR^{a}R^{a}, -OC-(=O) C₁₋₄-Alkyl oder -NR^{a}C(=O)C₁₋₄-Alkyl gewählt wird, und n 0, 1 oder 2 ist.

4. Verbindung, wie in Anspruch 1 angeführt, wobei:
R³ aus den unten dargelegten Formeln (i), (ii), (iii) oder (iv) gewählt wird: 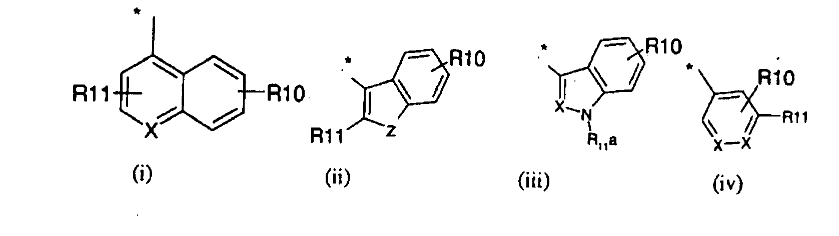
worin * der Ort ist, an welchem (i) oder (ii) oder (iii) oder (iv) an der Strukturformel (I) gebunden ist und X C oder N ist; und Z O oder S ist, wobei R¹⁰ an jeder beliebigen Position auf dem Ring ist und R¹⁰ und R¹¹ unabhängig bei jedem Auftreten H, R^{a}, Halogen, -CN, Nitro, OR^{a}, CF₃, -NR^{a}R^{a}, -C(=O)OR^{a}, -C(=O)R^{a}, -C(=O)NR^{a}R^{a}, -OC(=O)C₁₋₄-Alkyl, -NR^{a}C(=O)C₁₋₄ -Alkyl oder -S(=O)ₙR^{c} sind; und worin R^{11a} R^{a} -S-(=O)₂NR^{a}R^{a} oder -S(=O)ₙR^{c} ist und n 1 oder 2 ist.

5. Verbindung, wie in Anspruch 1 angegeben, worin:
R⁴ aus den unten dargelegten Formeln (a) bis (z) oder (aa) oder (ab) gewählt wird: 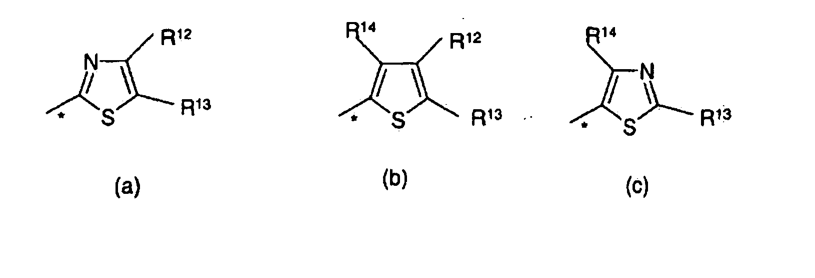 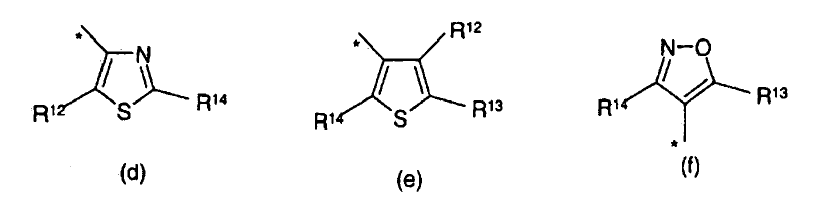 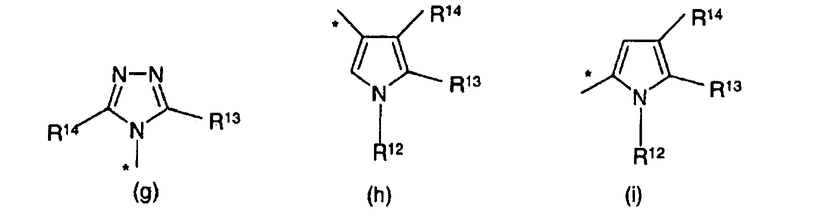 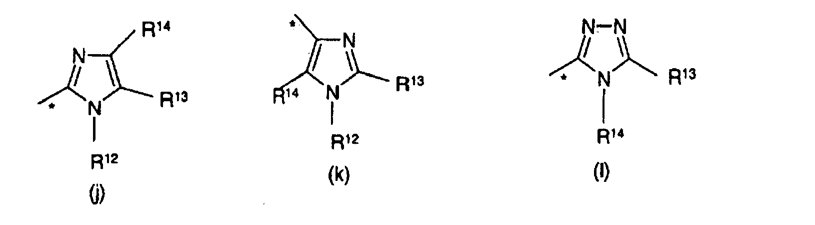 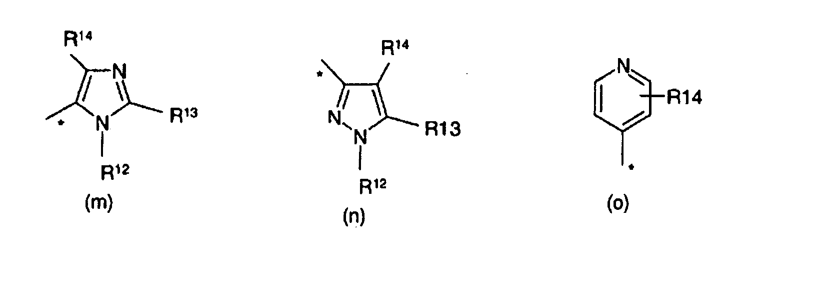 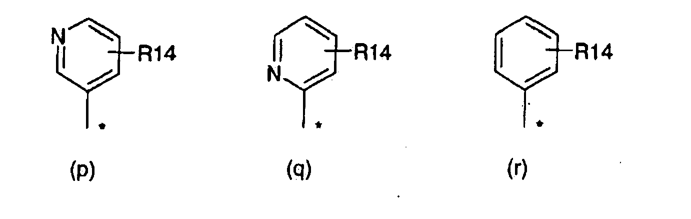 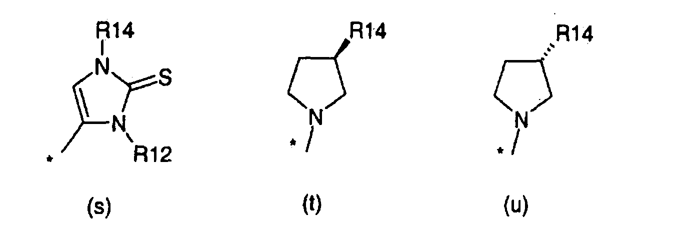 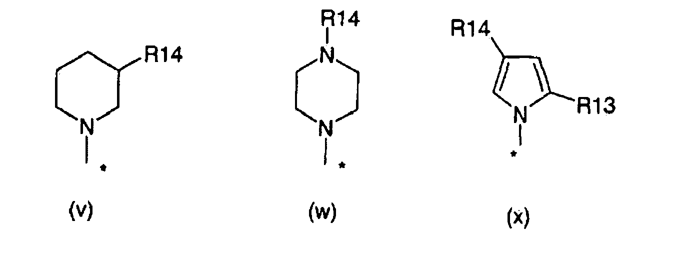 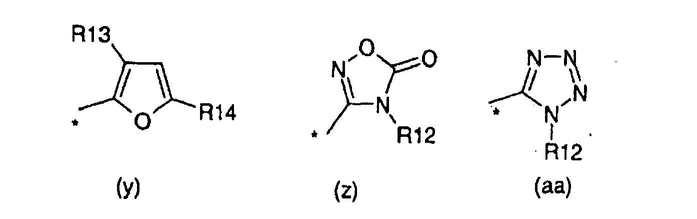 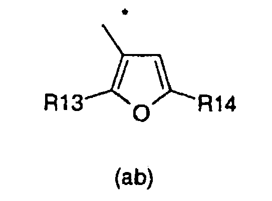 worin * der Ort ist, an dem R⁴ an das Ringsystem gebunden ist, und wobei
R¹², R¹³ und R¹⁴ jeweils unabhängig für H, Het, C₁₋₆-Alkyl, -CN, -NR^{a}R^{a}, -Nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), oder-S(=O)₂NR^{a}(R^{b}C(=O)OR^{a} stehen.

6. Verbindung, wie in Anspruch 1 angegeben, worin:
X S, O oder NR²⁰ ist, mit der Maßgabe, dass wenn W O ist, dann X nicht 0 ist; oder X und die Doppelbindung, an welches es gebunden ist, 2 Wasserstoffatome sein können;
W S, O oder NR²⁰ ist, mit der Maßgabe, dass wenn X 0 ist, dann W nicht 0 ist;
R²⁰ H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a} oder -OC(=O)R^{a} ist;
R¹ CH₃, CH₂CH₃, CH₂CN, CF₃, (CH₂)₂OH, Cyclopropyl, Isopropyl , CH₂CCH, (CH₂)₂N(CH₂)₂, (CH₂)₂N(C=NH)NH₂, -CH₂-2-Pyridyl, -CH₂-3-Pyridyl, -CH₂-4-Pyridyl, -(CH₂)₂-1-Imidazolyl, -(CH₂)₂-1-Pyrazolyl, (CH₂)₂-1-Piperidyl, -(CH₂)ₘ-(1-Methylpiperidin-4-yl), -CH₂-(1-Methylpiperidin-3-yl), -(CH₂)₂-(Morpholin-4-yl) ist;
R² -CH₂CH₂CH₃, -CH₂-Cyclopropyl, -CH₂CH(CH₃)₂, -CH₂CH₂-CH₂F, -CH₂-Cyclobutyl, -CH₂C(CH₃)₃, -CH₂CH₂CH(CH₃)₂, -CH₂CF₃, -CH₂-Methylphenyl, -CH₂-Phenol, -CH₂-(3,5-Dimethylisoxazol-4-yl), -CH₂-S-Phenyl, -CH₂-Phenyl-carboxyl oder -CH₂SCF₃ ist;
R³ aus den unten dargelegten Formeln (i), (ii), (iii) oder (iv) gewählt wird: 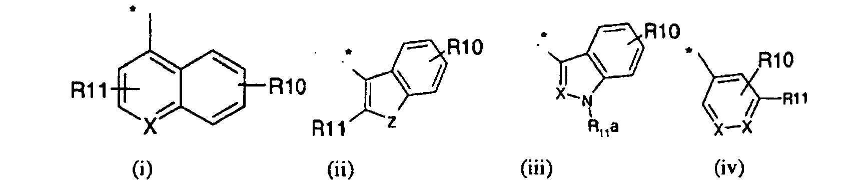 worin * der Ort ist, wo (i) oder (ii) oder (iii) oder (iv) an der Strukturformel (I) gebunden ist und X C oder N ist; und Z O oder S ist, wobei R¹⁰ an jeder beliebigen Position auf dem Ring ist und R¹⁰ und R¹¹ unabhängig bei jedem Auftreten H, R^{a}, Halogen, -CN, Nitro, OR^{a}, CF₃, -NR^{a}R^{a}, -C(=O)OR^{a}, -C(=O)R^{a}, -C(=O)NR^{a}R^{a}, -OC(=O)C₁₋₄-Alkyl, -NR^{a}C(=O)C₁₋₄ -Alkyl oder -S(=O)ₙR^{c} sind; und worin R^{11a} R^{a}, -S-(=O)₂NR^{a}R^{a} oder -S(=O)ₙR^{c} ist und n 1 oder 2 ist;
R⁴ aus den unten dargelegten Formeln (a) bis (z) oder (aa) oder (ab) gewählt wird: 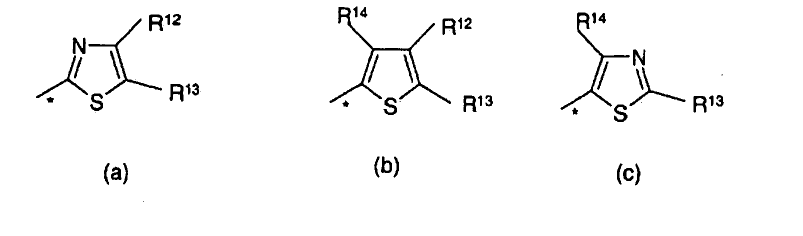 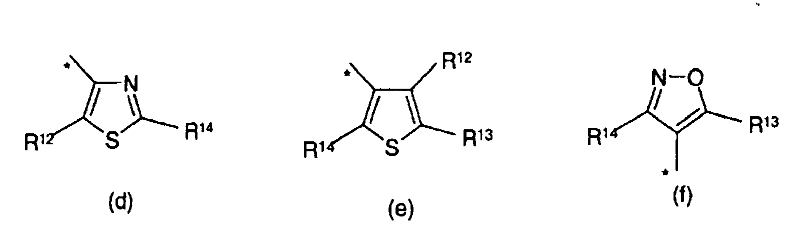 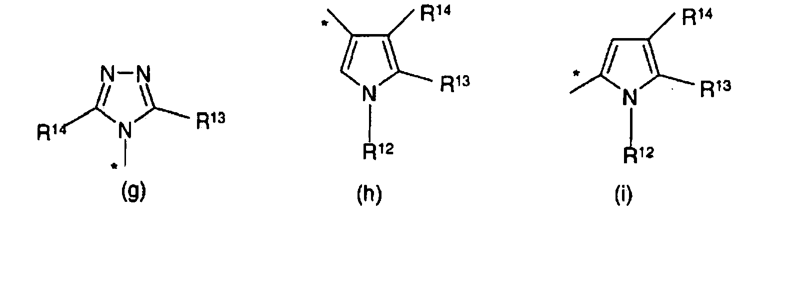 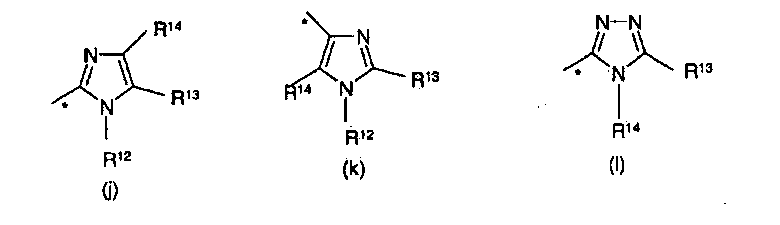 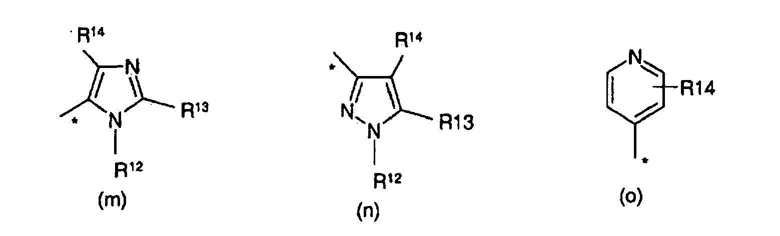 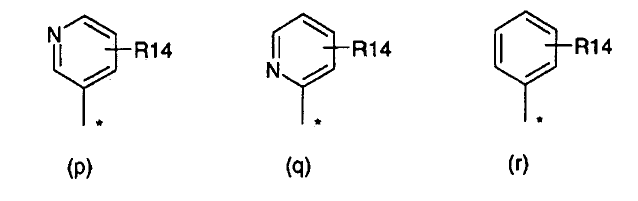 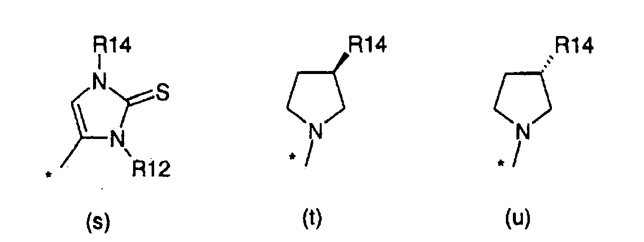 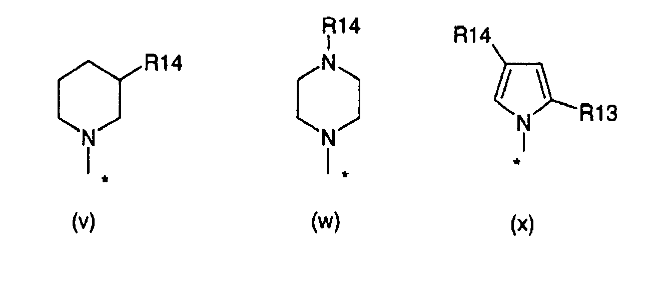 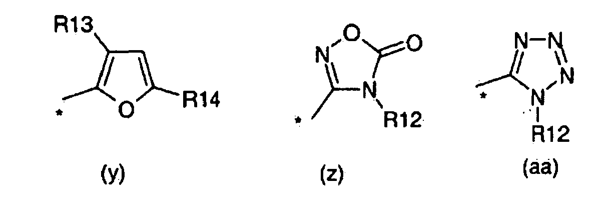 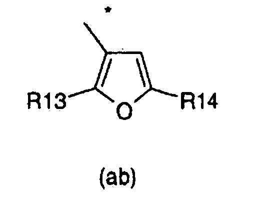
worin * der Ort ist, bei dem R⁴ an das Ringsystem gebunden ist, und wobei R¹² , R¹³ und R¹⁴ jeweils unabhängig für H, Het, C₁₋₆-Alkyl, -CN, -NR^{a}R^{a}, -Nitro. -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a} , -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), oder S(=O)₂NR^{a}(R^{b}C(=O)OR^{a} stehen.

7. Verbindung der Formel (I), gewählt aus:
5-[2-[(6-Chlorchinolin-4-yl)methyl]-7-(cyclopropylmethyl)-6-oxo-4-thioxo-4,5,6,7-tetrahydro-2H-pyrazolo[3,4-d]pyrimidin-3-yl]-1-methyl-1H-pyrrol-3-carbonitril;
5-[2-[(6-Chlorchinolin-4-yl)methyl]-7-(cyclopropylmethyl)-4-imino-5-methyl-6-oxo-4,5,6,7-tetrahydro-2H-pyrazolo[3,4-d]pyrimidin-3-yl]-1-methyl-1H-pyrrol-3-carbonitril;
5-[(4Z)-2-[(6-Chlorchinolin-4-yl)methyl]-7-(cyclopropylmethyl)-5-methyl-4-(methylimino)-6-oxo-4,5,6,7-tetrahydro-2H-pyrazolo[3,4-d]pyrimidin-3-yl]-1-methyl-1H-pyrrol-3-carbonitril;
5-[2-[(6-Chlorchinolin-4-yl)methyl]-7-(cyclopropylmethyl)-6-imino-5-methyl-4-oxo-4,5,6,7-tetrahydro-2H-pyrazolo[3,4-d]pyrimidin-3-yl]-1-methyl-1H-pyrrol-3-carbonitril;
5-[2-[(6-Chlorchinolin-4-yl)methyl]-7-(cyclopropylmethyl)-5-methyl-4-oxo-6-thioxo-4,5,6,7-tetrahydro-2*H*-pyrazolo[3,4-*d*]pyrimidin-3-yl]-1-methyl-1*H*-pyrrol-3-carbonitril;
5-[(6Z)-2-[(6-Chlorchinolin-4-yl)methyl]-7-(cyclopropylmethyl)-5-methyl-6-(methylimino)-4-oxo-4,5,6,7-tetrahydro-2H-pyrazolo[3,4-d]pyrimidin-3-yl]-1-methyl-1H-pyrrol-3-carbonitril;
N-[(6Z)-2-[(6-Chlorchinolin-4-yl)methyl]-3-(4-cyano-1-methyl-1H-pyrrol-2-yl)-7-(cyclopropylmethyl)-5-methyl-4-oxo-2,4,5,7-tetrahydro-6H-pyrazolo[3,4-d]pyrimidin-6-yliden]acetamid;
N-[(6Z)-2-[(6-Chlorchinolin-4-yl)methyl]-3-(4-cyano-1-methyl-1H-pyrrol-2-yl)-7-(cyclopropylmethyl)-5-methyl-4-oxo-2,4,5,7-tetrahydro-6H-pyrazolo[3,4-d]pyrimidin-6-yliden]methansulfonamid;
5-((6Z)-2-[(6-Chlorchinolin-4-yl)methyl]-7-(cyclopropylmethyl)-6-{[2-(dimethylamino)ethyl]imino}-5-methyl-4-oxo-4,5,6,7-tetrahydro-2H-pyrazolo[3,4-d]pyrimidin-3-yl)-1-methyl-1H-pyrrol-3-carbonitril;
N~1~-[2-[(6-Chlorchinolin-4-yl)methyl]-3-(4-cyano-1-methyl-1H-pyrrol-2-yl)-7-(cyclopropylmethyl)-5-methyl-4-oxo-2,4,5,7-tetrahydro-6H-pyrazolo[3,4-d]pyrimidin-6-yliden]-N~2~, N~2~-dimethylglycinamid;
5-[2-[(6-Chlorchinolin-4-yl)methyl]-7-(cyclopropylmethyl)-6-oxo-4,5,6,7-tetrahydro-2H-pyrazolo[3,4-d]pyrimidin-3-yl]-1-methyl-1H-pyrrol-3-carbonitril;
5-[2-[(6-Chlorchinolin-4-yl)methyl]-7-(cyclopropylmethyl)-5-methyl-6-oxo-4,5,6,7-tetrahydro-2H-pyrazolo[3,4-d]pyrimidin-3-yl]-1-methyl-1H-pyrrol-3-carbonitril;
5-[2-[(6-Chlorchinolin-4-yl)methyl]-7-(cyclopropylmethyl)-6-oxo-6,7-dihydro-2H-pyrazolo[3,4-d]pyrimidin-3-yl]-1-methyl-1H-pyrrol-3-carbonitril;
2-[(6-Chlorchinolin-4-yl)methyl]-7-(cyclopropylmethyl)-5-methyl-3-(1-methyl-1H-imidazol-5-yl)-4-thioxo-2,4,5,7-tetrahydro-6H-pyrazolo[3,4-d]pyrimidin-6-on;
(4Z)-2-[(6-Chlorchinolin-4-yl)methyl]-7-(cyclopropylmethyl)-5-methyl-3-(1-methyl-1H-imidazol-5-yl)-4-(methylimino)-2,4,5,7-tetrahydro-6 H-pyrazolo[3,4-d]pyrimidin-6-on.

8. Verbindung mit der Strukturformel (II): 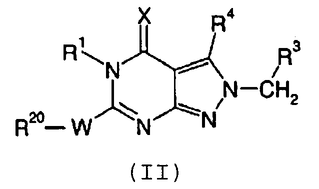 worin
X S, O oder NR²⁰ ist;
X und die Doppelbindung, an welche es gebunden ist, durch 2 Wasserstoffatome ersetzt werden können;
W S, O oder NR²¹ ist;
R¹ H, wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, Hydroxy, Amino oder wahlweise substituierter Heterocyclus ist, wobei die Substitution aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl, C₂₋₄-Alkenyl, C₂₋₄-Alkinyl, C₁₋₄ -Alkoxy, C₁₋₄-Alkanoyl, C₁₋₄-Alkanoyloxy, NH(C₁₋₄-Alkyl), N(C₁₋₄-Alkyl)₂, C₁₋₄-Alkanoylamino, (C₁₋₄-Alkanoyl)₂-amino, N-(C₁₋₄-Alkyl)carbamoyl, N,N-(C₁₋₄-Alkyl)₂-carbamoyl, (C₁₋₄)S, (C₁₋₄-Alkyl)S(O), (C₁₋₄-Alkyl)S(O)₂, (C₁₋₄)-Alkoxycarbonyl, N-(C₁₋₄-Alkyl)-sulfamoyl, N,N-(C₁₋₄-Alkyl)sulfamoyl, C₁₋₄-Alkyl-sulfonylamino und Heterocyclus gewählt wird;
R³ ein monocyclisches oder bicyclisches, gesättigtes oder ungesättigtes Ringsystem ist, welches 0, 1, 2 oder 3 Heteroatome umfasst, die unabhängig aus N, 0 oder S gewählt werden, wobei der Ring durch 0, 1, 2 oder 3 Substituenten substituiert ist, welche aus =O, Halogen, -OR^{a}, C₁₋₆-Alkyl, C₁₋₆-Halogenalkyl, -CN, Nitro, -S(O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, Aminocarbonyl, Phenyl, Benzyl gewählt werden; oder R³ durch -Het, -Het-Het, R⁵, -R⁵-Het; -Het-R⁵, -Het-O-R⁵, -R⁵-R⁵, -R⁵-OR⁵ angegeben wird;
R⁴ ein monocyclisches oder bicyclisches, gesättigtes oder ungesättigtes Ringsystem oder ein Vizinalkondensiertes Derivat davon ist, welches 5 bis 12, vorzugsweise 5 bis 10, Ringatome enthält, wobei 0, 1, 2, 3 oder 4 davon Heteroatome sind, die unabhängig aus N, O oder S gewählt werden, wobei das Ringsystem durch 0, 1, 2 oder 3 Substituenten substituiert wird, welcher von B(OH)₂, vizinalem
-OCH₂CH₂O-, vizinalem -OC₁₋₂-Halogenalkylo-, vizinalem -OCH₂O-, vizinalem -CH₂OCH₂O-, =O, Halogen, -R^{b}OR^{a}, -SR^{a}, -OR^{a}, C₁₋₆-Alkyl, C₁₋₆-Halogenalkyl, -CN, -S(=O)ₙR^{c}, -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)OR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(RS(=O)ₙR^{a}), -C(=O)NR^{a}(R^{b}Het), - C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, - C(=NCN)R^{a}, -S(=O)₂NR^{a}R^{a},-NR^{a}S(=O)₂R^{a}, -S(=O)₂NR^{a}(R^{b}C(=O)NR^{a}R^{a}), -S(=O)₂NR^{a}(R^{b}C(=O)OR^{a}), Aminocarbonyl, Phenyl, Benzyl gewählt werden; oder R⁴ wird durch - (CH₂) ₙR⁵-Het, -(CH₂)ₙR^{d} -Het, -Het-Het, R⁵, -R⁵-Het, -Het-R⁵, -Het-OR⁵, -R⁵-R⁵ oder -R⁵-OR⁵ angegeben; oder R⁴ wird durch C₁₋₆-Alkyl , -NC₁₋₆-Alkyl oder -N(C₁₋₆-Alkyl)₂ angeben, wobei das C₁₋₆-Alkyl, -NC₁₋₆-Alkyl, -N(C₁₋₆-Alkyl) durch 0, 1 oder 2 Substituenten substituiert werden, die aus R^{a}, OR^{a}, Halogen oder Phenyl gewählt werden, wobei R⁴ nicht -(CH₂)_{z}CH₃, -(CH₂)_{z}CH₂OH, -(CH₂)_{z}CO₂H, oder -(CH₂)_{z}CO₂C₁₋₆-Alkyl ist, wobei z 1, 2, 3, 4, 5 oder 6 ist;
R⁵ unabhängig bei jedem Auftreten Phenyl, substituiert durch 0, 1, 2 oder 3 Gruppen, gewählt aus Halogen, C₁₋₆-Halogenalkyl, -OC₁₋₆-Halogenalkyl, C₁₋₆-Alkyl, -CN, Nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a},-C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a},oder -S(=O)₂NR^{a}R^{b}C=O)OR^{a} ist;
R²⁰ unabhängig bei jedem Auftreten H, -CN, -S(=O)ₙR^{c}, -C(=O)R^{a}, -C(=O)NR^{a}R^{a}, -C(=O)OR^{a}, -NR^{a}C(=O)R^{a}, oder -OC(=O)R^{a}, wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, wahlweise substituierter Heterocyclus ist, wobei die Substitution aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl, C₂₋₄-Alkenyl, C₂₋₄-Alkinyl, C₁₋₄ -Alkoxy, C₁₋₄-Alkanoyl, C₁₋₄-Alkanoyloxy, NH(C₁₋₄-Alkyl), N(C₁₋₄-Alkyl)₂, C₁₋₄-Alkanoylamino, (C₁₋₄-Alkanoyl)₂-amino, N-(C₁₋₄-Alkyl)carbamoyl, N,N-(C₁₋₄-Alkyl)₂carbamoyl, (C₁₋₄)S, (C₁₋₄-Alkyl) S(O), (C₁₋₄-Alkyl) S (O)₂, (C₁₋₄)-Alkoxycarbonyl , N-(C₁₋₄-Alkyl)sulfamoyl, N,N-(C₁₋₄-Alkyl)sulfamoyl,C₁₋₄-Alkylsulfonylamino und Heterocyclus gewählt wird;
R²¹ unabhängig bei jedem Auftreten H, -CN, -S(=O)ₙR^{c}, -C(=O)R^{a}, -C(=O)NR^{a}R^{a}, -C(=O)OR^{a}, -NR^{a}C(=O)R^{a} oder -OC(=O)R^{a}; wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, wahlweise substituierter Heterocyclus ist, wobei die Substitution aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl, C₂₋₄-Alkenyl, C₂₋₄-Alkinyl, C₁₋₄ -Alkoxy, C₁₋₄-Alkanoyl, C₁₋₄-Alkanoyloxy, NH (C₁₋₄-Alkyl), N(C₁₋₄-Alkyl)₂, C₁₋₄-Alkanoylamino, (C₁₋₄-Alkanoyl)₂-amino, N-(C₁₋₄-Alkyl)carbamoyl, N,N-(C₁₋₄-Alkyl)₂-carbamoyl, (C₁₋₄)S, (C₁₋₄-Alkyl)S(O), (C₁₋₄-Alkyl)S(O)₂, (C₁₋₄)-Alkoxycarbonyl, N-(C₁₋₄-Alkyl)sulfamoyl, N,N-(C₁₋₄-Alkyl)sulfamoyl, C₁₋₄-Alkylsulfonylamino und Heterocyclus gewählt wird;
R²⁰ und R²¹ und das N, an welche sie in Kombination gebunden sind, ebenfalls einen 3- bis 10-gliedrigen, N-verknüpften, gesättigten oder ungesättigten Heterocyclus mit entweder 1 oder 2 Heteroatomen, die unabhängig aus N, O oder S gewählt werden, bilden können, wobei der Heterocyclus durch R^{e} substituiert ist;
R^{a} unabhängig bei jedem Auftreten H, C₁₋₆-Alkyl, -C(=O)C₁₋₄-Alkyl, C₁₋₄-Halogenalkyl, Phenyl, Benzyl oder ein 5- oder 6-gliedriger Ring, gesättigter oder ungesättigter Heterocyclus mit 1, 2, 3 oder 4 Heteroatomen, die unabhängig aus N, O oder S gewählt werden, ist;
R^{b} unabhängig bei jedem Auftreten C₁₋₆-Alkyl , -C(=O)-C₁₋₄-Alkyl, C₁₋₄-Halogenalkyl, Phenyl, Benzyl oder ein 5- oder 6-gliedriger Ring, gesättigter oder ungesättigter Heterocyclus mit 1, 2, 3 oder 4 Heteroatomen, die unabhängig aus N, O oder S gewählt werden, ist;
R^{c} C₁₋₆-Alkyl, C₁₋₄-Halogenalkyl, Phenyl oder Benzyl ist;
R^{d} Phenyl, substituiert durch 0, 1 oder 2 Gruppen, die aus -CN, Halogen, Nitro, C₁₋₆-Alkyl, C₁₋₄-Halogenalkyl, -OH, -OR^{c}, -NR^{a}R^{a}, -S(=O)ₙR^{c}, -C (=O-)OR^{a}, -NR^{a}C(=O)R^{a}, -OC (=O) R^{a}, B(OH)₂, Vizinyl-OCH₂CH₂O-, Vizinyl-OC₁₋₂-HalogenalkylO-, Vizinyl-OCH₂O-, Vicynyl-CH₂OCH₂O-, Phenyl, Benzyl und einem 5- oder 6-gliedrigen Ring, gesättigtem oder ungesättigtem Heterocyclus, der 1, 2, 3 oder 4 Heteroatome enthält, welche unabhängig aus N, O oder S gewählt werden, ist;
R^{e} unabhängig bei jedem Auftreten H, C₁₋₆-Alkyl, -C(=O)C₁₋₄-Alkyl, C₁₋₄-Halogenalkyl, Phenyl, Benzyl oder ein 5- oder 6-gliedriger Ring, gesättigter oder ungesättigter Heterocyclus mit 1, 2, 3 oder 4 Heteroatomen, die unabhängig aus N, 0 oder S gewählt werden, ist;
m 1, 2 oder 3 ist;
n 0, 1 oder 2 ist;
oder ein pharmazeutisch annehmbares Salz davon.

9. Verbindung, wie in Anspruch 8 angegeben, wobei:
R¹ H oder C₁₋₆-Alkyl oder -(CH₂)ₙCycloalkyl ist, wobei C₁₋₆-Alkyl oder -(CH₂)ₙCycloalkyl wahlweise durch 1, 2 oder 3 Substituenten substituiert ist, welcher aus Het, Halogen, -CN, -OR^{a}, -NR^{a}R^{a}, -C(=O)OR^{a}, -C(=O)NR^{a}R^{a}, -OC(=O) C₁₋₄-Alkyl oder -NR^{a}C(=O)C₁₋₄-Alkyl gewählt sind, und n 0, 1 oder 2 ist.

10. Verbindung, wie in Anspruch 8 angegeben, wobei:
R³ aus den unten dargelegten Formeln (i), (ii), (iii) oder (iv) gewählt wird: 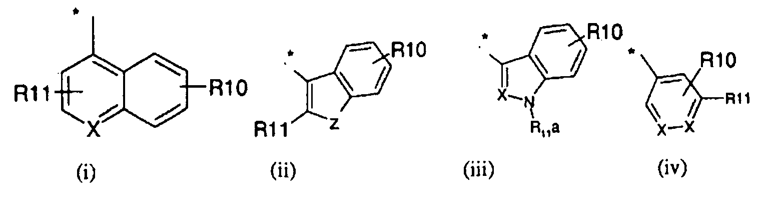
worin * der Ort ist, an dem (i) oder (ii) oder (iii) oder (iv) an der Strukturformel (I) gebunden ist und X C oder N ist; und Z O oder S ist, wobei R¹⁰ an jeder beliebigen Position auf dem Ring ist und R¹⁰ und R¹¹ unabhängig bei jedem Auftreten H, R^{a}, Halogen, -CN, Nitro, OR^{a}, CF₃, -NR^{a}R^{a}, -C(=O)OR^{a}, -C(=O)R^{a}, -C(=O)NR^{a}R^{a}, -OC(=O)C₁₋₄-Alkyl, -NR^{a}C(=O)C₁₋₄ -Alkyl oder -S(=O)ₙR^{c} sind; und worin R^{11a} R^{a}, -S(=O)₂NR^{a}R^{a} oder -S(=O)ₙR^{c} ist und n = 1 oder 2 ist.

11. Verbindung, wie in Anspruch 8 angegeben, wobei:
R⁴ aus den unten dargelegten Formeln (a) bis (z) oder (aa) oder (ab) gewählt wird: 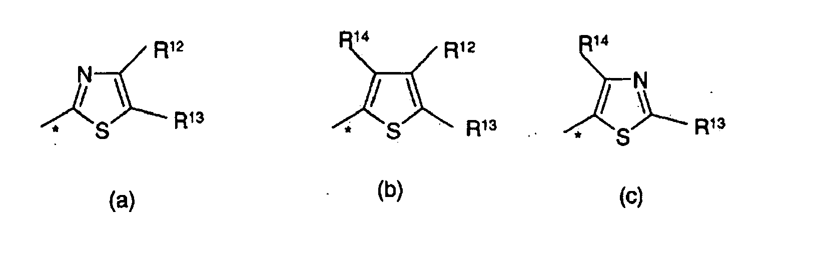 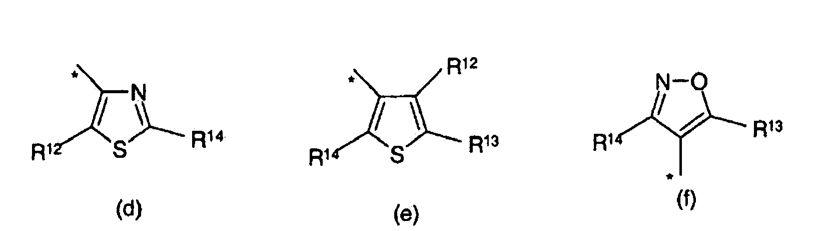 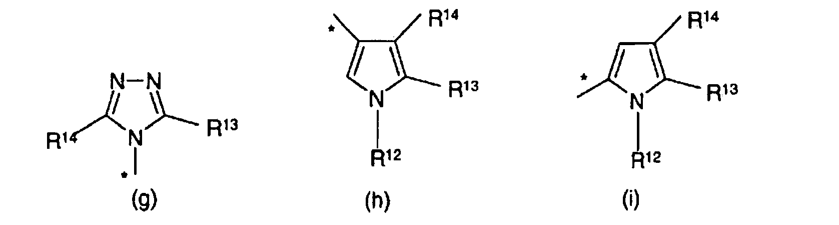 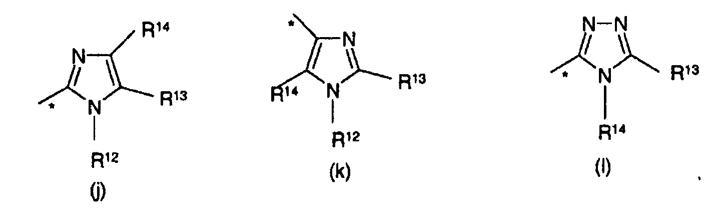 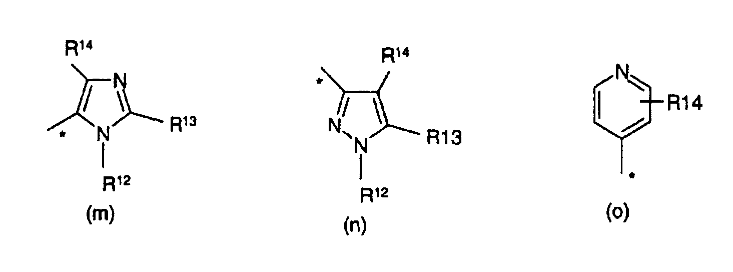 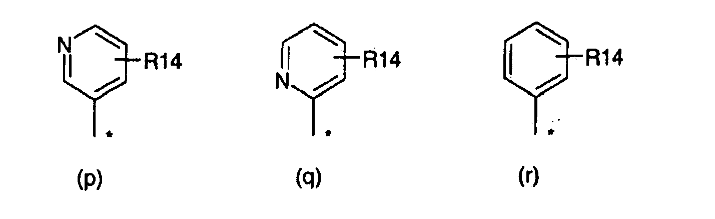 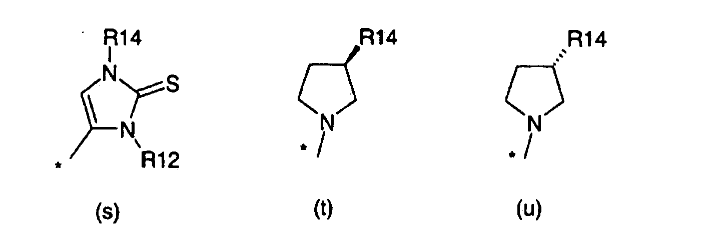 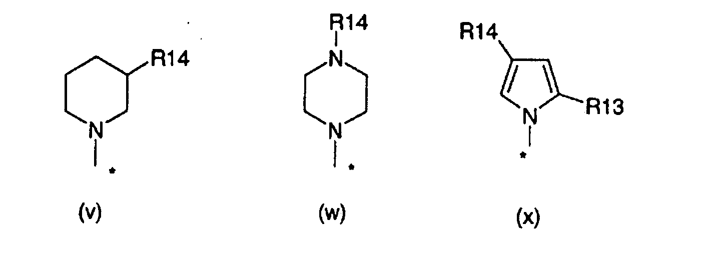 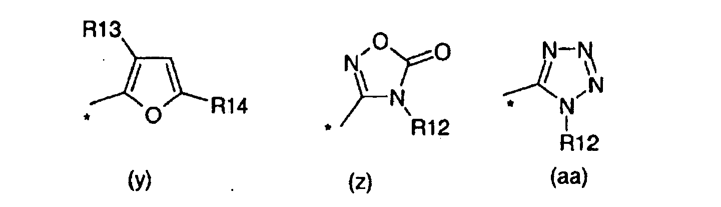 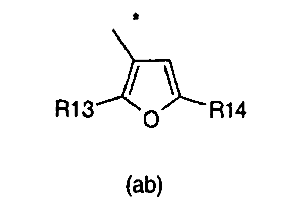
worin * der Ort ist, an dem R⁴ and das Ringsystem gebunden ist und wobei R¹², R¹³ und R¹⁴ jeweils unabhängig für H, Het, C₁₋₆-Alkyl, -CN, -NR^{a}R^{a}, -Nitro, -C(=O)R^{a}, -C(=O)NR^{a}R^{a}, -C(=O)NR^{a}S(=O)₂R^{a}, -C(=O)NR^{a}=Het, -C(=O)NR^{a}NR^{a}R^{a}, - C(=O)NR^{a}(R^{b}NR^{a}R^{a} -C(=O)NR^{a}(R^{b}OR^{a}), -C(=O)NR^{a}(R^{b}S(=O)₂R^{a}). -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}; -C(=O)OR^{b}NR^{a}R^{a}, C(=O)R^{a}, -OC(=O)R^{a}, -C(=O)R^{a}-SR^{a}, =S, -NR^{a}C(=O)R^{a}, -NR^{a}C(=O)OR^{a} , -NR^{a}S(=O)₂R^{b}, - C(=NOR')R', -S(=O)₂R^{a}, -S(=O)₂NR^{a}R^{a}, -S(=O)₂NR^{a}(R^{b}C(=O)NR^{a}R^{a}), ode r-S(=O)₂NR^{a}(R^{b}C(=O)OR^{a} stehen.

12. Verbindung, wie in Anspruch 8 angegeben, worin:
X S, 0 oder NR²⁰ ist; oder X und die Doppelbindung, an welche es gebunden ist, 2 Wasserstoffatome sein können;
W S, O oder NR²¹ ist;
R²⁰ H, -CN, R^{a}, -OR^{a}, -NR^{a}R^{a}, -Het, -S(=O)ₙR^{c}, -C(=O)-R^{a}, -C(=O)NR^{a}R^{a}, -C(=O)OR^{a}, -NR^{a}C(=O)R^{a} oder -OC (=O)R^{a} ist;
R²⁰ H, -CN, R^{a}, -OR^{a}, -NR^{a}R^{a}, -Het, -S(=O)ₙR^{c}, -C (=O) - R^{a}, -C(=O)NR^{a}R^{a}, -C(=O)OR^{a}, -NR^{a}C(=O)R^{a} oder -OC(=O)R^{a} ist;
R²⁰ und R²¹ und das N, an welche sie gebunden sind, in Kombination ebenfalls einen 3- bis 10-gliedrigen, N-verknüpften, gesättigten oder ungesättigten Heterocyclus mit entweder 1 oder 2 Heteroatomen bilden können, der unabhängig aus N, O oder S gewählt wird, wobei der Heterocyclus durch R^{e} substituiert ist;
R¹ CH₃, CH₂CH₃, CH₂CN, CF₃, (CH₂)₂OH, Cyclopropyl, Isopropyl, CH₂CCH, (CH₂)₂N(CH₂)₂, (CH₂)₂N(C=NH)NH₂, -CH₂-2-Pyridyl, -CH₂-3-Pyridyl, -CH₂-4-Pyridyl, -(CH₂)₂-1-Imidazolyl, -(CH₂)₂-1-Pyrazolyl, -(CH₂)₂-1-Piperidyl, -(CH₂)ₘ-(1-Methylpiperidin-4-yl), -CH₂-(1-Methylpiperidin-3-yl), -(CH₂)₂-(Morpholin-4-yl) ist;
R³ aus den unten dargelegten Formeln (i), (ii), (iii) oder (iv) gewählt ist: 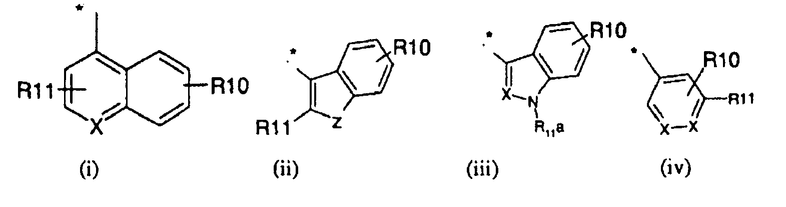 worin * der Ort ist, an dem (i) oder (ii) oder (iii) oder (iv) an der Strukturformel (I) gebunden ist, und X C oder N ist; und Z O oder S ist, wobei R¹⁰ jeder beliebige Ort auf dem Ring ist und R¹⁰ und R¹¹ bei jedem Auftreten unabhängig H, R^{a}, Halogen, -CN, Nitro, OR^{a}, CF₃, -NR^{a}R^{a}, -C (=O) OR^{a}, -C (=O) R^{a}, -C (=O) NR^{a}R^{a}, -OC (=O) C₁₋₄-Alkyl, -NR^{a}C (=O) C₁₋₄-Alkyl oder -S(=O)ₙR^{c} ist; und worin R^{11a} R^{a}, -S (=O) ₂NR^{a}R^{a} oder -S(=O)ₙR^{c} und n 1 oder 2 ist;
R⁴ aus den unten dargelegten Formeln (a) bis (z) oder (aa) oder (ab) gewählt wird: 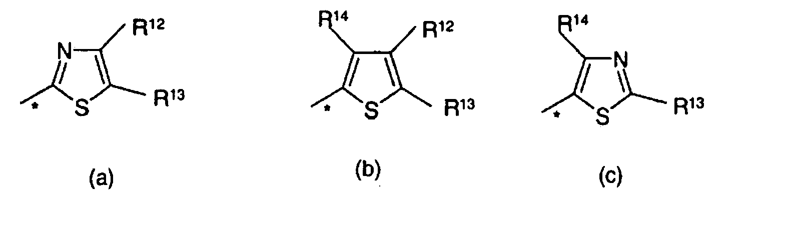 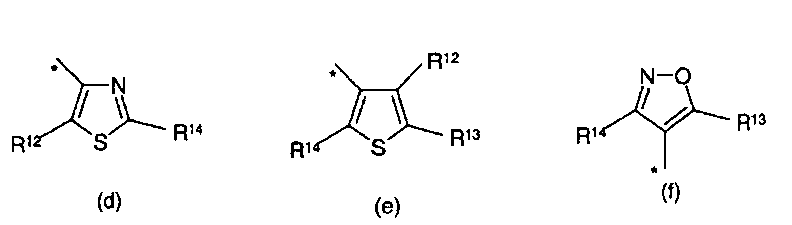 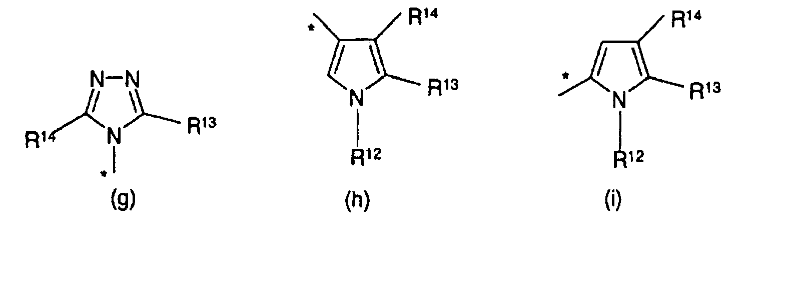 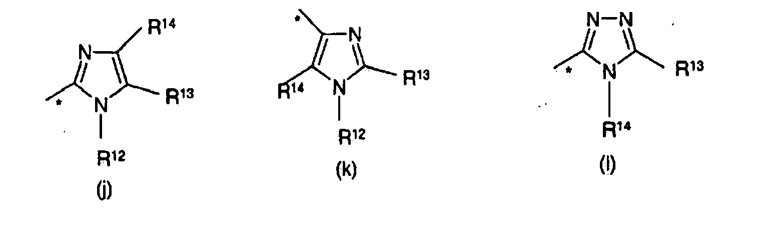 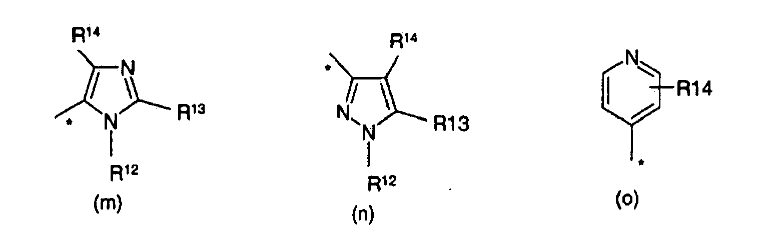 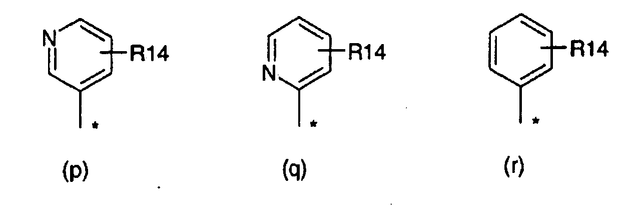 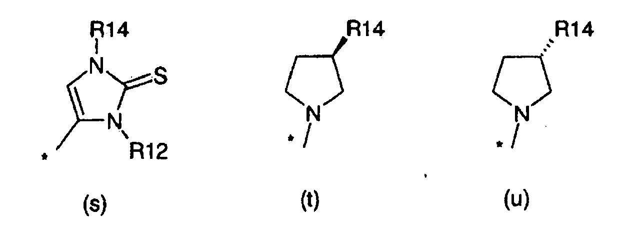 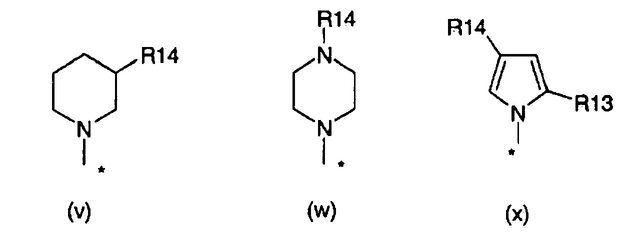 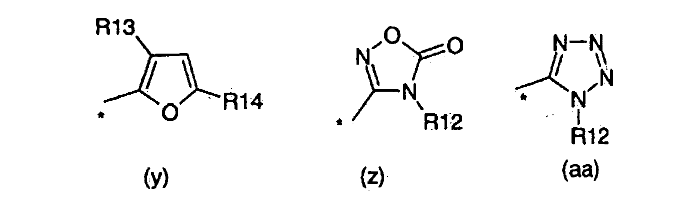 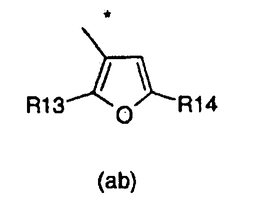
worin * der Ort ist, an dem R⁴ an das Ringsystem gebunden ist, und wobei R¹² , R¹³ und R¹⁴ jeweils unabhängig für H, Het, C₁₋₆-Alkyl, -CN, -NR^{a}R^{a}, -Nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R²), -C(=O)NR^{a}R^{b}Het, C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), oder-S(=O)₂NR^{a}(R^{b}C(=O)OR^{a} stehen.

13. Verbindung der Formel (II), gewählt aus:
5-{6-Amino-2-[(6-Chlorchinolin-4-yl)methyl]-5-methyl-4-oxo-4,5-dihydro-2H-pyrazolo[3,4-d]pyrimidin-3-yl}-1-methyl-1H-pyrrol-3-carbonitril; N-[2-[(6-Chlorchinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-3-methylbutanamid;
N,N-[2-[(6-Chlorchinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-3-methylbutanamid;
N'-[2-[(6-Chlorchinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-N,N-dimethylimidoformamid;
5-{2-[(6-Chlorchinolin-4-yl)methyl]-6-[(cyclopropylmethyl)(methyl)amino]-5-methyl-4-oxo-4,5-dihydro-2H-pyrazolo[3,4-d]pyrimidin-3-yl}-1-methyl-1H-pyrrol-3-carbonitril;
5-{2-[(6-Chlorchinolin-4-yl)methyl]-6-[(cyclopropylmethyl)amino]-5-methyl-4-oxo-4,5-dihydro-2H-pyrazolo[3,4-d]pyrimidin-3-yl}-1-methyl-1H-pyrrol-3-carbonitril;
N-[2-[(6-Chlorchinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]propan-1-sulfonamid;
Ethyl-2-[(6-Chlorchinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-ylcarbamat;
N-[2-[(6-Chlorchinolin-4-yl)methyl]-3-(4-cyano-1-methyl-1H-pyrrol-2-yl)-5-methyl-4-oxo-4,5-dihydro-2H-pyrazolo[3,4-d]pyrimidin-6-yl]-N'-ethylharnstoff;
5-[(4Z)-2-[(6-Chlorchinolin-4-yl)methyl]-6-[(cyclopropylmethyl)amino]-5-methyl-4-(methylimino)-4,5-dihydro-2H-pyrazolo[3,4-*d*] pyrimidin-3-yl]-1-methyl-1*H*-pyrrol-3-carbonitril;
5-[(4Z,6Z)-2-[(6-Chlorchinolin-4-yl)methyl]-7-(cyclopropylmethyl)-5-methyl-4,6-bis(methylimino)-4,5,6,7-tetrahydro-2*H*-pyrazolo[(3,4-*d*]pyrimidin-3-yl]-1-methyl-1H-pyrrol-3-carbonitril.

14. Verbindung der Strukturformel (III): 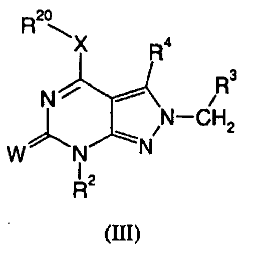 worin
X S, O, NR²¹ ist; oder XR²⁰ Wasserstoff ist;
W S, O oder NR²⁰ ist;
R² H, wahlweise substituiertes Alkyl, wahlweise substituiertes Alkylcycloalkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, oder wahlweise substituiertem Heterocyclus ist;
R³ ein monocyclisches oder bicyclisches, gesättigtes oder ungesättigtes Ringsystem ist, welches 0, 1, 2 oder 3 Heteroatome umfasst, die unabhängig aus N, O oder S gewählt werden, wobei der Ring durch 0, 1, 2 oder 3 Substituenten substituiert ist, welche aus =O, Halogen, -OR^{a}, C₁₋₆-Alkyl, C₁₋₆-Halogenalkyl, -CN, Nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, Aminocarbonyl, Phenyl, Benzyl gewählt werden; oder R³ durch -Het, -Het-Het, R⁵, -R⁵-Het, -Het-R⁵, -Het-O-R⁵, -R⁵-R⁵ -R⁵-OR⁵ angegeben wird;
R⁴ ein monocyclisches oder bicyclisches, gesättigtes oder ungesättigtes Ringsystem oder ein Vizinalkondensiertes Derivat davon ist, welches 5 bis 12, vorzugsweise 5 bis 10, Ringatome enthält, wobei 0, 1, 2, 3 oder 4 davon Heteroatome sind, die unabhängig aus N, O oder S gewählt werden, wobei das Ringsystem durch 0, 1, 2 oder 3 Substituenten substituiert wird, welcher von B(OH)₂, vizinalem -OCH₂CH₂O-, vizinalem -OC₁₋₂-HalogenalkylO-, vizinalem -OCH₂O-, vizinalem -CH₂OCH₂O-, =O, Halogen, -R^{b}OR^{a}, -SR^{a}, -OR^{a}, C₁₋₆-Alkyl, C₁₋₆-Halogenalkyl, -CN, -S(=O)ₙR^{c}, -O(CH₂)ₘHet, -O(CH₂)ₘC(=O) Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), - C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, - C(=NCN)R^{a}, -S(=O)₂NR^{a}R^{a},-NR^{a}S(=O)₂R^{a}, -S(=O)₂NR^{a}(R^{b}C(=O)NR^{a}R^{a}), -S(=O)₂NR^{a}(R^{b}C(=O)OR^{a}), Aminocarbonyl, Phenyl, Benzyl gewählt werden; oder R⁴ wird durch-(CH₂)ₙR⁵-Het, -(CH₂)ₙR^{d}, -Het, -Het-Het, R⁵, -R⁵-Het, -Het-R⁵, -Het-OR⁵, -R⁵-R⁵ oder -R⁵-OR⁵ angegeben; oder R⁴ wird durch C₁₋₆-Alkyl, -NC₁₋₆-Alkyl oder -N(C₁₋₆-Alkyl)₂ angeben, wobei das C₁₋₆-Alkyl, -NC₁₋₆-Alkyl, -N(C₁₋₆-Alkyl) durch 0, 1 oder 2 Substituenten substituiert werden, die aus R^{a}, OR^{a}, Halogen oder Phenyl gewählt werden, wobei R⁴ nicht -(CH₂)_{z}CH₃, -(CH₂)_{z}CH₂OH, -(CH₂)_{z}-CO₂H, oder -(CH₂)_{z}CO₂C₁₋₆-Alkyl ist, wobei z 1, 2, 3, 4, 5 oder 6 ist;
R⁵ unabhängig bei jedem Auftreten Phenyl, substituiert durch 0, 1, 2 oder 3 Gruppen, gewählt aus Halogen, C₁₋₆-Halogenalkyl, -OC₁₋₆-Halogenalkyl, C₁₋₆-Alkyl, -CN, Nitro, -OR^{a}, -S (=O) ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR², -S(CH₂)ₘNR^{a}R^{a}, -S(CH₂)ₘOR^{a}, -R^{b}OR^{a}, -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, -C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, - C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a},oder-S(=O)₂NR^{a}R^{b}C(=O)OR^{a} ist;
R²⁰ unabhängig bei jedem Auftreten H, -CN, wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, wahlweise substituierter Heterocyclus, -S(=O)ₙR^{c}, -C(=O)R^{a}, -C(=O)NR^{a}R^{a} , -NR^{a}C(=O)R^{a} oder -OC(O=)R^{a} ist;
R²¹ unabhängig bei jedem Auftreten H, -CN, wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, wahlweise substituierter Heterocyclus, -S(=O)ₙR^{c}, -C(=O)R^{a}, -C(=O)NR^{a}R^{a}, -C(=O)OR^{a}, -NR^{a}C(=O)R^{a} oder -OC(=O)R^{a} ist; oder
R²⁰ und R²¹ und das N, an welches sie gebunden sind, in Kombination einen 3- bis 10-gliedrigen, N-verknüpften, gesättigten oder ungesättigten Heterocyclus mit entweder 1 oder 2 Heteroatomen, die unabhängig aus N, O oder S gewählt werden, sind, wobei der Heterocyclus durch R^{e} substituiert ist;
R^{a} unabhängig bei jedem Auftreten H, C₁₋₆-Alkyl, -C(=O)C₁₋₄-Alkyl, C₁₋₄-Halogenalkyl, Phenyl, Benzyl oder ein 5- oder 6-gliedriger Ring, gesättigter oder ungesättigter Heterocyclus, der 1, 2, 3 oder 4 Heteroatome enthält, unabhängig gewählt aus N, O oder S, ist;
R^{b} unabhängig bei jedem Auftreten C₁₋₆-Alkyl, -C(=O)-C₁₋₄-Alkyl, C₁₋₄-Halogenalkyl, Phenyl, Benzyl oder ein 5- oder 6-glieder Ring, gesättigter oder ungesättigter Heterocyclus, der 1, 2, 3 oder 4 Heteroatome enthält, unabhängig gewählt aus N, 0 oder S, ist; R^{c} C₁₋₆-Alkyl, C₁₋₄-Halogenalkyl, Phenyl oder Benzyl ist;
R^{d} Phenyl, substituiert durch 0, 1 oder 2 Gruppen, gewählt aus -CN, Halogen, Nitro, C₁₋₆-Alkyl, C₁₋₄-Halogenalkyl, -OH, -OR^{c}, -NR^{a}R^{a}, -S(=O)ₙR^{c}, -C(=O)-NR^{a}R^{a}, -C(=O)OR^{a}, -NR^{a}C(=O)R^{a}, -OC(=O)R^{a}, B(OH)₂, Vizinyl-OCH₂CH₂O-, Vizinyl-OC₁₋₂-HalogenalkylO-, Vizinyl-OCH₂O-, Vizinyl-CH₂OCH₂O-, Phenyl, Benzyl und einem 5- oder 6-gliedrigen Ring, gesättigtem oder ungesättigtem Heterocyclus mit 1, 2, 3 oder 4 Heteroatomen, die unabhängig aus N, O oder S gewählt werden, ist;
R^{e} unabhängig bei jedem Auftreten H, C₁₋₆-Alkyl, -C(=O)C₁₋₄-Alkyl, C₁₋₄-Halogenalkyl, Phenyl, Benzyl oder einem 5- oder 6-gliedrigem Ring, gesättigtem oder ungesättigtem Heterocyclus mit 1, 2, 3 oder 4 Heteroatomen, die unabhängig aus N, 0 oder S gewählt werden, ist;
m 1, 2 oder 3 ist;
n 0, 1 oder 2 ist.
Wenn "wahlweise substituiert" verwendet wird, bezieht es sich mindestens auf einen Substituenten, der gewählt ist aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl, C₂₋₄-Alkenyl, C₂₋₄-Alkinyl, C₁₋₄ -Alkoxy, C₁₋₄-Alkanoyl, C₁₋₄-Alkanoyloxy, NH (C₁₋₄-Alkyl), N(C₁₋₄-Alkyl)₂, C₁₋₄-Alkanoylamino, (C₁₋₄-Alkanoyl)₂-amino, N-(C₁₋₄-Alkyl)carbamoyl, N,N-(C₁₋₄-Alkyl)₂-carbamoyl, (C₁₋₄)S, (C₁₋₄-Alkyl) S(O), (C₁₋₄-Alkyl)S(O)₂, (C₁₋₄)-Alkoxycarbonyl, N-(C₁₋₄-Alkyl)-sulfamoyl, N,N-(C₁₋₄-Alkyl)sulfamoyl, C₁₋₄-Alkyl-sulfonylamino und einen Heterocyclus,
oder ein pharmazeutisch annehmbares Salz davon.

15. Verbindung, wie in Anspruch 14 angegeben, worin:
R² -(CH₂)₁₋₃-Cycloalkyl oder -C₁₋₁₂-Alkyl ist, worin -(CH₂)₁₋₃-Cycloalkyl oder -C₁₋₁₂-Alkyl wahlweise substituiert ist durch 0, 1, 2 oder 3 Substituenten, die gewählt sind aus Het, S(=O)ₙR^{c}, Halogen, -CN, -OR^{a}, -NR^{a}R^{a}, -C(=O)OR^{a}, -C(=O)R^{a}, -C(=O)NR^{a}R^{a}, -OC (=O) C₁₋₄-Alkyl oder -NR^{a}C (=O) C₁₋₄-Alkyl und n 0, 1 oder 2 ist.

16. Verbindung, wie in Anspruch 14 angegeben, worin R³ aus den unten dargelegten Formeln (i), (ii), (iii) oder (iv) ausgewählt ist: 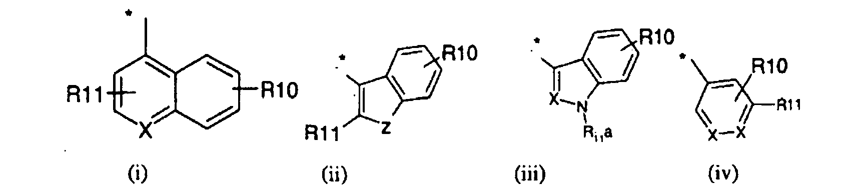 worin * der Ort ist, wo (i) oder (ii) oder (iii) oder (iv) an der Strukturformel (I) gebunden ist, und X C oder N ist; und Z O oder S ist, worin R¹⁰ an einer beliebigen Position auf dem Ring ist und R¹⁰ und R¹¹ unabhängig bei jedem Auftreten H, R^{a}, Halogen, -CN, Nitro, OR^{a}, CF₃, -NR^{a}R^{a}, -C(=O)OR^{a}, -C(=O)R^{a}, -C(=O)NR^{a}R^{a}, -OC(=O)C₁₋₄-Alkyl, -NR^{a}C(=O)C₁₋₄ -Alkyl oder -S(=O)ₙR^{c} sind; und worin R^{11a} R^{a}, -S(=O)₂NR^{a}R^{a} oder -S(=O)ₙR^{c} ist und n 1 oder 2 ist.

17. Verbindung, wie in Anspruch 14 angegeben, worin:
R⁴ aus den unten dargelegten Formeln (a) bis (z) oder (aa) oder (ab) ausgewählt ist: 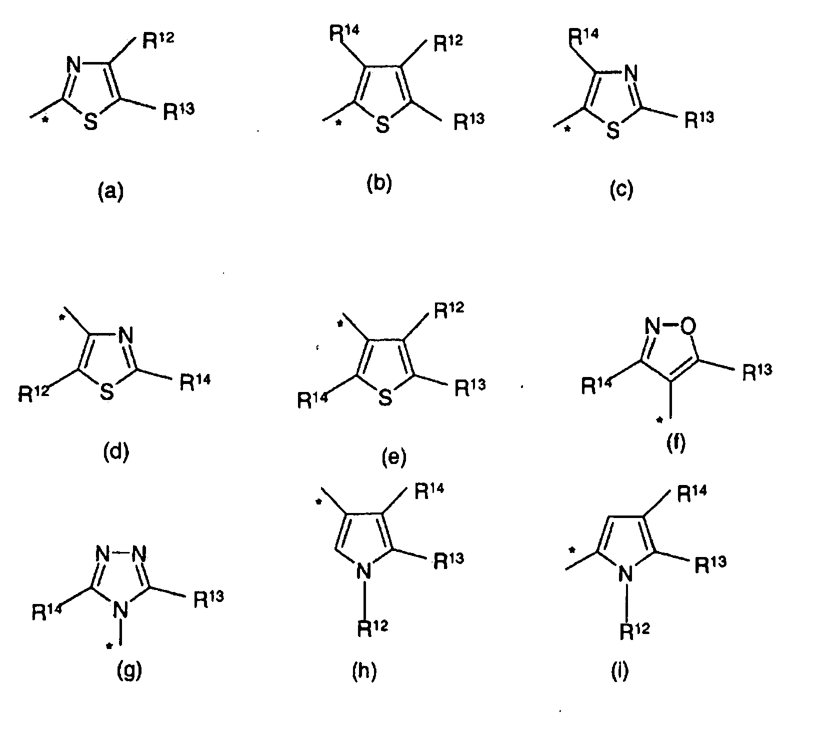 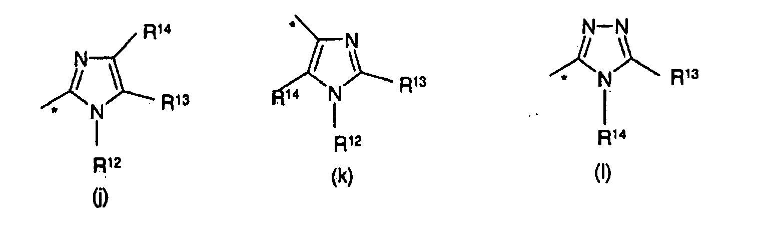 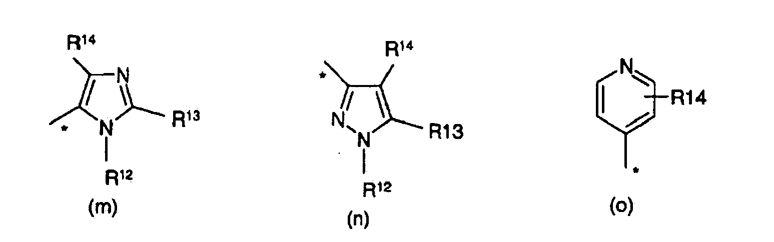 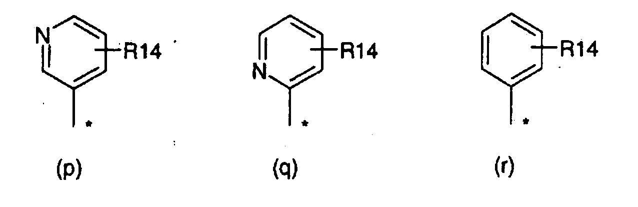 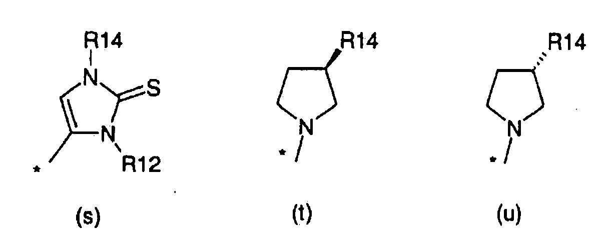 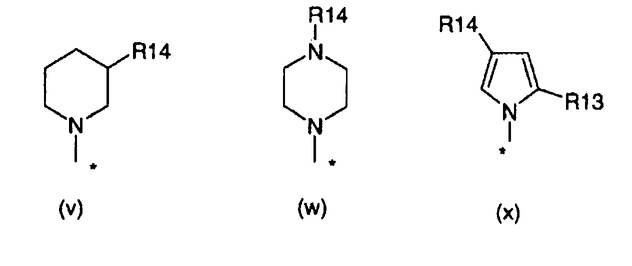 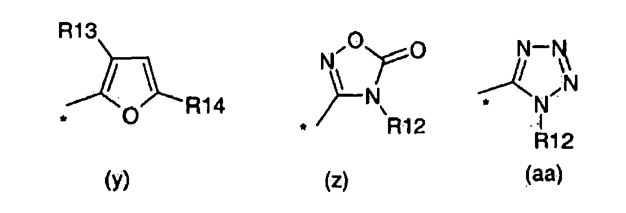 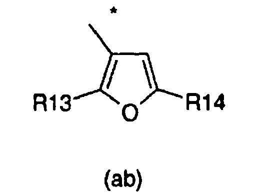
worin * der Ort ist, an dem R⁴ an das Ringsystem gebunden ist, und wobei R¹², R¹³ und R¹⁴ jeweils unabhängig für H, Het, C₁₋₆-Alkyl, -CN, -NR^{a}R^{a}, -Nitro, -C(=O)R^{a}. -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a} - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), oder S(=O)₂NR^{a}(R^{b}C(=O)OR^{a} stehen.

18. Verbindung, wie in Anspruch 14 angegeben, worin:
X S, 0 oder NR²¹ ist; oder XR²⁰ Wasserstoff ist;
W S, 0 oder NR²⁰ ist;
R²⁰ H, -CN, R^{a}, -OR^{a}, -NR^{a}R^{a}, -Het, -S(=O)ₙR^{c}, -C(=O) - R^{a}, -C(=O)NR^{a}R^{a}, -C(=O)OR^{a}, -NR^{a}C(=O)R^{a} oder -OC(=O)R^{a} ist;
R²⁰ H, -CN, R^{a}, -OR^{a}, -NR^{a}R^{a}, -Het, -S(=O)ₙR^{c}, -C(=O)-R^{a}, -C(=O)NR^{a}R^{a}, -C(=O)OR^{a}, -NR^{a}C(=O)R^{a} oder -OC(=O)R^{a} ist;
R²⁰ und R²¹ und das N, an welches sie gebunden sind, in Kombination ebenfalls einen 3- bis 10-gliedrigen, N-verknüpften, gesättigten oder ungesättigten Heterocyclus mit entweder 1 oder 2 Heteroatomen bilden können, welche unabhängig aus N, O oder S gewählt sind, wobei der Heterocyclus durch R^{e} substituiert ist;
R¹ CH₃, CH₂CH₃, CH₂CN, CF₃, (CH₂)₂OH, Cyclopropyl, Isopropyl, CH₂CCH, (CH₂)₂N(CH₂)₂, (CH₂)₂N(C=NH)NH₂, -CH₂-2-Pyridyl, -CH₂-3-Pyridyl, -CH₂-4-Pyridyl, -(CH₂)₂-1-Imidazolyl, -(CH₂)₂-1-Pyrazolyl, -(CH₂)₂-1-Piperidyl, -(CH₂)ₘ-(1-Methylpiperidin-4-yl) -CH₂-(1-Methylpiperidin-3-yl), -(CH₂)₂-(Morpholin-4-yl) ist,
R² -CH₂CH₂CH₃, -CH₂-Cyclopropyl, -CH₂CH(CH₃)₂, -CH₂CH₂-CH₂F, -CH₂-Cyclobutyl, -CH₂C(CH₃)₃, -CH₂CH₂CH(CH₃)₂, -CH₂CF₃, -CH₂-Methylphenyl, -CH₂-Phenol, -CH₂-(3,5-Dimethylisoxazol-4-yl), -CH₂-S-Phenyl, -CH₂-Phenyl-carboxyl oder -CH₂SCF₃ ist;
R³ aus den unten beigelegten Formeln (i), (ii), (iii) oder (iv) gewählt ist: 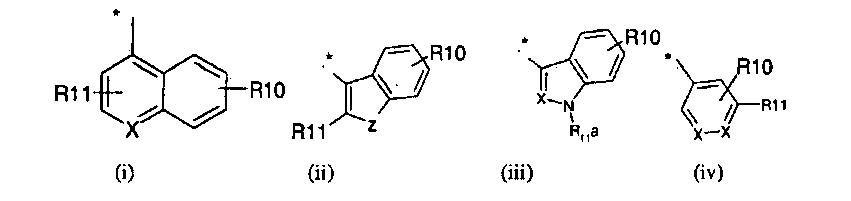
worin * der Ort ist, an dem (i) oder (ii) oder (iii) oder (iv) an die Strukturformel (I) gebunden ist, und X C oder N ist; und Z O oder S ist, wobei R¹⁰ an einer beliebigen Position auf dem Ring ist und R¹⁰ und R¹¹ unabhängig bei jedem Auftreten H, R^{a}, Halogen, -CN, Nitro, OR^{a}, CF₃, -NR^{a}R^{a}, -C (=O) OR^{a}, -C(=O)R^{a}, -C(=O)NR^{a}R^{a}, -OC(=O)C₁₋₄-Alkyl, -NR^{a}C(=O)C₁₋₄ -Alkyl oder -S(=O)ₙR^{c} sind; und worin R^{11a} R^{a}, -S(=O)₂NR^{a}R^{a} oder -S(=O)ₙR^{c} ist und n 1 oder 2 ist; R⁴ aus den unten dargelegten Formeln (a) bis (z) oder (aa) oder (ab) gewählt ist: 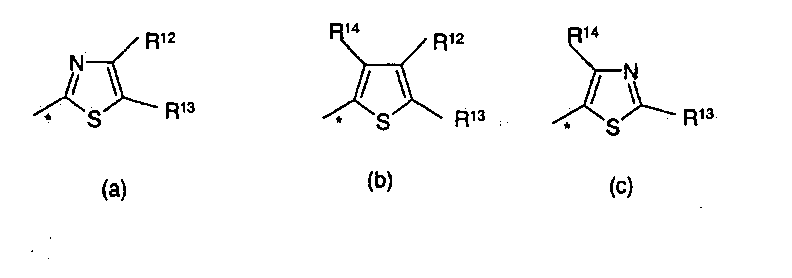 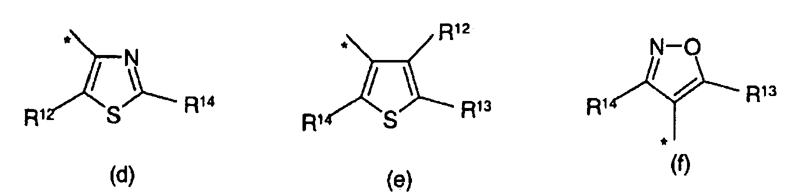 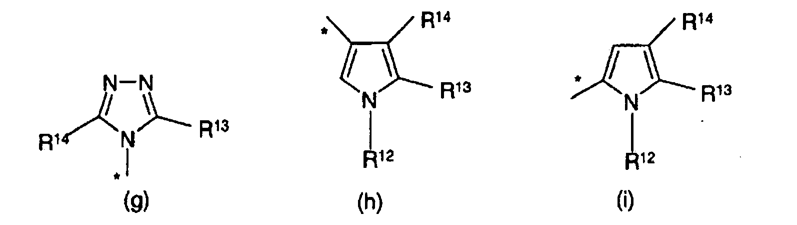 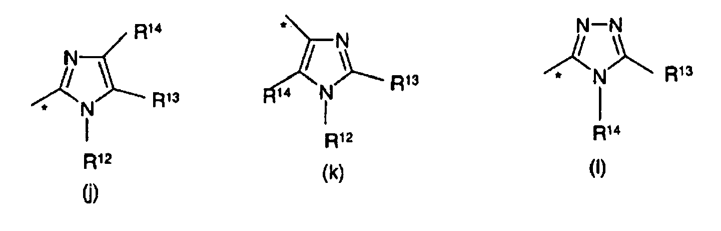 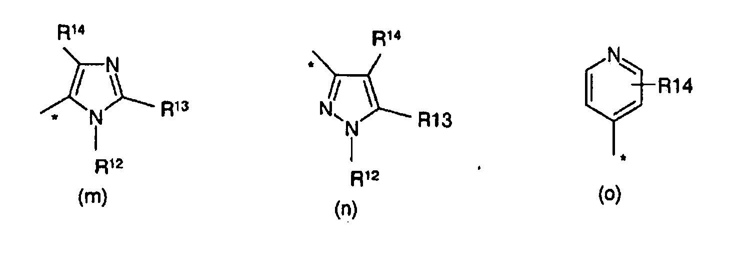 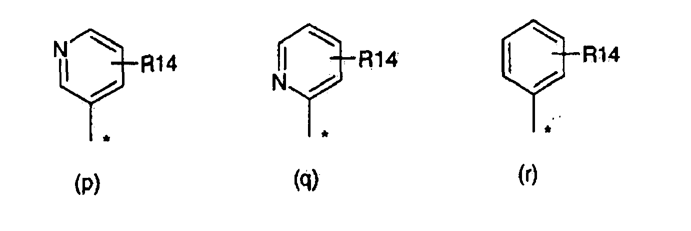 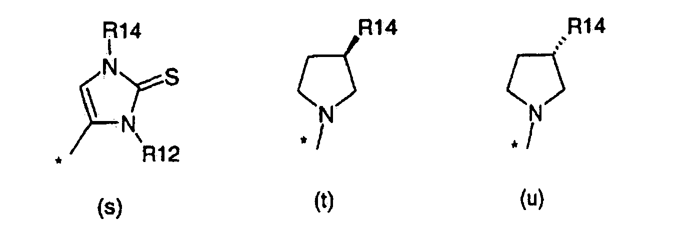 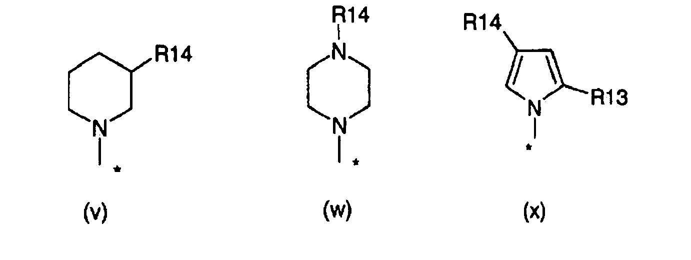 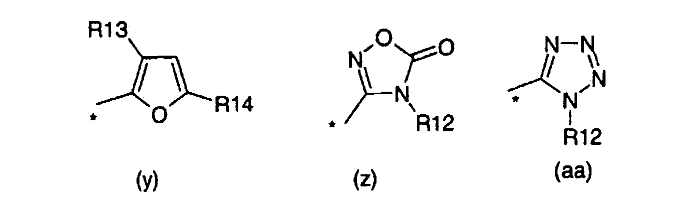 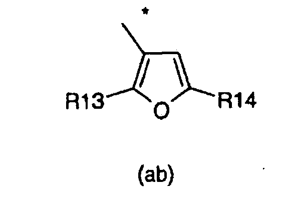 worin * der Ort ist, an dem R⁴ an das Ringsystem gebunden ist, und wobei R¹², R¹³ und R¹⁴ jeweils unabhängig für H, Het, C₁₋₆-Alkyl, -CN, -NR^{a}R^{a}, -Nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), oder-S(=O)₂NR^{a}(R^{b}C(=O)OR^{a} stehen.

19. Verbindung der Formel (III), gewählt aus:
4-Amino-7-isobutyl-2-(1-naphthylmethyl)-3-pyridin-4-yl-2,7-dihydro-6H-pyrazolo[3,4-d]pyrimidin-6-on; 7-Isobutyl-4-(methylamino)-2-(1-naphthylmethyl)-3-pyridin-4-yl-2,7-dihydro-6H-pyrazolo[3,4-d]pyrimidin-6-on;
4-(Dimethylamino)-7-isobutyl-2-(1-naphthylmethyl)-3-pyridin-4-yl-2,7-dihydro-6H-pyrazolo[3,4-d]pyrimidin-6-on;
7-Isobutyl-4-(4-methylpiperazin-1-yl)-2-(1-naphthylmethyl)-3-pyridin-4-yl-2,7-dihydro-6H-pyrazolo[3,4-d]pyrimidin-6-on;
4-Amino-2-[(6-chlorchinolin-4-yl)methyl]-7-isobutyl-3-(1-methyl-1H-pyrrol-2-yl)-2,7-dihydro-6H-pyrazolo[3,4-d]pyrimidin-6-on;
5-{4-Amino-2-[(6-chlorchinolin-4-yl)methyl]-7-isobutyl-6-oxo-6,7-dihydro-2H-pyrazolo[3,4-d]pyrimidin-3-yl}-1-methyl-1H-pyrrol-3-carbonitril;
5-[2-[(6-Chlorchinolin-4-yl)methyl]-7-isobutyl-4-(methylamino)-6-oxo-6,7-dihydro-2H-pyrazolo[3,4-d]pyrimidin-3-yl]-1-methyl-1H-pyrrol-3-carbonitril;
5-[2-[(6-Chlorchinolin-4-yl)methyl]-4-(dimethylamino)-7-isobutyl-6-oxo-6,7-dihydro-2H-pyrazolo[3,4-d]pyrimidin-3-yl]-1-methyl-1H-pyrrol-3-carbonitril;
5-[2-[(6-Chlorchinolin-4-yl)methyl]-7-isobutyl-6-oxo-4-(propylamino)-6,7-dihydro-2H-pyrazolo[3,4-d]pyrimidin-3-yl]-1-methyl-1H-pyrrol-3-carbonitril;
5-{2-[(6-Chlorchinolin-4-yl)methyl]-4-[(2-hydroxyethyl)amino]-7-isobutyl-6-oxo-6,7-dihydro-2H-pyrazolo[3,4-d]pyrimidin-3-yl}-1-methyl-1H-pyrrol-3-carbonitril;
5-[2-[(6-Chlorchinolin-4-yl)methyl]-4-(hydroxyamino)-7-isobutyl-6-oxo-6,7-dihydro-2H-pyrazolo[3,4-d]pyrimidin-3-yl]-1-methyl-1H-pyrrol-3-carbonitril;
5-[2-[(6-Chlorchinolin-4-yl)methyl]-4-(cyclopropylamino)-7-isobutyl-6-oxo-6,7-dihydro-2H-pyrazolo[3,4-d]pyrimidin-3-yl]-1-methyl-1H-pyrrol-3-carbonitril;
5-{2-[(6-Chlorchinolin-4-yl)methyl]-4-hydrazino-7-isobutyl-6-oxo-6,7-dihydro-2H-pyrazolo[3,4-d]pyrimidin-3-yl}-1-methyl-1H-pyrrol-3-carbonitril;
5-[2-[(6-Chlorchinolin-4-yl)methyl]-4-(2,2-dimethylhydrazino)-7-isobutyl-6-oxo-6,7-dihydro-2H-pyrazolo[3,4-d]pyrimidin-3-yl]-1-methyl-1H-pyrrol-3-carbonitril;
N-[2-[(6-Chlorchinolin-4-yl)methyl]-3-(4-cyano-1-methyl-1H-pyrrol-2-yl)-7-isobutyl-6-oxo-6,7-dihydro-2H-pyrazolo[3,4-d]pyrimidin-4-yl]acetamid;
5-[2-[(6-Chlorchinolin-4-yl)methyl]-7-(cyclopropylmethyl)-4-(methylthio)-6-oxo-6,7-dihydro-2H-pyrazolo[3,4-d]pyrimidin-3-yl]-1-methyl-1H-pyrrol-3-carbonitril;
5-{2-[(6-Chlorchinolin-4-yl)methyl]-7-(cyclopropylmethyl)-4-[(2-hydroxybutyl)amino]-6-oxo-6,7-dihydro-2H-pyrazolo[3,4-d]pyrimidin-3-yl}-1-methyl-1H-pyrrol-3-carbonitril;
5-(2-[(6-Chlorchinolin-4-yl)methyl]-7-(cyclopropylmethyl)-4-{[(2R)-2-hydroxypropyl]amino}-6-oxo-6,7-dihydro-2H-pyrazolo[3,4-d]pyrimidin-3-yl)-1-methyl-1H-pyrrol-3-carbonitril;
5-[2-[(6-Chlorchinolin-4-yl)methyl]-7-(cyclopropylmethyl)-4-methoxy-6-oxo-6,7-dihydro-2H-pyrazolo[3,4-d]pyrimidin-3-yl]-1-methyl-1H-pyrrol-3-carbonitril;
5-[2-[(6-Chlorchinolin-4-yl)methyl]-7-(cyclopropylmethyl)-6-oxo-4-(1*H*-pyrrol-1-yl)-6,7-dihydro-2H-pyrazolo[3,4-d]pyrimidin-3-yl]-1-methyl-1H-pyrrol-3-carbonitril;
5-[(6Z)-2-[(6-Chlorchinolin-4-yl)methyl-7-(cyclopropylmethyl)-4-(methylamino)-6-(methylimino)-6,7-dihydro-2H-pyrazolo[3,4-d]pyrimidin-3-yl]-1-methyl-1H-pyrrol-3-carbonitril;
5-[4-Amino-2-[(6-chlorchinolin-4-yl)methyl]-7-(cyclopropylmethyl)-6-oxo-6,7-dihydro-2H-pyrazolo[3,4-*d*]pyrimidin-3-yl]-1-methyl-1*H*-pyrrol-3-carbonitril.

20. Verbindung mit der Strukturformel (IV): 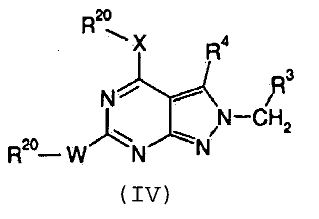 worin
X S, O, NR²¹ ist; oder XR²⁰ Wasserstoff ist;
W S, O oder NR²¹ ist;
R³ ein monocyclisches oder bicyclisches, gesättigtes oder ungesättigtes Ringsystem ist, welches 0, 1, 2 oder 3 Heteroatome umfasst, welche unabhängig aus N, O oder S gewählt sind, wobei der Ring durch 0, 1, 2 oder 3 Substituenten substituiert ist, welche aus =O, Halogen, -OR^{a}, C₁₋₆-Alkyl, C₁₋₆-Halogenalkyl, -CN, Nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{b}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, Aminocarbonyl, Phenyl, Benzyl gewählt sind; oder R³ für -Het, -Het-Het, R⁵, -R⁵-Het, -Het-R⁵, -Het-OR⁵, -R⁵-R⁵, -R⁵-OR⁵ steht;
R⁴ ein monocyclisches oder bicyclisches, gesättigtes oder ungesättigtes Ringsystem oder ein Vizinalkondensiertes Derivat davon ist, welches 5 bis 12, vorzugsweise 5 bis 10, Ringatome enthalten kann, wobei 0, 1, 2, 3 oder 4 davon Heteroatome sind, die unabhängig aus N, 0 oder S gewählt sind, wobei das Ringsystem durch 0, 1, 2 oder 3 Substituenten substituiert ist, welche von B(OH)₂, vizinalem -OCH₂CH₂O-, vizinalem -OC₁₋₂-HalogenalkylO-, vizinalem -OCH₂O-, vizinalem -CH₂OCH₂O-, =O, Halogen, -R^{b}OR^{a}, -SR^{a}, -OR^{a}, C₁₋₆-Alkyl, C₁₋₆haloalkyl, -CN, -S(=O)ₙR^{c}, -O(CH₂)ₘHet, -O(CH₂)ₘC(=O)Het, - O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, - S(CH₂)ₘC(=O)Het, -S(CH₂)mC(=O)NR^{a}R^{a}, -S(CH₂)ₘC(=O)OR^{a}, -S(CH₂)ₘNR^{a}R^{a}, S(CH₂)ₘOR^{a}, -NR^{a}R^{a}, -NHC(=O)R^{a}, NHC(=O)OR^{a}, N=NR^{a}, NO₂, -C(=O)NR^{a}R^{a}, -C(=O)NR^{a}OR^{a}, - C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), - C(=O)OR^{a},-OC(=O)R^{a}, -C(=O)OR^{b}NR^{a}R^{a}, -C(=O)R^{a}, -C(=O)R^{b}NR^{a}R^{a}, -C(=NOR^{a})R^{a},-C(=NCN)R^{a}, -S(=O)₂NR^{a}R^{a}, -NR^{a}S(=O)₂R^{a}, -S(=O)₂NR^{a}(R^{b}C(=O)NR^{a}R^{a}). -S(=O)₂NR^{a}(R^{b}C(=O)OR^{a}), Aminocarbonyl, Phenyl, Benzyl gewählt werden; oder R⁴ für- (CH₂)ₙR⁵-Het , -(CH₂)nR^{d}, -Het, -Het-Het, R⁵, -R⁵-Het, -Het-R⁵, -Het-OR⁵, -R⁵-R⁵ oder -R⁵-OR⁵ steht; oder R⁴ für C₁₋₆-Alkyl, -NC₁₋₆-Alkyl oder -N(C₁₋₆-Alkyl)₂ steht, wobei C₁₋₆-Alkyl, -NC₁₋₆-Alkyl, -N(C₁₋₆-Alkyl) durch 0, 1 oder 2 Substituenten substituiert sind, die aus R^{a}, OR^{a}, Halogen oder Phenyl gewählt sind, wobei R⁴ nicht -(CH₂)_{z}CH₃, -(CH₂)_{z}CH₂OH, -(CH₂)_{z}CO₂H, oder -(CH₂)_{z}CO₂C₁₋₆-Alkyl ist, wobei z 1, 2, 3, 4, 5 oder 6 ist;
R⁵ unabhängig bei jedem Auftreten Phenyl, substituiert durch 0, 1, 2 oder 3 Gruppen, die gewählt sind aus Halogen, C₁₋₆-Halogenalkyl, -OC₁₋₆-Halogenalkyl, C₁₋₆-Alkyl, -CN, Nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, - C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, - C(=O)NR^{a}R^{b}Het, -C(=O)OR^{a}, -OC(=O)R^{a}, -C(-O)OR^{b}NR^{a}R^{a}, -C(=O)R⁸, -C(=O)R^{b}NR^{a}R^{a}, - C(=NOR^{a})R^{a}, -C(=NCN)R², -S(=O)₂NR^{a}R^{a}, -NR^{a}S(=O)₂R^{a},-S(=O)₂NR^{a}R^{b}C(=O)NR^{a}R^{a}, oder-S(=O)₂NR^{a}R^{b}C(=O)OR^{a} gewählt sind, ist;
R²⁰ unabhängig bei jedem Auftreten H, -CN, -S(=O)ₙR^{c}, -C(=O)R^{a}, -C(=O)NR^{a}R^{a}, -C(=O)OR^{a}, -NR^{a}C (=O)R^{a} oder -OC(=O)R^{a}, wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, wahlweise substituierter Heterocyclus ist, wobei die Substitution aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl, C₂₋₄-Alkenyl, C₂₋₄-Alkinyl, C₁₋₄ -Alkoxy, C₁₋₄-Alkanoyl, C₁₋₄-Alkanoyloxy, NH (C₁₋₄-Alkyl), N (C₁₋₄-Alkyl) ₂, C₁₋₄-Alkanoylamino, (C₁₋₄-Alkanoyl)₂-amino, N-(C₁₋₄-Alkyl)carbamoyl, N,N-(C₁₋₄-Alkyl)₂-carbamoyl, (C₁₋₄)S, (C₁₋₄-Alkyl)S(O), (C₁₋₄-Alkyl)S(O)₂, (C₁₋₄)-Alkoxycarbonyl, N- (C₁₋₄-Alkyl) sulfamoyl, N,N-(C₁₋₄-Alkyl)sulfamoyl, C₁₋₄-Alkylsulfonylamino und einem Heterocyclus ist;
R²¹ unabhängig bei jedem Auftreten H, -CN, -S(=O)ₙR^{c}, -C(=O)R^{a}, -C(=O)NR^{a}R^{a}, -C(=O)OR^{a}, -NR^{a}C(=O)R^{a}, oder -OC(=O)R^{a}; wahlweise substituiertes Alkyl, wahlweise substituiertes Alkenyl, wahlweise substituiertes Alkinyl, wahlweise substituiertes Cycloalkyl, wahlweise substituiertes Cycloalkenyl, wahlweise substituiertes Cycloalkinyl, wahlweise substituiertes Aryl, wahlweise substituiertes Alkoxy, wahlweise substituiertes Amino, wahlweise substituierter Heterocyclus ist, wobei die Substitution aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl , C₂₋₄-Alkenyl, C₂₋₄-Alkinyl , C₁₋₄ -Alkoxy, C₁₋₄-Alkanoyl, C₁₋₄-Alkanoyloxy, NH(C₁₋₄-Alkyl), N(C₁₋₄-Alkyl)₂, C₁₋₄-Alkanoylamino, (C₁₋₄-Alkanoyl)₂-amino, N-(C₁₋₄-Alkyl)carbamoyl, N,N-(C₁₋₄-Alkyl)₂-carbamoyl, (C₁₋₄)S, (C₁₋₄-Alkyl)S(O), (C₁₋₄-Alkyl) S (O)₂, (C₁₋₄)-Alkoxycarbonyl, N-(C₁₋₄-Alkyl)-sulfamoyl, N,N-(C₁₋₄-Alkyl)sulfamoyl, C₁₋₄-Alkyl-sulfonylamino und ein Heterocyclus ist;
R²⁰ und R²¹ und das N, an welches sie gebunden sind, in Kombination ebenfalls einen 3- bis 10-gliedrigen, N-verknüpften, gesättigten oder ungesättigten Heterocyclus mit entweder 1 oder 2 Heteroatomen, die unabhängig aus N, O oder S gewählt werden, bilden können, wobei der Heterocyclus durch R^{e} substituiert ist;
R^{a} unabhängig bei jedem Auftreten H, C₁₋₆-Alkyl, -C(=O)C₁₋₄-Alkyl, C₁₋₄-Halogenalkyl, Phenyl, Benzyl oder ein 5- oder 6-gliedriger Ring, gesättigter oder ungesättigter Heterocyclus mit 1, 2, 3 oder 4 Heteroatomen, die unabhängig aus N, O oder S gewählt werden, ist;
R^{b} unabhängig bei jedem Auftreten C₁₋₆-Alkyl, -C(=0)-C₁₋₄-Alkyl, C₁₋₄-Halogenalkyl, Phenyl, Benzyl oder ein 5- oder 6-gliedriger Ring, gesättigter oder ungesättigter Heterocyclus mit 1, 2, 3 oder 4 Heteroatomen, die unabhängig aus N, O oder S gewählt werden, ist;
R^{c} C₁₋₆-Alkyl , C₁₋₄-Halogenalkyl, Phenyl oder Benzyl ist;
R^{d} Phenyl ist, welches durch 0, 1 oder 2 Gruppen substituiert ist, die gewählt werden aus -CN, Halogen, Nitro, C₁₋₆-Alkyl, C₁₋₄-Halogenalkyl, -OH, -OR^{c}, -NR^{a}R^{a} , -S(=O)ₙR^{c}, -C(=O)NR^{a}R^{a}, -C(=O)OR^{a}, -NR^{a}C(=O)R^{a}, -OC(=O)R^{a}, B(OH)₂, Vizinyl-OCH₂CH₂O-, Vizinyl-OC₁₋₂-HalogenalkylO-, Vizinyl-OCH₂O-, Vizinyl-CH₂OCH₂O-, Phenyl, Benzyl und einem 5- oder 6-gliedrigen Ring, gesättigtem oder ungesättigtem Heterocyclus mit 1, 2, 3 oder 4 Heteroatome, die unabhängig aus N, O oder S gewählt werden;
R^{e} unabhängig bei jedem Auftreten H, C₁₋₆-Alkyl, -C(=O)C₁₋₄-Alkyl, C₁₋₄-Halogenalkyl, Phenyl, Benzyl oder einen 5- oder 6-gliedrigen Ring, gesättigtem oder ungesättigtem Heterocyclus mit 1, 2, 3 oder 4 Heteroatome, die unabhängig aus N, O oder S gewählt werden, ist;
m 1, 2 oder 3 ist;
n 0, 1 oder 2 ist.
Wenn "wahlweise substituiert" verwendet wird, bezieht es sich mindestens auf einen Substituenten, der aus Cyclopropyl, Halogen, Nitro, Cyano, Hydroxy, Trifluormethyl, Amino, Carboxy, Carboxamido, Amidino, Carbamoyl, Mercapto, Sulfamoyl, C₁₋₄-Alkyl, C₂₋₄-Alkenyl, C₂₋₄-Alkinyl, C₁₋₄ -Alkoxy, C₁₋₄-Alkanoyl, C₁₋₄-Alkanoyloxy, NH(C₁₋₄-Alkyl), N(C₁₋₄-Alkyl)₂, C₁₋₄-Alkanoylamino, (C₁₋₄-Alkanoyl)₂-amino, N-(C₁₋₄-Alkyl)carbamoyl, N,N-(C₁₋₄-Alkyl)₂-carbamoyl, (C₁₋₄) S, (C₁₋₄-Alkyl)S(O), (C₁₋₄-Alkyl)S(O)₂, (C₁₋₄)-Alkoxycarbonyl, N-(C₁₋₄-Alkyl)sulfamoyl, N,N-(C₁₋₄-Alkyl)sulfamoyl, C₁₋₄-Alkylsulfonylamino und einem Heterocyclus gewählt wird,
oder ein pharmazeutisch annehmbares Salz davon.

21. Verbindung, wie in Anspruch 20 angegeben, worin:
R³ aus den unten dargelegten Formeln (i), (ii), (iii) oder (iv) gewählt wird: 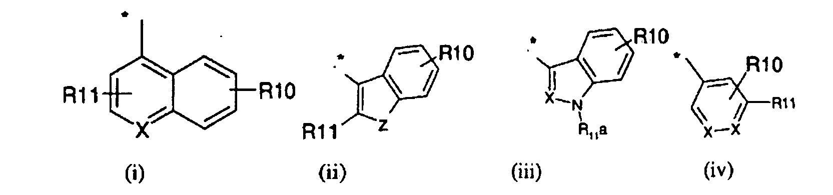
worin * der Ort ist, an dem (i) oder (ii) oder (iii) oder (iv) an der Strukturformel (I) gebunden ist, und X C oder N ist; und Z O oder S ist, wobei R¹⁰ an einer beliebigen Position auf dem Ring ist und R¹⁰ und R¹¹ unabhängig bei jedem Auftreten H, R^{a}, Halogen, -CN, Nitro, OR^{a}, CF₃, -NR^{a}R^{a}, -C(=O)OR^{a}, -C(=O)R^{a}, -C(=O)NR^{a}R^{a}, -OC(=O)C₁₋₄-Alkyl, -NR^{a}C(=O)C₁₋₄ -Alkyl oder -S(=O)ₙR^{c} sind; und worin R^{11a} R^{a}, -S(=O)₂NR^{a}R^{a} oder -S(=O)ₙR^{c} ist und n 1 oder 2 ist.

22. Verbindung, wie in Anspruch 20 angegeben, worin:
R⁴ aus den unten dargelegten Formeln (a) bis (z) oder (aa) oder (ab) gewählt wird: 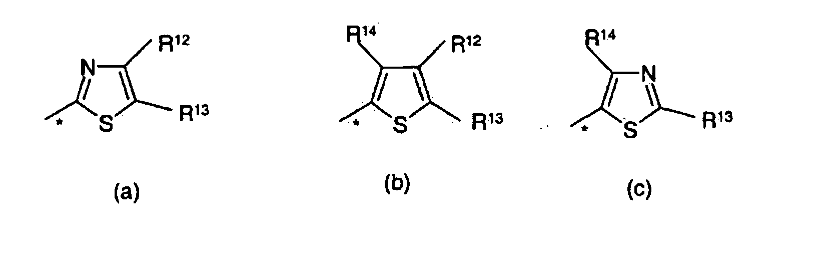 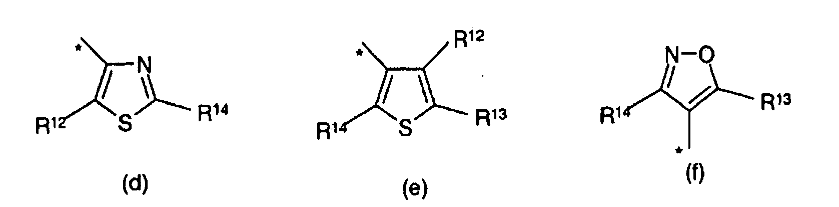 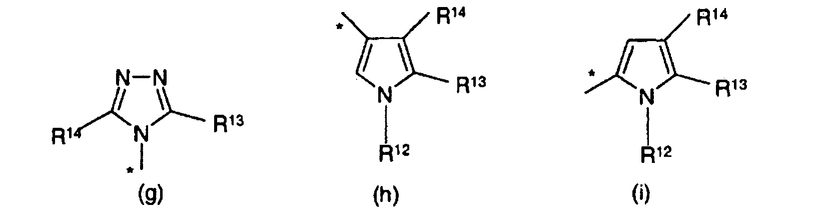 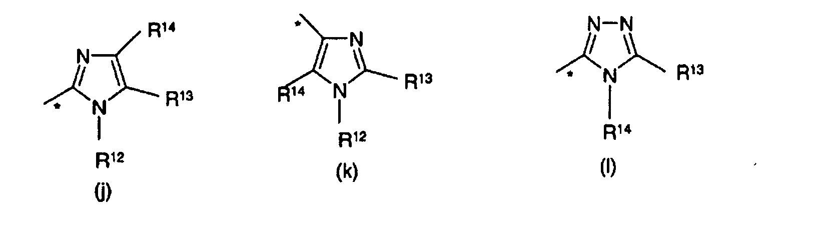 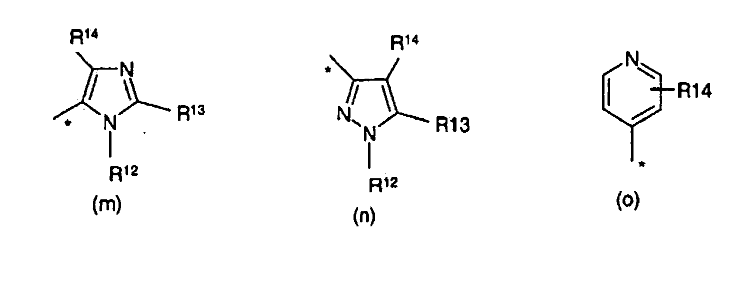 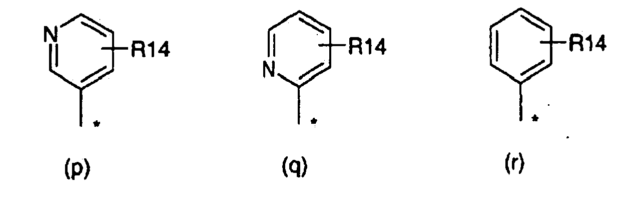 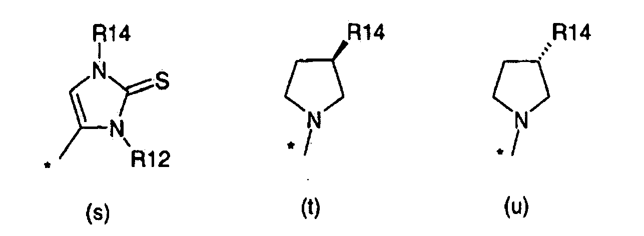 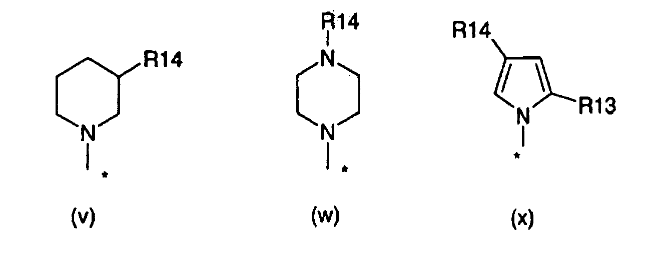 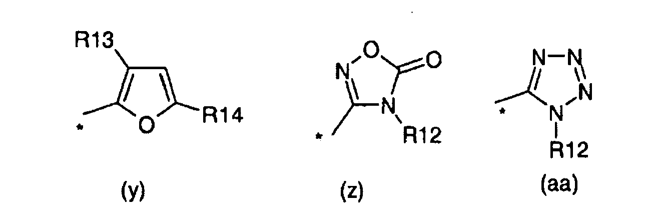 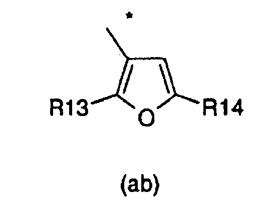
worin * der Ort ist, an dem R⁴ an das Ringsystem gebunden ist, und wobei R¹², R¹³ und R¹⁴ jeweils unabhängig für H, Het, C₁₋₆-Alkyl, -CN, -NR^{a}R^{a}, -Nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), oder-S(=O)₂NR^{a}(R^{b}C(=O)OR^{a} stehen.

23. Verbindung, wie in Anspruch 20 angegeben, worin:
X S, O oder NR²¹ ist; oder X-R²⁰ Wasserstoff ist;
W S, O oder NR²¹ ist;
R¹ CH₃, CH₂CH₃, CH₂CN, CF₃ , (CH₂)₂OH, Cyclopropyl, Isopropyl, CH₂CCH, (CH₂)₂N(CH₂)₂, (CH₂)₂N(C=NH)NH₂, - CH₂-2-Pyridyl, -CH₂-3-Pyridyl, -CH₂-4-Pyridyl, -(CH₂)₂-1-Imidazolyl, -(CH₂)₂-1-Pyrazolyl, -(CH₂)₂-1-Piperidyl, -(CH₂)ₘ-(1-Methylpiperidin-4-yl), -CH₂-(1-Methylpiperidin-3-yl), -(CH₂)₂-(Morpholin-4-yl) ist;
R² -CH₂CH₂CH₃, -CH₂-Cyclopropyl, -CH₂CH(CH₃)₂, -CH₂CH₂-CH₂F, -CH₂-Cyclobutyl, -CH₂C(CH₃) ₃, -CH₂CH₂CH(CH₃)₂, -CH₂CF₃, -CH₂-Methylphenyl, -CH₂-Phenol, -CH₂-(3,5-Dimethylisoxazol-4-yl), -CH₂-S-Phenyl, -CH₂-Phenyl-carboxyl oder -CH₂SCF₃ ist;
R³ aus den unten dargelegten Formeln (i), (ii), (iii) oder (iv) gewählt ist: 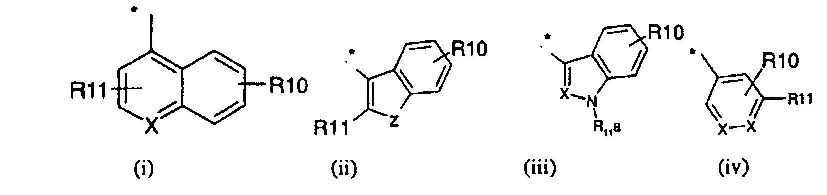 worin * der Ort ist, an dem (i) oder (ii) oder (iii) oder (iv) an der Strukturformel (I) gebunden ist, und X C oder N ist; und Z O oder S ist, wobei R¹⁰ an einer beliebigen Position auf dem Ring ist und R¹⁰ und R¹¹ unabhängig bei jedem Auftreten H, R^{a}, Halogen, -CN, Nitro, OR^{a}, CF₃, -NR^{a}R^{a}, -C(=O)OR^{a}, -C(=O)R^{a}, -C(=O)NR^{a}R^{a}, -OC(=O)C₁₋₄-Alkyl, -NR^{a}C(=O)C₁-4-Alkyl oder -S(=O)ₙR^{c} sind; und worin R^{11a} R^{a}, -S(=O)₂NR^{a}R^{a} oder -S(=O)ₙR^{c} ist und n 1 oder 2 ist.
R⁴ aus den unten dargelegten Formeln (a) bis (z) oder (aa) oder (ab) gewählt wird: 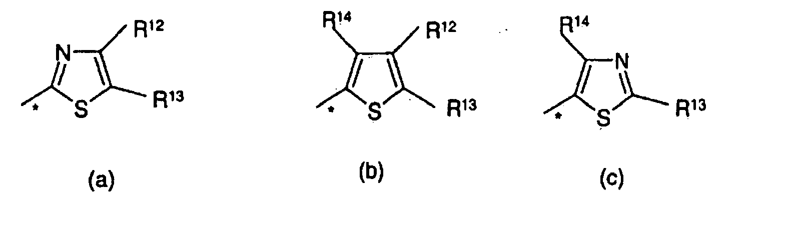 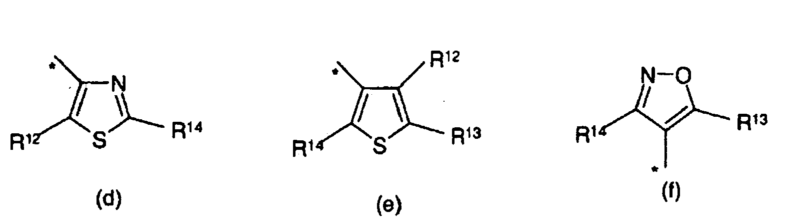 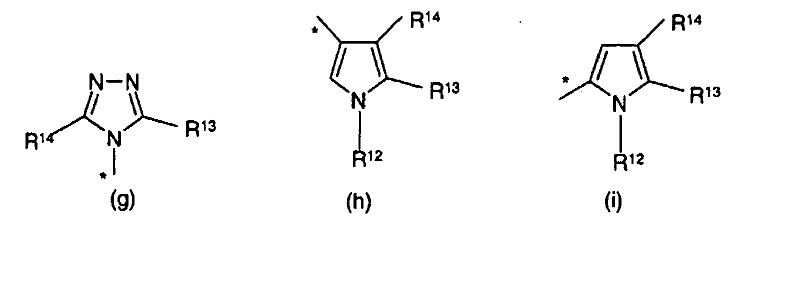 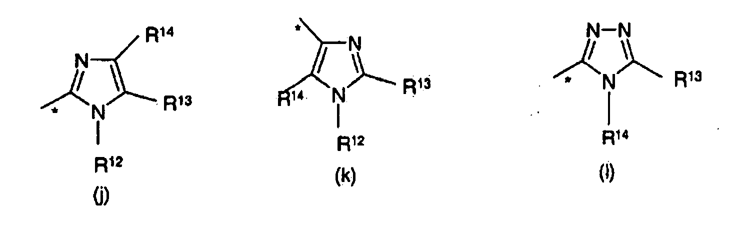 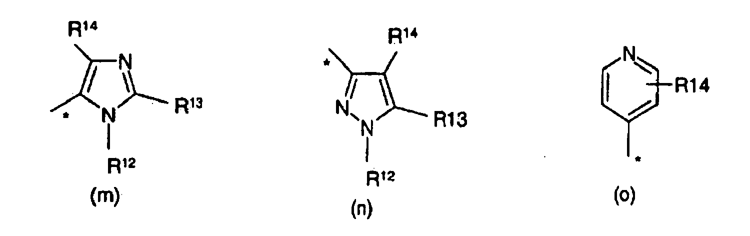 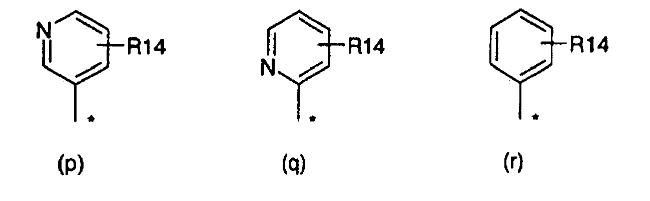 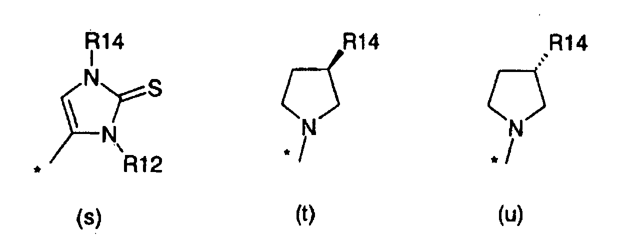 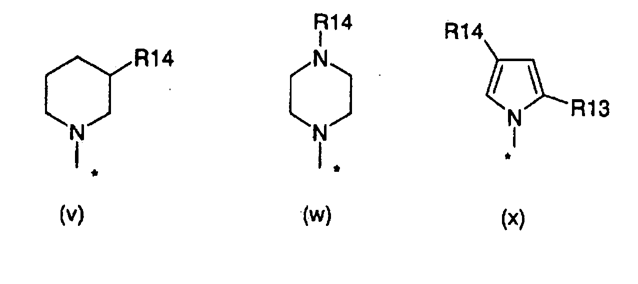 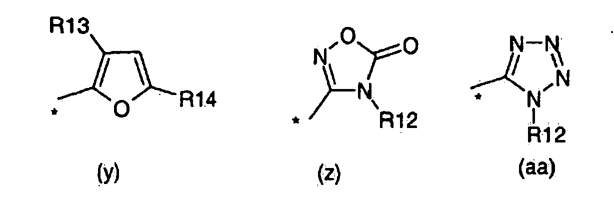 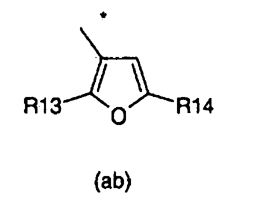 worin * der Ort ist, an dem R⁴ an das Ringsystem gebunden ist, und wobei R¹² , R¹³ und R¹⁴ jeweils unabhängig für H, Het, C₁₋₆-Alkyl, -CN, -NR^{a}R^{a}, -Nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, - C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, - C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, - C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, - C(=NOR^{a})R^{a}, -S(=O)₂R^{a}, -S(=O)₂NR^{a}R^{a}, -S(=O)₂NR^{a}(R^{b}C(=O)NR^{a}R^{a}), oder-S(=O)₂NR^{a}(R^{b}C(=O)OR^{a} stehen.
R²⁰ H, -CN, R^{a}, -OR^{a}, -NR^{a}R^{a}, -Het, -S(=O)ₙR^{a}, -C (=O) - R^{a}, -C(=O)NR^{a}R^{a}, -C(=O)OR^{a}, -NR^{a}C (=O) R^{a} oder -OC(=O)_{R}^{a} ist;
R²⁰ H, -CN, R^{a}, -OR^{a}, -NR^{a}R^{a}, -Het, -S(=O)ₙR^{c}, -C(=O)-R^{a}, -C(=O)NR^{a}R^{a}, -C(=O)OR^{a}, -NR^{a}C(=O)R^{a} oder -OC(=O)R^{a} ist;
R²⁰ und R²¹ und das N, an welches sie gebunden sind, in Kombination ebenfalls einen 3- bis 10-gliedrigen, N-verknüpften, gesättigten oder ungesättigten Heterocyclus mit entweder 1 oder 2 Heteroatomen, die unabhängig aus N, 0 oder S gewählt werden, sind, wobei der Heterocyclus durch R^{e} substituiert ist;
R^{e} unabhängig bei jedem Auftreten H, C₁₋₆-Alkyl, -C(=O)C₁₋₄-Alkyl, C₁₋₄-Halogenalkyl, Phenyl, Benzyl oder ein 5- oder 6-gliedriger Ring, gesättigter oder ungesättigter Heterocyclus mit 1, 2, 3 oder 4 Heteroatomen, die unabhängig aus N, 0 oder S gewählt werden, ist.

24. Verbindung der Formel (IV), gewählt aus:
5-[2-[(6-Chlorchinolin-4-yl)methyl]-6-[(cyclopropylmethyl)amino]-4-(methylamino)-2*H-*pyrazolo[3,4-*d*]pyrimidin-3-yl]-1-methyl-1*H*-pyrrol-3-carbonitril;
N-{3-(4-Acetyl-1-methyl-1*H*-pyrrol-2-yl)-2-[(6-chlorchinolin-4-yl)methyl]-4-methoxy-2*H-*pyrazolo[3,4,-*d*]pyrimidin-6-yl}-2-cyclopropylacetamid.

25. Verbindung gemäß mindestens einem der Ansprüche 1 bis 24, zur Verwendung als ein Medikament.

26. Verwendung einer Verbindung, wie in mindestens einem der Ansprüche 1 bis 24 definiert, bei der Herstellung eines Medikaments zur Behandlung oder Prophylaxe von Störungen, die mit einer *H. pylori-*Infektion assoziiert sind.

27. Pharmazeutische Zusammensetzung, die eine Verbindung umfasst, wie sie in mindestens einem der Ansprüche 1 bis 24 definiert ist, und zwar zusammen mit mindestens einem pharmazeutisch annehmbaren Träger, Verdünnungsmittel oder Exzipient.

## Revendications

1. Composé ayant la formule structurale (I) : 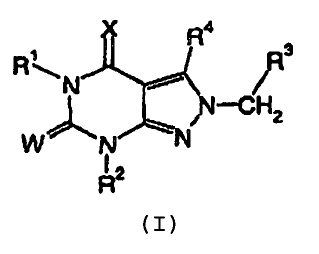 dans laquelle,
X est S, O, ou NR²⁰, à condition que lorsque W est 0, alors X n'est pas 0,
X et la double liaison à laquelle il est lié peuvent être remplacés par 2 atomes d'hydrogène,
W est S, O, ou NR²⁰, à condition que lorsque X est 0, alors W n'est pas O ;
R¹ est H, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un hydroxy, un amino, ou un hétérocycle facultativement substitué ;
R² est H, un alkyle facultativement substitué, un alkylcycloalkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, ou un hétérocycle facultativement substitué ;
R³ est un système cyclique, saturé ou insaturé, monocyclique ou bicyclique, comprenant 0, 1, 2 ou 3 hétéroatomes indépendamment choisis parmi N, 0, ou S, le cycle étant substitué par 0, 1, 2 ou 3 substituants choisis parmi =O, un halogène, -OR^{a}, un alkyle en C₁₋₆, un halogénoalkyle en C₁₋₆, -CN, le nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂) ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, l'aminocarbonyle, le phényle ou le benzyle ; ou R³ est représenté par -Het, -Het-Het, R⁵, -R⁵-Het, -Het-R⁵, -Het-O-R⁵, -R⁵-R⁵, -R⁵-OR⁵ ;
R⁴ est un système cyclique, saturé ou insaturé, monocyclique ou bicyclique, ou un dérivé vicinal condensé de celui-ci, qui peut contenir de 5 à 12, de préférence de 5 à 10, atomes cycliques, dont 0, 1, 2, 3 ou 4 sont des hétéroatomes indépendamment choisis parmi N, O, ou S, le système cyclique étant substitué par 0, 1, 2 ou 3 substituants choisis parmi B(OH)₂, -OCH₂CH₂O-vicinal, -O(halogénoalkyle en C₁₋₂)O- vicinal, -OCH₂O-vicinal, -CH₂OCH₂O- vicinal, =0, un halogène, -R^{b}OR^{a}, -SR^{a}, -OR^{a}, un alkyle en C₁₋₆, un halogénoalkyle en C₁₋₆, -CN, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a} , -NHC(=O)R^{a}, -NHC(=O)OR^{a}, N=NR^{a} , NO₂, -C(=O)NR^{a}R^{a}, -C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), -C(=O)OR^{a}, -OC(=O)R^{a}, -C(=O)OR^{b}R^{a}R^{a}, -C(=O)R^{a}, -C(=O)R^{b}R^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), -S(=O)₂NR^{a}(R^{b}C(=O)OR^{a}), l'aminocarbonyle, le phényle ou le benzyle ; ou R⁴ est représenté par -(CH₂)ₙR⁵-Het, -(CH₂)ₙR^{d}, -Het, -Het-Het, R⁵, -R⁵-Het, -Het-R⁵, -Het-OR⁵, R⁵-R⁵, ou -R⁵-OR⁵ ; ou R⁴ est représenté par un alkyle en C₁₋₆, -N(alkyle en C₁₋₆), ou -N(alkyle en C₁₋₆)₂ dans lequel alkyle en C₁₋₆, -N(alkyle en C₁₋₆), ou -N(alkyle en C₁₋₆)₂ sont substitués par 0, 1 ou 2 substituants choisis parmi R^{a}, OR^{a}, un halogène ou un phényle où R⁴ n'est pas -(CH₂)_{z}CH₃, -(CH₂)_{z}CH₂OH, -(CH₂)_{z}CO₂H, ou -(CH₂)_{z}CO₂(alkyle en C₁₋₆) où z est 1, 2, 3, 4, 5, ou 6 ;
R⁵ est, indépendamment dans chaque cas, un phényle substitué par 0, 1, 2 ou 3 groupes choisis parmi un halogène, un halogénoalkyle en C₁₋₆, -0(halogénoalkyle en C₁₋₆), un alkyle en C₁₋₆, -CN, le nitro, -OR^{a}, -S(=O)ₙR^{c}, -O(CH₂)ₘHet, -O(CH₂)ₘC(=O) Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet , -S(CH₂)ₘC(=O)Het, -S(CH₂)ₘC(=O)NR^{a}R^{a},-S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, -C(=O)NR^{a}OR^{a}, -C(=O)NR^{a}R^{b}NR^{a}R^{a}, -C(=)NR^{a}R^{b}OR^{a}, -C(=O)NR^{a}R^{b}S(=O)ₙR^{a}, -C(=O)NR^{a}R^{b}Het, -C(=O)OR^{a}, -OC(=O)R^{a}, -C(=O)OR^{b}R^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, ou -S(=O)₂NR^{a}R^{b}C(=O)OR^{a} ;
R²⁰ est, indépendamment dans chaque cas, H, -CN, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, un hétérocycle facultativement substitué, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a};
R^{a} est, indépendamment dans chaque cas, H, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, O, ou S;
R^{b} est, indépendamment dans chaque cas, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, 0, ou S ;
R^{c} est un alkyle en C₁₋₆, un halogénoalkyle en C₁₋₄, le phényle ou le benzyle ;
R^{d} est un phényle substitué par 0, 1 ou 2 groupes choisis parmi -CN, un halogène, le nitro, un alkyle en C₁₋₆, un halogénoalkyle en C₁₋₄, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, -OCH₂CH₂O- vicinal, -O(halogénoalkyle en C₁₋₂)O-vicinal, -OCH₂O- vicinal, -CH₂OCH₂O- vicinal, le phényle, le benzyle, et un hétérocycle saturé ou insaturé, à cycle à 5 ou 6 chaînons contenant 1, 2, 3 ou 4 hétéroatomes indépendamment choisis parmi N, 0, ou S ;
m est 1, 2 ou 3 ;
n est 0, 1 ou 2 ;
lorsque "facultativement substitué" est utilisé, il désigne au moins un substituant choisi parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyle en C₁₋₄), N(alkyle en C₁₋₄)₂, un alcanoylamino en C₁₋₄, (alcanoyle en C₁₋₄ )₂amino, N-(alkyle en C₁₋₄)carbamoyle, N,N-(alkyle en C₁₋₄)₂carbamoyle, (C₁₋₄)S, (alkyle en C₁₋₄)S(O), (alkyle en C₁₋₄)S(O)₂, (alcoxy en C₁₋₄)carbonyle, N- (alkyle en C₁₋₄) sulfamoyle, N,N-(alkyle en C₁₋₄)sulfamoyle, (alkyle en C₁₋₄)sulfonylamino, et un hétérocycle
ou un sel pharmaceutiquement acceptable de celui-ci.

2. Composé selon la revendication 1 dans lequel :
R¹ est H, ou un alkyle en C₁₋₆, ou -(CH₂)ₙcycloalkyle ou -(CH₂)₁₋₂Het dans lequel l'alkyle en C₁₋₆ ou -(CH₂)ₙcycloalkyle ou -(CH₂)₁₋₂Het est facultativement substitué par 1, 2 ou 3 substituants choisis parmi Het, un halogène, -CN, -OR^{a}, -NR^{a}R^{a}, -C (=O) OR^{a}, -C (=O) NR^{a}R^{a}, -OC(=O)(alkyle en C₁₋₄), -S(=O)ₙR^{c}, -S(=O)ₙNR^{a}R^{a} ou -NRₐC (=O) (alkyle en C₁₋₄) et n est 0, 1 ou 2.

3. Composé selon la revendication 1 dans lequel :
R² est -(CH₂)₁₋₃cycloalkyle ou un alkyle en C₁₋₁₂ dans lequel - (CH₂) ₁₋₃cycloalkyle ou l' alkyle en C₁₋₁₂ est facultativement substitué par 0, 1, 2 ou 3 substituants choisis parmi Het, -S(=O)ₙR^{c}, -S(=O)ₙNR^{a}R^{a}, un halogène, -CN , -OR^{a} , -NR^{a}R^{a} , -C(=O)OR^{a}, -C(=O)R^{a}, -C(=O)NR^{a}R^{a}, -OC (=O) (alkyle en C₁₋₄), ou -NR^{a}C(=O) (alkyle en C₁₋₄) et n est 0, 1 ou 2.

4. Composé selon la revendication 1 dans lequel :
R³ est choisi parmi les formules (i), (ii), (iii) ou (iv) décrites ci-dessous : 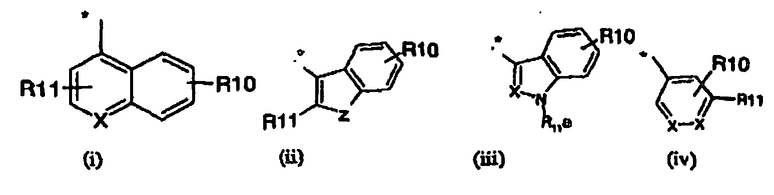
dans lesquelles * est l'emplacement où (i) ou (ii) ou (iii) ou (iv) est lié à la formule structurale (I), et X est C ou N ; et Z est O ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C (=O)OR^{a}, -C(=O)R^{a}, -C(=O)NR^{a}R^{a}, -OC(=O)(alkyle en C₁₋₄), -NR^{a}C(=O)(alkyle en C₁₋₄) ou -S(=O)ₙR^{c} ; et dans lesquelles R^{11a} est R^{a}, -S (=O)₂NR^{a}R^{a} ou -S(=O)ₙR^{c} et n = 1 ou 2.

5. Composé selon la revendication 1 dans lequel :
R⁴ est choisi parmi les formules (a) à (z) ou (aa) ou (ab) décrites ci-dessous : 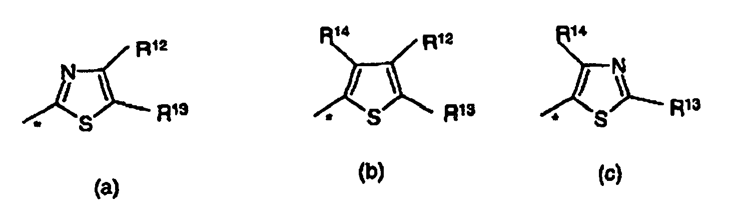 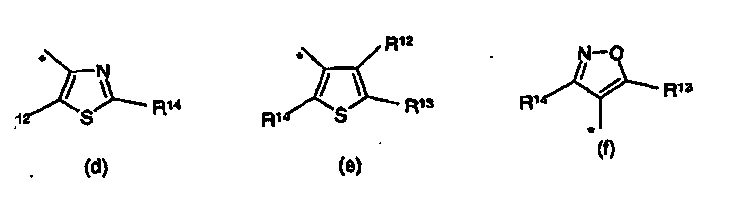 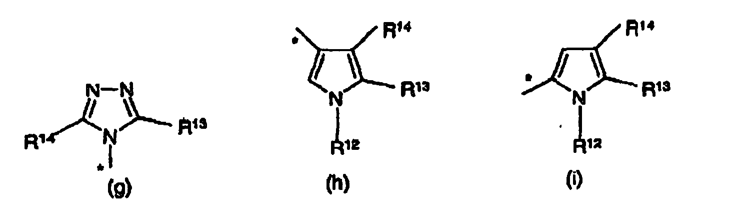 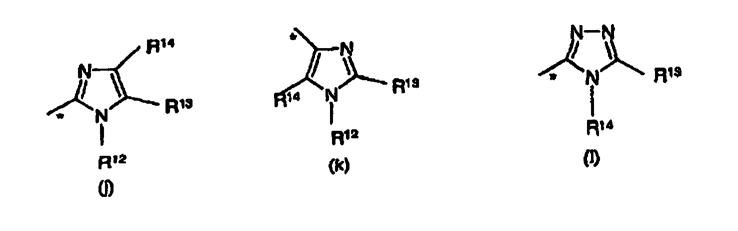 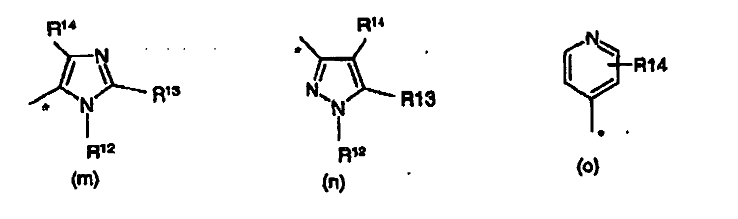 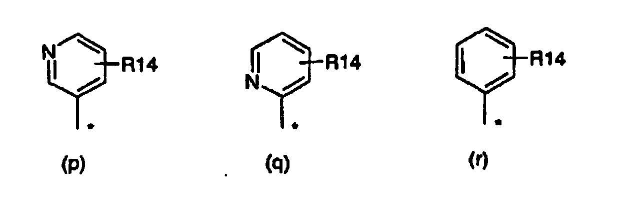 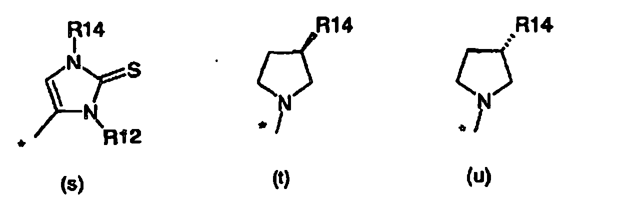 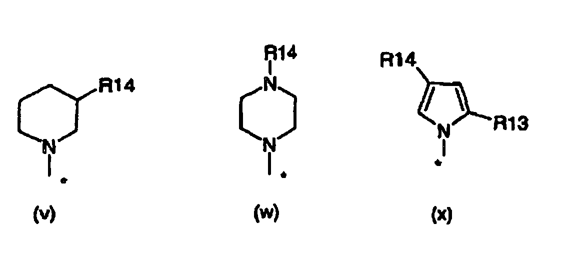 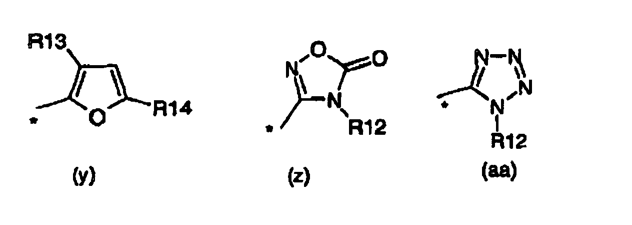 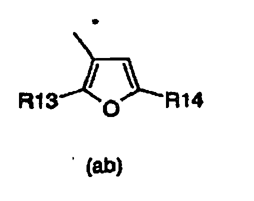
dans lesquelles * est l'emplacement auquel R⁴ est lié au système cyclique et dans lesquelles R¹², R¹³ et R¹⁴ sont chacun indépendamment représentés par H, Het, un alkyle en C₁₋₆, -CN, -NR^{a}R^{a}, le nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).

6. Composé selon la revendication 1 dans lequel :
X est S, O, ou NR²⁰, à condition que lorsque W est 0, alors X n'est pas O ; ou X et la double liaison à laquelle il est lié peuvent être 2 atomes d'hydrogène,
W est S, O, ou NR²⁰, à condition que lorsque X est 0, alors W n'est pas O ;
R²⁰ est H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a};
R¹ est CH₃, CH₂CH₃, CH₂CN, CF₃, (CH₂)₂OH, le cyclopropyle, l'isopropyle, CH₂CCH, (CH₂) ₂N (CH₂) ₂, (CH₂)₂N(C=NH)NH₂, -CH₂-2-pyridyle, -CH₂-3-pyridyle, -CH₂-4-pyridyle, -(CH₂)₂-1-imidazolyle, -(CH₂)₂-1-pyrazolyle, -(CH₂)₂-1-pipéridyle, -(CH₂)ₘ-(1-méthylpipéridin-4-yle), -CH₂-(1-méthylpipéridin-3-yle), -(CH₂)₂-(morpholin-4-yle),
R² est -CH₂CH₂CH₃, -CH₂-cyclopropyle, -CH₂CH(CH₃)₂, -CH₂CH₂CH₂F, -CH₂-cyclobutyle, -CH₂C(CH₃)₃, -CH₂CH₂CH(CH₃)₂, -CH₂CF₃, -CH₂-méthylphényle, -CH₂-phénol, -CH₂-(3,5-diméthylisoxazol-4-yle), -CH₂-S-phényle, -CH₂-phénylcarboxyle, ou -CH₂SCF₃ ;
R³ est choisi parmi les formules (i), (ii), (iii) ou (iv) décrites ci-dessous : 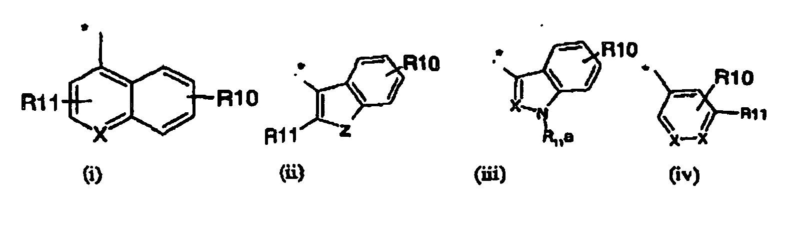 dans lesquelles * est l'emplacement où (i) ou (ii) ou (iii) ou (iv) est lié à la formule structurale (I), et X est C ou N ; et Z est 0 ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C(=O)OR^{a}, -C(=O)R^{a}, -C(=O)NR^{a}R^{a}, -OC(=O)(alkyle en C₁₋₄), -NR^{a}C(=O)(alkyle en C₁₋₄) ou -S(=O)ₙR^{c} ; et dans lesquelles R^{11a} est R^{a}, -S(=O)₂NR^{a}R^{a} ou -S(=O)ₙR^{c} et n = 1 ou 2,
R⁴ est choisi parmi les formules (a) à (z) ou (aa) ou (ab) décrites ci-dessous : 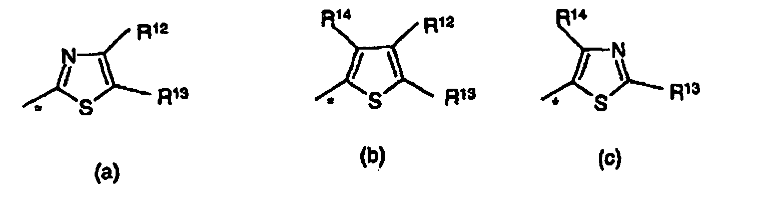 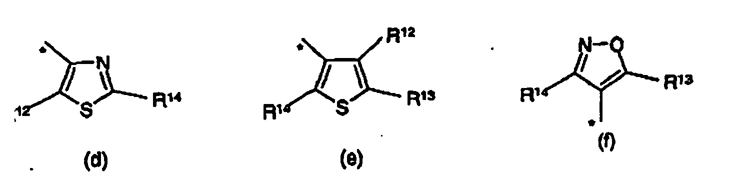 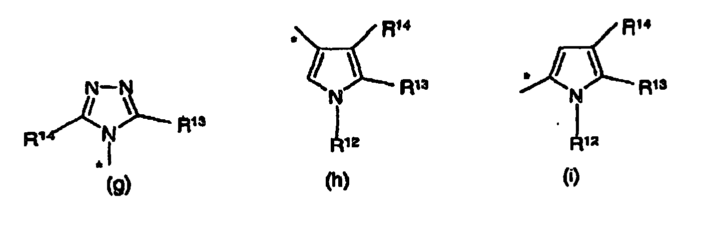 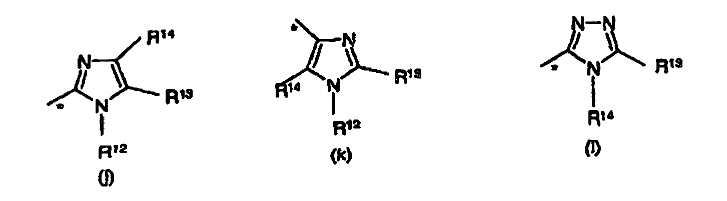 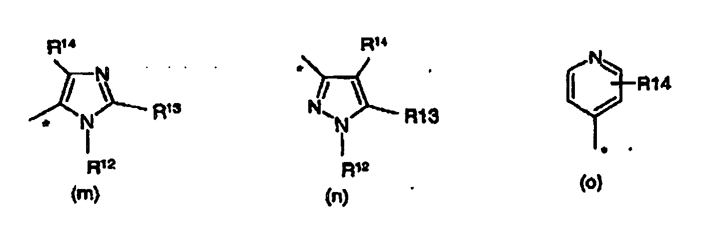 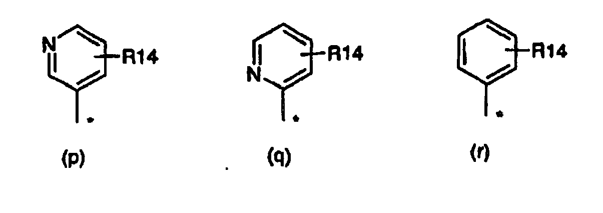 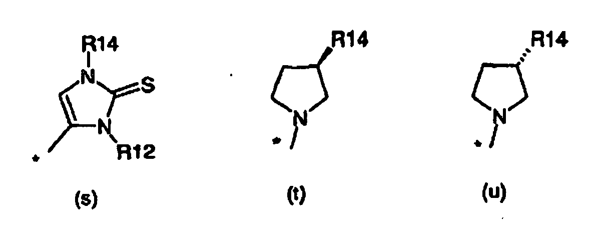 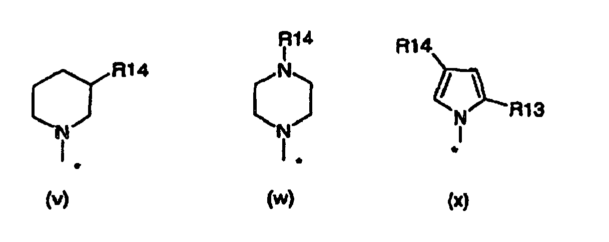 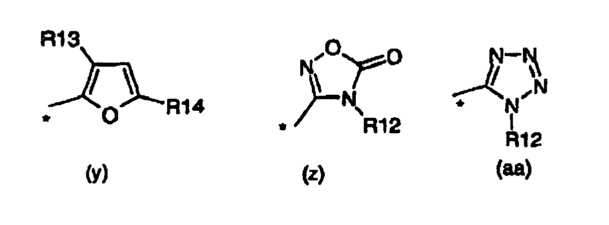 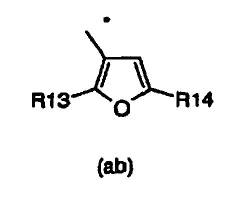
dans lesquelles * est l'emplacement auquel R⁴ est lié au système cyclique et dans lesquelles R¹², R¹³ et R¹⁴ sont chacun indépendamment représentés par H, Het, un alkyle en C₁₋₆, -CN, -NR^{a}R^{a}, le nitro, -C(=O)R^{a}, -C(=O) NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a} , -C(=O)C)RbNR^{a}R^{a}, -C (=O) R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).

7. Composé de formule (I) choisi parmi :
le 5-[2-[(6-chloroquinoléin-4-yl)méthyl]-7-(cyclopropylméthyl)-6-oxo-4-thioxo-4,5,6,7-tétrahydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-(cyclopropylméthyl)-4-imino-5-méthyl-6-oxo-4,5,6,7-tétrahydro-2H-pyrazolo[3,4-d]pyrimidin-3-yl]-1-méthyl-1H-pyrrole-3-carbonitrile ;
le 5-[(4Z)-2-[(6-chloroquinoléin-4-yl)méthyl]-7-(cyclopropylméthyl)-5-méthyl-4-(méthylimino)-6-oxo-4,5,6,7-tétrahydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-(cyclopropylméthyl)-6-imino-5-méthyl-4-oxo-4,5,6,7-tétrahydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-(cyclopropylméthyl)-5-méthyl-4-oxo-6-thioxo-4,5,6,7-tétrahydro-2H-pyrazolo[3,4-d]pyrimidin-3-yl]-1-méthyl-1H-pyrrole-3-carbonitrile ;
le 5-[(6Z)-2-[(6-chloroquinoléin-4-yl)méthyl]-7-(cyclopropylméthyl)-5-méthyl-6-(méthylimino)-4-oxo-4,5,6,7-tétrahydro-2H-pyrazolo[3,4-d]pyrimidin-3-yl]-1-méthyl-1H-pyrrole-3-carbonitrile ;
le N-[(6Z)-2-[(6-chloroquinoléin-4-yl)méthyl]-3-(4-cyano-1-méthyl-1H-pyrrol-2-yl)-7-(cyclopropylméthyl)-5-méthyl-4-oxo-2,4,5,7-tétrahydro-6H-pyrazolo[3,4-d]pyrimidin-6-ylidène]acétamide ;
le N-[(6Z)-2-[(6-chloroquinoléin-4-yl)méthyl]-3-(4-cyano-1-méthyl-1H-pyrrol-2-yl)-7-(cyclopropylméthyl)-5-méthyl-4-oxo-2,4,5,7-tétrahydro-6H-pyrazolo[3,4-d]pyrimidin-6-ylidène]méthanesulfonamide ;
le 5-[(6Z)-2-[(6-chloroquinoléin-4-yl)méthyl]-7-(cyclopropylméthyl)-6-{[2-(diméthylamino)éthyl]imino}-5-méthyl-4-oxo-4,5,6,7-tétrahydro-2H-pyrazolo[3,4-d]pyrimidin-3-yl]-1-méthyl-1H-pyrrole-3-carbonitrile ;
le N~1~-[2-[(6-chloroquinoléin-4-yl)méthyl]-3-(4-cyano-1-méthyl-1H-pyrrol-2-yl)-7-(cyclopropylméthyl)-5-méthyl-4-oxo-2,4,5,7-tétrahydro-6H-pyrazolo[3,4-d]pyrimidin-6-ylidène]-N~2~,N~2~-diméthylglycinamide;
le 5-[2-[(6-chloroquinoléin-4-yl)méthyl]-7-(cyclopropylméthyl)-6-oxo-4,5,6,7-tétrahydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-(cyclopropylméthyl)-5-méthyl-6-oxo-4,5,6,7-tétrahydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-(cyclopropylméthyl)-6-oxo-6,7-dihydro-2H-pyrazolo[3,4-d]pyrimidin-3-yl]-1-méthyl-1H-pyrrole-3-carbonitrile ;
la 2-[(6-chloroquinoléin-4-yl)méthyl]-7-(cyclopropylméthyl)-5-méthyl-3-(1-méthyl-1H-imidazol-5-yl)-4-thioxo-2,4,5,7-tétrahydro-6H-pyrazolo[3,4-d]pyrimidin-6-one ;
la (4Z)-2-[(6-chloroquinoléin-4-yl)méthyl]-7-(cyclopropylméthyl)-5-méthyl-3-(1-méthyl-1H-imidazol-5-yl)-4-(méthylimino)-2,4,5,7-tétrahydro-6H-pyrazolo[3,4-d]pyrimidin-6-one.

8. Composé ayant la formule structurale (II) : 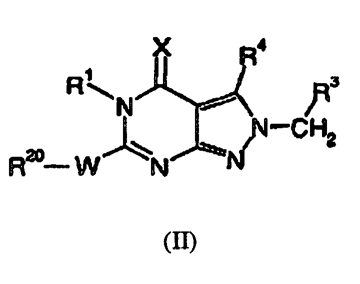 dans lequel,
X est S, O, ou NR²⁰,
X et la double liaison à laquelle il est lié peuvent être remplacés par 2 atomes d'hydrogène,
W est S, O, ou NR²¹;
R¹ est H, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un hydroxy, un amino, ou un hétérocycle facultativement substitué ; dans lequel le substituant est choisi parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyleenC₁₋₄), N(alkyle en C₁₋₄)₂, un alcanoylamino en C₁₋₄, (alcanoyle en C₁₋₄ )₂amino, N-(alkyle en C₁₋₄)carbamoyle, N,N-(alkyle en C₁₋₄)₂carbamoyle, (C₁₋₄)S, (alkyle en C₁₋₄)S(O), (alkyle en C₁₋₄)S(O)₂, (alcoxy en C₁₋₄)carbonyle, N- (alkyle en C₁₋₄)sulfamoyle, N,N- (alkyle en C₁₋₄) sulfamoyle, (alkyle en C₁₋₄)sulfonylamino, et un hétérocycle ;
R³ est un système cyclique, saturé ou insaturé, monocyclique ou bicyclique, comprenant 0, 1, 2 ou 3 hétéroatomes indépendamment choisis parmi N, 0, ou S, le cycle étant substitué par 0, 1, 2 ou 3 substituants choisis parmi =0, un halogène, -OR^{a}, un alkyle en C₁₋₆, un halogénoalkyle en C₁₋₆, -CN, le nitro, -S(=O)ₙR^{c}, -O(CH₂)ₘHet , -O(CH₂)ₘC(=O)Het, -O(CH₂)ₘC(=O)NR^{a}R^{a}, -O(CH₂)ₘC(=O)OR^{a}, -O(CH₂)NR^{a}R^{a}, -O(CH₂)ₘOR^{a}, -S(CH₂)Het, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, l'aminocarbonyle, le phényle ou le benzyle ; ou R³ est représenté par -Het, -Het-Het, R⁵, -R⁵-Het, -Het-R⁵, -Het-O-R⁵, -R⁵-R⁵ -R⁵-OR⁵ ;
R⁴ est un système cyclique, saturé ou insaturé, monocyclique ou bicyclique, ou un dérivé vicinal condensé de celui-ci, qui peut contenir de 5 à 12, de préférence de 5 à 10, atomes cycliques, dont 0, 1, 2, 3 ou 4 sont des hétéroatomes indépendamment choisis parmi N, O, ou S, le système cyclique étant substitué par 0, 1, 2 ou 3 substituants choisis parmi B(OH)₂, -OCH₂CH₂O-vicinal, -O(halogénoalkyle en C₁₋₂)O- vicinal, -OCH₂O-vicinal, -CH₂OCH₂O- vicinal, =0, un halogène, -R^{b}OR^{a}, -SR^{a}, -OR^{a}, un alkyle en C_{1-6,} un halogénoalkyle en C₁₋₆, -CN, -S(=O)ₙR^{c}, -O(CH₂)ₘHet, -O(CH₂)ₘC(=O)Het, -O(CH₂)ₘC(=O)NR^{a}R^{a}, -O(CH₂)C(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a} , NO₂, -C(=O)NR^{a}R^{a}, -C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), -C(=O)OR^{a}, -OC(=O)R^{a}, -C(=O)OR^{b}R^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), -S(=O)₂NR^{a}(R^{b}C(=O)OR^{a}), l'aminocarbonyle, le phényle ou le benzyle ; ou R⁴ est représenté par -(CH₂)ₙR⁵-Het, -(CH₂)ₙR^{d}, -Het, -Het-Het, R⁵, -R⁵-Het, -Het-R⁵, -Het-OR⁵, R⁵-R⁵, ou -R⁵-OR⁵ ; ou R⁴ est représenté par un alkyle en C₁₋₆, -N(alkyle en C₁₋₆), ou -N(alkyle en C₁₋₆)₂ dans lequel alkyle en C₁₋₆, -N(alkyle en C₁₋₆), ou -N(alkyle en C₁₋₆)₂ sont substitués par 0, 1 ou 2 substituants choisis parmi R^{a}, OR^{a}, un halogène ou un phényle où R⁴ n'est pas -(CH₂)_{z}CH₃, -(CH₂)_{z}CH₂OH, -(CH₂)_{z}CO₂H, ou -(CH₂)_{z}CO₂ (alkyle en C₁₋₆) où z est 1, 2, 3, 4, 5, ou 6 ;
R⁵ est, indépendamment dans chaque cas, un phényle substitué par 0, 1, 2 ou 3 groupes choisis parmi un halogène, un halogénoalkyle en C₁₋₆, -O(halogénoalkyle en C₁₋₆), un alkyle en C₁₋₆, -CN, le nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)_{O}R^{a}, -S(CH₂)ₘNR^{a}R^{a}, -S(CH₂)ₘOR^{a}, -R^{b}OR^{a}, -SR^{a}, -C(=O)NR^{a}R^{a}, -C(=O)NR^{a}OR^{a}, -C(=O)NR^{a}R^{b}R^{a}R^{a}, -C(=O)NR^{a}R^{b}OR^{a}, -C(=O)NR^{a}R^{b}S(=O)ₙR^{a}, -C(=O)NR^{a}R^{b}Het, -C(=O)OR^{a}, -OC(=O)R^{a}, -C (=O) OR^{b}NR^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, ou -S(=O)₂NR^{a}R^{b}C(=O)OR^{a};
R²⁰ est, indépendamment dans chaque cas, H, -CN, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a}, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, un hétérocycle facultativement substitué, dans lequel la substitution est choisie parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyle en C₁₋₄), N(alkyle en C₁₋₄)₂, un alcanoylamino en C₁₋₄, (alcanoyle en C₁₋₄)₂amino, N- (alkyle en C₁₋₄) carbamoyle, N,N-(alkyle en C₁₋₄)₂carbamoyle, (C₁₋₄) S, (alkyle en C₁₋₄)S(O), (alkyle en C₁₋₄)S(O)₂, (alcoxy en C₁₋₄) carbonyle, N-(alkyle en C₁₋₄)sulfamoyle, N,N-(alkyle en C₁₋₄) sulfamoyle, (alkyle en C₁₋₄) sulfonylamino, et un hétérocycle ;
R²¹ est, indépendamment dans chaque cas, H, -CN, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a}, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, un hétérocycle facultativement substitué, dans lequel la substitution est choisie parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyle en C₁₋₄), N(alkyle en C₁₋₄)₂, un alcanoylamino en C₁₋₄, (alcanoyle en C₁₋₄)₂amino, N- (alkyle en C₁₋₄)carbamoyle, N,N-(alkyle en C₁₋₄)₂carbamoyle, (C₁₋₄)S, (alkyle en C₁₋₄)S(O), (alkyle en C₁₋₄)S(O)₂, (alcoxy en C₁₋₄) carbonyle, N- (alkyle en C₁₋₄)sulfamoyle, N,N- (alkyle en C₁₋₄) sulfamoyle, (alkyle en C₁₋₄)sulfonylamino, et un hétérocycle ;
R²⁰ et R²¹ et le N auquel ils sont liés en combinaison peuvent également former un hétérocycle saturé ou insaturé à liaison N de 3 à 10 chaînons ayant 1 ou 2 hétéroatomes indépendamment choisis parmi N, O ou S l'hétérocycle étant substitué par R^{e} ;
R^{a} est, indépendamment dans chaque cas, H, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, 0, ou S;
R^{b} est, indépendamment dans chaque cas, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, O, ou S;
R^{c} est un alkyle en C₁₋₆, un halogénoalkyle en C₁₋₄, le phényle ou le benzyle;
R^{d} est un phényle substitué par 0, 1 ou 2 groupes choisis parmi -CN, un halogène, le nitro, un alkyle en C₁₋₆, un halogénoalkyle en C₁₋₄, -OH, -OR^{c}, -NR^{a}R^{a}, -S(=O)nR^{c}, -C(=O)NR^{a}R^{a}, -C(=O)OR^{a}, -NR^{a}C(=O)R^{a}, -OC(=O)R^{a}, B(OH)₂, -OCH₂CH₂O- vicinal, -O (halogénoalkyle en C₁₋₂)O-vicinal, -OCH₂O- vicinal, -CH₂OCH₂O- vicinal, le phényle, le benzyle, et un hétérocycle saturé ou insaturé, à cycle à 5 ou 6 chaînons contenant 1, 2, 3 ou 4 hétéroatomes indépendamment choisis parmi N, O, ou S ;
R^{e} est, indépendamment dans chaque cas, H, un alkyle en C₁₋₆, -C (=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, O, ou S ;
m est 1, 2 ou 3 ;
n est 0, 1 ou 2 ;
ou un sel pharmaceutiquement acceptable de celui-ci.

9. Composé selon la revendication 8 dans lequel :
R¹ est H, ou un alkyle en C₁₋₆, ou -(CH₂)ₙcycloalkyle dans lequel l'alkyle en C₁₋₆ ou -(CH₂)ₙcycloalkyle est facultativement substitué par 1, 2 ou 3 substituants choisis parmi Het, un halogène, -CN, -OR^{a}, -NR^{a}R^{a}, -C(=O)OR^{a}, -C(=O)NR^{a}R^{a}, -OC(=O) (alkyle en C₁₋₄), ou -NR^{a}C(=O) (alkyle en C₁₋₄) et n est 0, 1 ou 2.

10. Composé selon la revendication 8 dans lequel :
R³ est choisi parmi les formules (i), (ii), (iii) ou (iv) décrites ci-dessous : 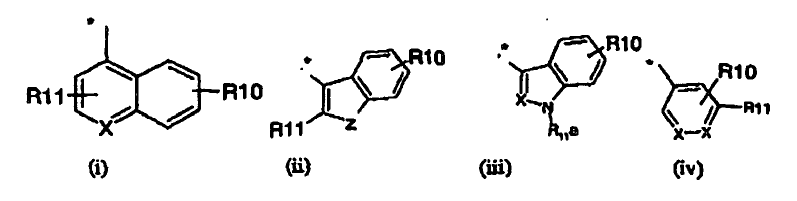
dans lesquelles * est l'emplacement où (i) ou (ii) ou (iii) ou (iv) est lié à la formule structurale (I), et X est C ou N ; et Z est 0 ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C(=O)OR^{a}, -C(=O)R^{a}, -C(=O)NR^{a}R^{a}, -OC(=O) (alkyle en C₁₋₄), -NR^{a}C (=O) (alkyle en C₁₋₄) ou -S(=O)ₙR^{c} ; et dans lesquelles R^{11a} est R^{a}, -S(=O)₂NR^{a}R^{a} ou -S(=O)ₙR^{c} et n = 1 ou 2.

11. Composé selon la revendication 8 dans lequel :
R⁴ est choisi parmi les formules (a) à (z) ou (aa) ou (ab) décrites ci-dessous : 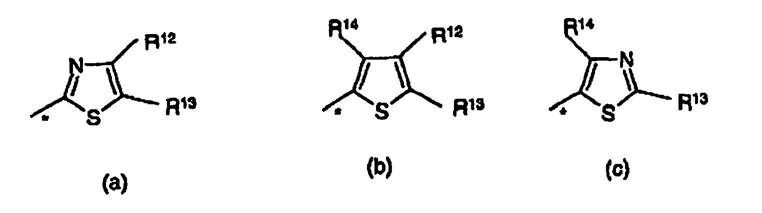 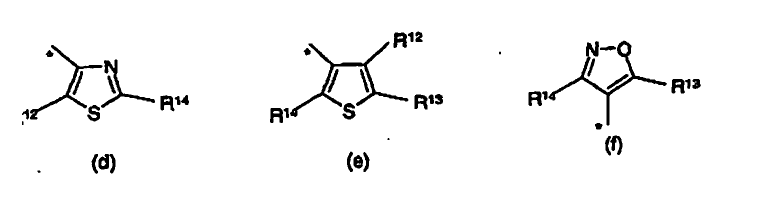 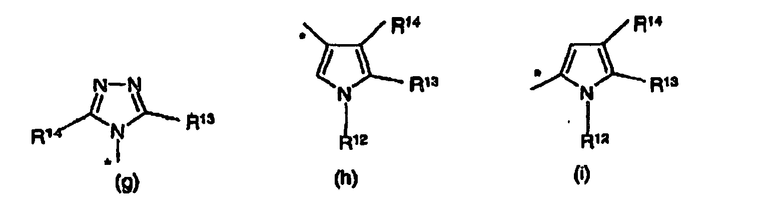 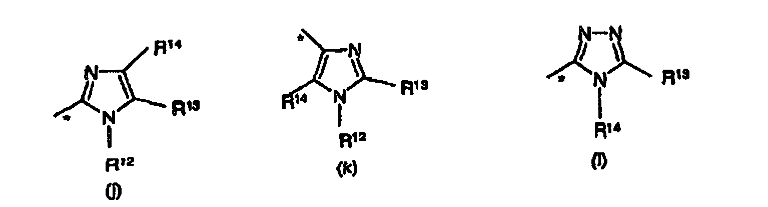 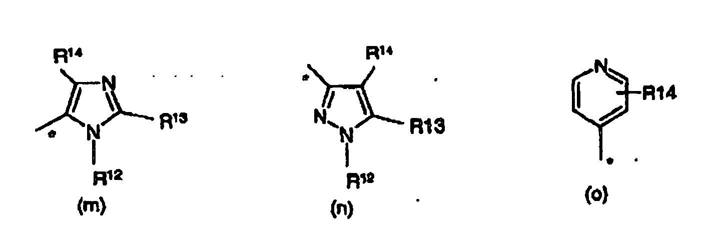 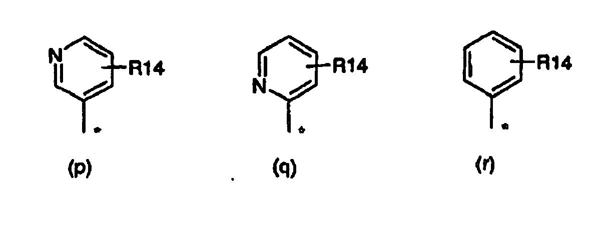 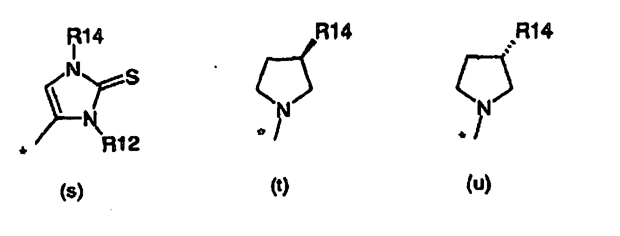 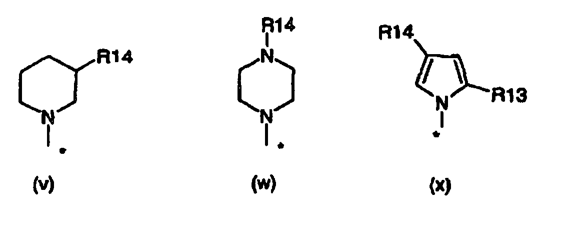 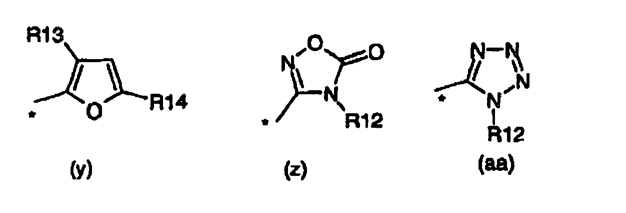 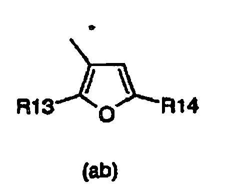
dans lesquelles * est l'emplacement auquel R⁴ est lié au système cyclique et dans lesquelles R¹², R¹³ et R¹⁴ sont chacun indépendamment représentés par H, Het, un alkyle en C₁₋₆, -CN, -NR^{a}R^{a}, le nitro, -C (=O) R^{a}, -C (=O) NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}R^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).

12. Composé selon la revendication 8 dans lequel :
X est S, O, ou NR²⁰ ; ou X et la double liaison à laquelle il est lié peuvent être 2 atomes d'hydrogène,
W est S, O, ou NR²¹;
R²⁰ est H, -CN, R^{a}, -OR^{a}, -NR^{a}R^{a}, -Het, -S(=O)ₙR^{c}, -C(=O)R^{a} , -C(=O)NR^{a}R^{a}, -C(=O)OR^{a}, -NR^{a}C(=O)R^{a}, ou -OC(=O)R^{a};
R²⁰ est H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a};
R²⁰ et R²¹ et le N auquel ils sont liés en combinaison peuvent également former un hétérocycle saturé ou insaturé à liaison N de 3 à 10 chaînons ayant 1 ou 2 hétéroatomes indépendamment choisis parmi N, O ou S l'hétérocycle étant substitué par R^{e} ;
R¹ est CH₃, CH₂CH₃, CH₂CN, CF₃, (CH₂)₂OH, le cyclopropyle, l'isopropyle, CH₂CCH, (CH₂)₂N(CH₂)₂, (CH₂)₂N(C=NH)NH₂, -CH₂-2-pyridyle, -CH₂-3-pyridyle, -CH₂-4-pyridyle, -(CH₂)₂-1-imidazolyle, -(CH₂)₂-1-pyrazolyle, -(CH₂)₂-1-pipéridyle, -(CH₂)ₘ-(1-méthylpipéridin-4-yle), -CH₂-(1-méthylpipéridin-3-yle), -(CH₂)₂-(morpholin-4-yle),
R³ est choisi parmi les formules (i), (ii), (iii) ou (iv) décrites ci-dessous : 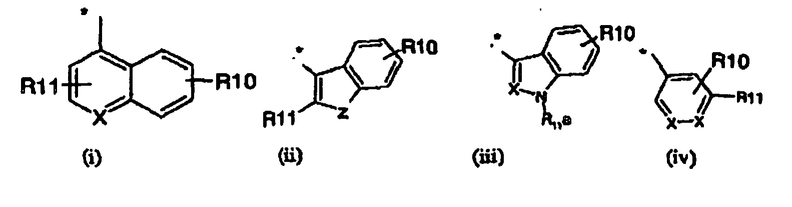 dans lesquelles * est l'emplacement où (i) ou (ii) ou (iii) ou (iv) est lié à la formule structurale (I), et X est C ou N ; et Z est 0 ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C(=O)OR^{a}, -C(=O)R^{a}, -C(=O)NR^{a}R^{a}, -OC(=O) (alkyle en C₁₋₄), -NR^{a}C(=O) (alkyle en C₁₋₄) ou -S(=O)ₙR^{c} ; et dans lesquelles R^{11a} est R^{a}, -S(=O)₂NR^{a}R^{a} ou -S(=O)ₙR^{c} et n = 1 ou 2,
R⁴ est choisi parmi les formules (a) à (z) ou (aa) ou (ab) décrites ci-dessous : 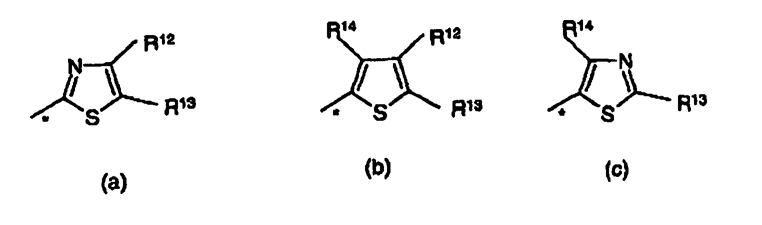 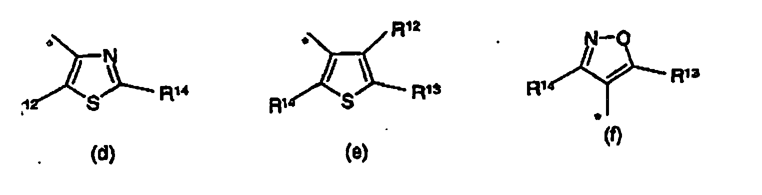 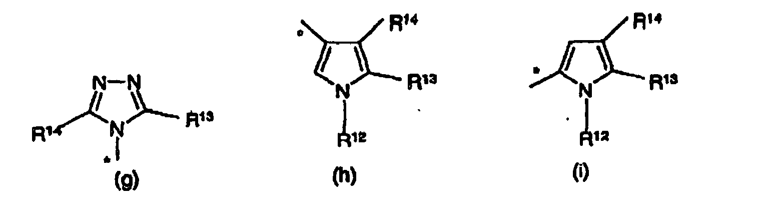 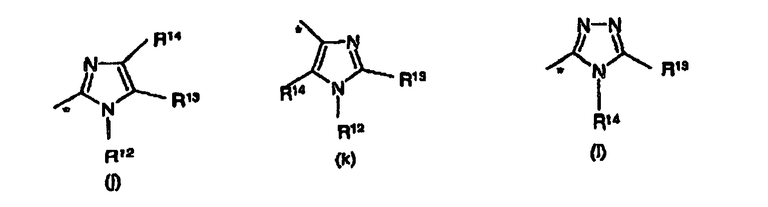 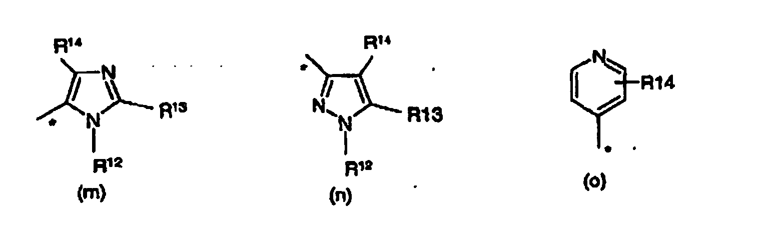 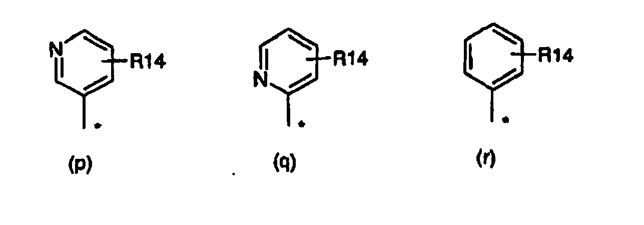 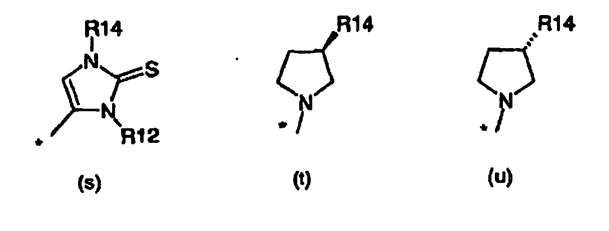 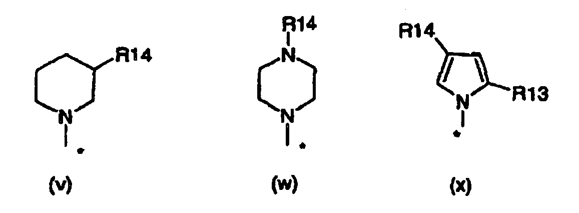 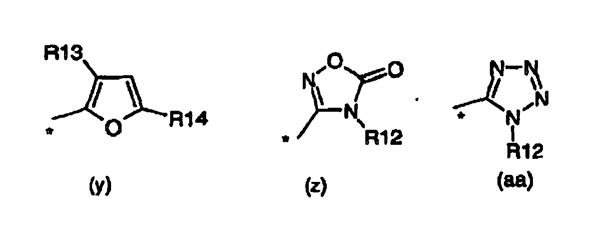 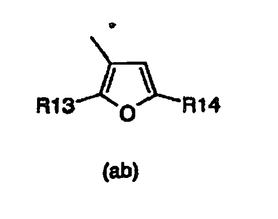
dans lesquelles * est l'emplacement auquel R⁴ est lié au système cyclique et dans lesquelles R¹², R¹³ et R¹⁴ sont chacun indépendamment représentés par H, Het, un alkyle en C₁₋₆, -CN, -NR^{a}R^{a}, le nitro, -C(=O)R^{a}, -C(=O)NR^{a}R^{a}, -C(=O)NR^{a}S(=O)₂R^{a}, -C(=O)NR^{a}- Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C (=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).

13. Composé de formule (II) choisi parmi :
le 5-{6-amino-2-[(6-chloroquinoléin-4-yl)méthyl]-5-méthyl-4-oxo-4,5-dihydro-2H-pyrazolo[3,4-d]pyrimidin-3-yl}-1-méthyl-1H-pyrrole-3-carbonitrile ;
le N-[2-[(6-chloroquinoléin-4-yl)méthyl]-3-(4-cyano-1-méthyl-1H-pyrrol-2-yl)-5-méthyl-4-oxo-4,5-dihydro-2H-pyrazolo[3,4-d]pyrimidin-6-yl]-3-méthylbutanamide ;
le N,N-[2-[(6-chloroquinoléin-4-yl)méthyl]-3-(4-cyano-1-méthyl-1H-pyrrol-2-yl)-5-méthyl-4-oxo-4,5-dihydro-2H-pyrazolo[3,4-d]pyrimidin-6-yl]-3-méthylbutanamide ;
le N'-[2-[(6-chloroquinoléin-4-yl) méthyl]-3-(4-cyano-1-méthyl-1H-pyrrol-2-yl)-5-méthyl-4-oxo-4,5-dihydro-2H-pyrazolo[3,4-d]pyrimidin-6-yl]-N,N-diméthylimidoformamide ;
le 5-{2-[(6-chloroquinoléin-4-yl)méthyl]-6-[(cyclopropylméthyl)(méthyl)amino]-5-méthyl-4-oxo-4,5-dihydro-2H-pyrazolo[3,4-d]pyrimidin-3-yl}-1-méthyl-1H-pyrrole-3-carbonitrile ;
le 5-{2-[(6-chloroquinoléin-4-yl)méthyl]-6-[(cyclopropylméthyl)amino]-5-méthyl-4-oxo-4,5-dihydro-2H-pyrazolo[3,4-d]pyrimidin-3-yl}-1-méthyl-1H-pyrrole-3-carbonitrile ;
le N-[2-[(6-chloroquinoléin-4-yl)méthyl]-3-(4-cyano-1-méthyl-1H-pyrrol-2-yl)-5-méthyl-4-oxo-4,5-dihydro-2H-pyrazolo[3,4-d]pyrimidin-6-yl]propane-1-sulfonamide ;
le 2-[(6-chloroquinoléin-4-yl)méthyl]-3-(4-cyano-1-méthyl-1H-pyrrol-2-yl)-5-méthyl-4-oxo-4,5-dihydro-2H-pyrazolo[3,4-d]pyrimidin-6-ylcarbamate d'éthyle ;
la N-[2-[(6-chloroquinoléin-4-yl)méthyl]-3-(4-cyano-1-méthyl-1H-pyrrol-2-yl)-5-méthyl-4-oxo-4,5-dihydro-2H-pyrazolo[3,4-d]pyrimidin-6-yl]-N'-éthylurée ;
le 5-[(4Z)-2-[(6-chloroquinoléin-4-yl)méthyl]-6-[(cyclopropylméthyl)amino]-5-méthyl-4-(méthylimino)-4,5-dihydro-2H-pyrazolo[3,4-d]pyrimidin-3-yl]-1-méthyl-1H-pyrrole-3-carbonitrile ;
le 5-[(4Z,6Z)-2-[(6-chloroquinoléin-4-yl)méthyl]-7-(cyclopropylméthyl)-5-méthyl-4,6-bis(méthylimino)-4,5,6,7-tétrahydro-2H-pyrazolo[3,4-d]pyrimidin-3-yl]-1-méthyl-1H-pyrrole-3-carbonitrile.

14. Composé ayant la formule structurale (III) : 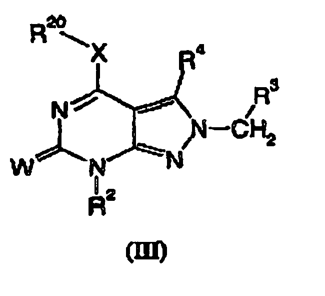 dans laquelle,
X est S, O, ou NR²¹ ; ou XR²⁰ est l'hydrogène ;
W est S, O, ou NR²⁰ ;
R² est H, un alkyle facultativement substitué, un alkylcycloalkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, ou un hétérocycle facultativement substitué ;
R³ est un système cyclique, saturé ou insaturé, monocyclique ou bicyclique, comprenant 0, 1, 2 ou 3 hétéroatomes indépendamment choisis parmi N, 0, ou S, le cycle étant substitué par 0, 1, 2 ou 3 substituants choisis parmi =0, un halogène, -OR^{a}, un alkyle en C₁₋₆, un halogénoalkyle en C₁₋₆, -CN, le nitro, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}, l'aminocarbonyle, le phényle ou le benzyle ; ou R³ est représenté par -Het, -Het-Het, R⁵, -R⁵-Het, -Het-R⁵, -Het-O-R⁵, -R⁵-R⁵, -R⁵-OR⁵;
R⁴ est un système cyclique, saturé ou insaturé, monocyclique ou bicyclique, ou un dérivé vicinal condensé de celui-ci, qui peut contenir de 5 à 12, de préférence de 5 à 10, atomes cycliques, dont 0, 1, 2, 3 ou 4 sont des hétéroatomes indépendamment choisis parmi N, 0, ou S, le système cyclique étant substitué par 0, 1, 2 ou 3 substituants choisis parmi B(OH)₂, -OCH₂CH₂O-vicinal, -O(halogénoalkyle en C₁₋₂)O- vicinal, -OCH₂O-vicinal, -CH₂OCH₂O- vicinal, =0, un halogène, -R^{b}OR^{a}, -SR^{a}, -OR^{a}, un alkyle en C₁₋₆, un halogénoalkyle en C₁₋₆, -CN, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, *-* S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC (=O) R^{a}, -NHC(=O)OR^{a}, N=NR^{a}, NO₂, -C(=O)NR^{a}R^{a}, -C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), -C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), -S(=O)₂NR^{a}(R^{b}C(=O)OR^{a}), l'aminocarbonyle, le phényle ou le benzyle ; ou R⁴ est représenté par -(CH₂)ₙR⁵-Het, -(CH₂)ₙR^{d}, -Het, -Het-Het, R⁵, -R⁵-Het, -Het-R⁵, -Het-OR⁵, R⁵-R⁵, ou -R⁵-OR⁵; ou R⁴ est représenté par un alkyle en C₁₋₆, -N (alkyle en C₁₋₆), ou -N (alkyle en C₁₋₆) ₂ dans lequel alkyle en C₁₋₆, -N(alkyle en C₁₋₆), ou -N(alkyle en C₁₋₆)₂ sont substitués par 0, 1 ou 2 substituants choisis parmi R^{a}, OR^{a}, un halogène ou un phényle où R⁴ n'est pas -(CH₂)_{z}CH₃, -(CH₂)_{z}CH₂OH, - (CH₂)_{z}CO₂H, ou -(CH₂)_{z}CO₂(alkyle en C₁₋₆) où z est 1, 2, 3, 4, 5, ou 6 ;
R⁵ est, indépendamment dans chaque cas, un phényle substitué par 0, 1, 2 ou 3 groupes choisis parmi un halogène, un halogénoalkyle en C₁₋₆, -O(halogénoalkyle en C₁₋₆), un alkyle en C₁₋₆, -CN, le nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, S(CH₂)ₘC(=O)OR^{a}-S(CH₂)ₘNR^{a}R^{a}, -S(CH₂)ₘOR^{a}, -R^{b}OR^{a}, -SR^{a}, -C(=O)NR^{a}R^{a}, -C(=O)NR^{a}OR^{a}, -C(=O)NR^{a}R^{b}NR^{a}R^{a}, -C(=O)NR^{a}R^{b}OR^{a}, -C(=O)NR^{a}R^{b}S(=O)ₙR^{a}, -C(=O)NR^{a}R^{b}Het, -C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, ou -S(=O)₂NR^{a}R^{b}C(=O)OR^{a};
R²⁰ est, indépendamment dans chaque cas, H, -CN, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, un hétérocycle facultativement substitué, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a} ;
R²¹ est, indépendamment dans chaque cas, H, -CN, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, un hétérocycle facultativement substitué, -S(=O)ₙR^{c}, -C(=O)R^{a}, -C(=O) NR^{a}R^{a}, -C(=O)OR^{a} , -NR^{a}C(=O)R^{a} , ou -OC(=O)R^{a} ; ou
R²⁰ et R²¹ et le N auquel ils sont liés en combinaison peuvent également former un hétérocycle saturé ou insaturé à liaison N de 3 à 10 chaînons ayant 1 ou 2 hétéroatomes indépendamment choisis parmi N, 0 ou S l'hétérocycle étant substitué par R^{e};
R^{a} est, indépendamment dans chaque cas, H, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, 0, ou S ;
R^{b} est, indépendamment dans chaque cas, un alkyle en C₁₋₆, -C(=O)(alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, 0, ou S ;
R^{c} est un alkyle en C₁₋₆, un halogénoalkyle en C₁₋₄, le phényle ou le benzyle ;
R^{d} est un phényle substitué par 0, 1 ou 2 groupes choisis parmi -CN, un halogène, le nitro, un alkyle en C₁₋₆, un halogénoalkyle en C₁₋₄, -OH, -OR^{c}, -NR^{a}R^{a}, -S(=O)ₙR^{c}, -C(=O)NR^{a}R^{a}, -C(=O)OR^{a}, -NR^{a}C(=O)R^{a}, -OC(=O)R^{a}, B(OH)₂, -OCH₂CH₂O- vicinal, -O (halogénoalkyle en C₁₋₂)O-vicinal, -OCH₂O- vicinal, -CH₂OCH₂O- vicinal, le phényle, le benzyle, et un hétérocycle saturé ou insaturé, à cycle à 5 ou 6 chaînons contenant 1, 2, 3 ou 4 hétéroatomes indépendamment choisis parmi N, 0, ou S ;
R^{e} est, indépendamment dans chaque cas, H, un alkyle en C₁₋₆, -C(=O)(alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, O, ou S ;
m est 1, 2 ou 3 ;
n est 0, 1 ou 2 ;
lorsque "facultativement substitué" est utilisé, il désigne au moins un substituant choisi parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyle en C₁₋₄), N(alkyle en C₁₋₄)₂, un alcanoylamino en C₁₋₄, (alcanoyle en C₁₋₄ )₂amino, N-(alkyle en C₁₋₄)carbamoyle, N,N-(alkyle en C₁₋₄)₂carbamoyle, (C₁₋₄)S, (alkyle en C₁₋₄)S(O), (alkyle en C₁₋₄)S(O)₂, (alcoxy en C₁₋₄) carbonyle, N-(alkyle en C₁₋₄)sulfamoyle, N,N- (alkyle en C₁₋₄)sulfamoyle, (alkyle en C₁₋₄)sulfonylamino, et un hétérocycle
ou un sel pharmaceutiquement acceptable de celui-ci.

15. Composé selon la revendication 14, dans lequel :
R² est -(CH₂)₁₋₃cycloalkyle ou un alkyle en C₁₋₁₂ dans lequel -(CH₂)₁₋₃cycloalkyle ou l' alkyle en C₁₋₁₂ est facultativement substitué par 0, 1, 2 ou 3 substituants choisis parmi Het, -S(=O)ₙR^{c}, un halogène, -CN, -OR^{a}, -NR^{a}R^{a}, -C(=O)OR^{a}, -C(=O)R^{a}, -C(=O)NR^{a}R^{a}, -OC(=O)(alkyle en C₁₋₄), ou -NR^{a}C(=O) (alkyle en C₁₋₄) et n est 0, 1 ou 2.

16. Composé selon la revendication 14 dans lequel :
R³ est choisi parmi les formules (i), (ii), (iii) ou (iv) décrites ci-dessous : 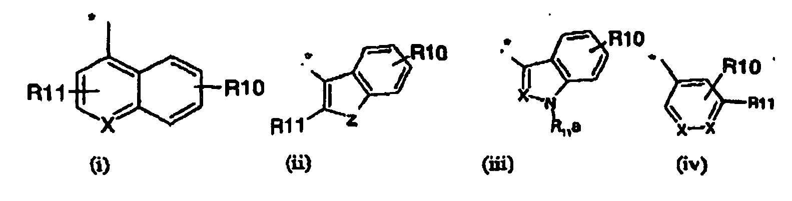
dans lesquelles * est l'emplacement où (i) ou (ii) ou (iii) ou (iv) est lié à la formule structurale (I), et X est C ou N ; et Z est O ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C(=O)OR^{a}, -C(=O)R^{a}, -C (=O) NR^{a}R^{a}, -OC (=O) (alkyle en C₁₋₄), -NR^{a}C(=O) (alkyle en C₁₋₄) ou -S(=O)ₙR^{c} ; et dans lesquelles R^{11a} est R^{a}, -S(=O)₂NR^{a}R^{a} ou -S(=O)ₙR^{c} et n = 1 ou 2.

17. Composé selon la revendication 14 dans lequel :
R⁴ est choisi parmi les formules (a) à (z) ou (aa) ou (ab) décrites ci-dessous : 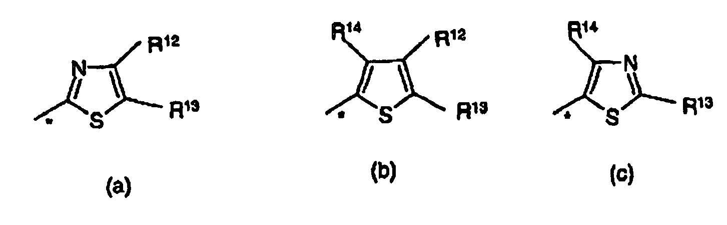 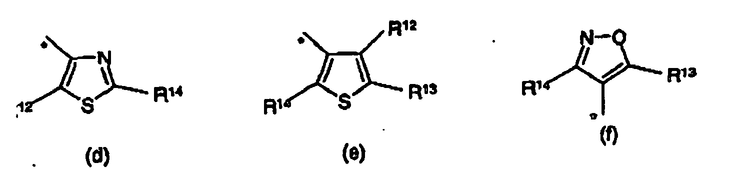 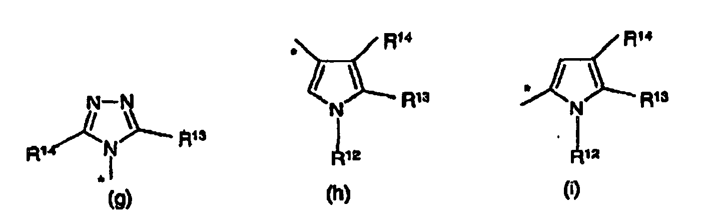 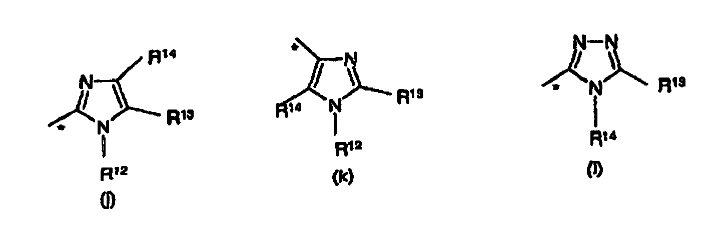 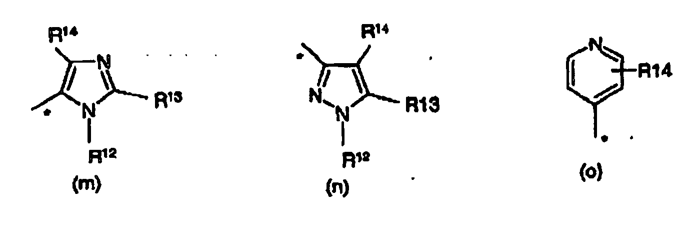 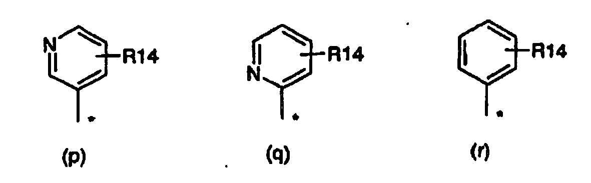 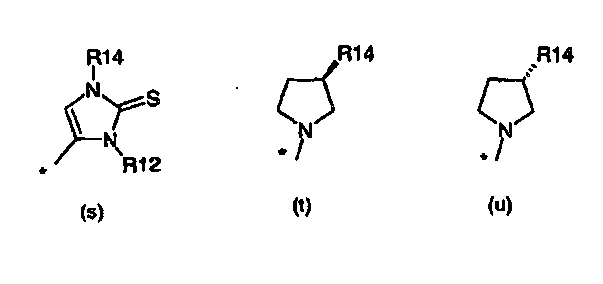 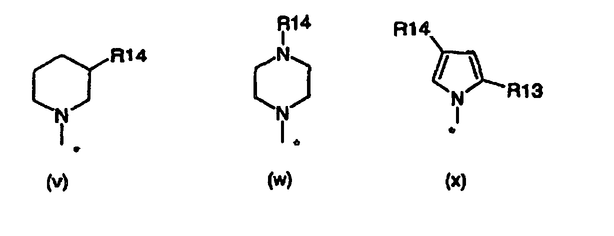 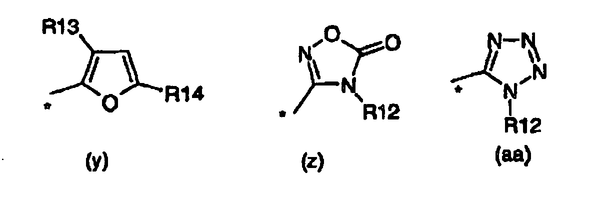 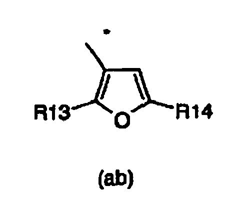
dans lesquelles * est l'emplacement auquel R⁴ est lié au système cyclique et dans lesquelles R¹², R¹³ et R¹⁴ sont chacun indépendamment représentés par H, Het, un alkyle en C₁₋₆, -CN, -NR^{a}R^{a}, le nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}R^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).

18. Composé selon la revendication 14 dans lequel :
X est S, O, ou NR²¹ ; ou XR²⁰ est l'hydrogène ;
W est S, O, ou NR²⁰;
R²⁰ est H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a};
R²⁰ est H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a} , -NR^{a}C(=O)R^{a}, ou -OC(=O)R^{a};
R²⁰ et R²¹ et le N auquel ils sont liés en combinaison peuvent également former un hétérocycle saturé ou insaturé à liaison N de 3 à 10 chaînons ayant 1 ou 2 hétéroatomes indépendamment choisis parmi N, 0 ou S l'hétérocycle étant substitué par R^{e} ;
R¹ est CH₃, CH₂CH₃, CH₂CN, CF₃, (CH₂)₂OH, le cyclopropyle, l'isopropyle, CH₂CCH, (CH₂)₂N(CH₂)₂, (CH₂)₂N(C=NH)NH₂, -CH₂-2-pyridyle, -CH₂-3-pyridyle, -CH₂-4-pyridyle, -(CH₂)₂-1-imidazolyle, -(CH₂)₂-1-pyrazolyle, -(CH₂)₂-1-pipéridyle, -(CH₂)ₘ- (1-méthylpipéridin-4-yle), -CH₂-(1-méthylpipéridin-3-yle), -(CH₂)₂-(morpholin-4-yle),
R² est -CH₂CH₂CH₃, -CH₂-cyclopropyle, -CH₂CH(CH₃)₂, -CH₂CH₂CH₂F, -CH₂-cyclobutyle, -CH₂C(CH₃)₃, -CH₂CH₂CH(CH₃)₂, -CH₂CF₃, -CH₂-méthylphényle, -CH₂-phénol, CH₂-(3,5-diméthylisoxazol-4-yle), -CH₂-S-phényle, -CH₂-phénylcarboxyle, ou -CH₂SCF₃ ;
R³ est choisi parmi les formules (i), (ii), (iii) ou (iv) décrites ci-dessous: 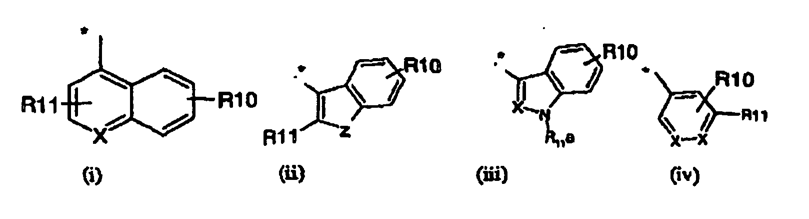 dans lesquelles * est l'emplacement où (i) ou (ii) ou (iii) ou (iv) est lié à la formule structurale (I), et X est C ou N ; et Z est 0 ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C (=O) OR^{a}, -C(=O)R^{a}, -C(=O)NR^{a}R^{a}, -OC(=O)(alkyle en C₁₋₄), -NR^{a}C (=O) (alkyle en C₁₋₄) ou -S(=O)ₙR^{c} ; et dans lesquelles R^{11a} est R^{a}, -S(=O)2NR^{a}R^{a} ou -S(=O)ₙR^{c} et n = 1 ou 2,
R⁴ est choisi parmi les formules (a) à (z) ou (aa) ou (ab) décrites ci-dessous : 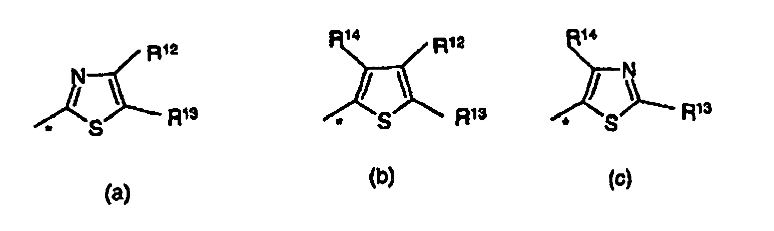 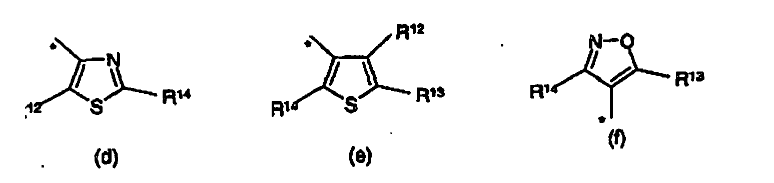 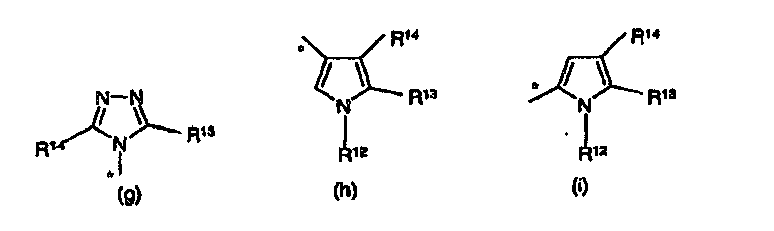 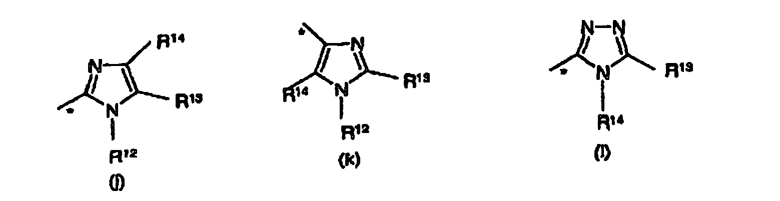 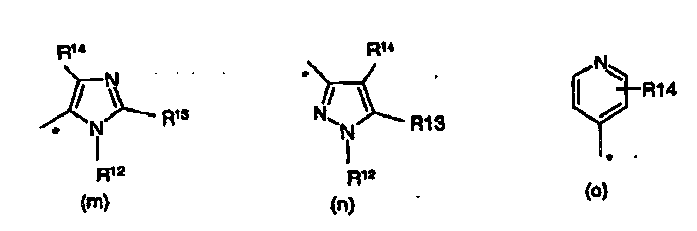 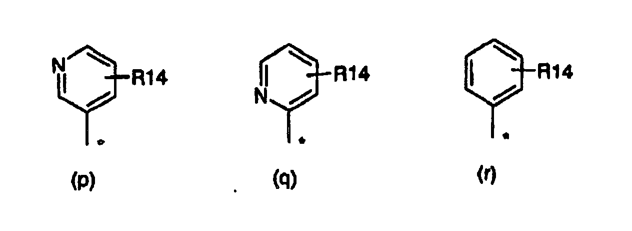 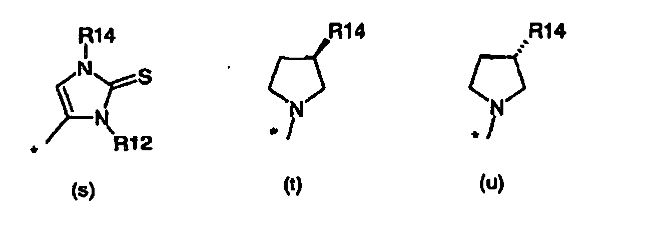 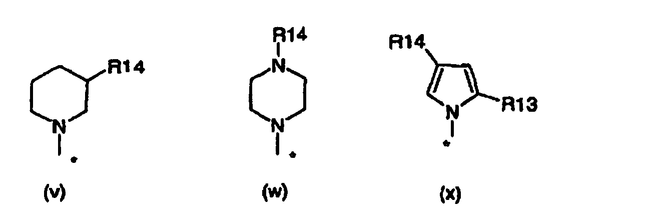 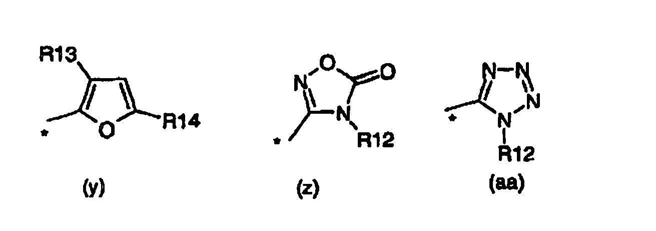 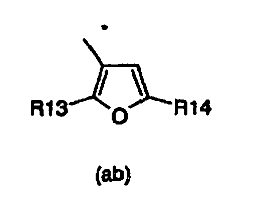
dans lesquelles * est l'emplacement auquel R⁴ est lié au système cyclique et dans lesquelles R¹², R¹³ et R¹⁴ sont chacun indépendamment représentés par H, Het, un alkyle en C₁₋₆, -CN, -NR^{a}R^{a}, le nitro, -C(=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a},-C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).

19. Composé de formule (III) choisi parmi :
la 4-amino-7-isobutyl-2-(1-naphtylméthyl)-3-pyridin-4-yl-2,7-dihydro-6H-pyrazolo[3,4-d]pyrimidin-6-one ; la 7-isobutyl-4-(méthylamino)-2-(1-naphtylméthyl)-3-pyridin-4-yl-2,7-dihydro-6H-pyrazolo[3,4-d]pyrimidin-6-one ;
la 4-(diméthylamino)-7-isobutyl-2-(1-naphtylméthyl)-3-pyridin-4-yl-2,7-dihydro-6H-pyrazolo[3,4-d]pyrimidin-6-one ;
la 7-isobutyl-4-(4-méthylpipérazin-1-yl)-2-(1-naphtylméthyl)-3-pyridin-4-yl-2,7-dihydro-6H-pyrazolo[3,4-d]pyrimidin-6-one ;
la 4-amino-2-[(6-chloroquinoléin-4-yl)méthyl]-7-isobutyl-3-(1-méthyl-1H-pyrrol-2-yl)-2,7-dihydro-6H-pyrazolo[3,4-d]pyrimidin-6-one ;
le 5-{4-amino-2-[(6-chloroquinoléin-4-yl)méthyl]-7-isobutyl-6-oxo-6,7-dihydro-2H-pyrazolo[3,4-d]pyrimidin-3-yl}-1-méthyl-1H-pyrrole-3-carbonitrile ;
le 5-[2-[(6-chloroquinoléin-4-yl)méthyl]-7-isobutyl-4-(méthylamino)-6-oxo-6,7-dihydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-4-(diméthylamino)-7-isobutyl-6-oxo-6,7-dihydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-isobutyl-6-oxo-4-(propylamino)-6,7-dihydro-2H-pyrazolo[3,4-d]pyrimidin-3-yl]-1-méthyl-1H-pyrrole-3-carbonitrile;
le 5-{2-[(6-chloroquinoléin-4-yl)méthyl]-4-[(2-hydroxyéthyl)amino]-7-isobutyl-6-oxo-6,7-dihydro-2H-pyrazolo[3,4-d]pyrimidin-3-yl}-1-méthyl-1H-pyrrole-3-carbonitrile ;
le 5-[2-[(6-chloroquinoléin-4-yl)méthyl]-4-(hydroxyamino)-7-isobutyl-6-oxo-6,7-dihydro-2H-pyrazolo[3,4-d]pyrimidin-3-yl]-1-méthyl-1H-pyrrole-3-carbonitrile;
le 5-[2-[(6-chloroquinoléin-4-yl)méthyl]-4-(cyclopropylamino)-7-isobutyl-6-oxo-6,7-dihydro-2H-pyrazolo[3,4-d]pyrimidin-3-yl]-1-méthyl-1H-pyrrole-3-carbonitrile ;
le 5-{2-[(6-chloroquinoléin-4-yl)méthyl]-4-hydrazino-7-isobutyl-6-oxo-6,7-dihydro-2H-pyrazolo[3,4-d]pyrimidin-3-yl}-1-méthyl-1H-pyrrole-3-carbonitrile ;
le 5-[2-[(6-chloroquinoléin-4-yl)méthyl]-4-(2,2-diméthylhydrazino)-7-isobutyl-6-oxo-6,7-dihydro-2H-pyrazolo[3,4-d]pyrimidin-3-yl]-1-méthyl-1H-pyrrole-3-carbonitrile ;
le N-[2-[(6-chloroquinoléin-4-yl)méthyl]-3-(4-cyano-1-méthyl-1H-pyrrol-2-yl)-7-isobutyl-6-oxo-6,7-dihydro-2H-pyrazolo[3,4-d]pyrimidin-4-yl]acétamide ;
le 5-[2-[(6-chloroquinoléin-4-yl)méthyl]-7-(cyclopropylméthyl)-4-(méthylthio)-6-oxo-6,7-dihydro-2H-pyrazolo[3,4-d]pyrimidin-3-yl]-1-méthyl-1H-pyrrole-3-carbonitrile ;
le 5-{2-[(6-chloroquinoléin-4-yl)méthyl]-7-(cyclopropylméthyl)-4-[(2-hydroxybutyl)amino]-6-oxo-6,7-dihydro-2H-pyrazolo[3,4-d]pyrimidin-3-yl}-1-méthyl-1H-pyrrole-3-carbonitrile ;
le 5-[2-[(6-chloroquinoléin-4-yl)méthyl]-7-(cyclopropylméthyl)-4-{[(2R)-2-hydroxypropyl]amino}-6-oxo-6,7-dihydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-(cyclopropylméthyl)-4-méthoxy-6-oxo-6,7-dihydro-2H-pyrazolo[3,4-d]pyrimidin-3-yl]-1-méthyl-1H-pyrrole-3-carbonitrile ;
le 5-[2-[(6-chloroquinoléin-4-yl)méthyl]-7-(cyclopropylméthyl)-6-oxo-4-(1H-pyrrol-1-yl)-6,7-dihydro-2H-pyrazolo[3,4-d]pyrimidin-3-yl]-1-méthyl-1H-pyrrole-3-carbonitrile ;
le 5-[(6Z)-2-[(6-chloroquinoléin-4-yl)méthyl]-7-(cyclopropylméthyl)-4-(méthylamino)-6-(méthylimino)-6,7-dihydro-2H-pyrazolo[3,4-d]pyrimidin-3-yl]-1-méthyl-1H-pyrrole-3-carbonitrile ;
le 5-[4-amino-2-[(6-chloroquinoléin-4-yl)méthyl]-7-(cyclopropylméthyl)-6-oxo-6,7-dihydro-2H-pyrazolo[3,4-d]pyrimidin-3-yl]-1-méthyl-1H-pyrrole-3-carbonitrile.

20. Composé ayant la formule structurale (IV) : 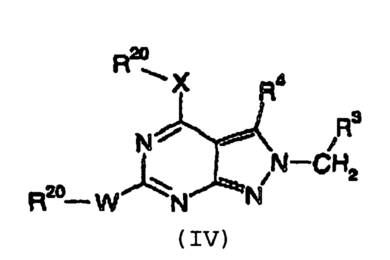 dans laquelle,
X est S, O, ou NR²¹ ; ou XR²⁰ est l'hydrogène ;
W est S, O, ou NR²¹ ;
R³ est un système cyclique, saturé ou insaturé, monocyclique ou bicyclique, comprenant 0, 1, 2 ou 3 hétéroatomes indépendamment choisis parmi N, 0, ou S, le cycle étant substitué par 0, 1, 2 ou 3 substituants choisis parmi =O, un halogène, -OR^{a}, un alkyle en C₁₋₆, un halogénoalkyle en C₁₋₆, -CN, le nitro, -S(=O)ₙR^{c}, -O(CH₂)ₘHet, -O(CH₂)ₘC(=O)Het, -O (CH₂) ₘC (=O) NR^{a}R^{a} , -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH2)ₘC(=0)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, N=NR^{a}. l'aminocarbonyle, le phényle ou le benzyle ; ou R³ est représenté par -Het, -Het-Het, R⁵, -R⁵-Het, -Het-R⁵, -Het-O-R⁵, -R⁵-R⁵, -R⁵-OR⁵ ;
R⁴ est un système cyclique, saturé ou insaturé, monocyclique ou bicyclique, ou un dérivé vicinal condensé de celui-ci, qui peut contenir de 5 à 12, de préférence de 5 à 10, atomes cycliques, dont 0, 1, 2, 3 ou 4 sont des hétéroatomes indépendamment choisis parmi N, 0, ou S, le système cyclique étant substitué par 0, 1, 2 ou 3 substituants choisis parmi B(OH)₂, -OCH₂CH₂O-vicinal, -O(halogénoalkyle en C₁₋₂)O- vicinal, -OCH₂O-vicinal, -CH₂OCH₂O- vicinal, =O, un halogène, -R^{b}OR^{a}, -SR^{a}, -OR^{a}, un alkyle en C₁₋₆, un halogénoalkyle en C₁₋₆, -CN, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -NR^{a}R^{a}, -NHC(=O)R^{a}, -NHC(=O)OR^{a}, N=NR^{a}, NO₂, -C(=O)NR^{a}R^{a}, -C(=O)NR^{a}OR^{a}, -C(=O)NR^{a}(R^{b}NR^{a}R^{a}), -C(=O)NR^{a}(R^{b}OR^{a}), -C(=O)NR^{a}(R^{b}S(=O)ₙR^{a}), -C(=O)NR^{a}(R^{b}Het), -C(=O)OR^{a}, -OC(=O)R^{a}, -C(=O)OR^{b}R^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}(R^{b}C(=O)NR^{a}R^{a}), -S(=O)₂NR^{a}(R^{b}C(=O)OR^{a}), l'aminocarbonyle, le phényle ou le benzyle ; ou R⁴ est représenté par -(CH₂)ₙR⁵-Het, -(CH₂)ₙR^{d}, -Het, -Het-Het, R⁵, -R⁵-Het, -Het-R⁵, -Het-OR⁵, R⁵-R⁵, ou -R⁵-OR⁵ ; ou R⁴ est représenté par un alkyle en C₁₋₆, -N (alkyle en C₁₋₆), ou -N(alkyle en C₁₋₆)₂ dans lequel alkyle en C₁₋₆, -N(alkyle en C₁₋₆), ou -N(alkyle en C₁₋₆)₂ sont substitués par 0, 1 ou 2 substituants choisis parmi R^{a}, OR^{a}, un halogène ou un phényle où R⁴ n' est pas -(CH₂)_{z}CH₃, -(CH₂)_{z}CH₂OH, -(CH₂)_{z}CO₂H, ou -(CH₂)_{z}CO₂(alkyle en C₁₋₆) où z est 1, 2, 3, 4, 5, ou 6;
R⁵ est, indépendamment dans chaque cas, un phényle substitué par 0, 1, 2 ou 3 groupes choisis parmi un halogène, un halogénoalkyle en C₁₋₆, -O(halogénoalkyle en C₁₋₆), un alkyle en C₁₋₆, -CN, le nitro, -OR^{a}, -S(=O)ₙR^{c}, -O(CH₂)ₘHet, -O(CH₂)ₘC(=O)Het, -O(CH₂)ₘC(=O)NR^{a}R^{a}, -O(CH₂)ₘC(=O)OR^{a}, -O(CH₂)ₘNR^{a}R^{a}, -O(CH₂)ₘOR^{a}, -S(CH₂)ₘHet, -S(CH₂)ₘC(=O)Het, -S(CH₂)ₘC(=O)NR^{a}R^{a}, -S(CH₂)ₘC(=O)OR^{a}, -S(CH₂)ₘNR^{a}R^{a}, -S(CH₂)ₘOR^{a}, -R^{b}OR^{a}, -SR^{a}, -C(=O)NR^{a}R^{a}, -C(=O)NR^{a}OR^{a}, -C(=O)NR^{a}R^{b}R^{a}R^{a}, -C(=O)NR^{a}R^{b}OR^{a}, -C(=O)NR^{a}R^{b}S(=O)ₙR^{a}, -C(=O)NR^{a}R^{b}Het , -C(=O)OR^{a}, -OC(=O)R^{a}, -C(=O)OR^{b}NR^{a}R^{a}, -C(=O)R^{a}, -C(=O)R^{b}NR^{a}R^{a}, -C(=NOR^{a})R^{a}, -C(=NCN)R^{a}, -S(=O)₂NR^{a}R^{a}, -NR^{a}S(=O)₂R^{a}, -S(=O)₂NR^{a}R^{b}C(=O)NR^{a}R^{a}, ou -S(=O)₂NR^{a}R^{b}C(=O)OR^{a} ;
R²⁰ est, indépendamment dans chaque cas, H, -CN, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a}, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, un hétérocycle facultativement substitué, dans lequel la substitution est choisie parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyle en C₁₋₄), N(alkyle en C₁₋₄)₂, un alcanoylamino en C₁₋₄, (alcanoyle en C₁₋₄)₂amino, N- (alkyle en C₁₋₄) carbamoyle, N, N- (alkyle en C₁₋₄)₂carbamoyle, (C₁₋₄) S, (alkyle en C₁₋₄)S(O), (alkyle en C₁₋₄)S(O)₂, (alcoxy en C₁₋₄)carbonyle, N- (alkyle en C₁₋₄)sulfamoyle, N,N- (alkyle en C₁₋₄)sulfamoyle, (alkyle en C₁₋₄)sulfonylamino, et un hétérocycle ;
R²¹ est, indépendamment dans chaque cas, H, -CN, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a}, un alkyle facultativement substitué, un alcényle facultativement substitué, un alcynyle facultativement substitué, un cycloalkyle facultativement substitué, un cycloalcényle facultativement substitué, un cycloalcynyle facultativement substitué, un aryle facultativement substitué, un alcoxy facultativement substitué, un amino facultativement substitué, un hétérocycle facultativement substitué, dans lequel la substitution est choisie parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyle en C₁₋₄), N(alkyle en C₁₋₄)₂, un alcanoylamino en C₁₋₄, (alcanoyle en C₁₋₄)₂amino, N-(alkyle en C₁₋₄) carbamoyle, N, N- (alkyle en C₁₋₄)₂carbamoyle, (C₁₋₄) S, (alkyle en C₁₋₄)S(O), (alkyle en C₁₋₄)S(O)₂, (alcoxy en C₁₋₄)carbonyle, N- (alkyle en C₁₋₄)sulfamoyle, N,N- (alkyle en C₁₋₄)sulfamoyle, (alkyle en C₁₋₄)sulfonylamino, et un hétérocycle ;
R²⁰ et R²¹ et le N auquel ils sont liés en combinaison peuvent également former un hétérocycle saturé ou insaturé à liaison N de 3 à 10 chaînons ayant 1 ou 2 hétéroatomes indépendamment choisis parmi N, O ou S l'hétérocycle étant substitué par R^{e} ;
R^{a} est, indépendamment dans chaque cas, H, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, O, ou S ;
R^{b} est, indépendamment dans chaque cas, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, 0, ou S ;
R^{c} est un alkyle en C₁₋₆, un halogénoalkyle en C₁₋₄, le phényle ou le benzyle ;
R^{d} est un phényle substitué par 0, 1 ou 2 groupes choisis parmi -CN, un halogène, le nitro, un alkyle en C₁₋₆, un halogénoalkyle en C₁₋₄, -OH, -OR^{c}, -NR^{a}R^{a}, -S(=O)ₙR^{c}, -C(=O)NR^{a}R^{a} , -C(=O)OR^{a}, -NR^{a}C(=O)R^{a}, -OC(=O)R^{a}, B(OH)₂, -OCH₂CH₂O- vicinal, -O(halogénoalkyle en C₁₋₂)O-vicinal, -OCH₂O- vicinal, -CH₂OCH₂O- vicinal, le phényle, le benzyle, et un hétérocycle saturé ou insaturé, à cycle à 5 ou 6 chaînons contenant 1, 2, 3 ou 4 hétéroatomes indépendamment choisis parmi N, 0, ou S ;
R^{e} est, indépendamment dans chaque cas, H, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, O, ou S ;
m est 1, 2 ou 3 ;
n est 0, 1 ou 2 ;
lorsque "facultativement substitué" est utilisé, il désigne au moins un substituant choisi parmi le cyclopropyle, un halogène, le nitro, le cyano, l'hydroxy, le trifluorométhyle, l'amino, le carboxy, le carboxamido, l'amidino, le carbamoyle, le mercapto, le sulfamoyle, un alkyle en C₁₋₄, un alcényle en C₂₋₄, un alcynyle en C₂₋₄, un alcoxy en C₁₋₄, un alcanoyle en C₁₋₄, un alcanoyloxy en C₁₋₄, NH(alkyle en C₁₋₄), N(alkyle en C₁₋₄)₂, un alcanoylamino en C₁₋₄, (alcanoyle en C₁₋₄ )₂amino, N-(alkyle en C₁₋₄)carbamoyle, N,N-(alkyle en C₁₋₄)₂carbamoyle, (C₁₋₄) S, (alkyle en C₁₋₄)S(O), (alkyle en C₁₋₄)S(O)₂, (alcoxy en C₁₋₄)carbonyle, N-(alkyle en C₁₋₄)sulfamoyle, N,N- (alkyle en C₁₋₄) sulfamoyle, (alkyle en C₁₋₄)sulfonylamino, et un hétérocycle
ou un sel pharmaceutiquement acceptable de celui-ci.

21. Composé selon la revendication 20 dans lequel :
R³ est choisi parmi les formules (i), (ii), (iii) ou (iv) décrites ci-dessous : 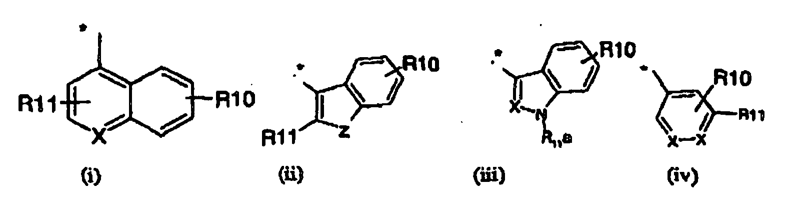
dans lesquelles * est l'emplacement où (i) ou (ii) ou (iii) ou (iv) est lié à la formule structurale (I), et X est C ou N ; et Z est O ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C(=O)OR^{a}, -C(=O)R^{a}, -C(=O)NR^{a}R^{a}, -OC(=O) (alkyle en C₁₋₄), -NR^{a}C(=O) (alkyle en C₁₋₄) ou -S(=O)ₙR^{c}; et dans lesquelles R^{11a} est R^{a}, -S(=O)₂NR^{a}R^{a} ou -S(=O)ₙR^{c} et n = 1 ou 2.

22. Composé selon la revendication 20 dans lequel :
R⁴ est choisi parmi les formules (a) à (z) ou (aa) ou (ab) décrites ci-dessous : 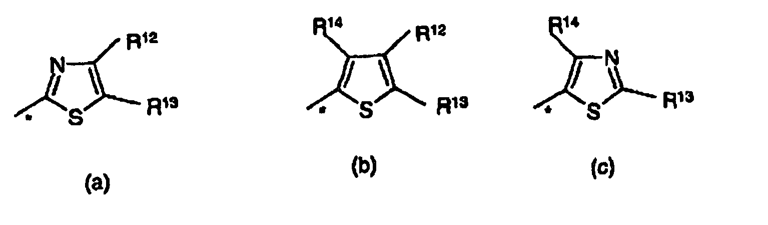 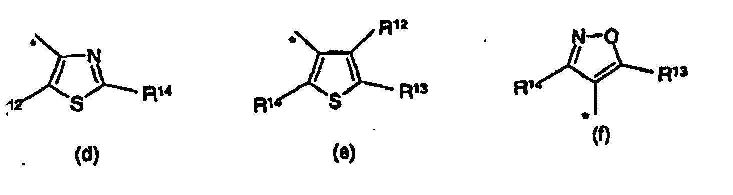 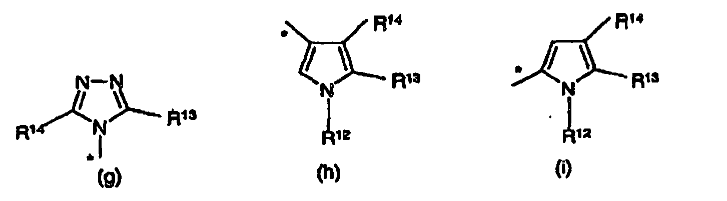 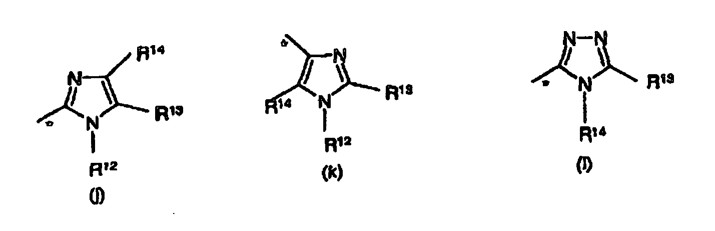 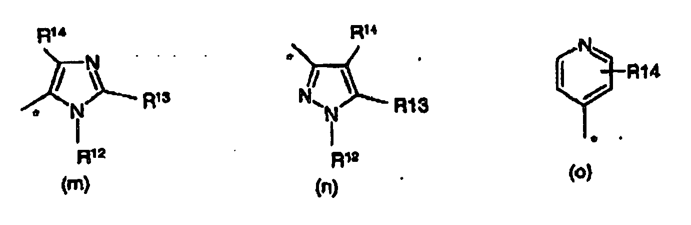 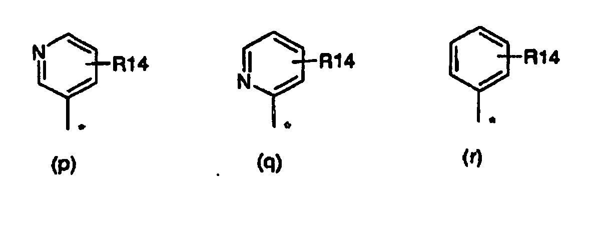 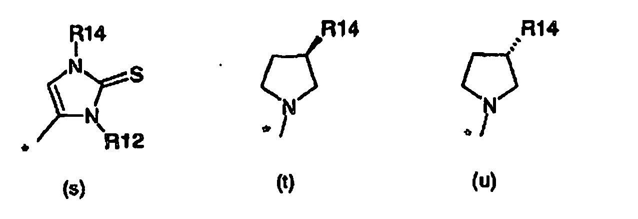 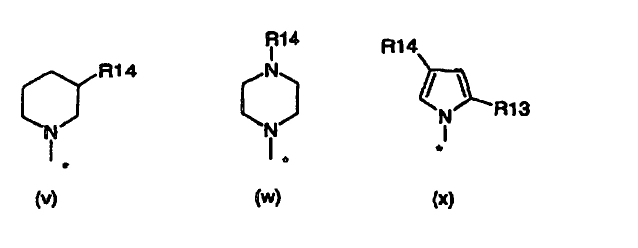 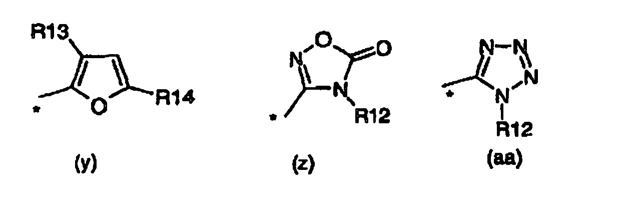 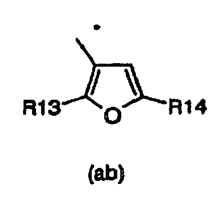
dans lesquelles * est l'emplacement auquel R⁴ est lié au système cyclique et dans lesquelles R¹² R¹³ et R¹⁴ sont chacun indépendamment représentés par H, Het, un alkyle en C₁₋₆, -CN, -NR^{a}R^{a}, le nitro, -C (=O)R^{a}, -C(=O)NR^{a}R^{a}, -C(=O)NR^{a}S(=O)₂R^{a}, -C(=O)NR^{a}-Het, -C(=O)NR^{a}NR^{a}R^{a}, -C(=O)NR^{a}(R^{b}OR^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{b}NR^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).

23. Composé selon la revendication 20, dans lequel :
X est S, O, ou NR²¹ ; ou X-R²⁰ est l'hydrogène ;
W est S, O, ou NR²¹ ;
R¹ est CH₃, CH₂CH₃, CH₂CN, CF₃, (CH₂)₂OH, le cyclopropyle, l'isopropyle, CH₂CCH, (CH₂)₂N(CH₂)₂, (CH₂)₂N(C=NH)NH₂, -CH₂-2-pyridyle, -CH₂-3-pyridyle, -CH₂-4-pyridyle, -(CH₂)₂-1-imidazolyle, -(CH₂)₂-1-pyrazolyle, -(CH₂)₂-1-pipéridyle, -(CH₂)ₘ-(1-méthylpipéridin-4-yle), -CH₂-(1-méthylpipéridin-3-yle), -(CH₂)₂-(morpholin-4-yle),
R² est -CH₂CH₂CH₃, -CH₂-cyclopropyle, -CH₂CH(CH₃)₂, -CH₂CH₂CH₂F, -CH₂-cyclobutyle, -CH₂C(CH₃)₃, -CH₂CH₂CH(CH₃)₂, -CH₂CF₃, -CH₂-méthylphényle, -CH₂-phénol, CH₂-(3,5-diméthylisoxazol-4-yle), -CH₂-S-phényle, -CH₂-phénylcarboxyle, ou -CH₂SCF₃ ;
R³ est choisi parmi les formules (i), (ii), (iii) ou (iv) décrites ci-dessous : 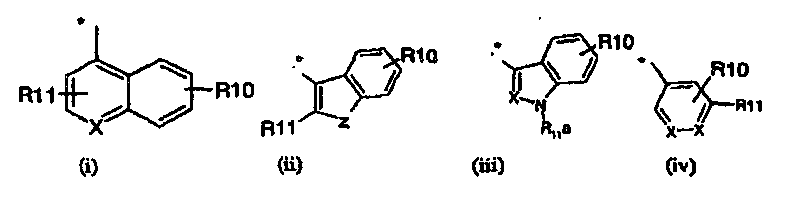 dans lesquelles * est l'emplacement où (i) ou (ii) ou (iii) ou (iv) est lié à la formule structurale (I), et X est C ou N ; et Z est O ou S, dans lesquelles R¹⁰ est à une position quelconque sur le cycle et R¹⁰ et R¹¹ sont indépendamment dans chaque cas H, R^{a}, un halogène, -CN, le nitro, OR^{a}, CF₃, -NR^{a}R^{a}, -C(=O)OR^{a}, -C(=O)R^{a}, -C(=O)NR^{a}R^{a}, -OC(=O) (alkyle en C₁₋₄), -NR^{a}C(=O)(alkyle en C₁₋₄) ou -S(=O)ₙR^{c} ; et dans lesquelles R^{11a} est R^{a}, -S(=O)₂NR^{a}R^{a} ou -S(=O)ₙR^{c} et n = 1 ou 2 ;
R⁴ est choisi parmi les formules (a) à (z) ou (aa) ou (ab) décrites ci-dessous : 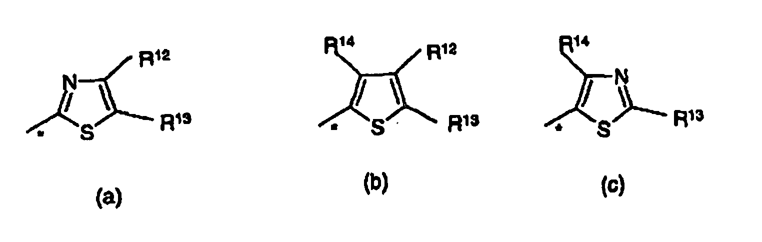 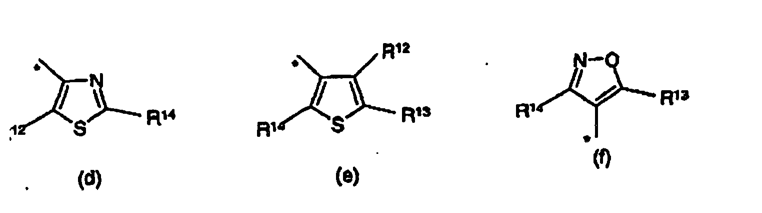 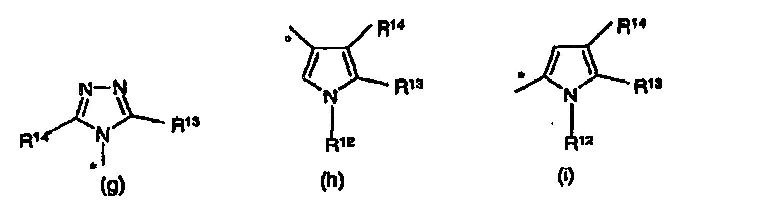 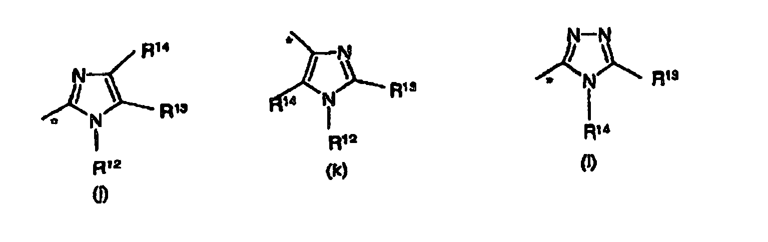 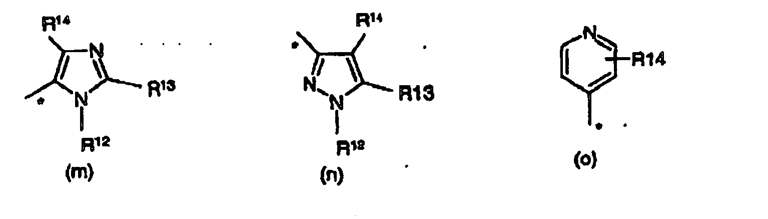 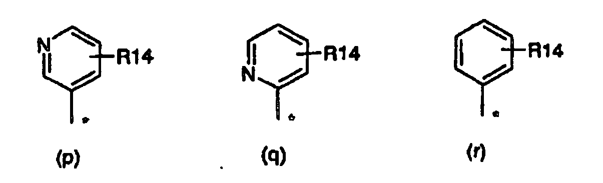 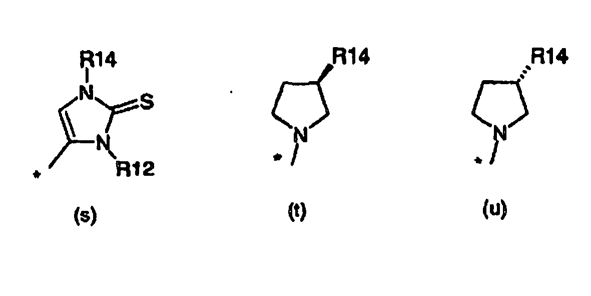 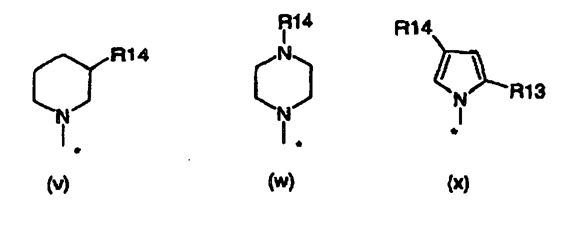 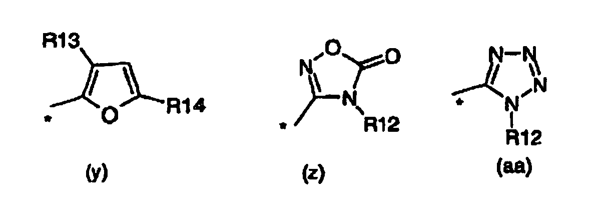 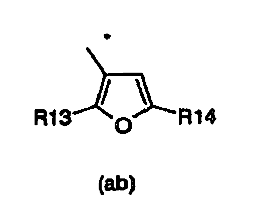 dans lesquelles * est l'emplacement auquel R⁴ est lié au système cyclique et dans lesquelles R¹², R¹³ et R¹⁴ sont chacun indépendamment représentés par H, Het, un alkyle en C₁₋₆, -CN, -NR^{a}R^{a}, le nitro, -C(=O)R^{a}, -C(=O)NR^{a}R^{a}, -C(=O)NR^{a}S(=O)2R^{a}, -C(=O)NR^{a}-Het, -C(=O)NR^{a}NR^{a}R^{a}, -C(=O)NR^{a}(R^{b}NR^{a}R^{a}), -C(=O)NR^{a}(R^{b}OR^{a}), -C(=O)NR^{a}(R^{b}S(=O)₂R^{a}), -C(=O)NR^{a}R^{b}Het, -C(=O)NR^{a}OR^{a}, -C(=O)R^{a}R^{a}, -C(=NOR^{a})R^{a}, -C(=NCN)R^{a}, -C(=O)OR^{a}, -C(=O)OR^{b}NR^{a}R^{a}, -C(=O)R^{a}, -OC(=O)R^{a}, -C(=O)R^{a}-SR^{a}, =S, -NR^{a}C(=O)R^{a}, -NR^{a}C(=O)OR^{a}, -NR^{a}S(=O)₂R^{b}, -C(=NOR^{a})R^{a}, -S(=O)₂R^{a}, -S(=O)₂NR^{a}R^{a}, -S(=O)₂NR^{a}(R^{b}C(=O)NR^{a}R^{a}), ou -S(=O)₂NR^{a}(R^{b}C(=O)OR^{a}).
R²⁰ est H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a};
R²⁰ est H, -CN, R^{a}, -OR^{a}, -NR^{a}R^{a}, -Het, -S(=O)ₙR^{c}, -C(=O)R^{a}, -C(=O)NR^{a}R^{a}, -C(=O)OR^{a}, -NR^{a}C(=O)R^{a}, ou -OC(=O)R^{a};
R²⁰ et R²¹ et le N auquel ils sont liés en combinaison peuvent également former un hétérocycle saturé ou insaturé à liaison N de 3 à 10 chaînons ayant 1 ou 2 hétéroatomes indépendamment choisis parmi N, O ou S l'hétérocycle étant substitué par R^{e} ;
R^{e} est, indépendamment dans chaque cas, H, un alkyle en C₁₋₆, -C(=O) (alkyle en C₁₋₄), un halogénoalkyle en C₁₋₄, le phényle, le benzyle, ou un hétérocycle saturé ou insaturé, à cycle à 5 ou 6 chaînons contenant 1, 2, 3 ou 4 hétéroatomes indépendamment choisis parmi N, 0, ou S.

24. Composé de formule (IV) choisi parmi :
le 5-[2-[(6-chloroquinoléin-4-yl)méthyl]-6-[(cyclopropylméthyl)amino]-4-(méthylamino)-2H-pyrazolo[3,4-d]pyrimidin-3-yl]-1-méthyl-1H-pyrrole-3-carbonitrile ;
le {3-(4-acétyl-1-méthyl-1H-pyrrol-2-yl)-2-[(6-chloroquinoléin-4-yl)méthyl]-4-méthoxy-2H-pyrazolo[3,4-d]pyrimidin-6-yl}-2-cyclopropylacétamide.

25. Composé selon l'une quelconque des revendications 1 à 24, pour utilisation en tant que médicament.

26. Utilisation d'un composé selon l'une quelconque des revendications 1 à 24 dans la fabrication d'un médicament pour le traitement ou la prophylaxie de troubles associés à une infection par *H. pylori.*

27. Composition pharmaceutique comprenant un composé selon l'une quelconque des revendications 1 à 24, conjointement avec au moins un véhicule, diluant ou excipient pharmaceutiquement acceptable.
